# EUROPEAN PATENT APPLICATION

(11) **EP 4 134 452 A1**
(43) Date of publication of application: **15.02.2023**
(21) Application number: 21190938.7
(22) Date of filing: 12.08.2021
(51) Int. Cl.: C12Q 1/6886

(54) **METHOD FOR CLASSIFICATION OF CANCER**

(71) Applicant: Deutsches Krebsforschungszentrum - Stiftung des öffentlichen Rechts / Universität Heidelberg, 69120 Heidelberg (DE)
(72) Inventor: Sill, Martin, 69120 Heidelberg (DE); Pfister, Stefan, 69120 Heidelberg (DE); Jones, David, 69120 Heidelberg (DE); von Deimling, Andreas, 69120 Heidelberg (DE); Capper, David, 69120 Heidelberg (DE); Hovestadt, Volker, Boston (US); Sahm, Felix, 69120 Heidelberg (DE); Schrimpf, Daniel, 69117 Heidelberg (DE)
(74) Representative: Legl, Stefan

(57) **Abstract**

The present disclosure pertains to an in vitro method for the diagnostic classification of cancer based on the biological state of specific genomic sites. The disclosure provides a method that allows for a classification of a tumour sample obtained from a patient by analysing a multitude, preferably genome wide, collection of gene sites, combining the biological state of the analysed gene sites into a biological state pattern and comparing with pre-determined biological state patterns pertaining to different cancer types or tumour species. The disclosure is in particular useful for classifying cancer e.g. of the central nervous system, such as brain tumour samples and tumours of the spinal cord, since these are characterized by a large variety of distinct tumour species which have different prognostic values and require a developed treatment regime for each species in the clinical context. However, other cancers could similarly profit from the disclosure, for example sarcomas.

## Description

### FIELD OF THE DISCLOSURE

The present disclosure pertains to the classification of cancer, in particular to a computer-implemented method for the diagnostic classification of cancer and/or an in vitro method for classification of cancer based on the biological state of specific genomic DNA sites or transcripts. The disclosure provides a method that allows for a classification of a tumour sample obtained from a patient by analysing a multitude, preferably genome wide, of gene sites, combining the biological state of the analysed gene sites into a biological state pattern and comparing it directly and/or indirectly with pre-determined biological state patterns pertaining to different cancer types or tumour species. The disclosure is in particular useful for classifying cancer of the central nervous system, i.e. brain tumour samples and/or tumours of the spinal cord, since these need to be correctly identified from a large variety of distinct tumour species which have different prognostic values and require a developed treatment regime for each species in the clinical context. However, other cancers could similarly profit from the disclosure, for example sarcomas.

### BACKGROUND

When looking at brain tumour entities alone, there are more than 100 different entities listed in the World Health Organisation classification. Many of these show complex patterns of potentially overlapping histological features. Moreover, even histologically identical tumours can belong to different molecular groups with very different treatment requirements and prognosis. The same is true for tumours of the spinal cord and tumours originating in tissues outside the central nervous system. Therefore, more advanced diagnostic tools are needed.

Epigenetic patterns, for example the epigenetic states of different gene sites, play a critical role in development, differentiation and pathogenesis of diseases such as multiple sclerosis, diabetes, schizophrenia, aging, and multiple forms of cancer including tumours of the central nervous system. Tumour entities originate from different precursor-cell populations which are transformed by genetic and epigenetic alterations. It is now recognized that many tumour entities, including the ones of the central nervous system, that are of distinct biological groups are not always distinguishable by their histology. Most tumour entities display varied histological spectra with no clear boundaries. Epigenetic modifications, such as methylation, preserve the information of the cell of origin, its original identity. Therefore, methylation data, for example DNA methylation patterns, have a great potential to identify molecular subgroups of tumours, such as tumours of the central nervous system. Similar results can be obtained by analysing the transcripts of the respective genes of interest.

Still, treatment planning and in particular treatment success in many cancers, and in particular in cancers of the central nervous system, is highly dependent on an early and accurate diagnosis and classification of the tumour. In view of the above, new methods that overcome at least some of the problems in the art are beneficial.

### SUMMARY

The present disclosure seeks to provide a strategy and method for the diagnostic classification of cancer samples with higher specificity and sensitivity.

This object of the present invention is solved by the features of the independent claims. Preferred embodiments are defined in the dependent claims. Any "aspect", "example" and "embodiment" of the description not falling within the scope of the claims does not form part of the invention and is provided for illustrative purposes only.

According to an independent aspect of the present disclosure, a computer-implemented method for diagnostic classification of cancer is provided. The method includes classifying a cancer using a classification algorithm based on biological states or biological state patterns of a set of gene sites of a cancer sample.

Preferably, the classification algorithm is trained using biological data derived from classified cancer types, such as pre-classified cancer types. In particular, the cancer types can be pre-classified and/or can be new cancer types which are identified using the classification algorithm. For example, the classification algorithm may classify a cancer sample as unknown, wherein such unknown cancer samples can then be further analysed to determine a cancer type thereof. The further analysis may be conducted by various means, such as software and/or medical personnel.

Preferably, the set of gene sites comprises at least 3 gene sites of the cancer sample genome selected from a list consisting of the gene sites in Table 1 of this document.

Preferably, the biological states of the gene sites comprise the biological states of the gene sites as listed in Table 1 of this document and preferably up to 20 (or 15 or 12 kb) upstream and/or downstream of each of said gene sites. For example, the biological states of the gene sites comprise the biological states of the gene sites as listed in Table 1 and up to 10 kb, preferably up to 8 kb or up to 6 kb or up to 4 kb or up to 2 kb, upstream and/or downstream of the gene sites.

According to some embodiments, which can be combined with other embodiments described herein, the method further includes determining biological states pertaining to the at least 3 gene sites of the cancer sample genome.

Additionally, or alternatively, the method further includes determining a biological state pattern of the set of gene sites based on the determined biological state(s) of each of the at least 3 gene sites.

According to another independent aspect of the present disclosure, a method for diagnostic classification of cancer is provided.

According to some embodiments, which can be combined with other embodiments described herein, the method for diagnostic classification of cancer is an *in-vitro* method.

In a preferred embodiment, the method includes:
- providing a cancer sample,
- determining biological states pertaining to at least 3 gene sites of the cancer sample genome, wherein the gene sites are selected from a list consisting of the gene sites in Table 1,
- determining a biological state pattern based on the determined biological state(s) of each of the at least 3 gene sites, and
- classifying a cancer type based on the determined biological state pattern and predetermined biological state patterns pertaining to different cancer types.

Preferably, the biological states of the gene sites comprise the biological states of the gene sites as listed in Table 1 of this document and preferably up to 20 kb (or 15 or 12 kb) upstream and/or downstream of each of said gene sites. For example, the biological states of the gene sites comprise the biological states of the gene sites as listed in Table 1 and preferably up to 10 kb, preferably up to 8 kb or up to 6 kb or up to 4 kb or up to 2 kb, upstream and/or downstream of the gene sites.

Preferably, the step of determining a biological state pattern comprises combining the biological state(s) of the gene sites into the biological state pattern.

Preferably, classifying a cancer type comprises comparing the biological state pattern of the set of gene sites with pre-determined biological state patterns derived from the biological state data pertaining to different cancer types.

Preferably, the cancer is classified as a specific cancer type if the biological state pattern of the set of gene sites differs from the biological state data derived from the pre-classified cancer type by at most 5 %, preferably at most 4 % or at most 3 % or at most 2 % or at most 1 %.

Preferably, the biological state is selected from a group including, or consisting of, epigenetic state, mutation state, copy number and RNA expression.

Preferably, the epigenetic state is a methylation state.

Preferably, the set of gene sites comprises at least 10, preferably at least 20 or at least 30 or at least 40 or at least 50 or at least 60 or at least 70 or at least 80 or at least 90 or at least 100 or all gene sites of the cancer sample genome in Table 1.

Preferably, the gene sites are the ones with the highest values of variable importance (imp_sum) in Tables 3 to 172 of this document.

Preferably, the biological states of the gene sites comprise exclusively the biological states of the gene sites as listed in Table 1 without any bases upstream and/or downstream of the gene sites.

Preferably, the biological state is a methylation state and/or the biological state pattern is a methylation state pattern.

Preferably, the cancer is a cancer of the central nervous system or a sarcoma.

Preferably, the cancer is a cancer listed in Table 2.

Preferably, the method further includes determining a further (second) biological state different from the (first) biological state and pertaining to at least one of the gene sites pertaining to the cancer sample genome.

Preferably, the method further includes correlating the further (second) biological state of the at least one gene site pertaining to the cancer sample genome with the classified cancer type.

Preferably, the method further includes defining the at least one gene site with the determined further (second) biological state as an alternative or additional biomarker in the diagnosis of the classified cancer types.

Preferably, the further (second) biological state is selected from the group including, or consisting of, epigenetic state, mutation state, RNA expression and copy number.

According to another independent aspect of the present disclosure, a computer-readable storage medium is provided. The computer-readable storage medium has computer-executable instructions stored, that, when executed, cause a computer to perform the methods described herein.

The term "computer-readable storage medium" may refer to any storage device used for storing data accessible by a computer, as well as any other means for providing access to data by a computer. Examples of a storage device-type computer-readable medium include: a magnetic hard disk; a floppy disk; an optical disk, such as a CD-ROM and a DVD; a magnetic tape; a memory chip.

According to another independent aspect of the present disclosure, a system for diagnosing cancer is provided. The system includes one or more processors and a memory coupled to the one or more processors and comprising instructions executable by the one or more processors to implement the methods described herein.

The system may be a computer system. The term a "computer system" may refer to a system having a computer, where the computer comprises a computer-readable storage medium embodying software to operate the computer.

The term "software" is used interchangeably herein with "program" and refers to prescribed rules to operate a computer. Examples of software include: software; code segments; instructions; computer programs; and programmed logic.

The embodiments of the present disclosure provide a classification of cancer samples in cancer diagnosis using a classification algorithm, which is a machine learning (ML) algorithm.

The term "classification" refers to a procedure and/or algorithm in which individual items are placed into groups or classes based on quantitative information on one or more characteristics inherent in the items (referred to as traits, variables, characters, features, etc.) and based on a statistical model and/or a training set of previously labelled items. Specifically in the context of the present disclosure, classification preferably means determining which specific cancer type, for example determined by its epigenetic features, a cancer sample belongs to.

The term "machine learning algorithm" as used throughout the present application refers to an algorithm that builds a model based on training data, in order to make predictions or decisions without being explicitly programmed to do so. In particular, the term "classification" refers to a machine learning algorithm in which individual items are placed into groups or classes based on quantitative information on one or more characteristics inherent in the items (referred to as traits, variables, characters, features, etc.) and based on a statistical model and/or a training data set of previously labelled items. Specifically in the context of the present invention, classification preferably means determining which specific cancer type, for example determined by its epigenetic state pattern, a cancer sample belongs to.

The term "training data set" in context of the invention refers to a set of biological state data, such as genomic methylation data, of a multitude of tumours that were classified by prior art methods, and therefore are of known tumour species.

The classification algorithm can be any appropriate algorithm for establishing a correlation between datasets, namely the biological state(s) or biological state pattern(s) of the cancer sample and the biological state data derived from pre-classified cancer types, which can be pre-determined biological state(s) or biological state patterns. Methods for establishing correlation between datasets include, but are not limited to, discriminant analysis (DA) (e.g., linear-, quadratic-, regularized-DA), Discriminant Functional Analysis (DFA), Kernel Methods (e.g., SVM), Multidimensional Scaling (MDS), Nonparametric Methods (e.g., k-Nearest-Neighbour Classifiers), PLS (Partial Least Squares), Tree-Based Methods (e.g., Logic Regression, CART, Random Forest Methods, Boosting/Bagging Methods), Generalized Linear Models (e.g., Logistic Regression), Principal Components based Methods (e.g., SIMCA), Generalized Additive Models, Fuzzy Logic based Methods, Neural Networks and Genetic Algorithms based methods.

The person skilled on the art will have no problem in selecting an appropriate method/algorithm to establish the correlation between the biological state(s) or biological state pattern(s) of the cancer sample and the biological state data derived from pre-classified cancer types of the present invention. In one embodiment, the method/algorithm used in a correlating the biological state(s) or biological state pattern(s) of the cancer sample and the biological state data derived from pre-classified cancer types of the present invention is selected from the group including (or consisting of) DA (e.g., Linear-, Quadratic-, Regularized Discriminant Analysis), DFA, Kernel Methods (e.g., SVM), MDS, Nonparametric Methods (e.g., k-Nearest-Neighbour Classifiers), PLS (Partial Least Squares), Tree-Based Methods (e.g., Logic Regression, CART, Random Forest Methods, Boosting Methods), Generalized Linear Models (e.g., Logistic Regression), and Principal Components Analysis.

In an exemplary embodiment, the classification algorithm uses random forest analysis. As used herein the term "random forest analysis" refers to a computational method that is based on the idea of using multiple different decision trees to compute the overall most predicted class (the mode). In a specific application, the mode will be either tumour species or class based on how many decision trees predicted the samples to match a specific class. The class predicted by the majority is selected as the predicted class for the sample. The different decision trees used in this algorithm are trained on a randomly generated subset of the training data set and on a randomly selected set of the variables. This is why this algorithm relies on two hyperparameters: the number of random trees to use, and the number of random variables used to train the different trees.

The term "biological state" may refer to an epigenetic state, mutation state, RNA expression or copy number of a gene or gene site.

The term "epigenetic state" refers to a measure for epigenetic changes (or for functionally relevant changes of an upregulation and/or downregulation) of the gene activity of a particular gene site and/or gene in the genome of a cancer sample. The epigenetic state comprises an epigenetic downregulation and/or upregulation of the gene site's activity in the cancer sample in comparison to that same gene site's activity in physiological tissue. Such downregulation and/or upregulation can for example be due to DNA methylation, histone modification or other epigenetic effects.

The term "epigenetic state pattern" refers to a combination of the epigenetic state(s) of a plurality of gene sites and/or genes. It comprises an overview of the epigenetic state(s) of the gene sites and/or genes. An epigenetic state pattern can therefore in its simplest form comprise information about which of the gene sites and/or genes of the plurality in question have an activation which is epigenetically modified in comparison to the physiological state and which do not. The epigenetic state pattern could also comprise information about which of the gene sites and/or genes are epigenetically upregulated and/or downregulated, for example in terms of hypermethylation (resulting in downregulation) or hypomethylation (resulting in upregulation) when DNA methylation is used as measure for epigenetic influence on gene or gene site activity.

In some embodiments, the classification algorithm of the present disclosure can be trained using epigenetic data derived from classified cancer types, such as pre-classified cancer types. The epigenetic data may be provided in the form of a predetermined pattern or predetermined epigenetic state pattern. The term "predetermined pattern" or "predetermined epigenetic state pattern" refers to an epigenetic state pattern that has been determined beforehand and that is typical of a specific cancer type, for example one of the types mentioned in Table 2 (and, for example, Tables 3 to 172). The first iteration of predetermined patterns has been determined by the inventors and has been used to train the classification algorithm.

In a preferred embodiment of the disclosure, the predetermined epigenetic state patterns pertain to the cancer types listed in Table 2 (and, for example, Tables 3 to 172, respectively). Furthermore, the predetermined epigenetic state pattern comprises essentially the same gene sites as the set of gene sites of the cancer sample being analysed. If, by determining the epigenetic state of the set of gene sites, as explained in more detail below, an epigenetic state pattern is obtained that corresponds to one of the predetermined patterns, the cancer pertaining to the sample can be classified as pertaining to this cancer type. The predetermined epigenetic state patterns are preferably determined by the classification algorithm. This means that the predetermined epigenetic state patterns are not in itself accessible by a user, but contained in the results of the classification algorithm, which, for example, employs machine learning and continually updates its own reference material. The predetermined epigenetic state patterns determined by the classification algorithm therefore change over time in an effort to increase sensitivity and specificity even further. It is therefore neither feasible nor useful to give an example of the predetermined patterns used in the invention as they are subject to continuous change. On the other hand, the skilled person is familiar with these aspects of machine learning and can easily provide for a classification algorithm to establish its own predetermined epigenetic state patterns as used herein.

Biological changes, such as epigenetic changes, in cancer tissue are known to be specific for certain cancer types or subtypes. The biological state(s) of a gene site can be determined using different methods known to the skilled person. For example, the biological state, such as the epigenetic state, of a gene site can be determined by assaying histone modifications, proteomics or transcriptomics. One approach could, for example, be based on an Assay for Trans-posase-Accessible Chromatin using sequencing (ATAC-seq). Another approach is assaying DNA methylation. As there are robust and reliable DNA methylation assays established and readily available, determining the epigenetic state of gene sites through determining methylation is the preferred approach used in the disclosure. However, it is not a single data point determined by any of the mentioned assays that determines the type of the cancer. The type of the cancer is determined by the epigenetic downregulation or upregulation of its gene sites, which in turn determines the metabolism and phenotype of the cancer. Gene site activation can, however, be determined by a number of different epigenetic approaches, as outlined above. To classify cancer types, it is therefore more prudent to determine the effect of the epigenetic changes on gene site activity rather than rely on the specific epigenetic changes measured by a specific type of assay. In theory, all of the epigenetic approaches should in the end imply the same gene sites as having a pathological activity, provided that all gene sites and their activity are equally accessible through the various assays. This pathological gene site activity is what makes and defines the cancer types.

In view of the above, the methods of the present disclosure classify a cancer based on the biological state of specific genomic DNA sites or transcripts.

In one embodiment of the disclosure, the inventors used DNA methylation to find gene sites with pathological activity within the cancer genome. The epigenetic state of these gene sites was then used to find patterns typical for different cancer types. Thus, the inventors found a set of gene sites having the biggest impact on differentiating between different cancer types.

To this end the inventors tested their approach using an Illumina methylation bead chip with which a multitude of classically classified tumour specimen were tested. Illumina's HumanMethylation450 (450k) BeadChip allows to assays DNA methylation at 482,421 CpG dinucleotides. The platform measures DNA methylation by genotyping sodium bisulfite treated DNA. To run the assay only a small amount of DNA is needed and it is possible to use both frozen and paraffin (FFPE) material. So far, approximately 90000 tumour samples have been profiled by the inventors and allowed the verification of the surprisingly superior approach of the herein disclosed disclosure.

As readily apparent to the skilled person, the classification according to the disclosure also means that a stratification and/or a diagnosis of the cancer is achieved. In the context of the present disclosure the term "stratification" refers to the classification or grouping of patients according to one or more predetermined criteria. In certain embodiments stratification is performed in a diagnostic setting in order to group a patient according to the prognosis of disease progression, either with or without treatment. In particular embodiments stratification is used in order to distribute patients enrolled for a clinical study according to their individual characteristics. In particular embodiments stratification is used in order to identify the best suitable treatment option for a patient.

The term "diagnosis" or "diagnostic" is used herein to refer to the identification or classification of a molecular or pathological state, disease or condition. For example, "diagnosis" may refer to identification of a particular type of cancer, e.g., a lung cancer. "Diagnosis" may also refer to the classification of a particular type of cancer, e.g., by histology (e.g., a non-small cell lung carcinoma), by molecular features (e.g., a lung cancer characterized by nucleotide and/or amino acid variation(s) in a particular gene or protein), or both. However, it is important to note that the disclosure is directed to a strictly in vitro method in all its embodiments. None of the method steps of any embodiment are performed on the human or animal body.

The term "cancer type", "tumour species" or "tumour class" shall refer to a specific kind of a tumour or subcategory of a tumour that can be classified based on its tissue origin, genetic makeup, histology etc. In particular in the field of brain tumours various distinct tumour species or classes of the central nervous system exist that can be differentiated via for example histopathology (1. Acta Neuropathol. 2007 Aug; 114(2):97-109. Epub 2007 Jul 6. "The 2007 WHO classification of tumours of the central nervous system." Louis DN(1), Ohgaki H, Wiestler OD, Cavenee WK, Burger PC, Jouvet A, Scheithauer BW, Kleihues P.). Specifically, the disclosure pertains to the cancer types as listed in Table 2.

The term "cancer sample" or "tumour sample" as used herein refers to a sample obtained from a patient. The tumour sample can be obtained from the patient by routine measures known to the person skilled in the art, i.e., biopsy (taken by aspiration or punctuation, excision or by any other surgical method leading to biopsy or resected cellular material). For those areas not easily reached via an open biopsy a surgeon can, through a small hole made in the skull, use stereotaxic instrumentation to obtain a "closed" biopsy. Stereotaxic instrumentation allows the surgeon to precisely position a biopsy probe in three-dimensional space to allow access almost anywhere in the brain. Therefore, it is possible to obtain tissue for the diagnostic method of the present disclosure. The actual removal of the sample from the patient is, however, not part of the inventive method. "Providing a cancer sample" therefore merely pertains to making a sample available for laboratory use without the step of obtaining it from a patient in the first place.

The term "cancer" or "tumour" is not limited to any stage, grade, histomorphological feature, invasiveness, aggressiveness or malignancy of an affected tissue or cell aggregation. In particular stage 0 cancer, stage I cancer, stage II cancer, stage III cancer, stage IV cancer, grade I cancer, grade II cancer, grade III cancer, malignant cancer, primary carcinomas, and all other types of cancers, malignancies etc. are included.

As used herein, the term "gene site" refers to a region of DNA comprising or consisting of a gene, particularly a gene or gene site as listed in Table 1. In particular, the term "gene site" refers to a DNA sequence with a genetic locus as defined in Table 1. A gene site may comprise additional base pairs upstream and/or downstream of a gene, for example up to 12 kb, preferably up to 10 kb up to 8 kb or up to 6 kb or up to 4 kb or up to 2 kb upstream and/or downstream. A biological state, such as an epigenetic state, of a gene site may therefore refer to the biological state of the gene itself and additionally to the biological state of the additional string of base pairs upstream and/or downstream of the gene. In preferred embodiments of the disclosure, the biological state of the gene sites in the set comprises the biological state of the gene sites as listed in Table 1 and up to 10 kb, preferably up to 8 kb or up to 6 kb or up to 4 kb or up to 2 kb, upstream and/or downstream of the genes. In a further preferred embodiment, the biological state of the gene sites comprises exclusively the biological state of the gene sites as listed in Table 1 without any bases upstream and/or downstream of the gene sites. In this case, only the biological state of the gene sites themselves are being used and the gene sites do not comprise any bases outside of the gene sites as listed in Table 1.

The term "set of gene sites" refers to a number of gene sites being grouped together. For example, it is the epigenetic state(s) of this set of gene sites that is being evaluated in the disclosure, then combined into a pattern and analysed by the classification algorithm.

As used herein, the term "CpG site" or "CpG position" refers to a region of DNA where a cytosine nucleotide occurs next to a guanine nucleotide in the linear sequence of bases along its length, the cytosine (C) being separated by only one phosphate (p) from the guanine (G). About 70% of human gene promoters have a high CpG content. Regions of the genome that have a higher concentration of CpG sites are known as "CpG islands". Cytosines in CpG dinucleotides can be methylated to form 5-methylcytosine. Methylation of (i.e., introduction of a methyl group in) the cytosines of CpG site within the promoters of genes can lead to gene silencing, a feature found in a number of human cancers. In contrast, the hypomethylation of CpG sites has generally been associated with the over-expression of oncogenes within cancer cells. The term "independent genomic CpG positions" shall in the context of the present disclosure mean that each CpG position of a group of genomic CpG positions can be probed separately for its methylation state.

The term "methylation state", as used herein describes the state of methylation of a CpG position, thus refers to the presence or absence of 5-methylcytosine at one CpG site within genomic DNA. When none of the DNA of an individual is methylated at one given CpG site, the position is 0% methylated. When all the DNA of the individual is methylated at that given CpG site, the position is 100% methylated. When only a portion, e.g., 50%, 75%, or 80%, of the DNA of the individual is methylated at that CpG site, then the CpG position is said to be 50%, 75%, or 80%, methylated, respectively. The term "methylation state" reflects any relative or absolute amount of methylation of a CpG position. Methylation of CpG positions can be assessed by any method used in the art. The terms "methylation" and "hypermethylation" are used herein interchangeably. When used in reference to a CpG positions, they refer to the methylation state corresponding to an increased presence of 5-methylcytosine at a CpG site within the DNA of a biological sample obtained from a patient, relative to the amount of 5-methylcytosine found at the CpG site within the same genomic position of a biological sample obtained from a healthy individual, or alternatively from an individual suffering from a tumour of a different class or species.

The term "biological sample" is used herein in its broadest sense. In the practice of the present disclosure, a biological sample is generally obtained from a subject. A sample may be any biological tissue or fluid with which the biological state(s) of gene sites of the present disclosure may be assayed. Frequently, a sample will be a "clinical sample" (i.e., a sample obtained or derived from a patient to be tested). The sample may also be an archival sample with known diagnosis, treatment, and/or outcome history. Examples of biological samples suitable for use in the practice of the present disclosure include, but are not limited to, bodily fluids, e.g., blood samples (e.g., blood smears), and cerebrospinal fluid, brain tissue samples, spinal cord tissue samples or bone marrow tissue samples such as tissue or fine needle biopsy samples. Biological samples may also include sections of tissues such as frozen sections taken for histological purposes. The term "biological sample" also encompasses any material derived by processing a biological sample. Derived materials include, but are not limited to, cells (or their progeny) isolated from the sample, as well as nucleic acid molecules (DNA and/or RNA) extracted from the sample. Processing of a biological sample may involve one or more of: filtration, distillation, extraction, concentration, inactivation of interfering components, addition of reagents, and the like.

The method according to some embodiments of the present disclosure includes a step of "determining an epigenetic state" of a set of gene sites. This can be achieved through any means suitable to assay epigenetically modified activity of the gene sites. In a preferred embodiment of the disclosure the epigenetic state of a set of gene sites is determined by assessing the DNA methylation state of a multitude of independent genomic CpG positions, particularly CpG positions within the gene sites as mentioned above, preferably within the gene sites listed in Table 1, in a biological sample obtained from a patient. Determination of the methylation state may be performed using any method known in the art to be suitable for assessing the methylation of cytosine residues in DNA. Such methods are known in the art and have been described; and one skilled in the art will know how to select the most suitable method depending on the number of samples to be tested, the quantity of sample available, and the like.

Thus, the methylation state of a genomic CpG position or a combination of genomic CpG positions according to the disclosure can be determined using any of a wide variety of methods that are generally divided into strategies based on methylation-specific PCR (MSP), and strategies employing PCR performed under methylation-independent conditions (MIP). Methylation-independent PCR (MIP) primers are used in most of the available PCR-based methods. They are designed for proportional amplification of methylated and unmethylated DNA. In contrast, methylation-specific PCR (MSP) primers are designed for the amplification of methylated template only.

Examples of methylation-independent PCR based techniques include, but are not limited to, direct bisulfite direct sequencing (Frommer et al., PNAS USA, 1992, 89: 1827-1831), pyrosequencing (Collela et al., Biotechniques, 2003, 35: 146-150; Uhlmann et al., Electrophoresis, 2002, 23: 4072-4079; Tost et al., Biotechniques, 2003, 35: 152-156), Combined Bisulfite Restriction Analysis or "COBRA" (Xiong et al., Nucleic Acids Res., 1997, 25: 2532-2534), Methylation-Sensitive Single-Nucleotide Primer Extension or "MS-SnuPE" (Gonzalgo et al., Nucleic Acids Res., 1997, 25: 2529-2531), Methylation-Sensitive Melting Curve Analysis or "MS-MSA" (Worm et al., Clin. Chem., 2001, 47: 1183-1189), Methylation-Sensitive High-Resolution Melting or "MS-HRM" (Wojdacz et al., Nucleic Acids Res., 2007, 35:e41), MALDI-TOF mass spectrometry with base-specific cleavage and primer extension (Ehrich et al., PNAS USA, 2005, 102: 15785-15790), and HeavyMethyl (Cottrell et al., Nucleic Acids Res., 2004, 32: e10).

Examples of methylation-specific PCR based techniques include for example methylation specific PCR or "MSP" (Herman et al., PNAS USA, 1996, 93: 9821-9826; Mackay et al., Hum. Genet., 2006, 120: 262-269; Mackay et al., Hum. Genet., 2005, 116: 255-261; Palmisano et al., Cancer Res., 2000, 60: 5954-5958; Voso et al., Blood, 2004, 103: 698-700), MethylLight (Eads et al., Nucleic Acids Res., 2000, 28:e32; Eads et al., Cancer Res., 1999, 59: 2302-2306; Lo et al., Cancer Res., 1999, 59: 3899-3903), Melting curve Methylation Specific PCR or "McMSP" (Akey et al., Genomics, 2002, 80: 376-384), Sensitive Melting Analysis after Real-Time MSP or "SMART-MSP" (Kristensen et al., Nucleic Acids Res., 2008, 36: e42), and Methylation-Specific Fluorescent Amplicon Generation or "MS-FLAG" (Bonanno et al., Clin. Chem., 2007, 53: 2119-2127).

Many of these methods rely on the prior treatment of DNA with sodium bisulphite. This treatment leads to the conversion of unmethylated cytosine to uracil, while methylated cytosine remains unchanged (Clark et al., Nucleic Acids Res., 1994, 22: 2990-2997). This change in the DNA sequence following bisulphite conversion can be detected using a variety of methods, including PCR amplification followed by DNA sequencing. It is safe to say that the use of bisulphite-converted DNA for DNA methylation analysis has surpassed almost every other methodology for DNA methylation analysis, thereby becoming the gold standard for detecting changes in DNA methylation. The protocol described by Frommer et al. (PNAS USA, 1992, 89: 1827-1831) has been widely used for sodium bisulphite treatment of DNA, and a variety of commercial kits are now available for this purpose.

Thus, in a method according to the disclosure, the step of determining the epigenetic state can be achieved by determining the methylation state of a gene promoter, or of a combination of gene promoters of the disclosure. It may be performed using any of the techniques described above or any combination of these techniques. One skilled in the art will recognized that when the methylation state of a combination of gene promoters has to be determined, the determinations may be performed using the same DNA methylation analysis technique or different DNA methylation analysis techniques. Other methods include oligonucleotide methylation tiling arrays, BeadChip assays, HPLC/MS methods, methylation-specific multiplex ligation-dependent probe amplification (MS-MPLA), bisulphite sequencing, and assays using antibodies to DNA methylation, i.e., ELISA assays.

By using the statistical model as described herein, the inventors found that the gene sites comprising the gene sites listed in Table 1 are sufficient to classify cancer samples into a large number of different cancer types. While it may be possible to classify even more cancer types by analysing the named gene sites, this has been validated for the cancer types listed in Table 2. To classify a cancer type, according to the disclosure, it is therefore only necessary to determine the epigenetic state of these selected gene sites, in particular of at least 3 gene sites. A full analysis of the whole genome of the cancer type can therefore be avoided. For a sufficiently specific classification, only those gene sites listed in Table 1 must be analysed, resulting in quicker and less laborious diagnosis.

The inventors further found that a set of gene sites comprising at least 3 of the gene sites listed in Table 1 is sufficient for the classification of the cancer sample. However, larger sets provide more accuracy. In preferred embodiments of the disclosure, the set of gene sites thus comprises at least 10, preferably at least 20 or at least 30 or at least 40 or at least 50 or at least 60 or at least 70 or at least 80 or at least 90 or at least 100, genes of the sample genome of the cancer being classified. A set of gene sites preferably comprising 100 or less, 90 or less, 80 or less, 70 or less, 60 or less, 50 or less, 40 or less, 30 or less, 20 or less, or 10 or less gene sites provide for a good balance between accuracy and work necessary. The embodiments of the present disclosure are not limited thereto, and the set may comprise more than 100 gene sites or all gene sites listed in Table 1.

While all of the gene sites or genes listed in Table 1 could be used to classify the cancer types as described herein in Table 2, the inventors identified subsets of the genes with higher importance, meaning resulting in more accuracy, when used to classify specific cancer types. It is therefore preferred that the predetermined pattern for a cancer type as listed in Table 2 comprises at least 3 gene sites for that specific cancer type. It is further preferred that the predetermined pattern for a cancer type comprises at least 3 gene sites for that specific cancer type selected from the gene sites listed in Tables 3 to 172, respectively. In a preferred embodiment the set of gene sites of the cancer sample genome being analysed comprises the exact same gene sites or genes as the predetermined pattern.

In a preferred embodiment, the statistical model employed by the inventors provides for a measure of the variable importance of the gene sites for each cancer.

As can be seen from Tables 3 to 172, the different gene sites have different importance for the classification. To improve the accuracy of the classification, it is therefore preferred that the epigenetic data for a cancer type comprises those gene sites listed in Tables 3 to 172 for that cancer type that are the ones with the highest values of variable importance for that cancer type.

As stated before, it is preferred that the set of gene sites of the cancer sample genome being analysed comprises the same genes or gene sites as the epigenetic data derived from pre-classified cancer types (predetermined pattern). The set of gene sites of the cancer sample genome being analysed, and the epigenetic data derived from pre-classified cancer types therefore preferably also comprise the same number of genes or gene sites.

While analysing gene sites of a set of genes comprising 3 genes is advantageous for being less laborious, the accuracy of the classification increases with more genes being analysed per cancer type. It is therefore preferred that the predetermined pattern for a cancer type listed in Table 2 comprises at least 10, preferably at least 20 or at least 30 or at least 40 or at least 50 or at least 60 or at least 70 or at least 80 or at least 90 or at least 100, gene sites or genes listed in Table 1. The preferred gene sites or genes "for a cancer type" are the ones listed for each cancer type in in Tables 3 to 172, respectively. As explained above for the set, 80 to 100 genes provide for a good balance between accuracy and workload.

The classification may include a direct or indirect comparison of the epigenetic state pattern of the set of gene sites with predetermined epigenetic state patterns, e.g. by determining the overlap of the two patterns, i.e., how much the two patterns are similar to or different from each other. This may, for example, be statistically determined and may be represented as a numerical value. Specifically, the difference between the patterns may be represented by a percentage. The accuracy of the classification can be influenced by allowing patterns with higher or lower difference from a predetermined pattern to still be classified as the cancer type the predetermined pattern pertains to. For a suitable accuracy, it is preferred that the cancer is classified as the cancer to which the predetermined pattern pertains if the epigenetic state pattern of the set of gene sites differs from the predetermined pattern by at most 5 %, preferably at most 4 % or at most 3 % or at most 2 % or at most 1 %. These values are both useful in practice and achievable by the inventive method.

As explained above, the predetermined epigenetic state patterns used for comparison have been determined by the inventors by analysing more than 90000 cancer samples from a range of different sources. As this process is also part of the present disclosure, it is explained in detail below.

In all embodiments the method of the disclosure is performed as an *ex-vivo* or *in-vitro* method.

Most preferably the methods according to the disclosure are used for the classification of tumours of the central nervous system, therefore, the tumour preferably is a brain tumour or a spinal cord tumour, and the tumour species is a brain tumour species or a spinal cord tumour species. As already noted herein before, these tumours are characterized by a huge epigenetic variety which has a significant impact on the development of treatment regimes in order to allow for the best treatment of the patient. If the tumour disease is a tumour of the central nervous system (CNS), it is preferred that said tumour species comprises at least 184 different classes of CNS tumours. Additionally, the disclosure is also applicable to sarcomas. In a preferred embodiment said CNS tumours are selected form the list of cancer types or tumour species of Table 2.

The determination of DNA methylation levels of the disclosure is performed preferably with a genomic array or chip comprising probes which are specific for the methylation of at least 1000 CpG positions. It is preferred to test as many positions as possible in order to allow for the generation of a highly specific classification. Genome-wide DNA methylation assays are therefore preferred, such as the HumanMethylation450k-chip (Illumina^{®}).

The classification algorithm may be based on random forest (RF). The training of the RF-based classification algorithm according to some embodiments of the disclosure may comprise a preceding step of selecting CpG position which of all CpG positions used provide the purest splitting rules, and using said selected CpG positions as a training-data-optimization-set to train a classification rule.

In other embodiments of the disclosure the training of the RF-based classification algorithm may comprise a step of down-sampling for each tumour species the number of bootstrap samples to the minority class, the minority class being the lowest sample size of a tumour species in the training dataset.

Another embodiment of the disclosure provides the above method and comprises the further step of including the methylation data of the tumour sample as classified into the training-data-set to obtain an enhanced-training-data-set and computing an enhanced-classification-rule by random forest analysis based on the enhanced-training-dataset. Optionally the classification of said tumour sample may be repeated with the enhanced-classification-rule. This embodiment serves the continuous development and improvement of the original training data set. Each further classified tumour species will have a genomic DNA methylation profile or epigenetic state pattern that further enhances the classification for that tumour species and that can then be used as a predetermined epigenetic state pattern in the present disclosure. Therefore, the disclosure in one preferred embodiment provides a classification system characterized by a self-learning classification rule.

In order to provide a classification rule with good specificity and sensitivity, the predetermined methylation data / epigenetic state pattern used in context of the present disclosure includes for each pre-classified cancer type the methylation state/levels at said CpG position of at least one, two, three, four, five, six or more independent samples.

Another aspect of the present disclosure then pertains to a method for stratifying the treatment of a tumour patient, comprising the classification of the tumour species / cancer type of the tumour of the patient according to the classification methods of the disclosure and stratifying the treatment of the patient in accordance with the diagnosed tumour species.

Yet a further aspect of the disclosure pertains to a computer-implemented method for generating a classification-rule for aiding the classification of tumour samples in cancer diagnosis, the method comprising providing DNA methylation data of a multitude of independent genomic CpG positions of genomes of a multitude of diverse pre-classified tumour species of the same tumour type (for example brain cancer, lung cancer, leukaemia, etc.); computing a random forest of binary decision trees from the DNA methylation data, wherein in each binary decision tree of said random forest each node is a CpG position, and each terminal leave a specific tumour species, and each binary splitting rule is a methylation state at said CpG position. This method can be used to create the predetermined epigenetic state patterns as explained above.

To learn a classification rule that allows predicting the class assignment of future diagnostic cases the inventor's applied the machine learning algorithm RandomForest (RF; Breiman, 2001). The RF algorithm is a so-called ensemble method that combines the predictions of several 'weak' classifiers to achieve improved prediction accuracy. The RF uses binary classification trees (Classification and Regression Trees (CART); Breiman et al., 1983) as 'weak' classifiers. Each of these trees is a sequence of binary splitting rules that are learned by recursive binary splitting. The CART algorithm starts with all samples assigned to a 'root' node and tries to find the variable, e.g., a measured CpG probe, and a corresponding cut-off that results in the purest split into the different classes. To measure this gain in class 'purity' the Gini index, a classical statistical measure for inequality, may be used. To fit a tree the CART algorithm iteratively repeats these steps until no further improvements can be made, i.e., only samples of the same class are assigned to the final 'leaf' node, or a pre-specified node size is achieved. To predict the class of a new diagnostic case the binary splitting rules are compared with the new data starting in the root node down to one of the leaf nodes. The tree then predicts or votes for the class dominating that leaf node.

Decision trees have the advantage that they are non-parametric and do not rely on any distributional assumptions. Moreover, trees allow to learn complex non-linear relationships and interactions, they are easy to interpret and can be efficiently fitted in large data sets. The main disadvantages of decision trees are that they often tend to overfit the data and that they have a weak prediction performance.

However, to improve the prediction accuracy of a single tree the RF algorithm combines thousands of trees by bootstrap aggregation (bagging). In brief, each tree is fitted using training data sets that are generated by drawing bootstrap samples, i.e., randomly selecting two-third of the data with replacement. In addition, at each node only a random subset of the available variables is used to find an optimal splitting rule. This additional source of randomization allows selecting variables with lower predictive value that would otherwise be ruled out by the most prominent variables. This feature guarantees that the resulting trees are decorrelated, i.e., they use different variables to find an optimal prediction rule. Taking the majority vote over thousands of bootstrap aggregated and decorrelated trees greatly improves the prediction accuracy of the RF. The majority vote, i.e., the proportion of trees voting for a class, can be used as empirical class probabilities or scores that turned out to be a very useful tool for diagnosis.

To validate the resulting RF classifier, a repeated five-fold cross-validation is applied. In each cross-validation the reference set is randomly split into five parts. Then four-fifth of the data is used to train the RF classifier and one-fifth is used for prediction. Currently the estimated test error of the classifier is around 3.1%.

Alternatively, the resulting RF classifier is validated by a repeated threefold cross-validation. In each cross validation the reference set is randomly split into three parts. Then two-third of the data is used to train the RF classifier and one-third is used for prediction. Currently the estimated test error of the classifier is around 4.9%.

The classification scores generated by the RF, i.e., the proportion of trees voting for a class, are typically unequally distributed between classes. Furthermore, if interpreted as class probabilities, the scores often fail to estimate the actual class probabilities and are thus said to be not well-calibrated. However, to judge the classification of a single case in the context of clinical diagnosis, the uncertainties associated with an individual prediction in terms of a confidence scores, or estimated class probability is needed. To receive recalibrated scores that are comparable between classes and that are improved estimates of the certainty of individual predictions, the inventors fit a calibration model to raw RF scores. This calibration model is a multinomial logistic regression model, which takes the tumour subclasses as response variable and the 'raw' RF scores as explanatory variables. In addition, this model is fitted by incorporating a small ridge-penalty on the likelihood to prevent the model from over fitting as well as to stabilize estimation in situations where classes are perfectly separable. The amount of this regularization, i.e., the penalization parameter, is determined by running a ten-fold cross-validation and choosing the value that minimizes the misclassification error. To fit this model independent, 'raw' RF scores are needed, i.e., the scores need to be generated by an RF classifier that was not trained using the same samples, otherwise the RF scores will be systematically biased and not comparable to scores of unseen cases. To generate such independent 'raw' scores, the inventors apply a three-fold cross validation.

To validate the class predictions generated by using the recalibrated scores of the calibration model a three-fold nested cross-validation is applied. In each cross validation the reference set is randomly split into three parts. Then two-third of the data is used to train the RF classifier and one-third is used for prediction. Within each of these three cross-validation runs a nested three-fold cross-validation is applied to generate independent RF scores, which are used to train a calibration model. The predicted RF scores resulting from the outer cross-validation loop are then recalibrated by using the suitable calibration model, i.e. a model that was fitted using the RF scores generated by using the other two-third of the data in the inner loop. Currently the estimated test error of the classifier when using the recalibrated scores for prediction is around 3.2%.

Some embodiments of the disclosure pertain to a method where the diverse tumour species are selected from metastatic tumours, tumours stemming from specific tissues, tumours in a specific stage, recurrent tumours, tumours having a specific genetic mutation, tumours of patients having different gender, age or genetic background.

### BRIEF DESCRIPTION OF THE DRAWINGS

The present disclosure will now be further described in the following examples with reference to the accompanying figures and sequences, nevertheless, without being limited thereto. For the purposes of the present disclosure, all references as cited herein are incorporated by reference in their entireties. In the Figures:

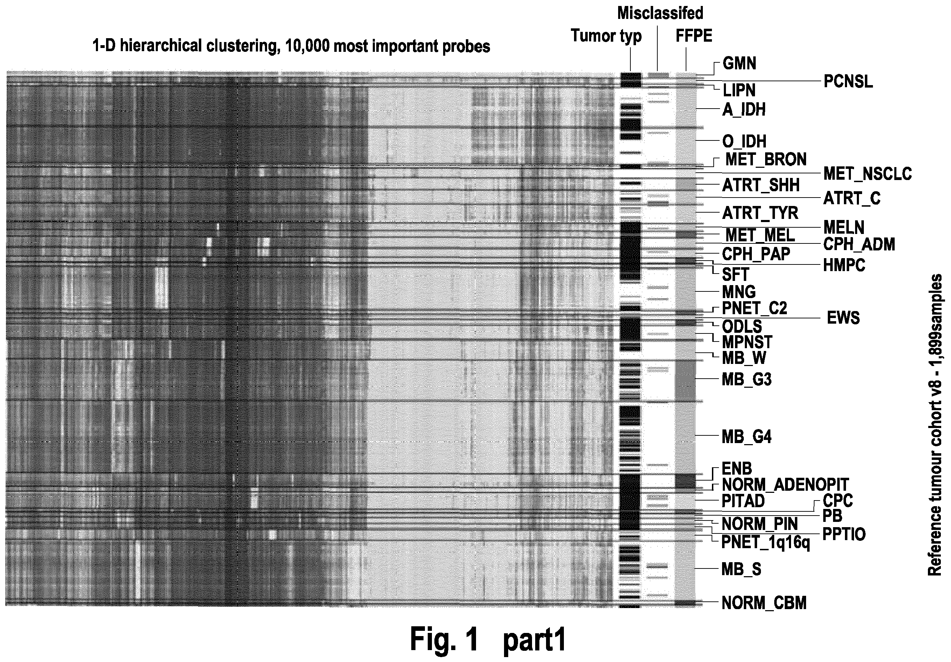

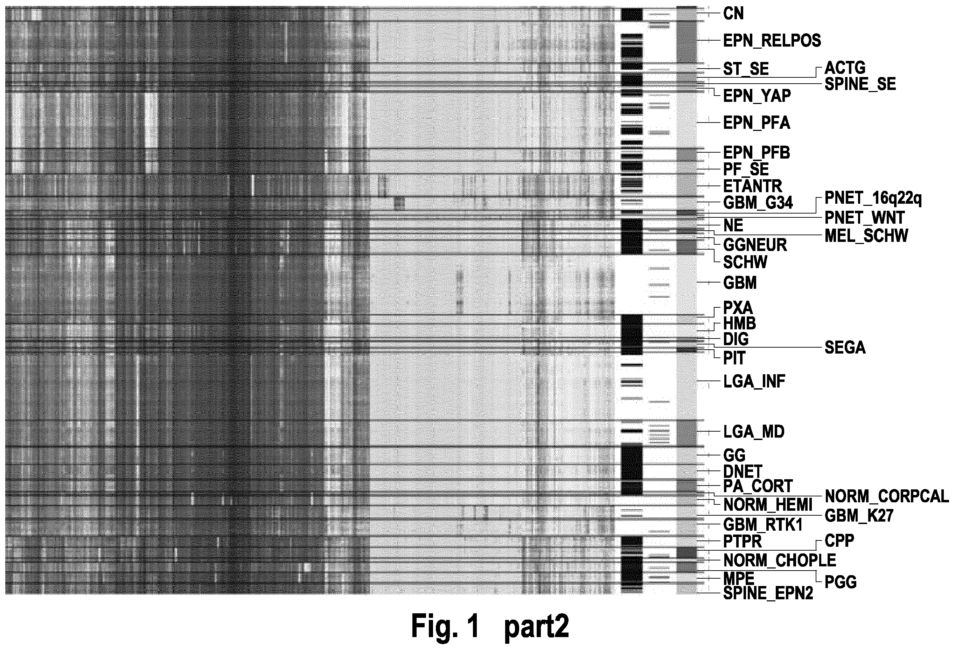

- **Figure 1:**: Heatmap representation of the reference set. The colour code indicates the different tumour classes, FFPE and frozen samples as well as samples that are misclassified in the cross validation. The heatmap shows the methylation profile of 10,000 CpG probes most important for the classification (highest average gain in Gini purity).

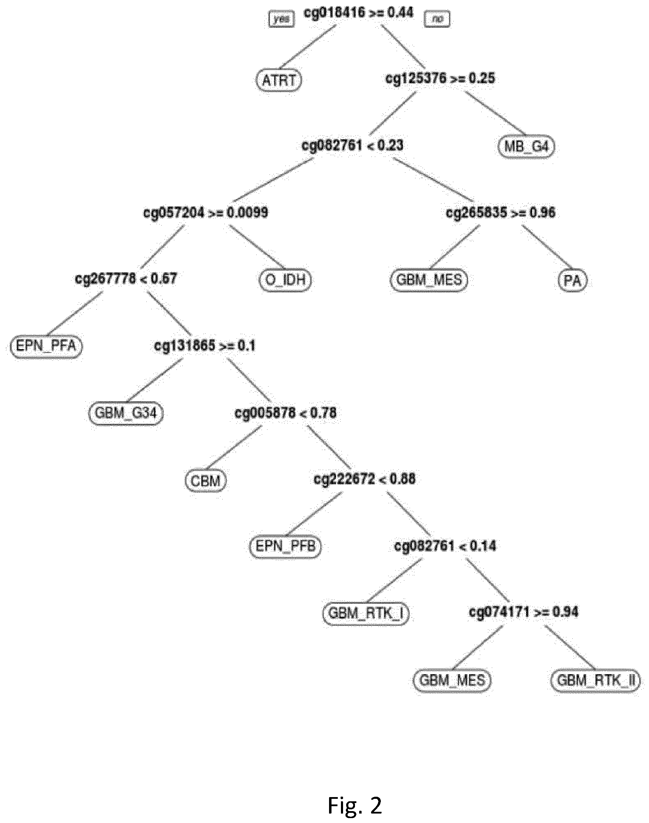

- **Figure 2:**: Example of a binary decision tree. At each node a CpG probe and corresponding cut-off is used to make a binary decision. The final leaf nodes display the abbreviation of the tumour subclass, i.e., EPN_PFA means posterior fossa ependymoma subtype A.

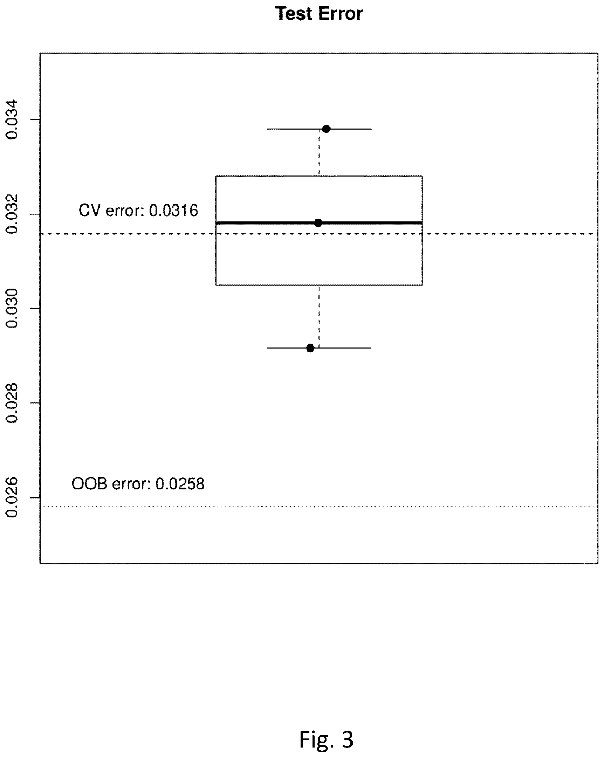

- **Figure 3:**: Median test error estimated by three five-fold cross validation runs.
- **Figure 4:**: The left panel shows a symbolically the histology of a WNT medulloblastoma and a Group 3 medulloblastoma which are not distinguishable. The right panel shows a multidimensional scaling (MDS) analysis of 107 medulloblastoma samples of all molecular subtypes using the 21,092 most variable CpG probes. WNT medulloblastoma are coloured in blue, SHH medulloblastoma in red, Group 3 medulloblastoma in yellow and Group 4 medulloblastoma in green.
- **Figure 5:**: A shows the result of the histology of the patient. B shows the classifier scores. Highlighted is the highest score entry.
- **Figure 6:**: A and B show the result of the histology of the patient. C shows the classifier scores. Highlighted is the highest score entry.
- **Figure 7:**: Schematic overview how the classifier is trained and validated by the three-fold nested-cross validation. In each outer cross validation run the training data is used for an inner three-fold cross-validation that generates independent RF scores. These scores are used to fit a calibration model which can then be applied to recalibrate the RF scores generated by predicting the test data in the outer loop. To fit a calibration model using all the data in the reference set, which is later used for new diagnostic cases, the RF scores generated in the outer loop can be used.
- **Figure 8:**: Genome plot showing the *PTPRN2* gene, CpG sites and RF variable importance measure.
- **Figure 9:**: Heatmap showing the methylation values of 100 CpGs located on *PAX6, PTPRN2* and *OSTM1* with highest standard deviation across 75 ATRT samples. Rows and columns have been reordered by applying hierarchical clustering with Euclidean distance as distance metric and complete linkage as linkage method. The class annotation colour code shows the previously known molecular subtypes, the gene annotation indicates the gene on which the CpGs are located.
- **Figure 10A:**: 75 ATRT tumour samples projected on to the first two PCs resulting from PCA.
- **Figure 10B:**: 75 ATRT tumour samples projected on to coordinates calculated by tSNE analysis.
- **Figure 10C:**: CART tree with two sequential splitting rules.
- **Figure 10D:**: Scatterplot of 75 ATRT tumour samples, the x and y-axes are the methylation value of the two CpG sites selected by the CART tree. The corresponding splitting rule cut-offs are displayed as dashed lines.
- **Figure 11:**: tSNE of 1167 samples for which DNA-methylation as well as gene expression data is available. The tSNE coordinates were calculated on the gene expression data of the 688 most important genes or gene sites. The class labels and colours correspond to classes predicted by the methylation classifier.
- **Figures 12A and B:**: Confusion matrices that show the results of a 3-fold cross validation to validate the RF and the multinomial logistic regression model. Like for classifiers trained on methylation data, most errors occur between closely related entities such as the MB group 3 and 4 subtypes.

### DETAILED DESCRIPTION OF EMBODIMENTS

### Infinium Methylation Assay

Genome-wide screening of DNA methylation patterns was performed by using the Infinium HumanMethylation450 BeadChips (Illumina, San Diego, US), allowing the simultaneous quantitative measurement of the methylation state at 485,577 CpG sites. By combining Infinium I and Infinium II assay chemistry technologies, the BeadChip provides coverage of 99% of RefSeq genes and 96 % of CpG islands.

DNA concentrations were determined using PicoGreen (Life Technologies, Darmstadt, Germany). The quality of genomic DNA samples was checked by agarose-gel analysis, and sampies with an average fragment size >3kb were selected for methylation analysis. For formalin-fixed paraffin-embedded (FFPE) DNA samples the quality was evaluated by real-time PCR analysis on Light Cycler 480 Real-Time PCR System (Roche, Mannheim, Germany) using the Infinium HD FFPE QC Kit (Illumina). The laboratory work was done in the Genomics and Proteomics Core Facility at the German Cancer Research Center, Heidelberg, Germany (DKFZ).

DNA (500 ng genomic DNA and 250 ng FFPE DNA, respectively) from each sample was bisulfite converted using the EZ-96 DNA Methylation Kit (Zymo Research Corporation, Orange, US) according to the manufacturer recommendations. Bisulfite treatment leads to the deamination of non-methylated cytosines to uracils, while methylated cytosines are refractory to the effects of bisulfite and remain cytosine. After bisulfite conversion, FFPE samples were treated with the Infinium HD DNA Restoration Kit (Illumina) according to the manufacturer recommendations. By using enzymatic reactions, degraded FFPE DNA is restored in preparation for the whole genome amplification.

Each sample was whole genome amplified and enzymatically fragmented following the instructions in the Illumina Infinium HD Assay Methylation Protocol Guide (genomic DNA) or Infinium HD FFPE Methylation Guide (FFPE DNA), respectively. The DNA was applied to Infinium HumanMethylation450 BeadChip and hybridization is performed for 16-24h at 48°C. During hybridization, the DNA molecules anneal to locus-specific DNA oligomers linked to individual bead types. One or two probes are used to interrogate CpG locus, depending on the probe design for a particular CpG site.

Allele-specific primer annealing is followed by single-base extension using DNP- and Biotin- labeled ddNTPs. For Infinium I assay design, both bead types (one each for the methylated and unmethylated states) for the same CpG locus incorporate the same type of labeled nucleotide, determined by the base preceding the interrogated "C" in the CpG locus, and therefore are detected in the same color channel. Infinium II uses only one bead type with a unique type of probe allowing detection of both alleles. The methylated and unmethylated signals are generated respectively in the green and the red channels.

After extension, the array is fluorescently stained, scanned, and the intensities at each CpGs were measured. Microarray scanning was done using an iScan array scanner (Illumina). DNA methylation values, described as beta values, are recorded for each locus in each sample. DNA methylation beta values are continuous variables between 0 and 1, representing the percentage of methylation of a given cytosine corresponding to the ratio of the methylated signal over the sum of the methylated and unmethylated signals.

### Data pre-processing

All data analysis was performed using the open-source statistical programming language R (R Core Team, 2014). Raw data files generated by the iScan array scanner were read and preprocessed using the capabilities of the Bioconductor package minfi (Aryee et al, 2014). With the minfi package the same pre-processing steps as recommended in Illumina's BeadStudio software were performed.

In addition, the following filtering criteria were applied: Removal of probes targeting the X and Y chromosomes (n = 11,551), removal of probes containing a single nucleotide polymorphism (dbSNP132 Common) within five base pairs of and including the targeted CpG-site (n = 24,536), and probes not mapping uniquely to the human reference genome (hg19) allowing for one mismatch (n = 9,993). In total, 438,370 probes were kept for analysis.

### Training the classifier

To learn a classification of 1899 samples that were assigned to 72 different brain tumour subtypes the Random Forest (RF) algorithm implemented in the R package randomForest (Liaw and Wiener, 2002) was used. The RF algorithm is a so-called ensemble method that combines the predictions of several 'weak' classifiers to achieve improved prediction accuracy. The RF uses binary classification trees (Classification and Regression Trees (CART); Breiman et al., 1983) as 'weak' classifiers. Each of these trees represents a sequence of binary splitting rules that are learned by recursive binary splitting. The CART algorithm starts with all samples assigned to a 'root' node and tries to find the variable, e.g., a measured CpG probe, and a corresponding cut-off that results in the purest split into the different classes. To measure this gain in class 'purity' the Gini index, a classical statistical measure for inequality, is used. To fit a tree the CART algorithm iteratively repeats these steps until no further improvements can be made, i.e., only samples of the same class are assigned to the final 'leaf' node, or a pre-specified node size is achieved. To predict the class of a new diagnostic case the binary splitting rules are compared with the new data starting in the root node down to one of the leaf nodes. The tree then predicts or votes for the class dominating that leaf node. However, to improve the prediction accuracy of a single tree the RF algorithm combines thousands of trees by bootstrap aggregation (bagging). In brief, each tree is fitted using training data sets that are generated by drawing bootstrap samples, i.e., randomly selecting two-third of the data with replacement. In addition, at each node only a random subset of the available variables are used to find an optimal splitting rule. To predict the class of a diagnostic sample the RF takes the majority vote of all trees in the forest.

To learn the classification the default parameter settings of the randomForest function were used and 10,000 decision trees were fitted. In addition, to take the highly imbalanced class sizes into account a down-sampling strategy was followed, i.e., to fit a decision tree the number of bootstrap samples drawn from each class was equal to the number of samples in the minority class. To further improve prediction performance of the classifier a variable selection was performed, i.e. in a first step the algorithm is used to calculate the variable importance, e.g. the average improvement in Gini purity of a CpG probe when used for a splitting rule. The final classifier was trained using only the 30,000 CpG probes with highest variable importance measure.

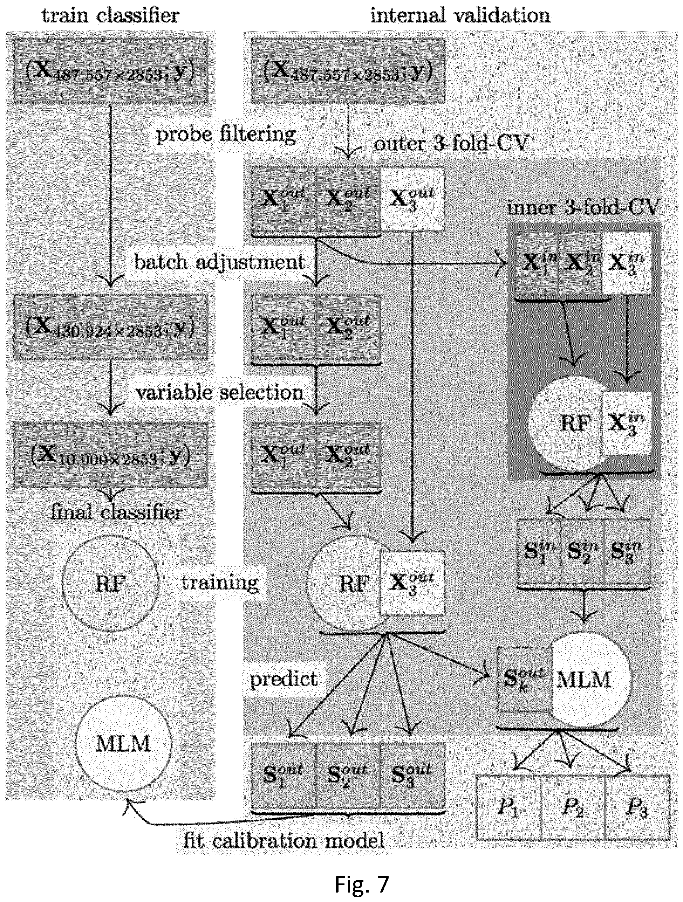

An overview of the training of the classifier is provided in Figure 7.

### Internal validation

To validate the resulting classifier and estimate its performance in predicting future diagnostic cases a repeated five-fold cross-validation was applied. In example, in each cross-validation run the reference set is randomly split into five parts. Then four-fifth of the data is used to train the RF classifier as described above and one-fifth is used for prediction. Currently the estimated test error of the classifier is around 3.1%.

### Example 1: Distinguishing WNT medulloblastoma from Group 3 medulloblastoma

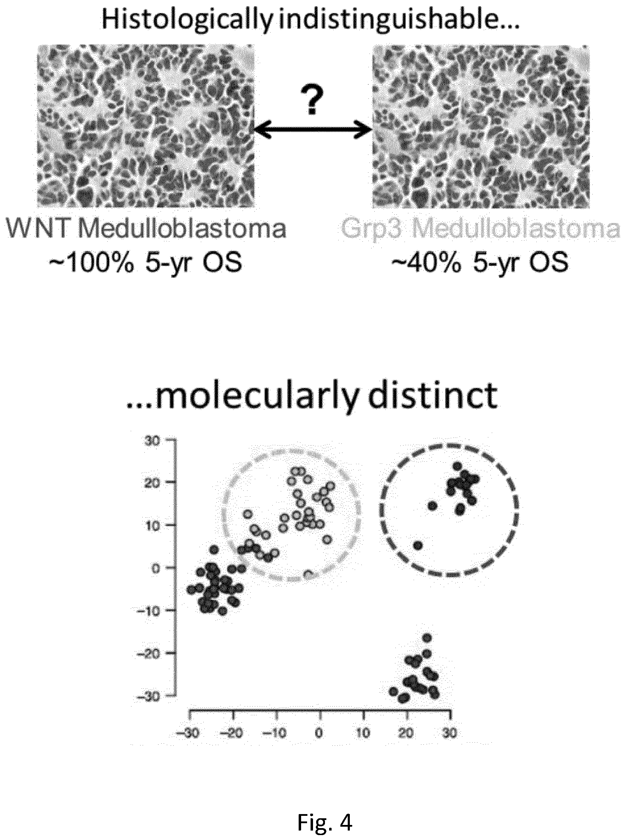

Medulloblastoma is the most common malignant paediatric brain tumour and comprises four distinct molecular variants. These variants are known as WNT, SHH, Group 3, and Group 4. These variants are histologically indistinguishable, but clearly separable by DNA methylation patterns (see Figure 4). WNT tumours show activated Wnt signalling pathway and carry a favourable prognosis. SHH medulloblastoma show Hedgehog signalling pathway activation and are known to have an intermediate to good prognosis. While both WNT and SSH variants are molecularly already well characterised, the genetic programs driving the pathogenesis of Group 3 and Group 4 medulloblastoma remain largely unknown.

### Example 2: Change of Diagnosis of an anaplastic astrocytoma WHO III

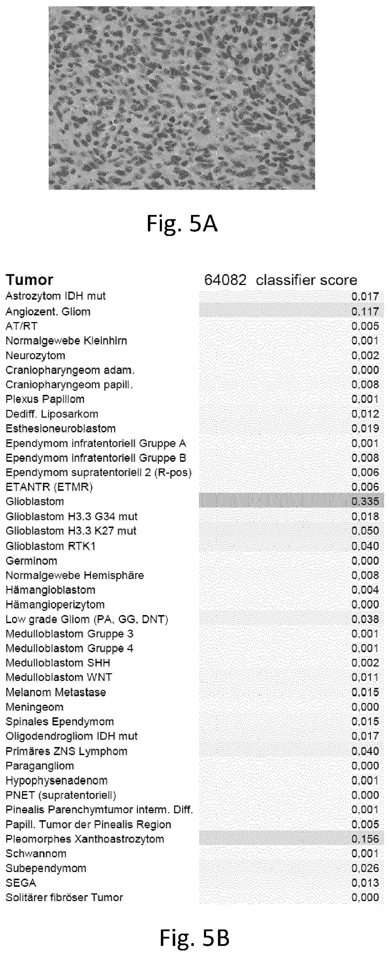

A 1944 born female brain tumour patient was diagnosed based on histology (see Figure 5A) to suffer from an anaplastic astrocytoma WHO III. Using the inventive classification procedure, a classifier score of 0.335 changed the diagnosis to Glioblastoma WHO IV (see Figure 5B).

### Example 3: Change of Diagnosis of Schwannoma

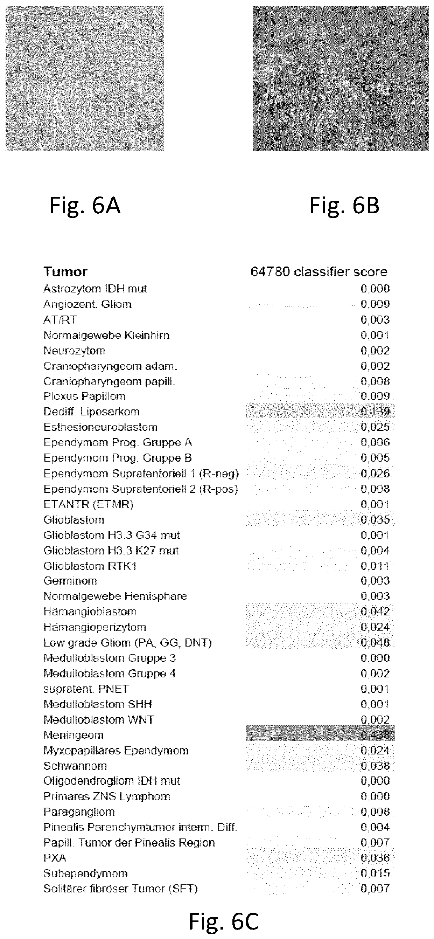

A 1969 born male patient was based on the histology diagnosed with Schwannoma (Figures 6A and 6B). The classification procedure of the present disclosure however was able to diagnose the patient to suffer from Meningioma WHO I (see Figure 6C).

### Example 4: DNA methylation-based classification of tumour entities using three gene sites

Atypical teratoid rhabdoid tumour (ATRT) is a rare paediatric brain tumour that can be subdivided into three molecular subgroups: ATRT-TYR, ATRT-SHH and ATRT-MYC (Ho et al. 2020, PMID: 31889194).

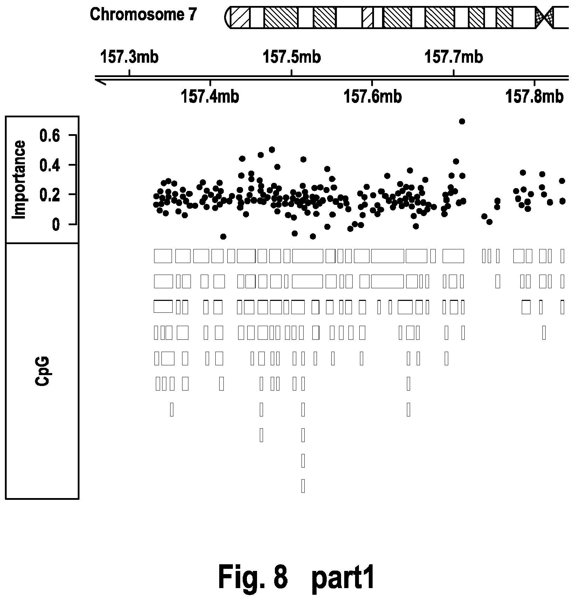

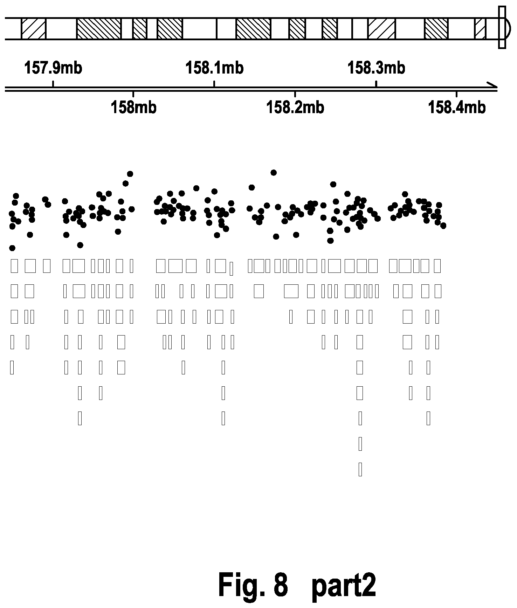

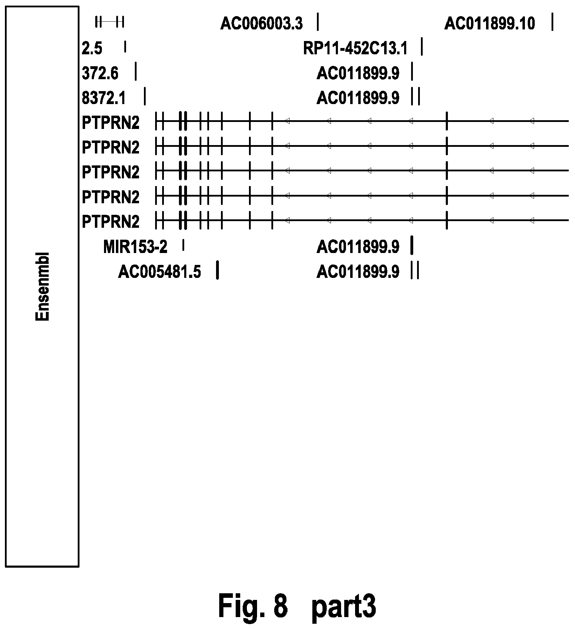

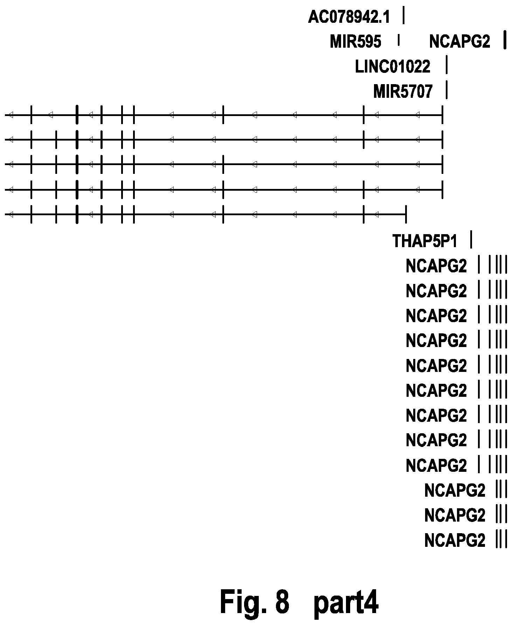

The inventors have identified genes that include CpG sites that are most important for the classification of brain tumours and molecular subtypes. The importance of these CpGs for the classification has been measured by applying the permutation-based variable importance measure of the Random Forest (RF) algorithm (Strobl et al. 2007, PMID: 17254353). Among others the three genes PAX6, PTPRN2 and OSTM1 include many important CpGs for the classification. Figure 8 displays the PTPRN2 gene and the CpG sites located on it. Most of the CpGs have a positive variable importance measure, indicating that these CpGs are predictive for the classification of brain tumours.

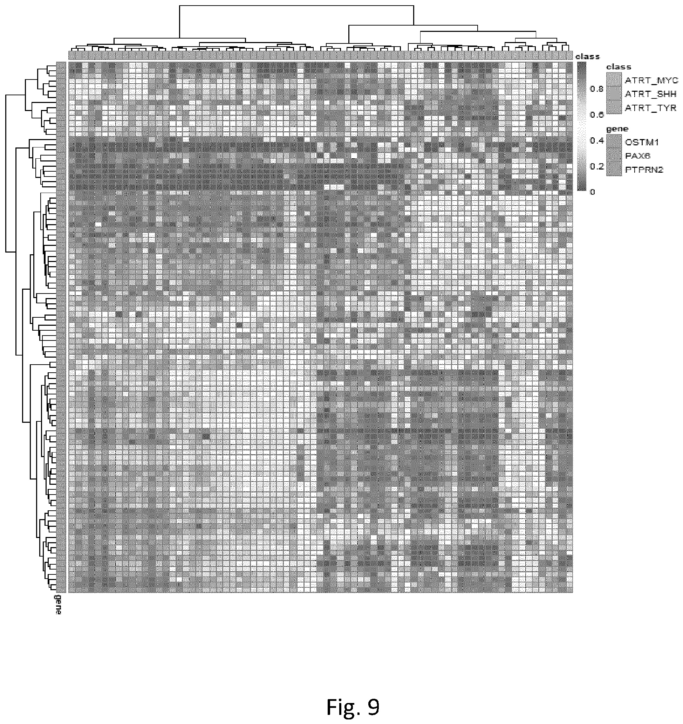

In the following it is demonstrated how the CpGs located on the three genes *PAX6, PTPRN2* and *OSTM1* can be used to classify ATRTs into their three molecular subtypes by applying different unsupervised and supervised statistical methods. After pre-processing, the inventors identified 1022 CpGs located on the three genes. Applying unsupervised, hierarchical clustering to the methylation values of the 100 CpGs with highest standard deviation across 75 ATRT samples, an almost perfect separation into the three molecular subtypes of ATRT can be found (Figure 9).

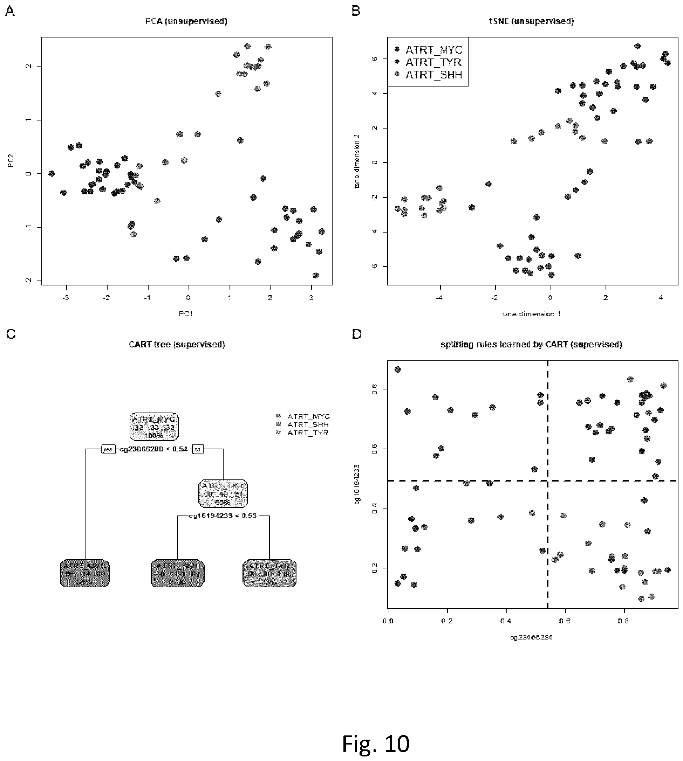

Next principal component analysis (PCA) is applied as an example for a linear dimension reduction method to the methylation values of all 1022 CpGs. Projecting the samples on the first two principal components (PC) that explain most of the variability in the data, a grouping into the three molecular subtypes can be found (Figure 10A). In addition, t-distributed stochastic neighbour embedding (t-SNE) has been applied, as an example for a non-linear dimension reduction method, to the methylation data and the resulting projection also shows a clustering of the three ATRT subtypes (Figure 10B). Other linear and non-linear dimension reduction methods that can be applied to achieve similar results are for example multidimensional scaling (MDS), factor analysis (FA), non-negative matrix factorization (NMF), truncated singular value decomposition (SVD), stochastic neighbour embedding (SNE), uniform manifold approximation and projection for dimension reduction (UMAP) and linear discriminant analysis (LDA).

To show how supervised statistical methods can be applied to fit a model that predicts ATRT subtypes, a classification and regression tree (CART) has been applied to methylation data (Figure 10C). At each node, the CART algorithm automatically tries out all available 1022 CpGs probes and possible cut-offs and selects the CpG probe and corresponding cut-off that leads to the purest split into the ATRT subtypes. The algorithm stops, as soon as the class purity measured by the Gini coefficient cannot be further improved. Here the CART algorithm found two sequential splitting rules (Figure 10D) that involve only two CpG probes that result in an almost perfect separation of the ATRT subclasses. Random Forests usually combine hundreds or thousands of CART trees by bootstrap aggregation (bagging) to achieve an improved prediction accuracy. Other supervised methods that can be applied to fit models with comparable prediction performance, are for example gradient boosting machines (GBM), support vector machines (SVM), multinomial logistic regression models and (deep) neural nets.

### Example 5: Gene expression data used for the classification of tumour entities originally identified in DNA-methylation data

By analysing DNA-methylation data and training machine learning models for the classification of brain tumours, 688 genes have been identified that include CpG sites that can be considered most important for the classification of molecular brain tumour types. To show that these brain tumour entities can also be recognized in gene expression data and that this data can be used to train similarly performing machine learning models, 1167 brain tumour samples were analysed for which both DNA-methylation as well as gene expression data is available. This paired gene expression and methylation data set includes samples from 79 of the in total 184 classes that were defined on the DNA-methylation level.

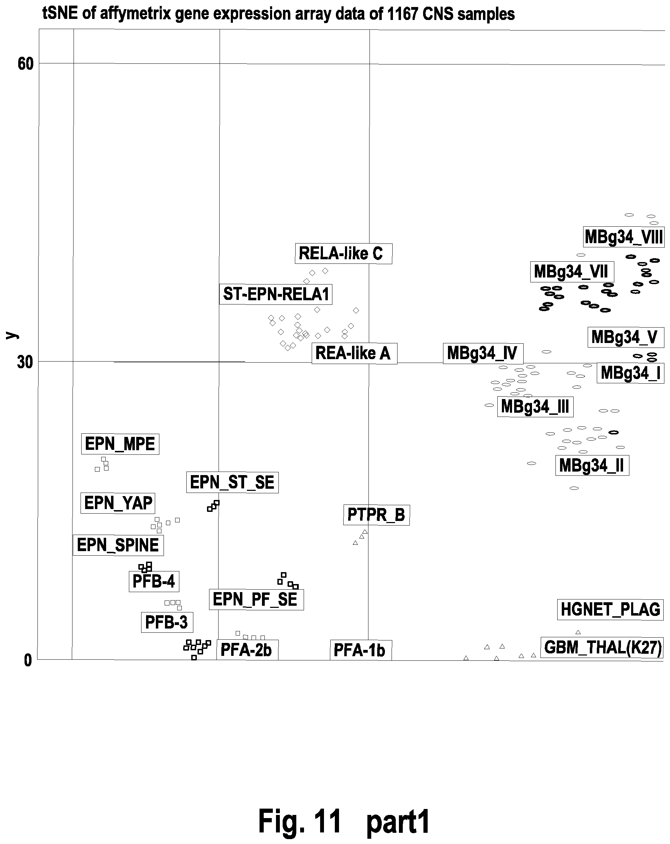

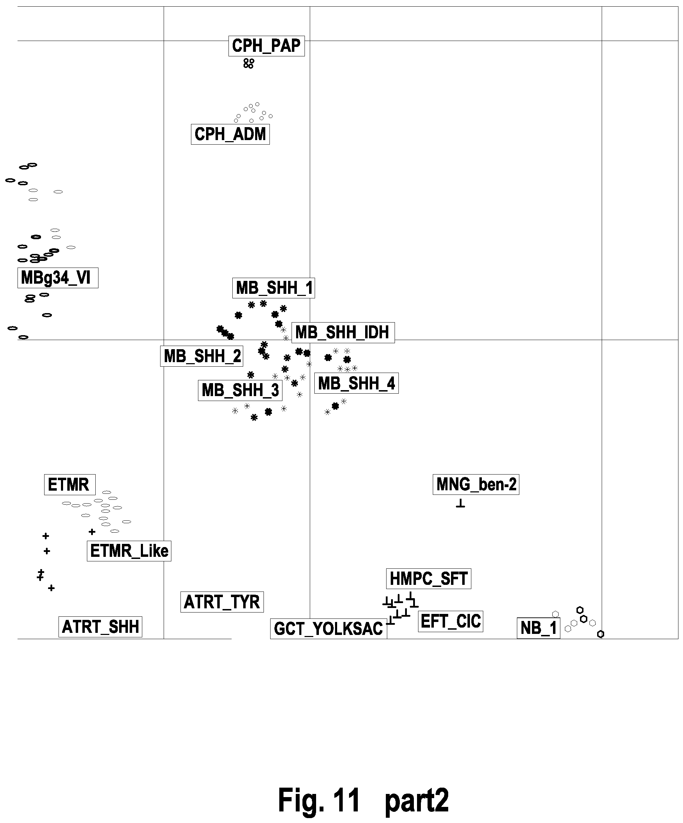

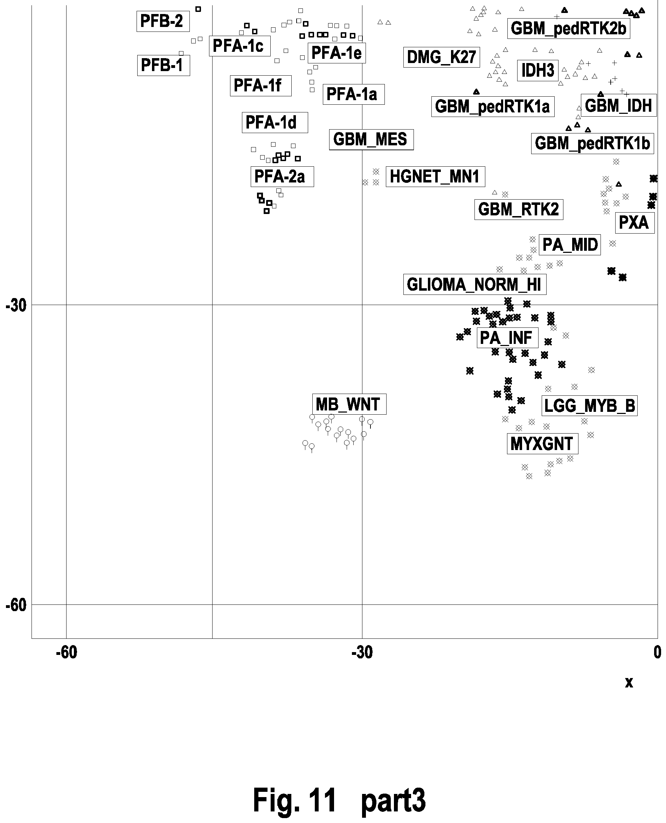

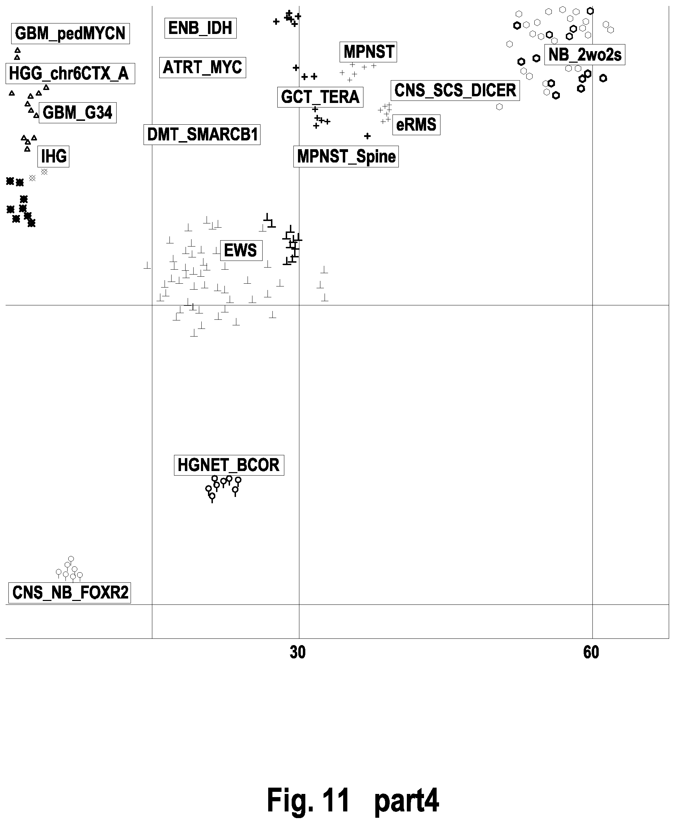

Figure 11 shows the 1167 samples projected onto a t-distributed stochastic neighbour embedding (tSNE) that was applied to the gene expression data of the 688 most important genes identified in the methylation data. The colouring and the labelling of the groups are according to the class, and the samples are classified by the DNA-methylation classifier. The general clustering of the classes is very similar to a tSNE performed on DNA-methylation and even new sub-entities such as the medulloblastoma (MB) group 3 and 4 subtypes I-VIII can be identified. This proves that the gene expression data of the 688 identified genes is highly predictive for the 184 classes.

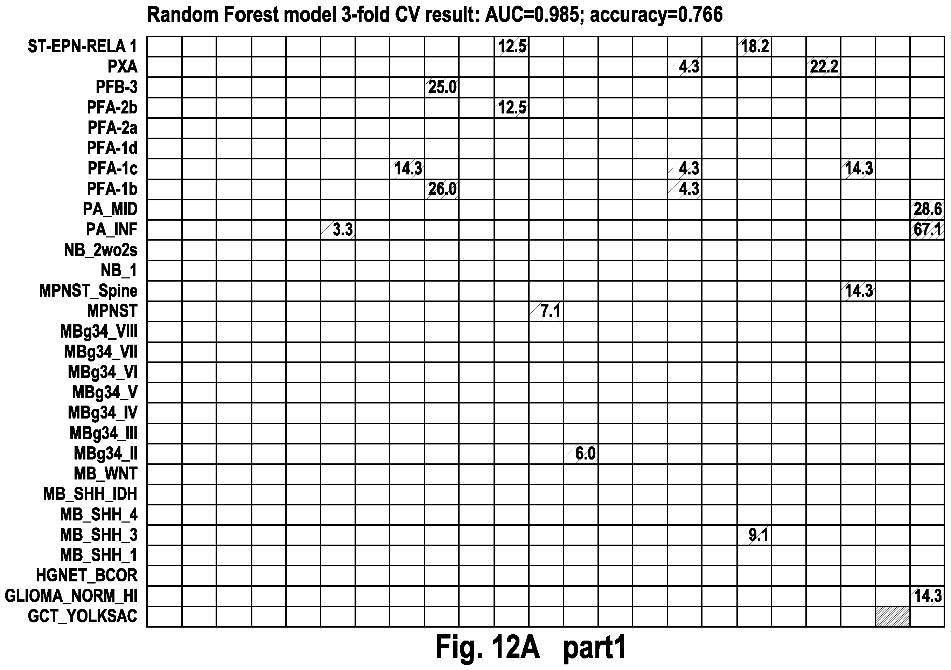

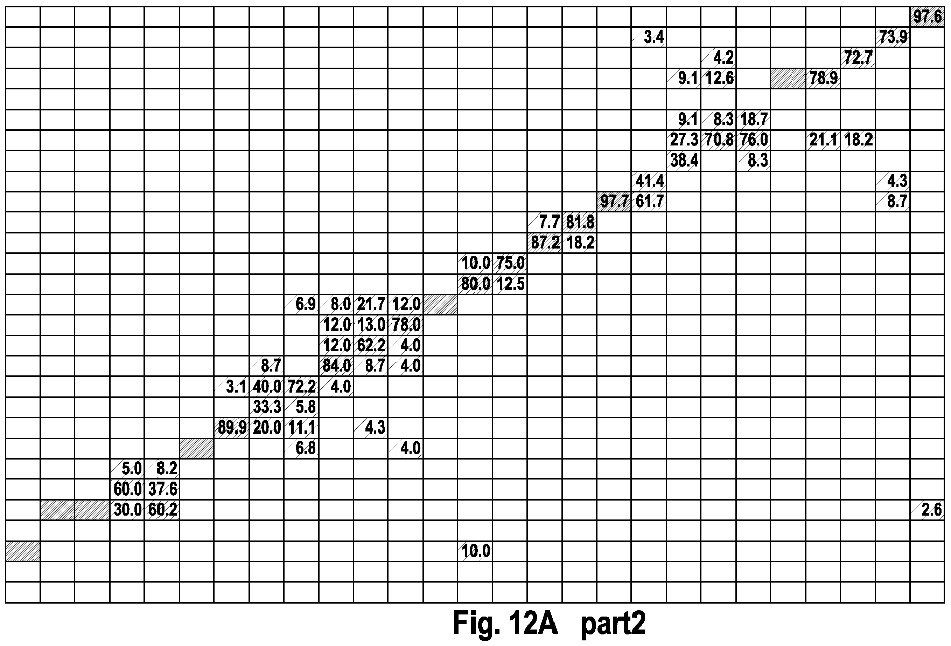

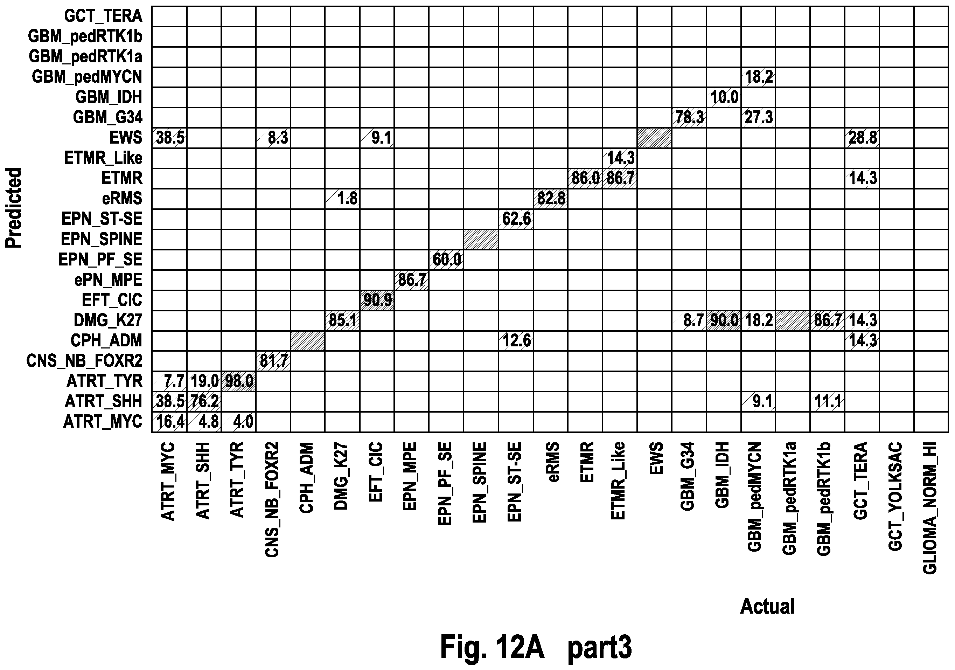

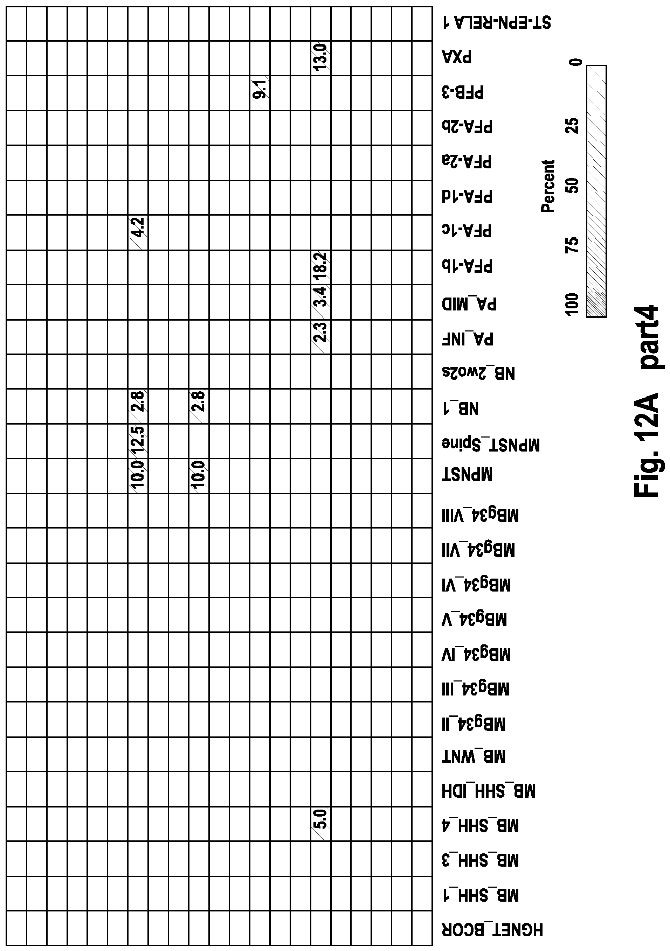

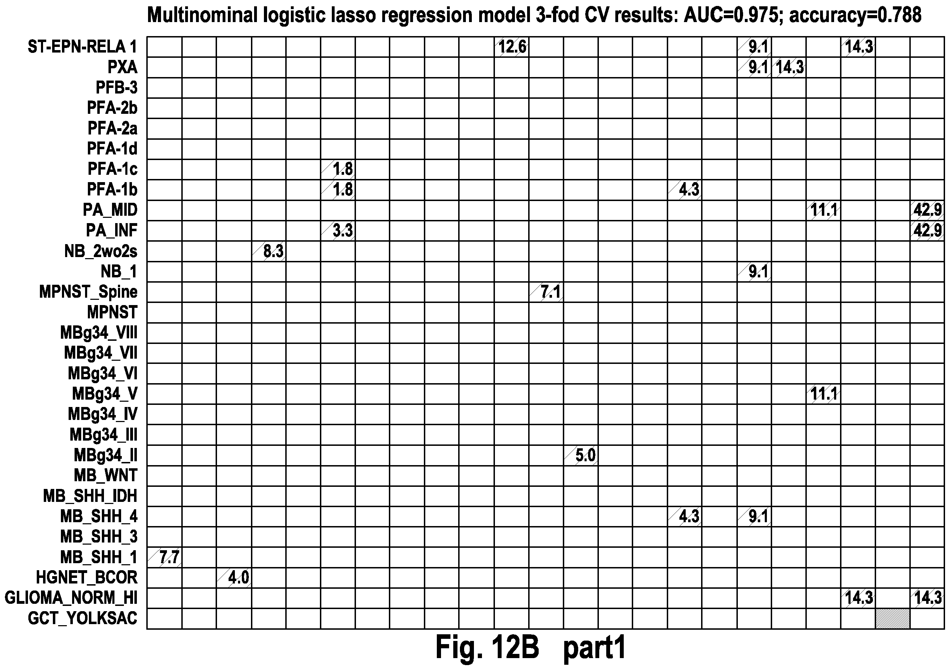

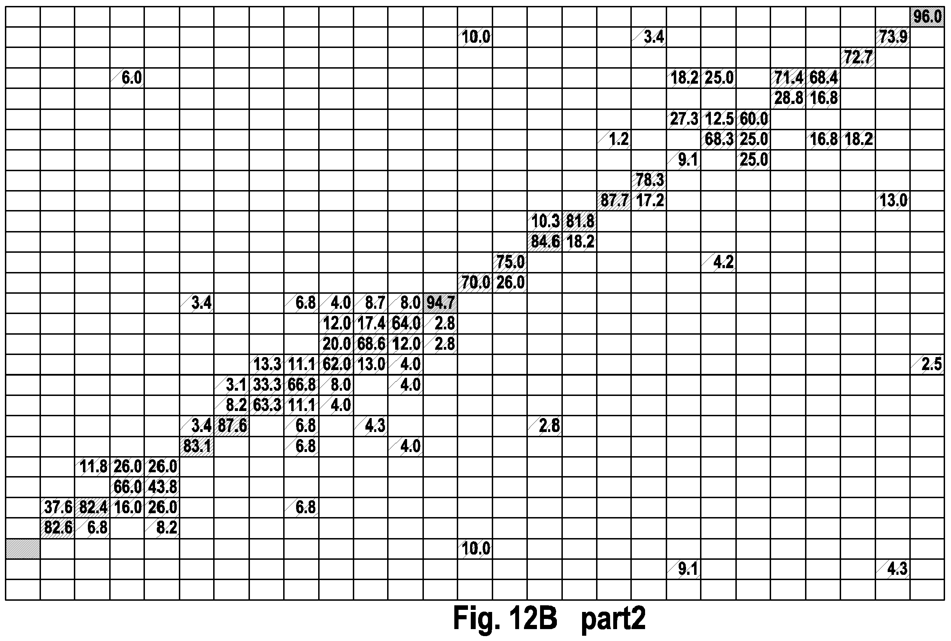

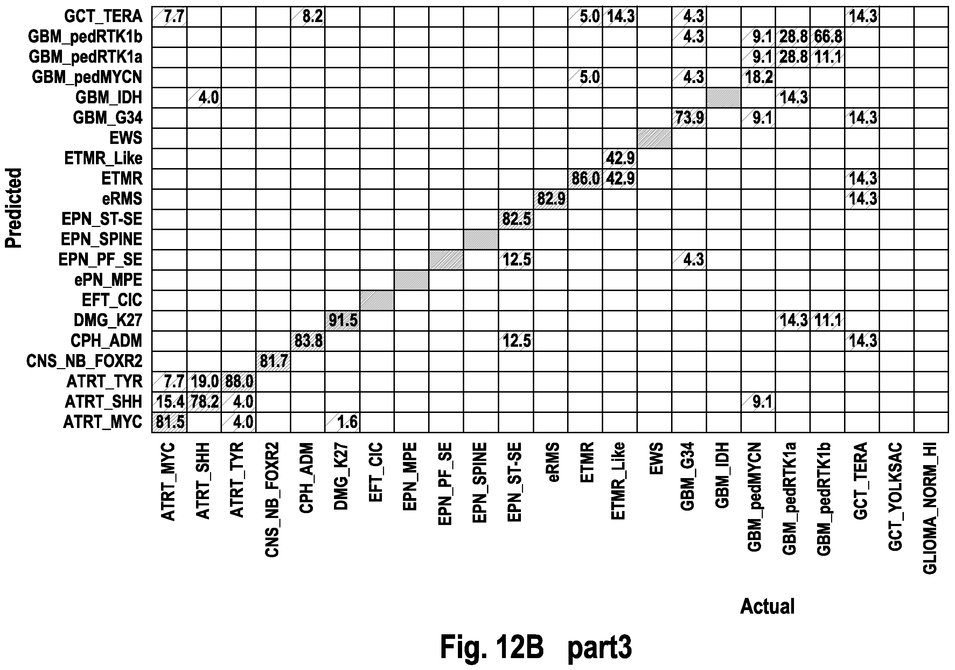

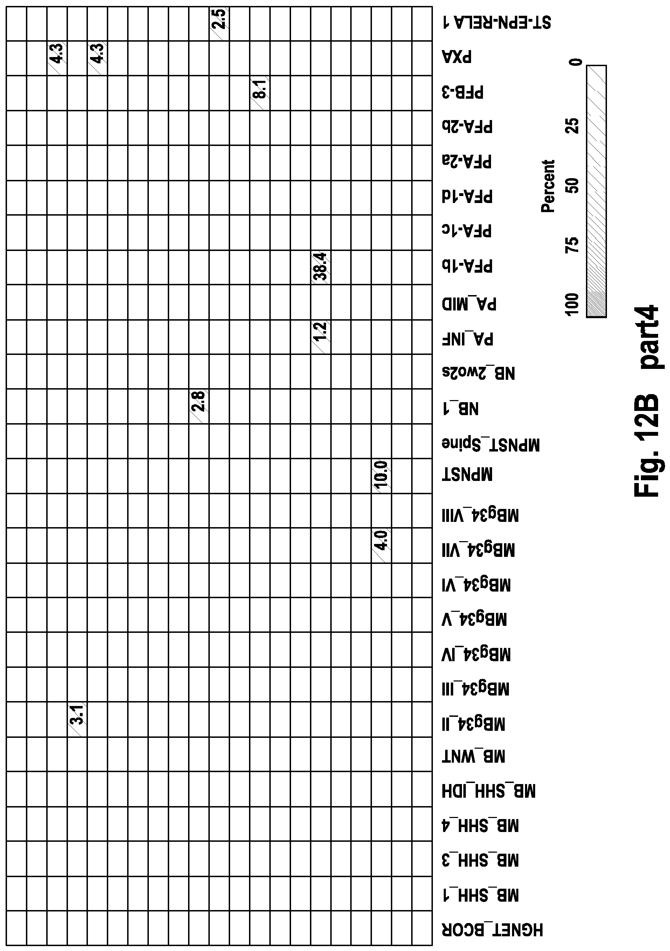

To show that the gene expression data can also be used to train supervised machine learning models, the gene expression data set was reduced to 1057 samples belonging to 50 classes with a minimal class sample size of 7 samples. The inventors then trained a basic random forest (RF) model and a lasso-penalized multinomial logistic regression model to this data set and validated the performance of both models by 3-fold cross-validation (CV). The CV estimated an accuracy of 0.788 for the RF (Figure 12B) and an accuracy 0.766 for the logistic regression model (Figure 12A), what proves that gene expression can be used to train similar classification models.

Accordingly, it has been shown by the inventors that the biological state used to train the classification algorithm is not limited to methylation, but can also be another biological state such as gene expression.

### References:

R Core Team (2014). R: A language and environment for statistical computing. R Foundation for Statistical Computing, Vienna, Austria. URL http://www.R-project.org/.
MJ Aryee, AE Jaffe, H Corrada-Bravo, C Ladd-Acosta, AP Feinberg, KD Hansen, RA Irizarry. Minfi: A flexible and comprehensive Bioconductor package for the analysis of Infinium DNA Methylation microarrays. Bioinformatics 2014, In press. doi: 10.1093/bioinformatics/btu049.
A. Liaw and M. Wiener (2002). Classification and Regression by randomForest. R News 2(3), 18--22.
Bioconductor: Open software development for computational biology and bioinformatics R. Gentleman, V. J. Carey, D. M. Bates, B. Bolstad, M. Dettling, S. Dudoit, B. Ellis, L. Gautier, Y. Ge, and others 2004, Genome Biology, Vol. 5, R80.

### Tables:

**Table 1: List of gene sites according to the disclosure including their genetic locus and Sequence ID in the sequence listing. The sequence listing associated with this application is filed in electronic format and hereby incorporated by reference into the specification in its entirety.**

| **Seq. ID No.** | **Gene site** | **Chromosome** | **Sequence start** | **Sequence end** |
|---|---|---|---|---|
| **1** | ABAT | chr16 | 8766944 | 8879932 |
| **2** | ABLIM2 | chr4 | 7965537 | 8162059 |
| **3** | ABR | chr17 | 905258 | 1092116 |
| **4** | ACAD10 | chr12 | 112122357 | 112196411 |
| **5** | ACMSD | chr2 | 135594686 | 135661102 |
| **6** | ACOT7 | chr1 | 6322832 | 6455326 |
| **7** | ACOX3 | chr4 | 8366509 | 8443952 |
| **8** | ACSL1 | chr4 | 185675249 | 185748715 |
| **9** | ACTR3C | chr7 | 149942801 | 150022258 |
| **10** | ADAMTS17 | chr15 | 100510143 | 100883683 |
| **11** | ADAMTS2 | chr5 | 178536352 | 178773931 |
| **12** | ADARB2 | chr10 | 1221753 | 1781170 |
| **13** | ADGRA1 | chr10 | 134882933 | 134946679 |
| **14** | ADGRB1 | chr8 | 143543877 | 143627868 |
| **15** | ADGRD1 | chr12 | 131436952 | 131627508 |
| **16** | AFF3 | chr2 | 100162216 | 100760537 |
| **17** | AGAP1 | chr2 | 236401233 | 237041944 |
| **18** | AGAP2 | chr12 | 58116576 | 58137444 |
| **19** | AGO2 | chr8 | 141539764 | 141647146 |
| **20** | AIRE | chr21 | 45704221 | 45719602 |
| **21** | AK1 | chr9 | 130627259 | 130641522 |
| **22** | AKAP13 | chr15 | 85922347 | 86294089 |
| **23** | ANAPC16 | chr10 | 73619647 | 73997118 |
| **24** | ANK1 | chr8 | 41509244 | 41755780 |
| **25** | ANK2 | chr4 | 113737739 | 114306396 |
| **26** | ANKLE2 | chr12 | 133300754 | 133339951 |
| **27** | ANKRD 11 | chr16 | 89332529 | 89558469 |
| **28** | ANKRD33B | chr5 | 10562935 | 10659428 |
| **29** | ANKS1A | chr6 | 34855538 | 35086820 |
| **30** | ANKS1B | chr12 | 99127069 | 100379932 |
| **31** | AP2A2 | chr11 | 924309 | 1013745 |
| **32** | APBA2 | chr15 | 29129668 | 29412016 |
| **33** | ARHGAP18 | chr6 | 129896740 | 130184192 |
| **34** | ARHGAP22 | chr10 | 49652568 | 49865810 |
| **35** | ARHGAP23 | chr17 | 36583220 | 36670128 |
| **36** | ARHGAP25 | chr2 | 68905246 | 69055457 |
| **37** | ARHGAP26 | chr5 | 142148792 | 142610072 |
| **38** | ARHGAP27P1 | chr17 | 62744280 | 62779617 |
| **39** | ARHGAP45 | chr19 | 1064422 | 1088127 |
| **40** | ARHGEF10 | chr8 | 1770649 | 1908307 |
| **41** | ARHGEF7 | chr13 | 111766124 | 111959581 |
| **42** | ARL6IP6 | chr2 | 153572907 | 153619267 |
| **43** | ARMC2 | chr6 | 109168119 | 109296852 |
| **44** | ASAP1 | chr8 | 131062851 | 131457406 |
| **45** | ASAP2 | chr2 | 9345394 | 9547312 |
| **46** | ASIC2 | chr17 | 31338606 | 32485325 |
| **47** | ASPSCR1 | chr17 | 79933926 | 79976782 |
| **48** | ATG4B | chr2 | 242575527 | 242614771 |
| **49** | ATP11A | chr13 | 113343143 | 113542982 |
| **50** | ATP2B4 | chr1 | 203594415 | 203714709 |
| **51** | ATXN7L1 | chr7 | 105243721 | 105518531 |
| **52** | AUTS2 | chr7 | 69062405 | 70259385 |
| **53** | AXIN2 | chr17 | 63523183 | 63638683 |
| **54** | BACH2 | chr6 | 90634747 | 91008127 |
| **55** | BAHCC1 | chr17 | 79372040 | 79434858 |
| **56** | BAIAP2 | chr17 | 79007447 | 79092732 |
| **57** | BCAR1 | chr16 | 75261428 | 75303451 |
| **58** | BCAT1 | chr12 | 24961458 | 25103893 |
| **59** | BCL11B | chr14 | 99634125 | 99739322 |
| **60** | BFSP2 | chr3 | 133117290 | 133195556 |
| **61** | BOC | chr3 | 112928912 | 113007805 |
| **62** | BOLA2 | chr16 | 29452726 | 30207127 |
| **63** | BTBD11 | chr12 | 107710697 | 108054919 |
| **64** | BTBD9 | chr6 | 38134727 | 38609424 |
| **65** | C10orf105 | chr10 | 73469958 | 73499081 |
| **66** | C10orf90 | chr10 | 128112074 | 128360579 |
| **67** | C19orf25 | chr19 | 1460262 | 1480728 |
| **68** | C1orf94 | chr1 | 34631124 | 34686231 |
| **69** | C6orf223 | chr6 | 43966837 | 43975194 |
| **70** | C7orf50 | chr7 | 1035123 | 1179393 |
| **71** | CABLES1 | chr18 | 20713028 | 20841934 |
| **72** | CACHD1 | chr1 | 64934976 | 65160241 |
| **73** | CACNA1C | chr12 | 2160916 | 2808615 |
| **74** | CACNA1D | chr3 | 53527576 | 53847992 |
| **75** | CACNA1H | chr16 | 1201741 | 1273272 |
| **76** | CACNA1I | chr22 | 39965258 | 40087240 |
| **77** | CACNA2D2 | chr3 | 50398731 | 50542392 |
| **78** | CACNA2D3 | chr3 | 54155193 | 55110084 |
| **79** | CACNA2D4 | chr12 | 1899623 | 2029370 |
| **80** | CACNB2 | chr10 | 18428106 | 18832188 |
| **81** | CADM1 | chr11 | 115038451 | 115376741 |
| **82** | CALD1 | chr7 | 134462664 | 134656980 |
| **83** | CAMK4 | chr5 | 110558447 | 110822248 |
| **84** | CAMTA1 | chr1 | 6843884 | 7831266 |
| **85** | CAPG | chr2 | 85620371 | 85642697 |
| **86** | CASC15 | chr6 | 21663503 | 22216234 |
| **87** | CASP8 | chr2 | 202096666 | 202153934 |
| **88** | CASZ1 | chr1 | 10695166 | 10858233 |
| **89** | CBFA2T3 | chr16 | 88939763 | 89045004 |
| **90** | CCDC140 | chr2 | 223161366 | 223171436 |
| **91** | CCDC167 | chr6 | 37449197 | 37469200 |
| **92** | CCDC177 | chr14 | 70035031 | 70043100 |
| **93** | CCDC85C | chr14 | 99976103 | 100072227 |
| **94** | CCDC88C | chr14 | 91736167 | 91885688 |
| **95** | CCND2 | chr12 | 4381402 | 4416022 |
| **96** | CCR6 | chr6 | 167411316 | 167554129 |
| **97** | CDC42BPB | chr14 | 103397216 | 103525242 |
| **98** | CDH4 | chr20 | 59825982 | 60517173 |
| **99** | CDK6 | chr7 | 92232735 | 92467441 |
| **100** | CDYL | chr6 | 4704893 | 4957278 |
| **101** | CELF4 | chr18 | 34821508 | 35147500 |
| **102** | CELSR1 | chr22 | 46755231 | 46934567 |
| **103** | CFAP46 | chr10 | 134620396 | 134757589 |
| **104** | CFLAR | chr2 | 201979377 | 202038911 |
| **105** | CHID1 | chr11 | 865857 | 916558 |
| **106** | CHN2 | chr7 | 29184700 | 29555444 |
| **107** | CHST11 | chr12 | 104849192 | 105157292 |
| **108** | CHTF18 | chr16 | 837122 | 849574 |
| **109** | CLDN10 | chr13 | 96084353 | 96233510 |
| **110** | CLYBL | chr13 | 100257419 | 100550888 |
| **111** | CMIP | chr16 | 81477275 | 81746867 |
| **112** | CNMD | chr13 | 53275900 | 53315447 |
| **113** | CNP | chr17 | 40117259 | 40131254 |
| **114** | CNPY1 | chr7 | 155292453 | 155328039 |
| **115** | COL23A1 | chr5 | 177663117 | 178019056 |
| **116** | COL26A1 | chr7 | 101004622 | 101203804 |
| **117** | COL4A1 | chr13 | 110799810 | 110960996 |
| **118** | COLEC11 | chr2 | 3640922 | 3693734 |
| **119** | COQ8A | chr | 227083089 | 227176746 |
| **120** | COROIC | chr12 | 109037385 | 109126826 |
| **121** | CORO2B | chr15 | 68869808 | 69021644 |
| **122** | CPE | chr4 | 166298597 | 166420982 |
| **123** | CPEB1-AS1 | chr15 | 83315021 | 83363072 |
| **124** | CPEB4 | chr5 | 173313831 | 173388813 |
| **125** | CPNE4 | chr3 | 131252077 | 132005754 |
| **126** | CPQ | chr8 | 97655999 | 98157222 |
| **127** | CPZ | chr4 | 8580717 | 8622988 |
| **128** | CRACR2A | chr12 | 3714818 | 3863866 |
| **129** | CRADD | chr12 | 94069651 | 94290116 |
| **130** | CRB2 | chr9 | 126116948 | 126142532 |
| **131** | CRISPLD2 | chr16 | 84852087 | 84944616 |
| **132** | CSMD1 | chr8 | 2791375 | 4853828 |
| **133** | CSRNP1 | chr3 | 39181842 | 39197553 |
| **134** | CTBP2 | chr10 | 126674918 | 126851124 |
| **135** | CTNNA2 | chr2 | 79410857 | 80877488 |
| **136** | CUEDC1 | chr17 | 55937104 | 56034184 |
| **137** | CUX1 | chr7 | 101457684 | 101928750 |
| **138** | CXXC5 | chr5 | 139026801 | 139064180 |
| **139** | CYBA | chr16 | 88708197 | 88718992 |
| **140** | CYREN | chr7 | 134775617 | 134857078 |
| **141** | CYTH1 | chr17 | 76668630 | 76779876 |
| **142** | DAGLB | chr7 | 6447247 | 6525349 |
| **143** | DDA1 | chr19 | 17418837 | 17435606 |
| **144** | DDT | chr22 | 24312054 | 24323519 |
| **145** | DDX31 | chr9 | 135468176 | 135547288 |
| **146** | DENND11 | chr7 | 141355028 | 141403453 |
| **147** | DENND2B | chr11 | 8713399 | 8933998 |
| **148** | DENND3 | chr8 | 142137220 | 142207406 |
| **149** | DERL3 | chr22 | 24175190 | 24182815 |
| **150** | DGKD | chr2 | 234261653 | 234382243 |
| **151** | DGKG | chr3 | 185863490 | 186081523 |
| **152** | DICER1 | chr14 | 95551065 | 95625847 |
| **153** | DIP2C | chr10 | 318630 | 737108 |
| **154** | DISCI | chr1 | 231761061 | 232178519 |
| **155** | DLEU1 | chr13 | 50654914 | 51104170 |
| **156** | DLG4 | chr17 | 7091710 | 7124869 |
| **157** | DLL1 | chr6 | 170589794 | 170601197 |
| **158** | DLX5 | chr7 | 96648202 | 96655643 |
| **159** | DLX6-AS1 | chr7 | 96596327 | 96644877 |
| **160** | DMRTA2 | chr1 | 50881723 | 50890619 |
| **161** | DNAAF5 | chr7 | 764838 | 827616 |
| **162** | DNAJB6 | chr7 | 157128210 | 157211633 |
| **163** | DNAJC17 | chr15 | 41058567 | 41101176 |
| **164** | DNAJC27 | chr2 | 25165005 | 25196463 |
| **165** | DNM3 | chr1 | 171809118 | 172389067 |
| **166** | DNMT3A | chr2 | 25454330 | 25566959 |
| **167** | DONSON | chr21 | 34946283 | 35286203 |
| **168** | DPP6 | chr7 | 153582919 | 154587495 |
| **169** | DPY19L1P1 | chr7 | 32619053 | 32760280 |
| **170** | DSE | chr6 | 116573836 | 116760942 |
| **171** | DTNA | chr18 | 32071754 | 32473308 |
| **172** | DUSP5 | chr10 | 112256125 | 112272802 |
| **173** | DUSP6 | chr12 | 89740337 | 89747796 |
| **174** | DUSP7 | chr3 | 52081437 | 52091961 |
| **175** | EBF1 | chr5 | 158121423 | 158528288 |
| **176** | EBF2 | chr8 | 25697746 | 25904140 |
| **177** | EBF3 | chr10 | 131631996 | 131763591 |
| **178** | EDNRB | chr13 | 78468116 | 78551164 |
| **179** | EGFR | chr7 | 55085225 | 55276531 |
| **180** | EML1 | chr14 | 100202569 | 100409895 |
| **181** | EMX2OS | chr10 | 119242304 | 119306079 |
| **182** | EOGT | chr3 | 69022868 | 69064274 |
| **183** | EPAS 1 | chr2 | 46523041 | 46615342 |
| **184** | EPHA10 | chr1 | 38178053 | 38232324 |
| **185** | EPHB1 | chr3 | 134512599 | 134980807 |
| **186** | ERI3 | chr1 | 44685242 | 44822439 |
| **187** | ESR1 | chr6 | 151976330 | 152425908 |
| **188** | ESRRG | chr1 | 216675088 | 217312597 |
| **189** | ETS1 | chr11 | 128327156 | 128458953 |
| **190** | EXPH5 | chr11 | 108374658 | 108465874 |
| **191** | EXT1 | chr8 | 118810102 | 119125558 |
| **192** | EXT2 | chr11 | 44095576 | 44268480 |
| **193** | F11R | chr1 | 160963501 | 161010274 |
| **194** | FAM181A | chr14 | 94383740 | 94397454 |
| **195** | FAM53B | chr10 | 126304149 | 126480407 |
| **196** | FAM83E | chr19 | 49102357 | 49118194 |
| **197** | FBRSL1 | chr12 | 133065657 | 133163273 |
| **198** | FBXL17 | chr5 | 107193234 | 107719299 |
| **199** | FBXL18 | chr7 | 5513928 | 5554899 |
| **200** | FEZ1 | chr11 | 125314141 | 125367706 |
| **201** | FGFR2 | chr10 | 123236344 | 123359472 |
| **202** | FHIT | chr3 | 59733536 | 61238633 |
| **203** | FLJ12825 | chr12 | 54450538 | 54517518 |
| **204** | FMN1 | chr15 | 33056245 | 33488434 |
| **205** | FMNL2 | chr2 | 153190251 | 153507848 |
| **206** | FOXK1 | chr7 | 4681888 | 4812574 |
| **207** | FOXO1 | chr13 | 41046631 | 41242234 |
| **208** | FOXP1 | chr3 | 71002365 | 71634640 |
| **209** | FOXP4 | chr6 | 41512664 | 41571622 |
| **210** | FRMD4A | chr10 | 13684206 | 14505643 |
| **211** | FRMPD2 | chr10 | 49363101 | 49484441 |
| **212** | FYN | chr6 | 111980035 | 112196155 |
| **213** | GABRB3 | chr15 | 26787194 | 27186186 |
| **214** | GAK | chr4 | 841565 | 927674 |
| **215** | GALK2 | chr15 | 49446476 | 49623502 |
| **216** | GALNT2 | chr1 | 230192036 | 230419375 |
| **217** | GALNT9 | chr12 | 132679417 | 132907405 |
| **218** | GAREM2 | chr2 | 26394460 | 26414032 |
| **219** | GAS7 | chr17 | 9812426 | 10103368 |
| **220** | GATA4 | chr8 | 11532968 | 11619009 |
| **221** | GATA6 | chr18 | 19747904 | 19783991 |
| **222** | GCK | chr7 | 44182370 | 44230522 |
| **223** | GCSAML | chr1 | 247668894 | 247741348 |
| **224** | GDF6 | chr8 | 97153058 | 97174520 |
| **225** | GDNF | chr5 | 37811279 | 37841282 |
| **226** | GLI2 | chr2 | 121491699 | 121751729 |
| **227** | GLI3 | chr7 | 41999048 | 42278118 |
| **228** | GLT8D2 | chr12 | 104381265 | 104459461 |
| **229** | GLUD1P2 | chr10 | 46763168 | 48959583 |
| **230** | GNA12 | chr7 | 2766241 | 2885459 |
| **231** | GNAO1 | chr16 | 56223751 | 56392856 |
| **232** | GNAS | chr20 | 57413295 | 57487750 |
| **233** | GNB5 | chr15 | 52411623 | 52485065 |
| **234** | GNG7 | chr19 | 2509718 | 2704246 |
| **235** | GPC6 | chr13 | 93877578 | 95061773 |
| **236** | GPR39 | chr2 | 133172647 | 133405669 |
| **237** | GRHL2 | chr8 | 102503168 | 102683452 |
| **238** | GRID1 | chr10 | 87357812 | 88127750 |
| **239** | GRIK2 | chr6 | 101845169 | 102519458 |
| **240** | GRIN 1 | chr9 | 140032109 | 140064714 |
| **241** | GRIN2B | chr12 | 13712910 | 14134522 |
| **242** | GRIP1 | chr12 | 66739711 | 67199394 |
| **243** | GRK5 | chr10 | 120965697 | 121216631 |
| **244** | GRTP1 | chr13 | 113977005 | 114019963 |
| **245** | GSE1 | chr16 | 85643529 | 85711312 |
| **246** | GSG1 | chr12 | 13234971 | 13258130 |
| **247** | GTF2E2 | chr8 | 30434531 | 30517238 |
| **248** | HBG2 | chr11 | 5268002 | 5668511 |
| **249** | HDAC4 | chr2 | 239968364 | 240324846 |
| **250** | HDAC7 | chr12 | 48175007 | 48215263 |
| **251** | HHEX | chr10 | 94448181 | 94456908 |
| **252** | HIVEP3 | chr1 | 41970536 | 42503096 |
| **253** | HK1 | chr10 | 71028256 | 71163137 |
| **254** | HLX | chr1 | 221051243 | 221059900 |
| **255** | HMGA2 | chr12 | 66215937 | 66361571 |
| **256** | HMGCR | chr5 | 74630654 | 74659426 |
| **257** | HNF1B | chr17 | 36044934 | 36106596 |
| **258** | HOTAIR | chr12 | 54354592 | 54370240 |
| **259** | HOTTIP | chr7 | 27238540 | 27247630 |
| **260** | HOXA-AS3 | chr7 | 27178483 | 27197047 |
| **261** | HOXA10-HOXA9 | chr7 | 27200557 | 27221380 |
| **262** | HOXA3 | chr7 | 27144309 | 27168139 |
| **263** | HOXB-AS1 | chr17 | 46620213 | 46630103 |
| **264** | HOXB-AS3 | chr17 | 46666323 | 46685274 |
| **265** | HOXB3 | chr17 | 46624732 | 46669131 |
| **266** | HOXB6 | chr17 | 46671599 | 46683854 |
| **267** | HOXC4 | chr12 | 54409142 | 54451314 |
| **268** | HOXD3 | chr2 | 177027305 | 177039326 |
| **269** | HOXD4 | chr2 | 177014613 | 177019449 |
| **270** | ICAM5 | chr19 | 10399155 | 10408954 |
| **271** | IDI2 | chr10 | 1063347 | 1073299 |
| **272** | IFFO1 | chr12 | 6646039 | 6666749 |
| **273** | IFT80 | chr3 | 159943741 | 160169126 |
| **274** | IGDCC4 | chr15 | 65672325 | 65716910 |
| **275** | IGF1R | chr15 | 99191261 | 99509259 |
| **276** | IGF2BP1 | chr17 | 47073274 | 47135007 |
| **277** | IGF2BP3 | chr7 | 23348328 | 23511495 |
| **278** | IGFBPL1 | chr9 | 38405025 | 38425944 |
| **279** | IGSF21 | chr1 | 18432740 | 18706477 |
| **280** | IL17D | chr13 | 21275982 | 21298737 |
| **281** | INPP5A | chr10 | 134349853 | 134598484 |
| **282** | IQCE | chr7 | 2597132 | 2655868 |
| **283** | IQSEC1 | chr3 | 12937042 | 13116117 |
| **284** | IRF6 | chr1 | 209957468 | 209981020 |
| **285** | ISLR2 | chr15 | 74420215 | 74430643 |
| **286** | ITGA5 | chr12 | 54787545 | 54814550 |
| **287** | ITPK1 | chr14 | 93401759 | 93583763 |
| **288** | ITPKB | chr1 | 226817891 | 226928376 |
| **289** | JAKMIP1 | chr4 | 6026426 | 6203818 |
| **290** | JPH3 | chr16 | 87633941 | 87733261 |
| **291** | JUP | chr17 | 39678369 | 39944464 |
| **292** | KAZN | chr1 | 14923713 | 15446044 |
| **293** | KCNAB2 | chr1 | 6050858 | 6162753 |
| **294** | KCNB1 | chr20 | 47987005 | 48101990 |
| **295** | KCNH2 | chr7 | 150640544 | 150676902 |
| **296** | KCNIP1 | chr5 | 169779381 | 170165136 |
| **297** | KCNIP4 | chr4 | 20728739 | 21951874 |
| **298** | KCNMA1 | chr10 | 78627859 | 79399077 |
| **299** | KCNQ1 | chr11 | 2464721 | 2871840 |
| **300** | KCNV2 | chr9 | 2716026 | 2731537 |
| **301** | KDM4B | chr19 | 4967624 | 5155108 |
| **302** | KIAA1522 | chr1 | 33206012 | 33242071 |
| **303** | KIF21B | chr1 | 200937014 | 200994328 |
| **304** | KIF26A | chr14 | 104603560 | 104648735 |
| **305** | KIF26B | chr1 | 245316787 | 245867928 |
| **306** | KIFC3 | chr16 | 57790629 | 57898233 |
| **307** | KIRREL3 | chr11 | 126291896 | 126874855 |
| **308** | KLHL25 | chr15 | 86301059 | 86339689 |
| **309** | KLHL26 | chr19 | 18746338 | 18782802 |
| **310** | KLHL29 | chr2 | 23606798 | 23932983 |
| **311** | KNDC1 | chr10 | 134972471 | 135041416 |
| **312** | LAIR1 | chr19 | 54863735 | 54883665 |
| **313** | LBX1-AS1 | chr10 | 102987851 | 103000116 |
| **314** | LDLRAD4 | chr18 | 13217229 | 13654253 |
| **315** | LHPP | chr10 | 126148841 | 126304210 |
| **316** | LHX2 | chr9 | 126772389 | 126796942 |
| **317** | LHX4 | chr1 | 180197933 | 180245688 |
| **318** | LHX5 | chr12 | 113899194 | 113911377 |
| **319** | LHX9 | chr1 | 197880135 | 197903215 |
| **320** | LIMCH1 | chr4 | 41361304 | 41703561 |
| **321** | LIN28A | chr1 | 26735769 | 26757719 |
| **322** | LINC00311 | chr16 | 85315064 | 85323185 |
| **323** | LINC00461 | chr5 | 87835097 | 87982120 |
| **324** | LINC00856 | chr10 | 80006882 | 80312612 |
| **325** | LINC01140 | chr1 | 87457190 | 87636386 |
| **326** | LINC01551 | chr14 | 29240410 | 29265500 |
| **327** | LINC01749 | chr20 | 61639235 | 61717923 |
| **328** | LIPE-AS 1 | chr19 | 42899800 | 43158007 |
| **329** | LMF1 | chr16 | 902135 | 1032818 |
| **330** | LMX1B | chr9 | 129375222 | 129464811 |
| **331** | LOC100130872 | chr4 | 1188071 | 1204250 |
| **332** | LOC100132215 | chr2 | 63269600 | 63277156 |
| **333** | LOC145845 | chr15 | 37155144 | 37180234 |
| **334** | LOC339874 | chr3 | 131042436 | 131101819 |
| **335** | LOC606724 | chr16 | 29459166 | 30202075 |
| **336** | LOC613038 | chr16 | 29474789 | 30219748 |
| **337** | LOXL3 | chr2 | 74758446 | 74782562 |
| **338** | LPCAT1 | chr5 | 1460042 | 1525576 |
| **339** | LPIN1 | chr2 | 11816205 | 11969033 |
| **340** | LPP | chr3 | 187870163 | 188609960 |
| **341** | LRBA | chr4 | 151184311 | 151938149 |
| **342** | LRMDA | chr10 | 77541019 | 78318626 |
| **343** | LRP2 | chr2 | 169982119 | 170220622 |
| **344** | LRRC61 | chr7 | 150005130 | 150036745 |
| **345** | LRRFIP1 | chr2 | 238534724 | 238691790 |
| **346** | LTF | chr3 | 46475996 | 46528224 |
| **347** | LYPD1 | chr2 | 133400837 | 133430570 |
| **348** | MACROD1 | chr11 | 63764530 | 63935085 |
| **349** | MAD1L1 | chr7 | 1853928 | 2274083 |
| **350** | MAML2 | chr11 | 95709940 | 96077844 |
| **351** | MAML3 | chr4 | 140636046 | 141076733 |
| **352** | MAP2K3 | chr17 | 21186468 | 21220051 |
| **353** | MAP3K3 | chr17 | 61698275 | 61775170 |
| **354** | MAPK8IP1 | chr11 | 45905547 | 45929516 |
| **355** | MAPK8IP3 | chr16 | 1754721 | 1821818 |
| **356** | MBP | chr18 | 74689289 | 74846274 |
| **357** | MCC | chr5 | 112356296 | 112826027 |
| **358** | MCF2L | chr13 | 113621314 | 113755553 |
| **359** | MCIDAS | chr5 | 54513925 | 54524643 |
| **360** | MCPH1 | chr8 | 6262613 | 6502640 |
| **361** | MDM4 | chr1 | 204484007 | 204679161 |
| **362** | MECOM | chr3 | 168799787 | 169383063 |
| **363** | MEGF6 | chr1 | 3403006 | 3529559 |
| **364** | MEIS1 | chr2 | 66661032 | 66801391 |
| **365** | MEIS2 | chr15 | 37181722 | 37395000 |
| **366** | METAP1D | chr2 | 172863304 | 172947087 |
| **367** | MGMT | chr10 | 131263954 | 131567283 |
| **368** | MIR100HG | chr11 | 121958311 | 122239967 |
| **369** | MIR124-2HG | chr8 | 65284275 | 65297342 |
| **370** | MIR548F5 | chr13 | 36046426 | 36516882 |
| **371** | MIR548G | chr3 | 99271653 | 99718559 |
| **372** | MIR548H4 | chr15 | 69114803 | 69491362 |
| **373** | MIR9-3HG | chr15 | 89909830 | 89943218 |
| **374** | MIRLET7BHG | chr22 | 46448226 | 46511308 |
| **375** | MLC1 | chr22 | 50496320 | 50525858 |
| **376** | MLLT1 | chr19 | 6208892 | 6281459 |
| **377** | MNX1 | chr7 | 156785245 | 156804847 |
| **378** | MOB2 | chr11 | 1489185 | 1787001 |
| **379** | MPP7 | chr10 | 28338423 | 28593495 |
| **380** | MRC2 | chr17 | 60703262 | 60772462 |
| **381** | MSC-AS1 | chr8 | 72753858 | 72970047 |
| **382** | MSI2 | chr17 | 55331712 | 55758799 |
| **383** | MSRA | chr8 | 9910330 | 10287901 |
| **384** | MTHFR | chr1 | 11844287 | 11867660 |
| **385** | MYO16 | chr13 | 109247000 | 109861855 |
| **386** | MYT1 | chr20 | 62781644 | 62875106 |
| **387** | MYT1L | chr2 | 1791385 | 2336545 |
| **388** | NAV1 | chr1 | 201615950 | 201797602 |
| **389** | NAV2 | chr11 | 19370771 | 20144647 |
| **390** | NBEA | chr13 | 35514924 | 36248374 |
| **391** | NCOR2 | chr12 | 124807457 | 125053510 |
| **392** | NDRG4 | chr16 | 58496049 | 58549023 |
| **393** | NDST1 | chr5 | 149875840 | 149939273 |
| **394** | NDUFA13 | chr19 | 19625519 | 19647453 |
| **395** | NEAT1 | chr11 | 65188769 | 65213528 |
| **396** | NFATC1 | chr18 | 77154272 | 77290823 |
| **397** | NFIB | chr9 | 14080342 | 14400482 |
| **398** | NFIX | chr19 | 13105084 | 13211110 |
| **399** | NHSL1 | chr6 | 138741681 | 138895168 |
| **400** | NKD2 | chr5 | 1007577 | 1040427 |
| **401** | NOTCH1 | chr9 | 139387396 | 139441738 |
| **402** | NPHP4 | chr1 | 5921370 | 6054033 |
| **403** | NR1D1 | chr17 | 38247537 | 38258478 |
| **404** | NR2E1 | chr6 | 108485715 | 108511513 |
| **405** | NR2F1-AS1 | chr5 | 92743565 | 92918503 |
| **406** | NR5A2 | chr1 | 199995230 | 200148050 |
| **407** | NRCAM | chr7 | 107786571 | 108098341 |
| **408** | NRG1 | chr8 | 31495411 | 32624058 |
| **409** | NRXN1 | chr2 | 50144143 | 51261174 |
| **410** | NRXN3 | chr14 | 78635216 | 80336133 |
| **411** | NTM | chr11 | 131238871 | 132208216 |
| **412** | NUDT1 | chr7 | 2280357 | 2292280 |
| **413** | NUMA1 | chr11 | 71712411 | 71793073 |
| **414** | NXN | chr17 | 701053 | 884498 |
| **415** | NXPH1 | chr7 | 8472085 | 8794093 |
| **416** | OBI1-AS1 | chr13 | 78492324 | 79192960 |
| **417** | OLFM1 | chr9 | 137965589 | 138014530 |
| **418** | OLIG2 | chr21 | 34396716 | 34403003 |
| **419** | ONECUT2 | chr18 | 55101417 | 55160030 |
| **420** | OPCML | chr11 | 132283375 | 133403903 |
| **421** | OSBPL3 | chr7 | 24834664 | 25021260 |
| **422** | OTP | chr5 | 76923037 | 76936022 |
| **423** | OTX1 | chr2 | 63275692 | 63286466 |
| **424** | OTX2-AS 1 | chr14 | 57277224 | 57399050 |
| **425** | PACRG | chr6 | 163146664 | 163738024 |
| **426** | PACS2 | chr14 | 105765670 | 105865984 |
| **427** | PARD3 | chr10 | 34396988 | 35105753 |
| **428** | PARD3B | chr2 | 205409016 | 206486386 |
| **429** | PAX1 | chr20 | 21684797 | 21700624 |
| **430** | PAX3 | chr2 | 223063106 | 223165215 |
| **431** | PAX6 | chr11 | 31804840 | 31841009 |
| **432** | PAX6-AS1 | chr11 | 31836614 | 31910087 |
| **433** | PBX1 | chr1 | 164527097 | 164855800 |
| **434** | PCCA | chr13 | 100739769 | 101184191 |
| **435** | PCDHA1 | chr5 | 140164376 | 140393429 |
| **436** | PCDHA2 | chr5 | 140172944 | 140393429 |
| **437** | PCDHA3 | chr5 | 140179283 | 140393429 |
| **438** | PCDHA4 | chr5 | 140185172 | 140393429 |
| **439** | PCDHGA1 | chr5 | 140708752 | 140894048 |
| **440** | PCDHGA10 | chr5 | 140791243 | 140894048 |
| **441** | PCDHGA11 | chr5 | 140799037 | 140894048 |
| **442** | PCDHGA12 | chr5 | 140808658 | 140894048 |
| **443** | PCDHGA2 | chr5 | 140716854 | 140894048 |
| **444** | PCDHGA3 | chr5 | 140722101 | 140894048 |
| **445** | PCDHGA4 | chr5 | 140733268 | 140894048 |
| **446** | PCDHGA5 | chr5 | 140742398 | 140894048 |
| **447** | PCDHGA6 | chr5 | 140752151 | 140894048 |
| **448** | PCDHGA7 | chr5 | 140760967 | 140894048 |
| **449** | PCDHGA8 | chr5 | 140765952 | 140894048 |
| **450** | PCDHGA9 | chr5 | 140781020 | 140894048 |
| **451** | PCDHGB1 | chr5 | 140728328 | 140894048 |
| **452** | PCDHGB2 | chr5 | 140738203 | 140894048 |
| **453** | PCDHGB3 | chr5 | 140748462 | 140894048 |
| **454** | PCDHGB4 | chr5 | 140765952 | 140894048 |
| **455** | PCDHGB5 | chr5 | 140776195 | 140894048 |
| **456** | PCDHGB6 | chr5 | 140786270 | 140894048 |
| **457** | PCDHGB7 | chr5 | 140795714 | 140894048 |
| **458** | PCDHGC3 | chr5 | 140854069 | 140894048 |
| **459** | PCSK9 | chr1 | 55503649 | 55532026 |
| **460** | PDE4B | chr1 | 66256693 | 66841762 |
| **461** | PDE4D | chr5 | 58263366 | 59785425 |
| **462** | PDE6B | chr4 | 617863 | 666181 |
| **463** | PDGFRA | chr4 | 54242320 | 55165912 |
| **464** | PER2 | chr2 | 239151179 | 239200243 |
| **465** | PHF19 | chr9 | 123616431 | 123641106 |
| **466** | PITPNC1 | chr17 | 65371897 | 65694879 |
| **467** | PITX2 | chr4 | 111537080 | 111564779 |
| **468** | PITX3 | chr10 | 103988446 | 104002731 |
| **469** | PLEC | chr8 | 144987821 | 145052413 |
| **470** | PLEKHM1P1 | chr17 | 62773689 | 62834802 |
| **471** | PLEKHO2 | chr15 | 65132582 | 65161701 |
| **472** | PLXNC1 | chr12 | 94540999 | 94702951 |
| **473** | POU6F2 | chr7 | 39016109 | 39505890 |
| **474** | PPM1H | chr12 | 63036263 | 63330165 |
| **475** | PPP2R2A | chr8 | 25228575 | 26231695 |
| **476** | PPP2R2B | chr5 | 145967567 | 146462583 |
| **477** | PRDM16 | chr1 | 2984242 | 3356685 |
| **478** | PRDM2 | chr1 | 14025235 | 14153074 |
| **479** | PRDM6 | chr5 | 122423341 | 122525245 |
| **480** | PRDM8 | chr4 | 81103939 | 81126982 |
| **481** | PRKAG2 | chr7 | 151251701 | 151575816 |
| **482** | PRKCA | chr17 | 64297426 | 64808362 |
| **483** | PRKCE | chr2 | 45877543 | 46416629 |
| **484** | PRKCH | chr14 | 61652787 | 62019198 |
| **485** | PRKCZ | chr1 | 1980409 | 2118334 |
| **486** | PRKN | chr6 | 161767090 | 163150334 |
| **487** | PRR5L | chr11 | 36316225 | 36488254 |
| **488** | PSD3 | chr8 | 18383313 | 18872696 |
| **489** | PTPN20 | chr10 | 46548623 | 48829424 |
| **490** | PTPRG | chr3 | 61545743 | 62282073 |
| **491** | PTPRN2 | chr7 | 157330250 | 158381982 |
| **492** | PVT1 | chr8 | 128805303 | 129114999 |
| **493** | PWWP2B | chr10 | 134209202 | 134232858 |
| **494** | RAB11FIP3 | chr16 | 474168 | 573981 |
| **495** | RABGAP1L | chr1 | 174127052 | 174965945 |
| **496** | RAD51B | chr14 | 68284996 | 69198435 |
| **497** | RADIL | chr7 | 4832785 | 4924835 |
| **498** | RAI1 | chr17 | 17583287 | 17716265 |
| **499** | RALGAPA2 | chr20 | 20368772 | 20694766 |
| **500** | RAPGEF4 | chr2 | 173599025 | 173919120 |
| **501** | RASA3 | chr13 | 114745694 | 114899595 |
| **502** | RASGRP3 | chr2 | 33659916 | 33791298 |
| **503** | RBFOX1 | chr16 | 6067632 | 7764840 |
| **504** | RBFOX3 | chr17 | 77083927 | 77513730 |
| **505** | RBM20 | chr10 | 112402655 | 112600727 |
| **506** | RBMS1 | chr2 | 161127162 | 161351818 |
| **507** | RBMS3 | chr3 | 29321303 | 30053386 |
| **508** | RCN1 | chr11 | 31836614 | 32128772 |
| **509** | RERE | chr1 | 8410964 | 8879199 |
| **510** | REXO1 | chr19 | 1813745 | 1849952 |
| **511** | RFX4 | chr12 | 106975185 | 107158082 |
| **512** | RGL1 | chr1 | 183603708 | 183899166 |
| **513** | RGL3 | chr19 | 11492273 | 11531518 |
| **514** | RGS12 | chr4 | 3293255 | 3443140 |
| **515** | RGS20 | chr8 | 54762868 | 54873363 |
| **516** | RIMBP2 | chr12 | 130879181 | 131202326 |
| **517** | RNF216 | chr7 | 5658172 | 5822861 |
| **518** | RNF4 | chr4 | 2469295 | 2519086 |
| **519** | RNLS | chr10 | 89890557 | 90344582 |
| **520** | ROR1 | chr1 | 64238190 | 64648679 |
| **521** | RORA | chr15 | 60778983 | 61523002 |
| **522** | RPS6KA2 | chr6 | 166821354 | 167277271 |
| **523** | RPTOR | chr17 | 78517125 | 78941673 |
| **524** | RREB1 | chr6 | 7106330 | 7253713 |
| **525** | RTEL1 | chr20 | 62287663 | 62330044 |
| **526** | RTEL1-TNFRSF6B | chr20 | 62287663 | 62331551 |
| **527** | RUBCN | chr3 | 197396759 | 197478068 |
| **528** | RUNDC3A | chr17 | 42384427 | 42397538 |
| **529** | RUNX1 | chr21 | 36158598 | 37358547 |
| **530** | RXRA | chr9 | 137207444 | 137333931 |
| **531** | SASH1 | chr6 | 148662229 | 148874684 |
| **532** | SATB2 | chr2 | 200132723 | 200337489 |
| **533** | SATB2-AS 1 | chr2 | 200331321 | 200338981 |
| **534** | SBNO2 | chr19 | 1106133 | 1175782 |
| **535** | SCG5 | chr15 | 32932370 | 32990798 |
| **536** | SCOC | chr4 | 141176940 | 141305210 |
| **537** | SDK1 | chr7 | 3339580 | 4310131 |
| **538** | SDK2 | chr17 | 71329023 | 71641727 |
| **539** | SEPTIN9 | chr17 | 75275992 | 75498178 |
| **540** | SFXN5 | chr2 | 73167665 | 73300465 |
| **541** | SH3BP4 | chr2 | 235859128 | 235965858 |
| **542** | SH3RF3 | chr2 | 109744497 | 110263707 |
| **543** | SHANK2 | chr11 | 70312461 | 70937342 |
| **544** | SHOX2 | chr3 | 157812300 | 157825452 |
| **545** | SHROOM3 | chr4 | 77354753 | 77705905 |
| **546** | SIM1 | chr6 | 100835250 | 100914305 |
| **547** | SIM2 | chr21 | 38070491 | 38124010 |
| **548** | SKI | chr1 | 2158634 | 2243152 |
| **549** | SKOR1 | chr15 | 68110542 | 68127674 |
| **550** | SLC12A9 | chr7 | 100448841 | 100466134 |
| **551** | SLC1A7 | chr1 | 53551351 | 53609789 |
| **552** | SLC22A18 | chr11 | 2919451 | 2947976 |
| **553** | SLC22A18AS | chr11 | 2907827 | 2926675 |
| **554** | SLC25A10 | chr17 | 79668900 | 79689546 |
| **555** | SLC25A22 | chr11 | 788975 | 799769 |
| **556** | SLC38A10 | chr17 | 79217299 | 79270596 |
| **557** | SLC4A8 | chr12 | 51783601 | 51911047 |
| **558** | SLC6A9 | chr1 | 44455672 | 44498664 |
| **559** | SLC7A5 | chr16 | 87862129 | 87904600 |
| **560** | SLC8A2 | chr19 | 47929779 | 47976807 |
| **561** | SLC9A3 | chr5 | 471834 | 526049 |
| **562** | SLX1A | chr16 | 29464322 | 30210387 |
| **563** | SLX1B-SULT1A4 | chr16 | 29464371 | 30217150 |
| **564** | SMAD3 | chr15 | 67356695 | 67489033 |
| **565** | SMAGP | chr12 | 51637633 | 51665702 |
| **566** | SMG1P2 | chr16 | 29452726 | 30283698 |
| **567** | SMURF1 | chr7 | 98623558 | 98743243 |
| **568** | SND1 | chr7 | 127290702 | 127734159 |
| **569** | SNTG2 | chr2 | 945054 | 1372884 |
| **570** | SNX29 | chr16 | 12069102 | 12669646 |
| **571** | SOGA1 | chr20 | 35404345 | 35493587 |
| **572** | SORBS2 | chr4 | 186505098 | 186879370 |
| **573** | SORCS2 | chr4 | 7192874 | 7746064 |
| **574** | SOX10 | chr22 | 38366819 | 38384929 |
| **575** | SOX2-OT | chr3 | 180772968 | 181461513 |
| **576** | SOX6 | chr11 | 15986495 | 16761690 |
| **577** | SPATA13 | chr13 | 24552339 | 24898169 |
| **578** | SPECC1 | chr17 | 19911149 | 20219572 |
| **579** | SPON2 | chr4 | 1159221 | 1204250 |
| **580** | SPPL2B | chr19 | 2268020 | 2356600 |
| **581** | SPTBN1 | chr2 | 54681954 | 54900083 |
| **582** | SPTBN4 | chr19 | 40970648 | 41083865 |
| **583** | SRCIN1 | chr17 | 36684759 | 36763683 |
| **584** | SRGAP3 | chr3 | 9020776 | 9292869 |
| **585** | SRRM3 | chr7 | 75829716 | 75918105 |
| **586** | SSBP3 | chr1 | 54689604 | 54873568 |
| **587** | STAP2 | chr19 | 4322540 | 4344283 |
| **588** | STARD13 | chr13 | 33675772 | 34252432 |
| **589** | STK10 | chr5 | 171467574 | 171616846 |
| **590** | STK24 | chr13 | 99100955 | 99230896 |
| **591** | STK32C | chr10 | 134019496 | 134147563 |
| **592** | STON1-GTF2A1L | chr2 | 48755564 | 49005156 |
| **593** | STOX2 | chr4 | 184825009 | 184940375 |
| **594** | STRA6 | chr15 | 74470308 | 74503546 |
| **595** | SYCP2L | chr6 | 10746495 | 10976041 |
| **596** | SYNJ2 | chr6 | 158401388 | 158521707 |
| **597** | TACC2 | chr10 | 123747189 | 124015557 |
| **598** | TAFA2 | chr12 | 62100529 | 62655425 |
| **599** | TBC1D16 | chr17 | 77904642 | 78011157 |
| **600** | TBC1D7 | chr6 | 13265274 | 13330287 |
| **601** | TBC1D9 | chr4 | 141540436 | 141678971 |
| **602** | TBCD | chr17 | 80708440 | 80902562 |
| **603** | TBR1 | chr2 | 162271120 | 162283073 |
| **604** | TBX15 | chr1 | 119424166 | 119533679 |
| **605** | TBX4 | chr17 | 59528279 | 59563971 |
| **606** | TBX5 | chr12 | 114790235 | 114847747 |
| **607** | TEAD1 | chr11 | 12694469 | 12967784 |
| **608** | TENM2 | chr5 | 166710343 | 167692662 |
| **609** | TENM3 | chr4 | 183063640 | 183725677 |
| **610** | TENM3-AS1 | chr4 | 183058659 | 183067168 |
| **611** | TENM4 | chr11 | 78362828 | 79153195 |
| **612** | TET1 | chr10 | 70318617 | 70455739 |
| **613** | TFAP2A | chr6 | 10395416 | 10421297 |
| **614** | TFAP2B | chr6 | 50784939 | 50816826 |
| **615** | TG | chr8 | 133877705 | 134148643 |
| **616** | TGFB3 | chr14 | 76422942 | 76449592 |
| **617** | THRA | chr17 | 38216663 | 38251620 |
| **618** | THRB | chr3 | 24157145 | 24537953 |
| **619** | TK1 | chr17 | 76168660 | 76184785 |
| **620** | TLX1NB | chr10 | 102847578 | 102892403 |
| **621** | TMBIM1 | chr2 | 219137417 | 219158780 |
| **622** | TMEM132C | chr12 | 128750448 | 129193960 |
| **623** | TMEM132D | chr12 | 129554771 | 130389712 |
| **624** | TNRC18P1 | chr4 | 141560845 | 141565734 |
| **625** | TNS3 | chr7 | 47313252 | 47623242 |
| **626** | TOLLIP | chr11 | 1294098 | 1332392 |
| **627** | TOM1L2 | chr17 | 17745322 | 17877284 |
| **628** | TOX2 | chr20 | 42541992 | 42699754 |
| **629** | TP73 | chr1 | 3567629 | 3654265 |
| **630** | TRABD2B | chr1 | 48224700 | 48464062 |
| **631** | TRAK1 | chr3 | 42131246 | 42268882 |
| **632** | TRAPPC12 | chr2 | 3381946 | 3490357 |
| **633** | TRAPPC9 | chr8 | 140741086 | 141470178 |
| **634** | TRIM2 | chr4 | 154072770 | 154261974 |
| **635** | TRIM34 | chr11 | 5639674 | 5667125 |
| **636** | TRIM6-TRIM34 | chr11 | 5616365 | 5667125 |
| **637** | TRIM65 | chr17 | 73883541 | 73894584 |
| **638** | TRIM71 | chr3 | 32858010 | 32935271 |
| **639** | TRIO | chr5 | 14142329 | 14510958 |
| **640** | TRIP6 | chr7 | 100463450 | 100472576 |
| **641** | TSC2 | chr16 | 2096490 | 2140213 |
| **642** | TSNAX-DISC1 | chr1 | 231662899 | 232178519 |
| **643** | TSPAN14 | chr10 | 82212538 | 82283891 |
| **644** | TSPAN4 | chr11 | 841324 | 868616 |
| **645** | TSPAN9 | chr12 | 3185021 | 3397230 |
| **646** | TSPEAR | chr21 | 45916275 | 46132995 |
| **647** | TSTD1 | chr1 | 161005922 | 161010274 |
| **648** | TTC12 | chr11 | 113183751 | 113245518 |
| **649** | TTLL10 | chr1 | 1107786 | 1134813 |
| **650** | TTLL11 | chr9 | 124582704 | 124857385 |
| **651** | TUBA1C | chr12 | 49620209 | 49668613 |
| **652** | TULP4 | chr6 | 158732192 | 158934356 |
| **653** | TXNRD1 | chr12 | 104604988 | 104745585 |
| **654** | UFSP2 | chr4 | 186319194 | 186348639 |
| **655** | UHRF1 | chr19 | 4908010 | 4963665 |
| **656** | UNQ6494 | chr9 | 92253198 | 92336174 |
| **657** | USP20 | chr9 | 132596196 | 132645617 |
| **658** | UTRN | chr6 | 144605990 | 145175670 |
| **659** | VAV2 | chr9 | 136625516 | 136858946 |
| **660** | VAX1 | chr10 | 118886532 | 118899312 |
| **661** | VAX2 | chr2 | 71126220 | 71162075 |
| **662** | VEPH1 | chr3 | 156976032 | 157222915 |
| **663** | VGLL4 | chr3 | 11596044 | 11763720 |
| **664** | VOPP1 | chr7 | 55536806 | 55641700 |
| **665** | VPS13D | chr1 | 12288613 | 12573598 |
| **666** | VRK2 | chr2 | 58133286 | 58388555 |
| **667** | WDR81 | chr17 | 1618317 | 1643393 |
| **668** | WFIKKN2 | chr17 | 48910511 | 48921209 |
| **669** | WNT16 | chr7 | 120963921 | 120982658 |
| **670** | WNT5A | chr3 | 55498243 | 55523170 |
| **671** | WNT6 | chr2 | 219723046 | 219740454 |
| **672** | WT1 | chr11 | 32407822 | 32458581 |
| **673** | WWOX | chr16 | 78131827 | 79248064 |
| **674** | WWP2 | chr16 | 69794687 | 69977144 |
| **675** | YJEFN3 | chr19 | 19625519 | 19649893 |
| **676** | ZAR1 | chr4 | 48490809 | 48497922 |
| **677** | ZBTB16 | chr11 | 113928931 | 114122897 |
| **678** | ZBTB20 | chr3 | 114055447 | 114867627 |
| **679** | ZC3H12D | chr6 | 149767266 | 149807648 |
| **680** | ZC3H3 | chr8 | 144518325 | 144625120 |
| **681** | ZIC4 | chr3 | 147102335 | 147126096 |
| **682** | ZIC5 | chr13 | 100613775 | 100625678 |
| **683** | ZMIZ1 | chr10 | 80827292 | 81077785 |
| **684** | ZNF280D | chr15 | 56920874 | 57212197 |
| **685** | ZNF423 | chr16 | 49523015 | 49893330 |
| **686** | ZNF536 | chr19 | 30861828 | 31050465 |
| **687** | ZNF664-RFLNA | chr12 | 124456262 | 124802070 |
| **688** | ZNF833P | chr19 | 11749091 | 11798884 |

**Table 2: List of cancer types according to the disclosure**

| Column 1 lists the abbreviations of the cancer types used herein. The WHO 2020 entity or cancer type names are shown in Column 2. Column 3 provides a descriptor for the molecular class and Column 4 lists the PubMed Number (PMID). Where no PMID number appears in Column 4 the method according to the disclosure uncovered cancer subspecies that where not known or published before and thus have no PMID. | | | |
|---|---|---|---|
| **Cancer Type** | **WHO_2020_entity** | **Molecular.class** | **PMID** |
| A_IDH | Astrocytoma, IDH-mutant | diffuse glioma, IDH-mutant and 1p19q retained [astroglial type] | 29539639 |
| A_IDH_HG | Astrocytoma, IDH-mutant | diffuse glioma, IDH-mutant and 1p19q retained [astroglial type], high grade | 29539639 |
| ANTCON | Anaplastic neuroepithelial tumour with condensed nuclei | anaplastic neuroepithelial tumour with condensed nuclei | |
| ATRT_MYC | Atypical teratoid/rhabdoid tumour | Atypical teratoid rhabdoid tumour, MYC activated | 29539639 |
| ATRT_SHH | Atypical teratoid/rhabdoid tumour | Atypical teratoid rhabdoid tumour, SHH activated | 29539639 |
| ATRT_TYR | Atypical teratoid/rhabdoid tumour | Atypical teratoid rhabdoid tumour, Tyrosinase activated | 29539639 |
| CHGL | Chordoid glioma | chordoid glioma of the 3rd ventricle | 29539639 |
| CHORDM | Chordoma (including poorly differentiated chordoma) | chordoma | 29539639 |
| CN | Central neurocytoma | central neurocytoma | 29539639 |
| CNS_NB_FOXR2 | CNS embryonal tumour (or CNS neuroblastoma), FOXR2-altered | CNS neuroblastoma, FOXR2-altered | 29539639 |
| CNS_SARC_DICER | Primary intracranial sarcoma, DICER1-mutant | CNS DICER1-associated sarcoma | 29881993 |
| CPC_A | Choroid plexus carcinoma | choroid plexus carcinoma | 29539639 |
| CPC_B | Choroid plexus carcinoma | choroid plexus carcinoma | 33249490 |
| CPH_ADM | Adamantinomatous Cranio-pharyngioma | adamantinomatous craniopharyngioma | 29539639 |
| CPH_PAP | Papillary Craniopharyngioma | papillary craniopharyngioma | 29539639 |
| CPP_AD | Choroid plexus papilloma | choroid plexus papilloma | 29539639 |
| CPP_INF | Choroid plexus papilloma | choroid plexus papilloma | 29539639 |
| CRINET | Cribriform neuroepithelial tumour | cribriform neuroepithelial tumour | |
| CTRL_ADENOPIT | Control tissue, pituitary gland (anterior lobe) | normal pituitary gland, anterior lobe | 29539639 |
| CTRL_BLOOD | Normal WBCs | control tissue, blood | |
| CTRL_CBM | Control tissue, cerebellum | control tissue, cerebellar hemisphere | 29539639 |
| CTRL_CORPCAL | Control tissue, corpus callosum | control tissue, white matter (corpus callosum) | 29539639 |
| CTRL_HEMI | Control tissue, cerebral hemisphere | control tissue, hemispheric cortex | 29539639 |
| CTRL_HYPOTHAL | Control tissue, hypothalamus | control tissue, hypothalamus | 29539639 |
| CTRL_INFLAM | Glioblastoma, IDH-wildtype | control tissue, inflammatory tumour microenvironment | 29539639 |
| CTRL_OPTIC | Control tissue, optic pathway | control tissue, optic pathway | |
| CTRL_PIN | Control tissue, pineal gland | control tissue, pineal gland | 29539639 |
| CTRL_PONS | Control tissue, pons | control tissue, pons | 29539639 |
| CTRL_REACTIVE | Control tissue, reactive tumour microenvironment | control tissue, reactive tumour microenvironment | 29539639 |
| DGONC | Diffuse glioneuronal tumour with oligodendroglioma-like features and nuclear clusters (DGONC) | Diffuse glioneuronal tumour with oligodendroglioma-like features and nuclear clusters | 31867747 |
| DLBCL | Diffuse large B-cell lymphoma of the CNS | diffuse large B cell lymphoma | 29539639 |
| DLGNT_1 | Diffuse leptomeningeal glioneuronal tumour | diffuse leptomeningeal glioneuronal tumour | 29539639 |
| DLGNT_2 | Diffuse leptomeningeal glioneuronal tumour | diffuse leptomeningeal glioneuronal tumour | 29766299 |
| DMG_EGFR | Bithalamic glioma, EGFR-mutant | diffuse midline glioma [(bi-)thalamic, EGFR altered] | 33130881 |
| DMG_K27 | Diffuse midline glioma, H3 K27M-mutant | diffuse midline glioma, H3 K27-mutant / EZHIP over-expressing | 29539639 |
| DMT_SMARCB1 | Desmoplastic myxoid tumour of the pineal region, SMARCB1-mutant | desmoplastic myxoid tumour, SMARCB1-altered | |
| DNET | Dysembryoplastic neuroepithelial tumour | dysembryoplastic neuroepithelial tumour | 29539639 |
| EFT_CIC | CIC sarcoma | Ewing family tumour with CIC alteration | 29539639 |
| EMB_ND_A | Embryonal tumour not otherwise specified, subtype A | embryonal tumour [non defined, type A] | |
| ENB | Esthesioneuroblastoma, IDH-wildtype | esthesioneuroblastoma | 29730775 |
| EPN_MPE | Myxopapillary ependymoma | myxopapillary ependymoma | 29539639 |
| EPN_PF_SE | Subependymoma | posterior fossa subependymoma | 29539639 |
| EPN_PFA_1a | Posterior fossa ependymoma Group PFA | posterior fossa ependymoma group A1 | 29909548 |
| EPN_PFA_1b | Posterior fossa ependymoma Group PFA | posterior fossa ependymoma group A1 | 29909548 |
| EPN_PFA_1c | Posterior fossa ependymoma Group PFA | posterior fossa ependymoma group A1 | 29909548 |
| EPN_PFA_1d | Posterior fossa ependymoma Group PFA | posterior fossa ependymoma group A1 | 29909548 |
| EPN_PFA_1e | Posterior fossa ependymoma Group PFA | posterior fossa ependymoma group A1 | 29909548 |
| EPN_PFA_1f | Posterior fossa ependymoma Group PFA | posterior fossa ependymoma group A1 | 29909548 |
| EPN_PFA_2a | Posterior fossa ependymoma Group PFA | posterior fossa ependymoma group A2 | 29909548 |
| EPN_PFA_2b | Posterior fossa ependymoma Group PFA | posterior fossa ependymoma group A2 | 29909548 |
| EPN_PFA_2c | Posterior fossa ependymoma Group PFA | posterior fossa ependymoma group A2 | 29909548 |
| EPN_PFB_1 | Posterior fossa ependymoma Group PFB | posterior fossa ependymoma group B1-B3 | 30019219 |
| EPN_PFB_2 | Posterior fossa ependymoma Group PFB | posterior fossa ependymoma group B1-B3 | 30019219 |
| EPN_PFB_3 | Posterior fossa ependymoma Group PFB | posterior fossa ependymoma group B1-B3 | 30019219 |
| EPN_PFB_4 | Posterior fossa ependymoma Group PFB | posterior fossa ependymoma group B4 | 30019219 |
| EPN_PFB_5 | Posterior fossa ependymoma Group PFB | posterior fossa ependymoma group B5 | 30019219 |
| EPN_RELA_Like_A | Supratentorial ependymoma C11orf95 fusion-positive | supratentorial ependymoma, c11orf95:RELA-like | 33879448 |
| EPN_RELA_Like_B | Supratentorial ependymoma C11orf95 fusion-positive | supratentorial ependymoma, c11orf95:RELA-like | 33879448 |
| EPN_RELA_Like_C | Supratentorial ependymoma C11orf95 fusion-positive | supratentorial ependymoma, c11orf95:RELA-like | 33879448 |
| EPN_SPINE | Spinal ependymoma | spinal ependymoma | 29539639 |
| EPN_SPINE_MYCN | Spinal ependymoma, MYCN-amplified | spinal ependymoma, MYCN-amplified | 31414211 |
| EPN_SPINE_SE_A | Subependymoma | spinal subependymoma [subtype B] | 31414211 |
| EPN_SPINE_SE_B | Subependymoma | spinal subependymoma [subtype A] | 29539639 |
| EPN_ST_ND_A | Supratentorial ependymoma | supratentorial ependymoma [non-defined type] | |
| EPN_ST_SE | Subependymoma | supratentorial subependymoma | 29539639 |
| EPN_YAP | Supratentorial ependymoma, YAP1 fusion-positive | supratentorial ependymoma, YAP1-fused | 29539639 |
| ERMS | Rhabdomyosarcoma | embryonal rhabdomyosarcoma | 33479225 |
| ETMR_Atyp | Embryonal tumour with multilayered rosettes | embryonal tumour with multilayered rosettes-like | 31802000 |
| ETMR_C19MC | Embryonal tumour with multilayered rosettes | embryonal tumour with multilayered rosettes, C19MC altered | 29539639 |
| EVNCYT | Extraventricular neurocytoma | extraventricular neurocytoma | |
| EWS | Ewing sarcoma | Ewing sarcoma | 29539639 |
| GBM_CBM | Glioblastoma, IDH-wildtype | high-grade diffuse glioma of the midline/posterior fossa; H3/IDH-wildtype | |
| GBM_G34 | Diffuse hemispheric glioma, H3 G34-mutant | high-grade diffuse glioma, H3 G34-mutant | 29539639 |
| GBM_MES_Atyp | Glioblastoma, IDH-wildtype | glioblastoma, IDH-wildtype, mesenchymal type | |
| GBM_MES_Typ | Glioblastoma, IDH-wildtype | glioblastoma, IDH-wildtype, mesenchymal type | 29539639 |
| GBM_ped_ND_A | Diffuse paediatric-type high grade glioma, H3 wildtype and IDH wild type | glioblastoma [pediatric-type; non-defined A] | |
| GBM_ped_ND_B | Diffuse paediatric-type high grade glioma, H3 wildtype and IDH wild type | glioblastoma [pediatric-type; non-defined B] | |
| GBM_pedMYCN | Diffuse paediatric-type high grade glioma, H3 wildtype and IDH wild type | glioblastoma [pediatric-type, MYCN activated] | 29539639 |
| GBM_pedRTK1a | Diffuse paediatric-type high grade glioma, H3 wildtype and IDH wild type | glioblastoma [pediatric-type, RTK1] | 28401334 |
| GBM_pedRTK1b | Diffuse paediatric-type high grade glioma, H3 wildtype and IDH wild type | glioblastoma [pediatric-type, RTK1] | 28401334 |
| GBM_pedRTK1c | Diffuse paediatric-type high grade glioma, H3 wildtype and IDH wild type | glioblastoma [pediatric-type, RTK1] | 28401334 |
| GBM_pedRTK2a | Diffuse paediatric-type high grade glioma, H3 wildtype and IDH wild type | glioblastoma [pediatric-type, RTK2] | 28401334 |
| GBM_pedRTK2b | Diffuse paediatric-type high grade glioma, H3 wildtype and IDH wild type | glioblastoma [pediatric-type, RTK2] | 28401334 |
| GBM_PNC | Glioblastoma, IDH-wildtype | glioblastoma, IDH-wildtype, with primitive neuronal component | |
| GBM_RTK1 | Glioblastoma, IDH-wildtype | glioblastoma, IDH-wildtype, RTK1 type | 29539639 |
| GBM_RTK2 | Glioblastoma, IDH-wildtype | glioblastoma, IDH-wildtype, RTK2 type | 29539639 |
| GCT_GERM_A | Germinoma | germinoma, type A | |
| GCT_GERM_B | Germinoma | germinoma, type B (KIT-mutant) | |
| GCT_TERA | Mature teratoma | teratoma | |
| GCT_YOLKSAC | Yolk sac tumour | yolk sac tumour | |
| GG | Ganglioglioma | ganglioglioma | 29539639 |
| GNT_ND | Glioneuronal tumour, not otherwise specified, subtype A | diffuse glioneuronal tumour, non defined type | |
| HGAP | High-grade astrocytoma with piloid features | high-grade astrocytoma with piloid features | 29539639 |
| HGNET_BCOR_Fus | CNS tumour with BCOR internal tandem duplication | neuroepithelial tumour with EP300:BCOR(L1) fusion | |
| HGNET_BCOR_ITD | CNS tumour with BCOR internal tandem duplication | neuroepithelial tumour with BCOR internal tandem duplication | 29539639 |
| HGNET_BEND2 | Astroblastoma | high-grade neuroepithelial tumour with MN1:BEND2 fusion | 29539639 |
| HGNET_CXXC5 | Astroblastoma | high-grade neuroepithelial tumour with MN1:CXXC5 fusion | |
| HGNET_ND_B | Glioblastoma, IDH-wildtype | diffuse high-grade neuroepithelial tumour [adult-type, non-defined type B] | |
| HGNET_ND_C | Glioblastoma, IDH-wildtype | diffuse high-grade neuroepithelial tumour [adult-type, non-defined type C] | |
| HGNET_ND_D | Glioblastoma, IDH-wildtype | diffuse high-grade neuroepithelial tumour [adult-type, non-defined type D] | |
| HGNET_PATZ | Neuroepithelial tumour, PATZ1 fusion-positive | neuroepithelial tumour with PATZ1 fusion | |
| HGNET_PLAG | Diffuse paediatric-type high grade glioma, H3 wildtype and IDH wild type | diffuse high-grade neuroepithelial tumour, PLAG-family amplified | |
| HMB | Haemangioblastoma | haemangioblastoma | 29539639 |
| IDH_B | Astrocytoma, IDH-mutant | diffuse glioma, IDH-mutant and 1p19q retained [astroglial type] | |
| IHG | Infant-type hemispheric glioma, H3-wildtype | infantile hemispheric glioma | 29539639 |
| IO_MEPL | Medulloepithelioma | intraocular medulloepithelioma | |
| LCH | Langerhans cell histiocytosis | Langerhans cell histiocytosis | |
| LGG_DIG_DIA | Desmoplastic infantile astrocytoma and ganglioglioma | desmoplastic infantile ganglioglioma/astrocytoma | 29539639 |
| LGG_MYB_A | Angiocentric glioma | diffuse glioma, MYB(L1)-altered, subtype A [angiocentric glioma-type] | 29539639 |
| LGG_MYB_B | Diffuse astrocytoma, MYB or MYBL1-altered | diffuse glioma, MYB(L1)-altered, subtype B [infratentorial-type] | |
| LGG_MYB_C | Diffuse astrocytoma, MYB or MYBL1-altered | diffuse glioma, MYB(L1)-altered, subtype C [isomorphic diffuse glioma-type] | |
| LGG_MYB_D | Diffuse astrocytoma, MYB or MYBL1-altered | diffuse glioma, MYB(L1)-altered, subtype D | |
| LIPN | Cerebellar liponeurocytoma | liponeurocytoma | 29539639 |
| MB_G34_I | Medulloblastoma, non-WNT/non-SHH | medulloblastoma Group 3 | 31076851 |
| MB_G34_II | Medulloblastoma, non-WNT/non-SHH | medulloblastoma Group 3 | 31076851 |
| MB_G34_III | Medulloblastoma, non-WNT/non-SHH | medulloblastoma Group 3 | 31076851 |
| MB_G34_IV | Medulloblastoma, non-WNT/non-SHH | medulloblastoma Group 3 | 31076851 |
| MB_G34_V | Medulloblastoma, non-WNT/non-SHH | medulloblastoma Group 4 | 31076851 |
| MB_G34_VI | Medulloblastoma, non-WNT/non-SHH | medulloblastoma Group 4 | 31076851 |
| MB_G34_VII | Medulloblastoma, non-WNT/non-SHH | medulloblastoma Group 4 | 31076851 |
| MB_G34_VIII | Medulloblastoma, non-WNT/non-SHH | medulloblastoma Group 4 | 31076851 |
| MB_MYO | Medulloblastoma, non-WNT/non-SHH | medullomyoblastoma | |
| MB_SHH_AD | Medulloblastoma, SHH-activated | medulloblastoma, SHH-activated [childhood/adult type] | 28609654 |
| MB_SHH_AD | Medulloblastoma, SHH-activated | medulloblastoma, SHH-activated [childhood/adult type] | 28609654 |
| MB_SHH_AD | Medulloblastoma, SHH-activated | medulloblastoma, SHH-activated [childhood/adult type] | 28609654 |
| MB_SHH_AD | Medulloblastoma, SHH-activated | medulloblastoma, SHH-activated [childhood/adult type] | 28609654 |
| MB_SHH_IDH | Medulloblastoma, SHH-activated | medulloblastoma, SHH-activated, IDH-mutant | |
| MB_WNT | Medulloblastoma, WNT-activated | medulloblastoma, WNT activated | 29539639 |
| MELN | Meningeal melanocytosis and melanomatosis | melanocytoma | 29539639 |
| MET_MEL | Metastases to the brain and spinal cord parenchyma | melanoma [metastatic] | 29539639 |
| MMNST | Malignant melanotic nerve sheath tumour | malignant melanotic nerve sheath tumour | 29539639 |
| MNG_ben-1 | Meningioma | meningioma, benign | 28314689 |
| MNG_ben-2 | Meningioma | meningioma, benign | 28314689 |
| MNG_ben-3 | Meningioma | meningioma, benign | 28314689 |
| MNG_int-A | Meningioma | meningioma, intermediate | 28314689 |
| MNG_int-B | Meningioma | meningioma, intermediate | 28314689 |
| MNG_mal | Meningioma | meningioma, malignant | 28314689 |
| MNG_SMARCE1 | Meningioma | meningioma, SMARCE1-altered | |
| MPNST_Atyp | Malignant peripheral nerve sheath tumour (MPNST) | malignant peripheral nerve sheath tumour [spinal or atypical type] | |
| MPNST_Typ | Malignant peripheral nerve sheath tumour (MPNST) | malignant peripheral nerve sheath tumour [typical type] | |
| MYXGNT | Myxoid glioneuronal tumour | myxoid glioneuronal tumour of the 3rd ventricle / septum pellucidum | |
| NB_MYCN | NB, MYCN | neuroblastoma | 27635046 |
| NB_TMMneg | NB, no-TMM | neuroblastoma | 27635046 |
| NB_TMMpos | NB, TERT | neuroblastoma | 27635046 |
| NFIB_PLEX | Hybrid nerve sheath tumours | plexiform neurofibroma | |
| O_IDH | Oligodendroglioma, IDH-mutant and 1p/19q-codeleted | diffuse glioma, IDH-mutant and 1p19q co-deleted [oli-godendroglial type] | 29539639 |
| OLIGOSARC_IDH | Oligodendroglioma, IDH-mutant and 1p/19q-codeleted | diffuse glioma, IDH-mutant and 1p19q co-deleted [oligodendroglial type] | |
| PA_CORT | Pilocytic astrocytoma | supratentorial pilocytic astrocytoma | 29539639 |
| PA_INF | Pilocytic astrocytoma | infratentorial pilocytic astrocytoma | 29539639 |
| PA_INF_FGFR | Pilocytic astrocytoma | infratentorial pilocytic astrocytoma | |
| PA_MID | Pilocytic astrocytoma | supratentorial midline pilocytic astrocytoma | 29539639 |
| PB_FOXR2 | Pineoblastoma | pineoblastoma, MYC/FOXR2-activated | 31768671 |
| PB_GrplA | Pineoblastoma | pineoblastoma, miRNA pathway altered, group 1 | 31768671 |
| PB_GrplB | Pineoblastoma | pineoblastoma, miRNA pathway altered, group 1 | 31768671 |
| PB_Grp2 | Pineoblastoma | pineoblastoma, miRNA pathway altered, group 2 | 31768671 |
| PGG | Paraganglioma | spinal paraganglioma | 29539639 |
| PGNT | Papillary glioneuronal tumour | papillary glioneuronal tumour | |
| PIN_CYT | Pineocytoma | pineocytoma | 31768671 |
| PIN_RB | Pineoblastoma | pineal retinoblastoma | 29539639 |
| PITAD_ACTH | Pituitary adenoma /PitNET | pituitary adenoma, ACTH-producing | 29539639 |
| PITAD_GON | Pituitary adenoma /PitNET | pituitary adenoma, gonadotrophin-producing | 29539639 |
| PITAD_PRL | Pituitary adenoma /PitNET | pituitary adenoma, prolactin-producing | 29539639 |
| PITAD_STH_DENSE1 | Pituitary adenoma /PitNET | pituitary adenoma, STH-producing | 29539639 |
| PITAD_STH_DENSE2 | Pituitary adenoma /PitNET | pituitary adenoma, STH-producing | 29539639 |
| PITAD_STH_SPARSE | Pituitary adenoma /PitNET | pituitary adenoma, STH-producing | 29539639 |
| PITAD_TSH | Pituitary adenoma /PitNET | pituitary adenoma, TSH-producing | 29539639 |
| PITUI | Pituicytoma | pituicytoma | 29539639 |
| PLASMACYT | Miscellaneous rare lymphomas in the CNS | plasmacytoma | 29539639 |
| PLNTY | Polymorphous low-grade neuroepithelial tumour of the young | polymorphous low-grade neuroepithelial tumour of the young | 27812792 |
| PPTID_A | Pineal parenchymal tumour of intermediate differentiation | pineal parenchymal tumour of intermediate differentia-tion | 31768671 |
| PPTID_B | Pineal parenchymal tumour of intermediate differentiation | pineal parenchymal tumour of intermediate differentiation | 31768671 |
| PTPR_A | Papillary tumour of the pineal region | papillary tumour of the pineal region | 29539639 |
| PTPR_B | Papillary tumour of the pineal region | papillary tumour of the pineal region | 29539639 |
| PXA | Pleomorphic xanthoastrocytoma | pleomorphic xanthoastrocytoma(-like) | 29539639 |
| RB | Retinoblastoma | retinoblastoma | 29539639 |
| RB_MYCN | Retinoblastoma, subtype MYCN-altered | retinoblastoma, MYCN-activated | 33879448 |
| RGNT | Rosette forming glioneuronal tumour | rosette-forming glioneuronal tumour | 29539639 |
| SCHW | Schwannoma | schwannoma | 29539639 |
| SEGA | Subependymal giant cell astrocytoma | subependymal giant cell astrocytoma | 29539639 |
| SFT_HMPC | Solitary fibrous tumour | solitary fibrous tumour / haemangiopericytoma | 29539639 |
| SNUC_IDH2 | Esthesioneuroblastoma, IDH-mutant | Sinonasal undifferentiated carcinoma, IDH2-mutant | 29730775 |
| ST_EPN_RELA_A | Supratentorial ependymoma C11orf95 fusion-positive | supratentorial ependymoma, c11orf95:RELA-fused | 29539639 |
| ST_EPN_RELA_B | Supratentorial ependymoma C11orf95 fusion-positive | supratentorial ependymoma, c11orf95:RELA-fused | 33879448 |
| VGLL | Low grade neuroepithelial tumour, subtype VGLL fused | intracranial schwannoma, VGLL-altered | |

**Tables 3 to 172:** Classification of cancer types listed in Table 2 according to the disclosure.

The classification data for each cancer type as listed in Table 2 is shown in an individual table. Each table comprises the following columns:
Column 1 shows the selected gene sites for the classification of the cancer type.
Column 2 shows the overall statistical importance (imp_sum) of a specific gene site for the classification of the cancer type. The overall importance of the specific gene site (imp_sum) is calculated by multiplying the number of single measurement points (n_probes) of Column 4 with the mean variable importance (imp_mean) of Column 3. Higher values represent more important gene sites.
Column 3 shows the mean variable importance (imp_mean) of all of the single measurement points (n_probes) of the specific gene sites according to the statistical model used (e.g. based on Random Forest)
Column 4 shows the number of single measurement points (n_probes; CpG site methylation probes that fall within the gene site).

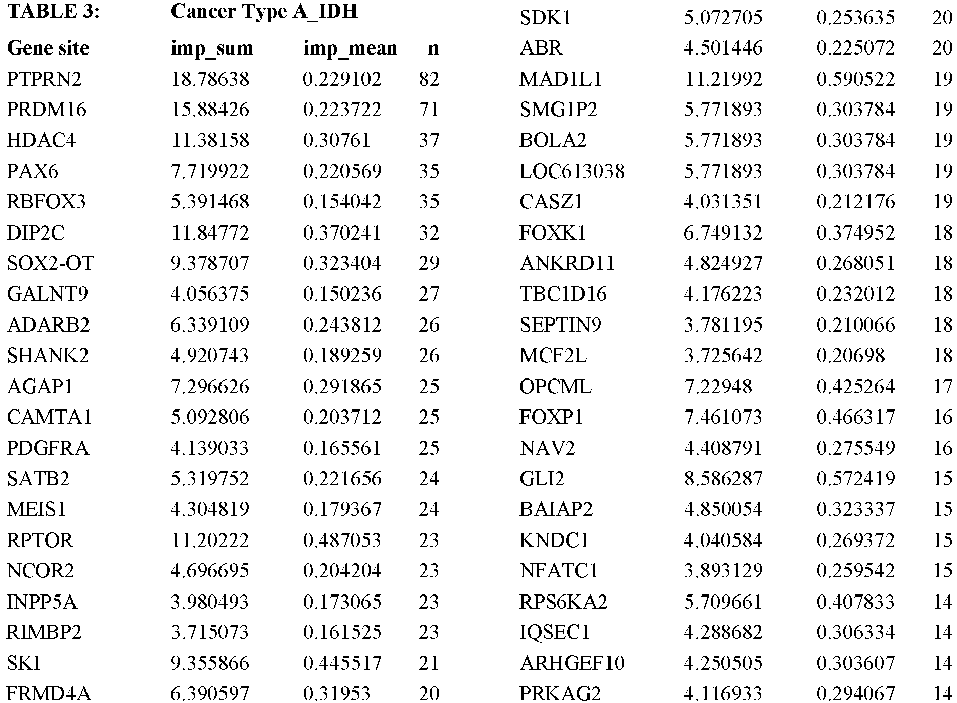

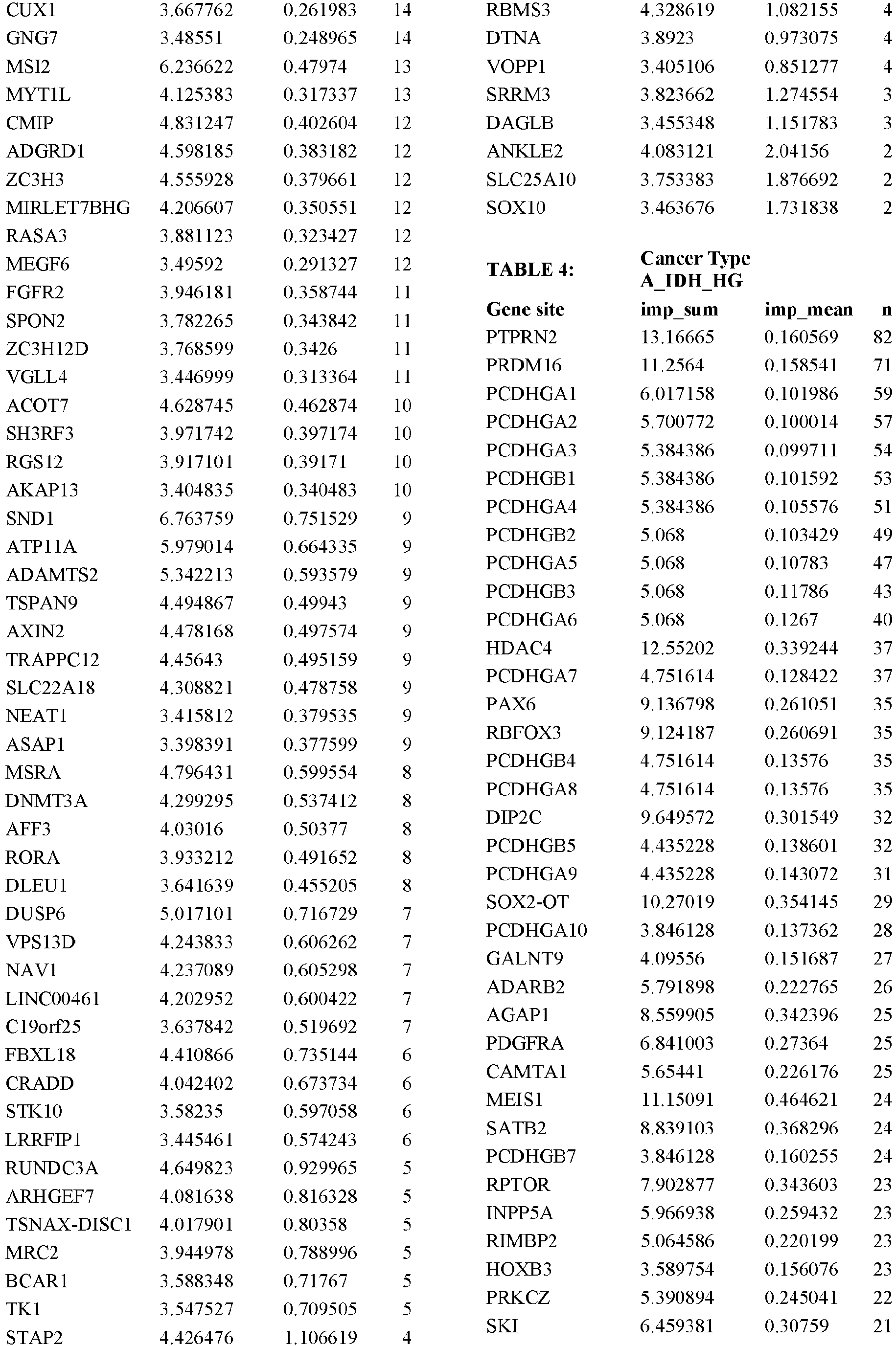

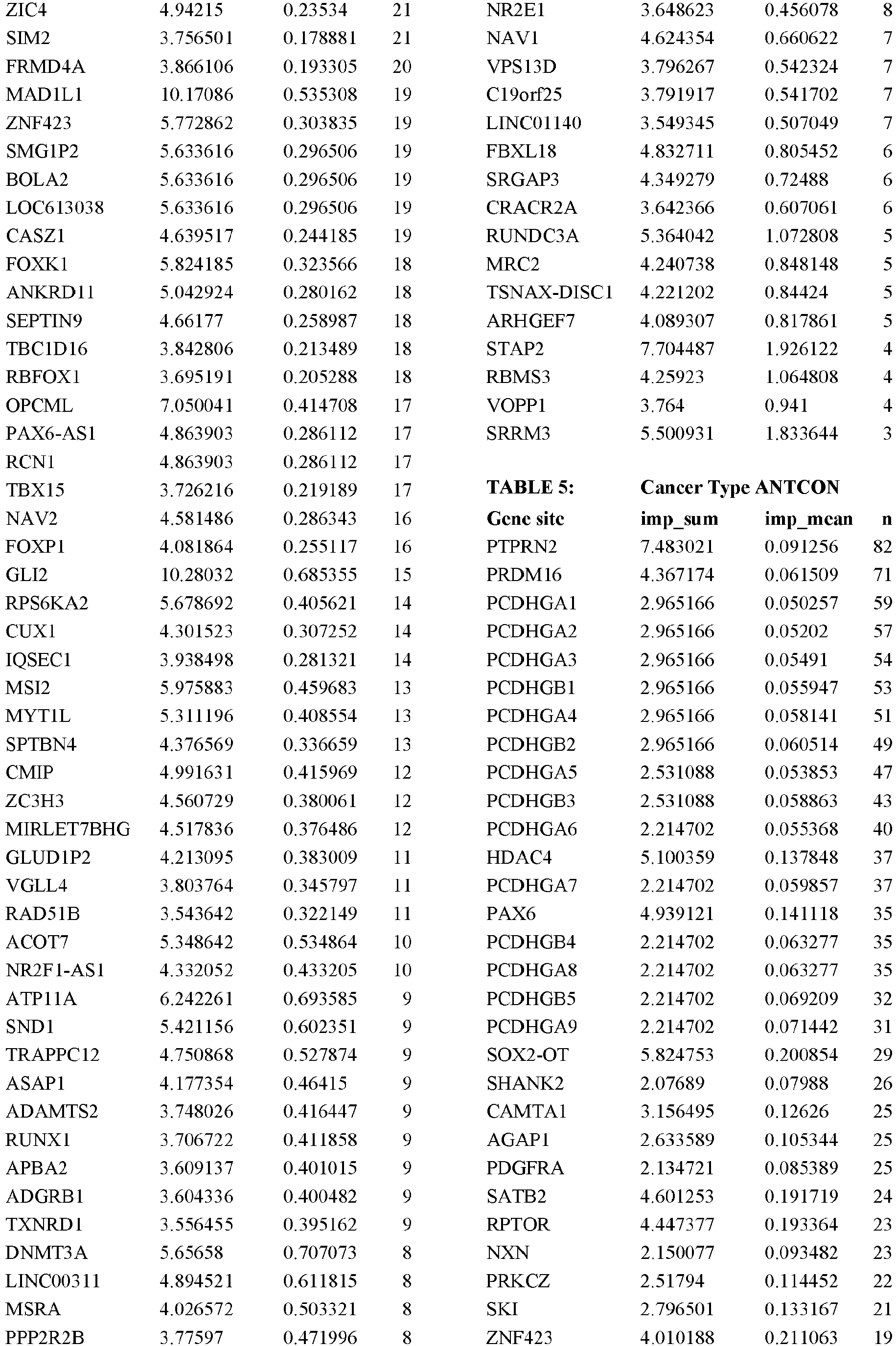

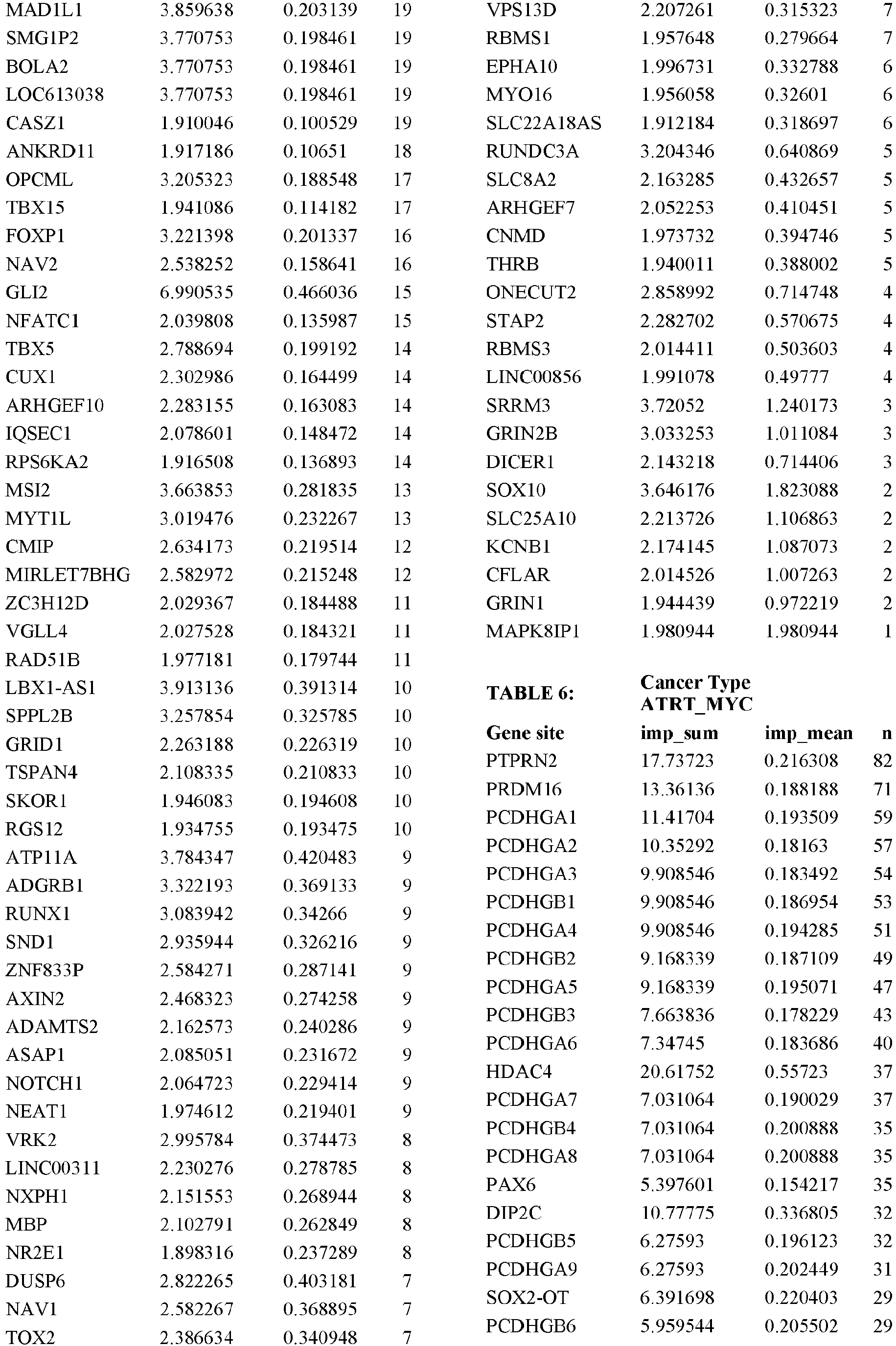

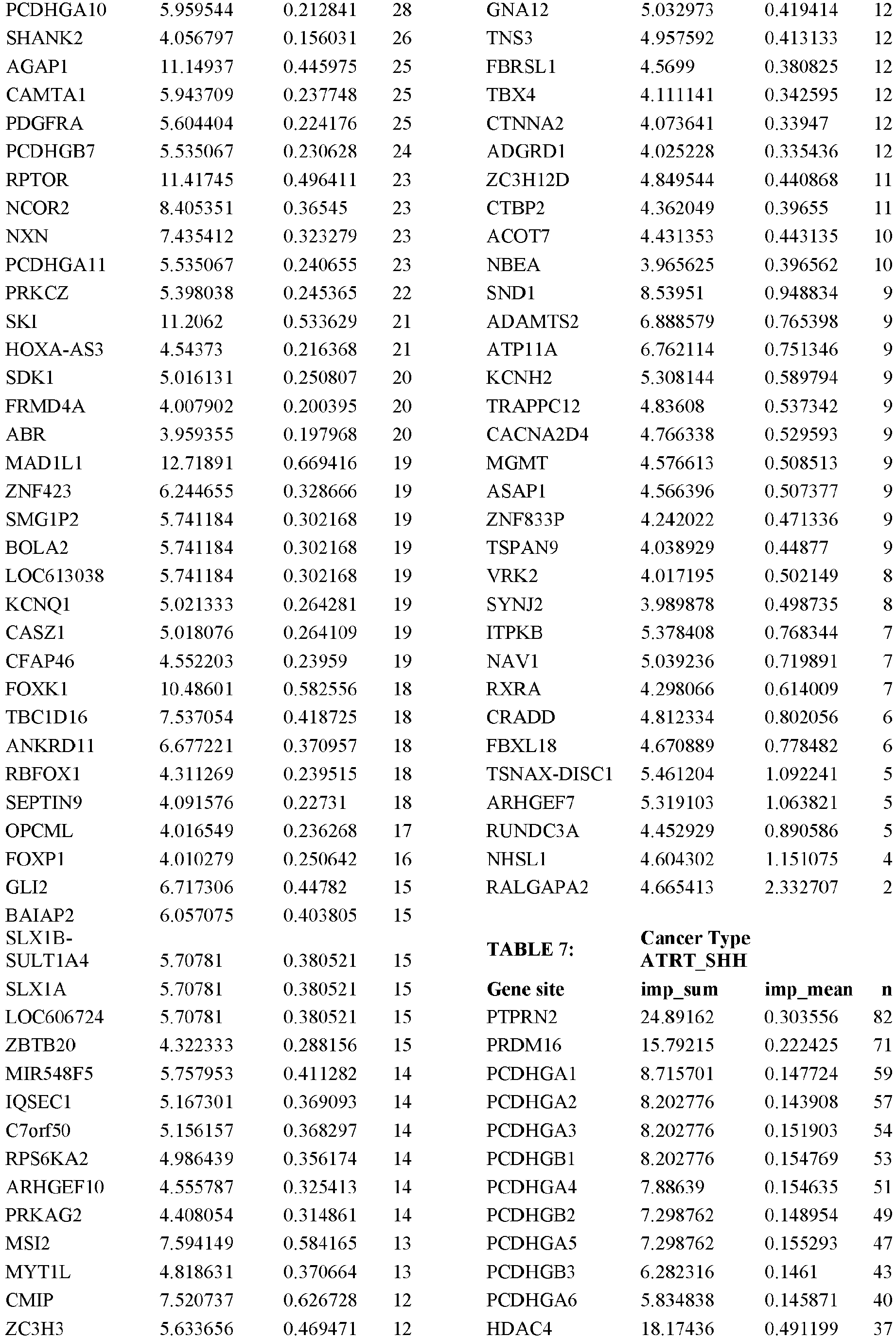

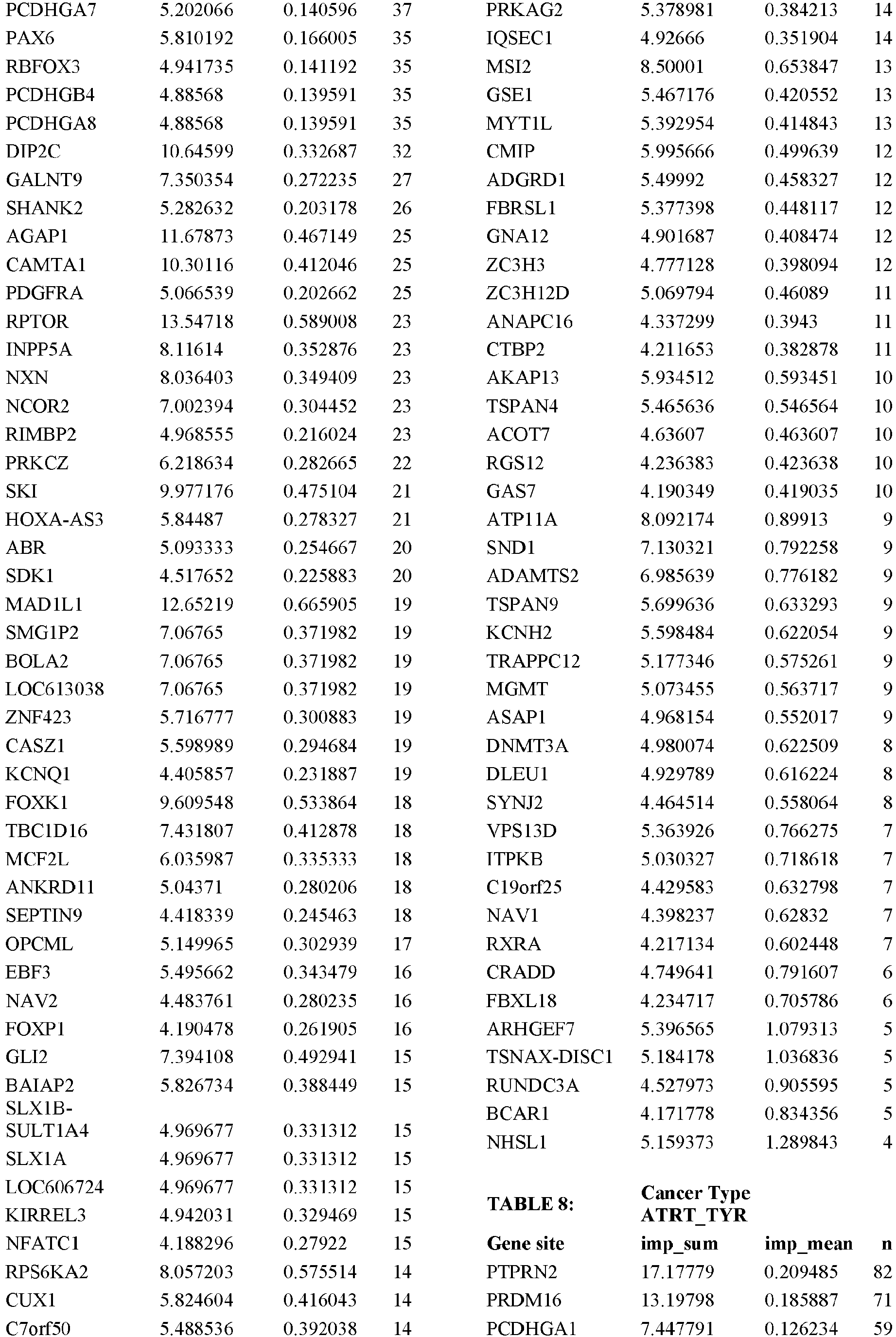

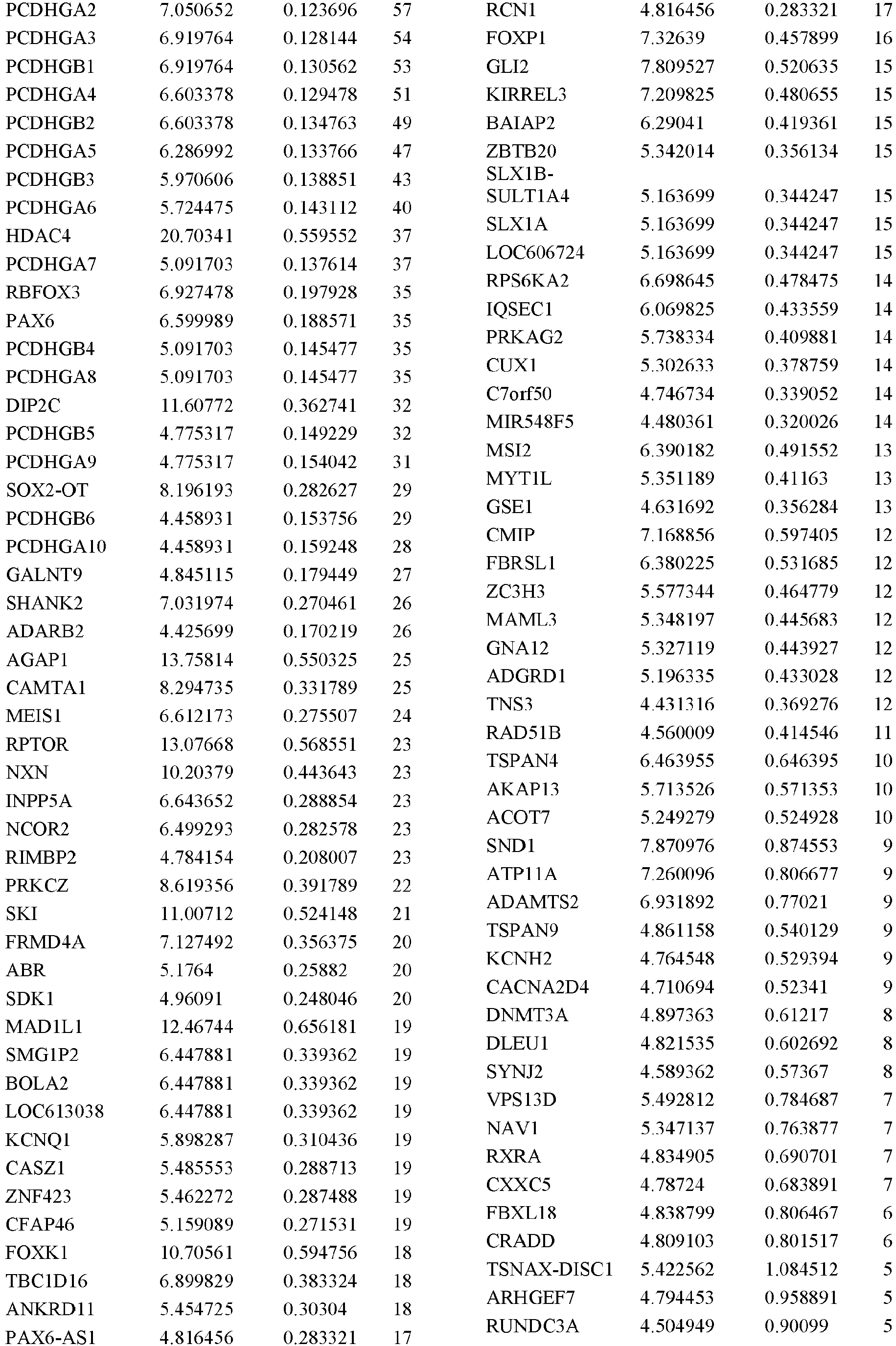

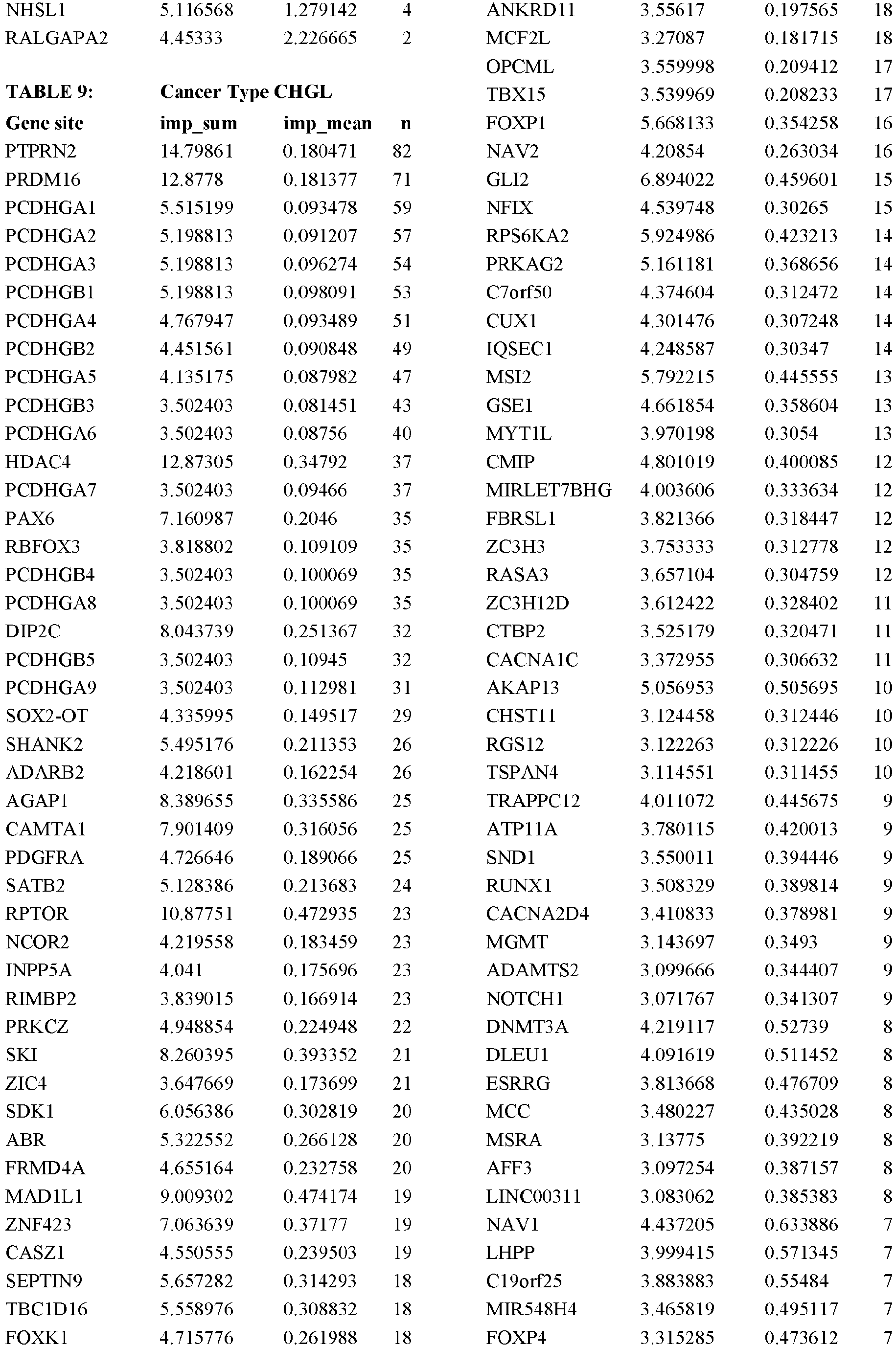

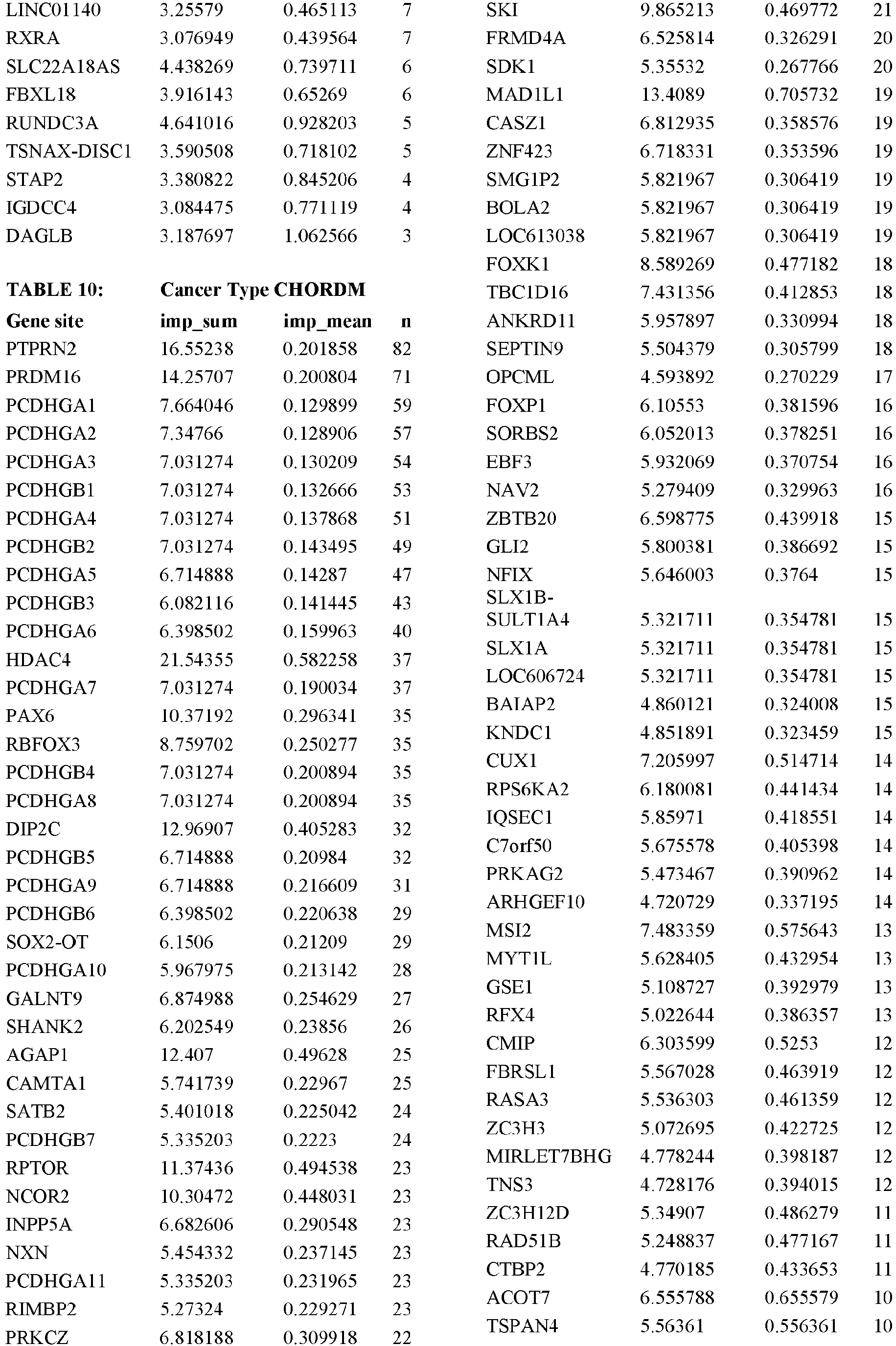

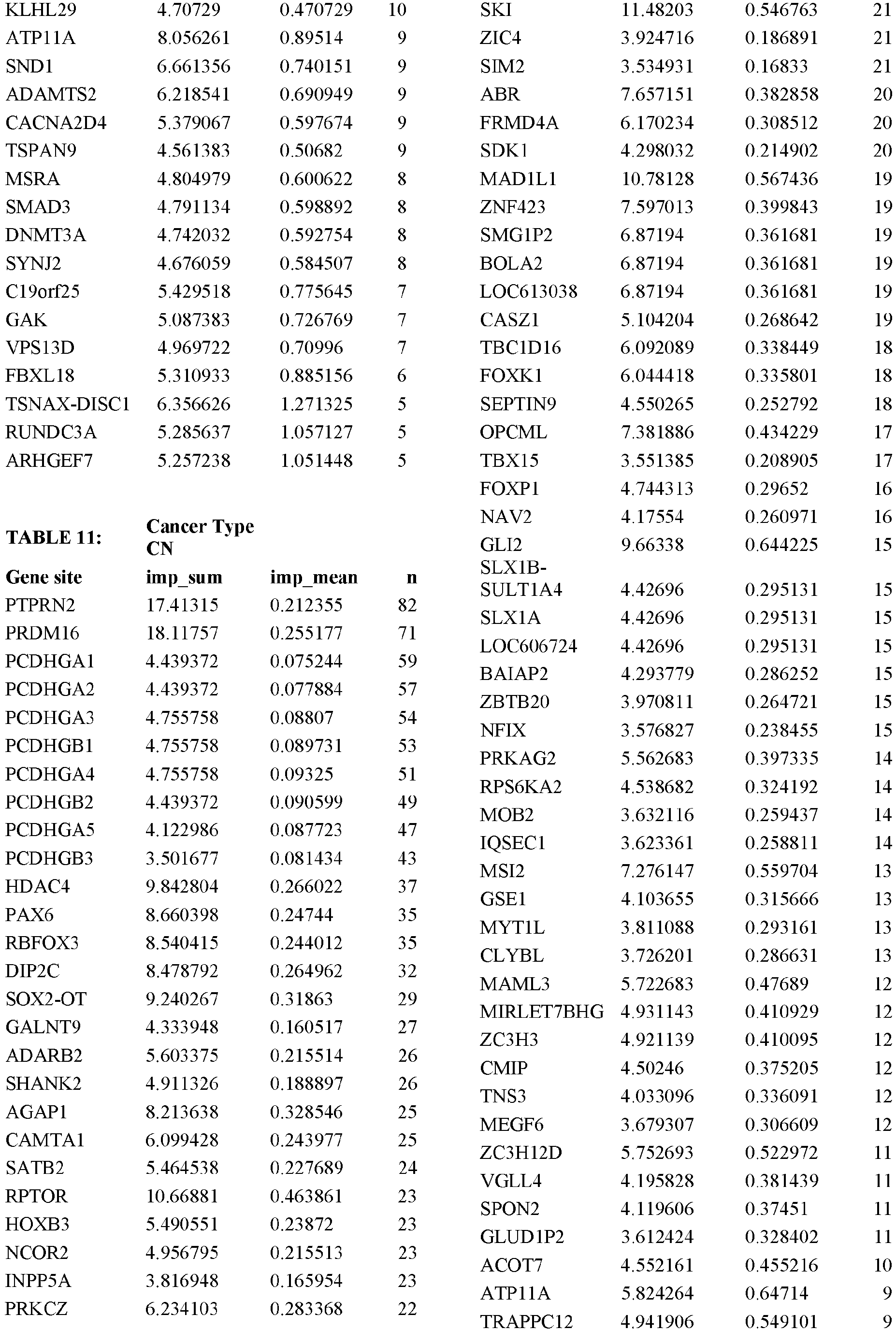

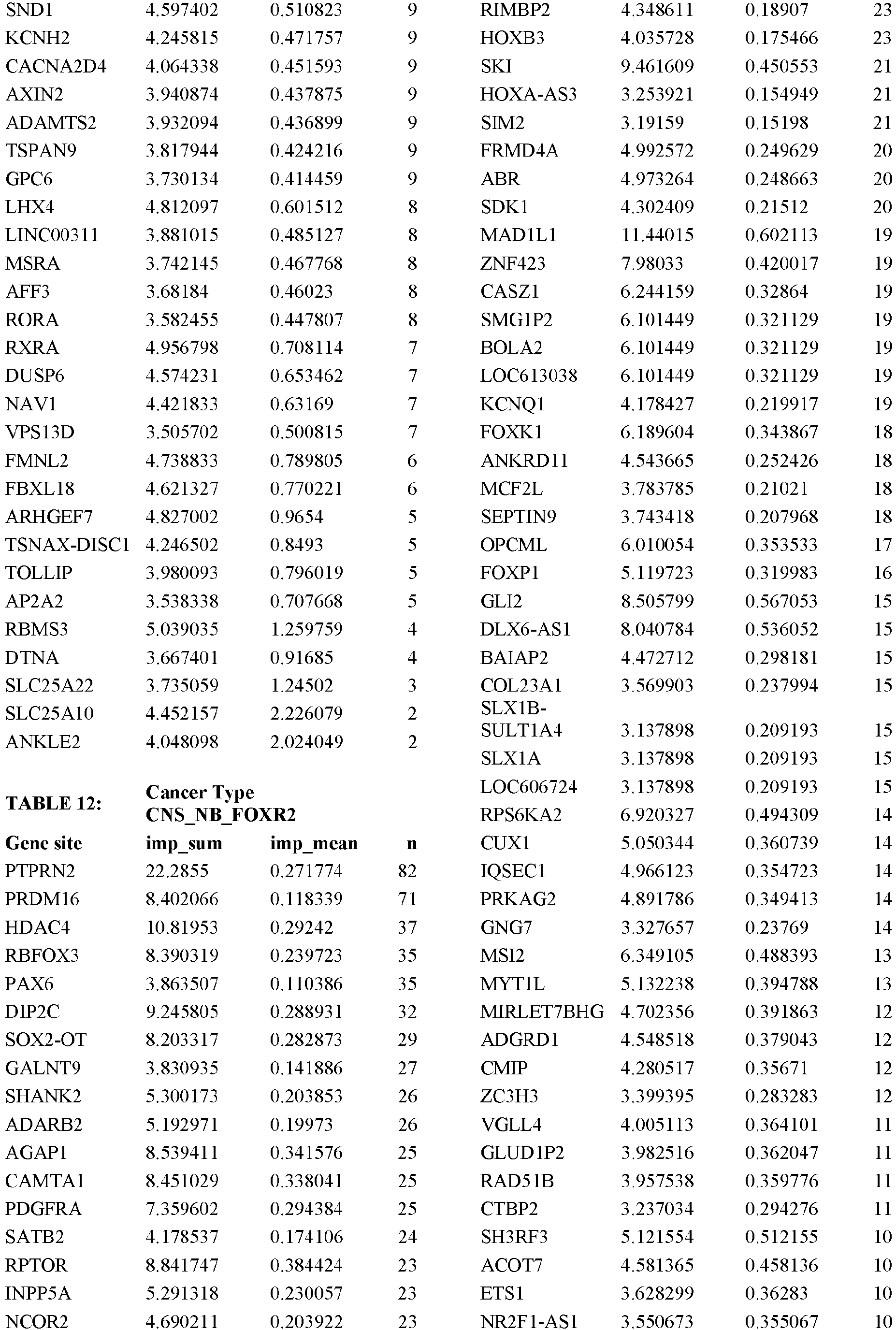

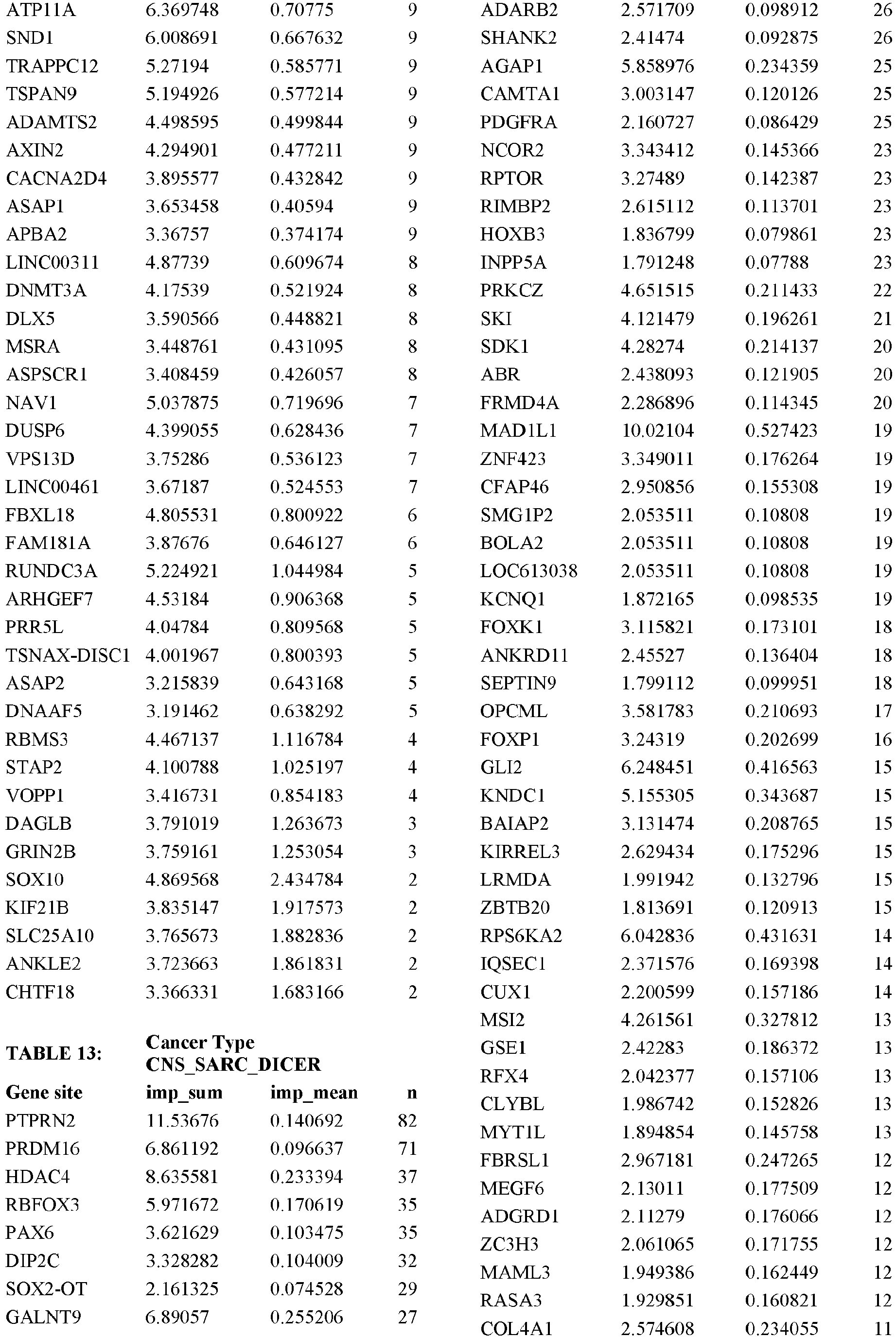

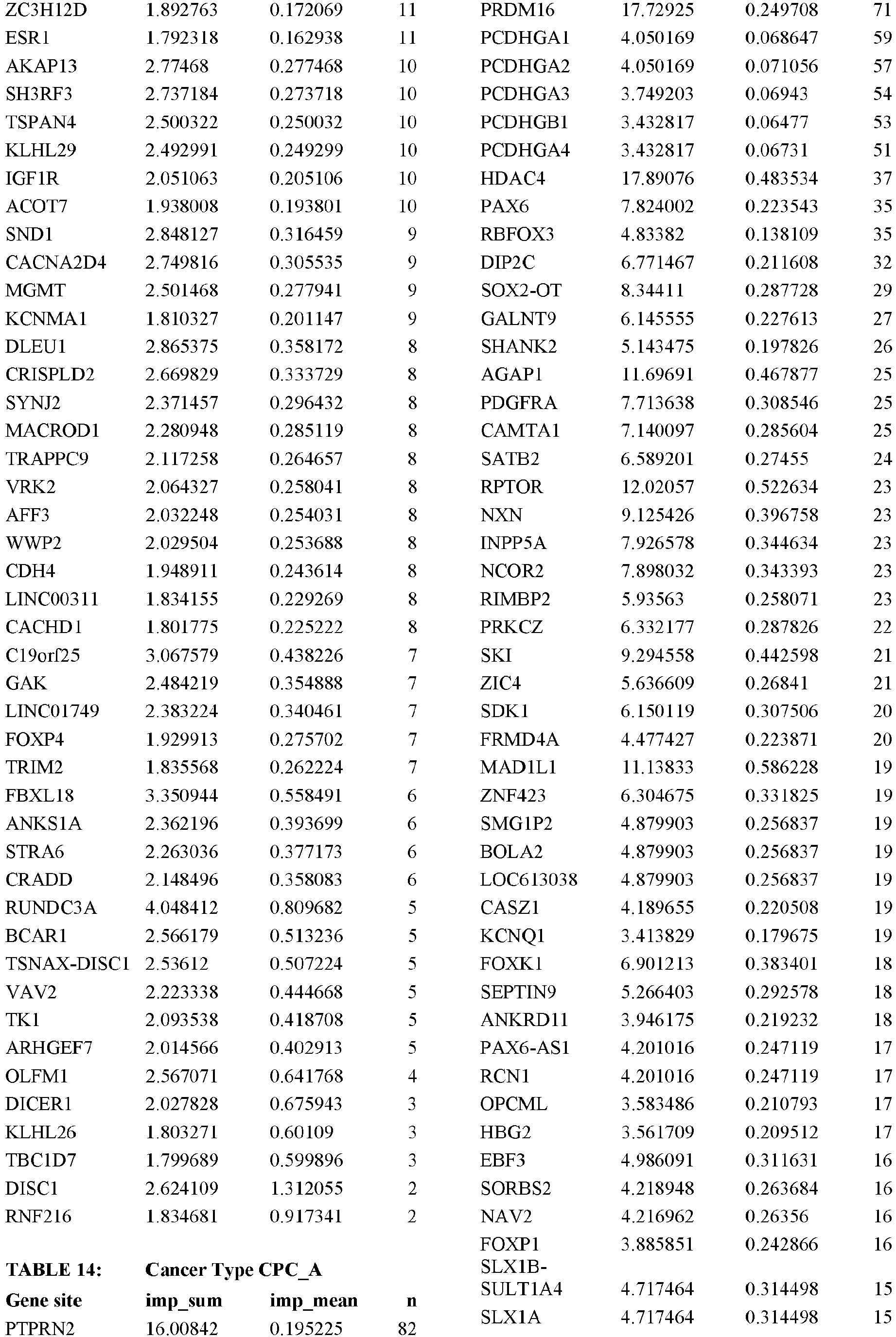

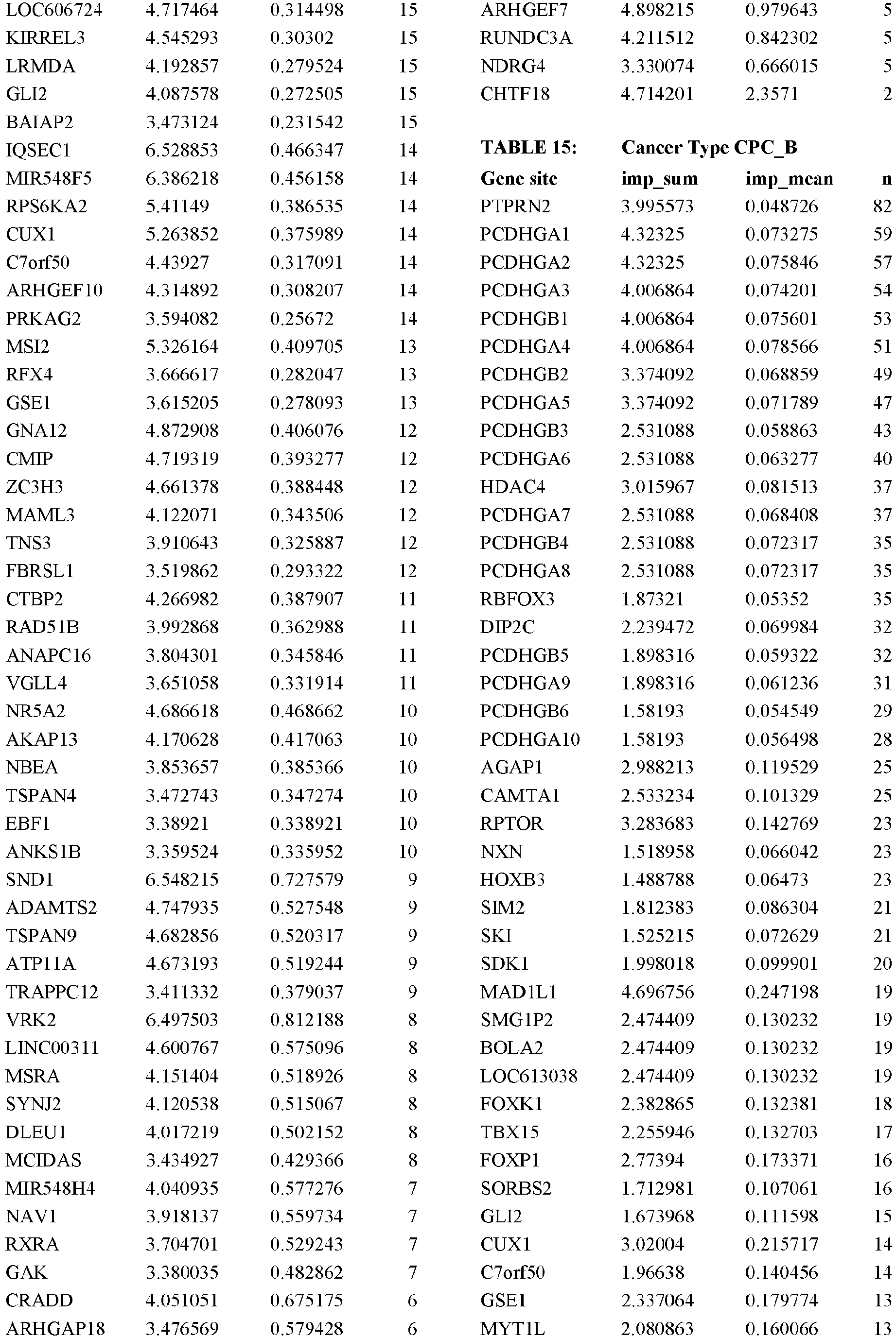

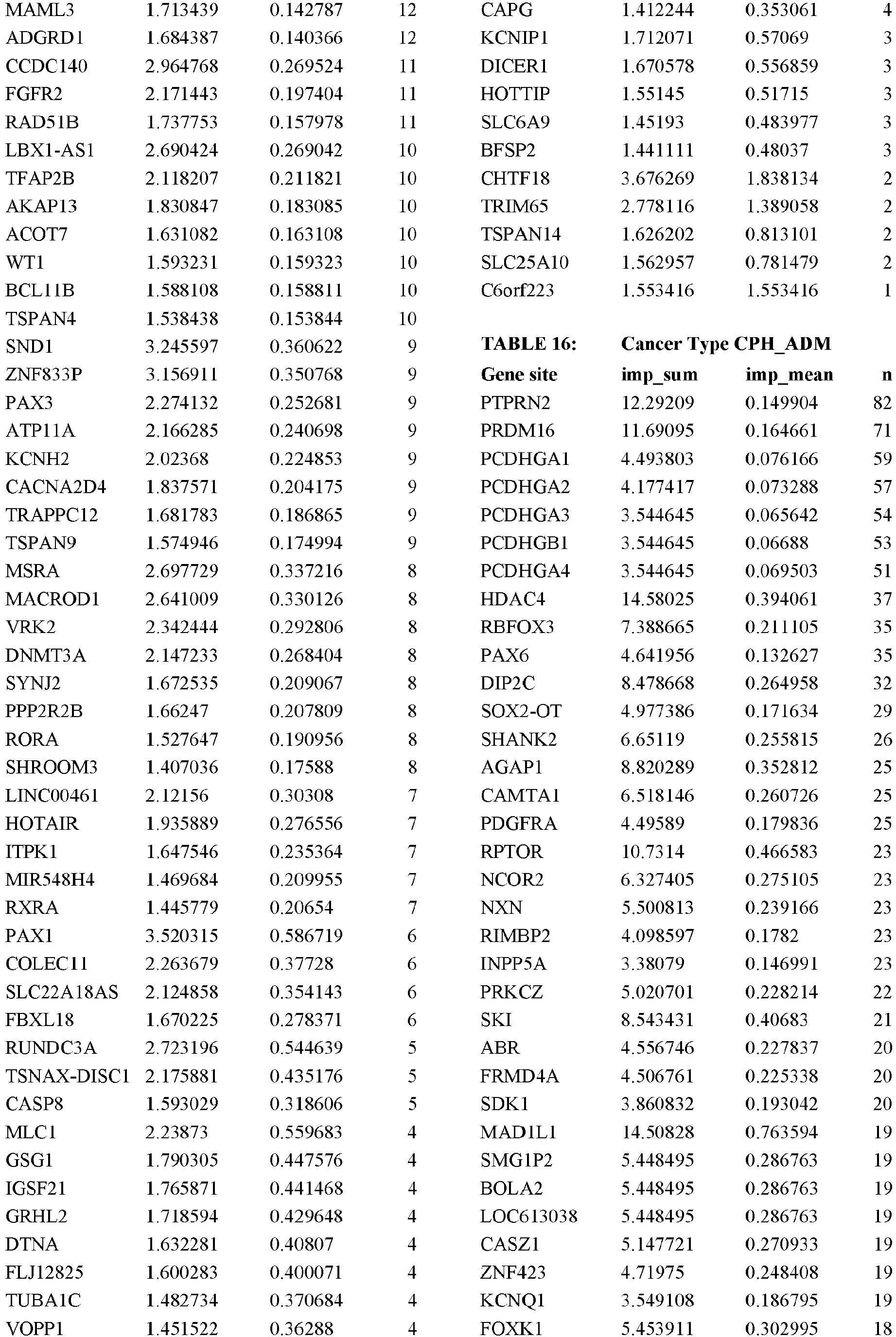

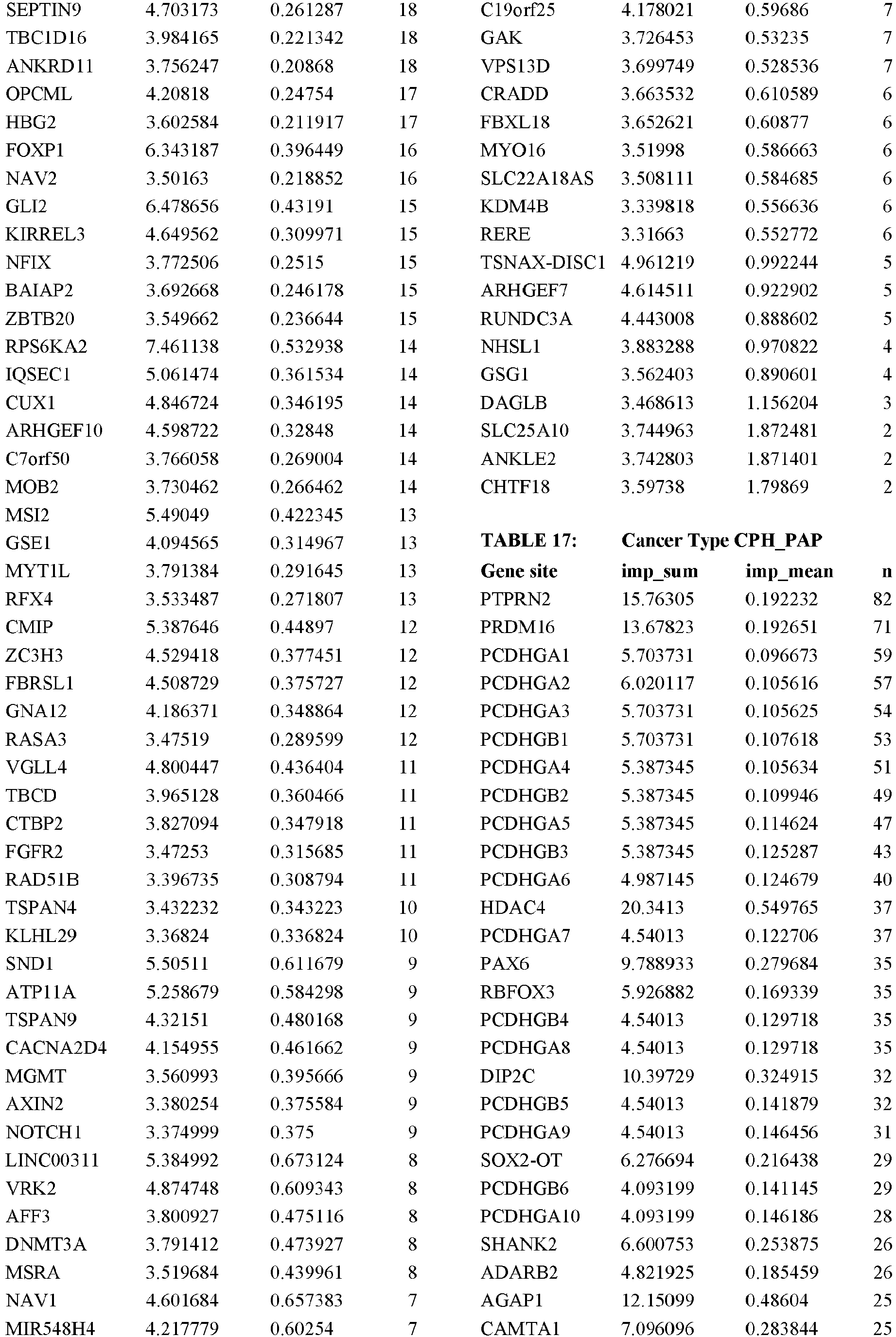

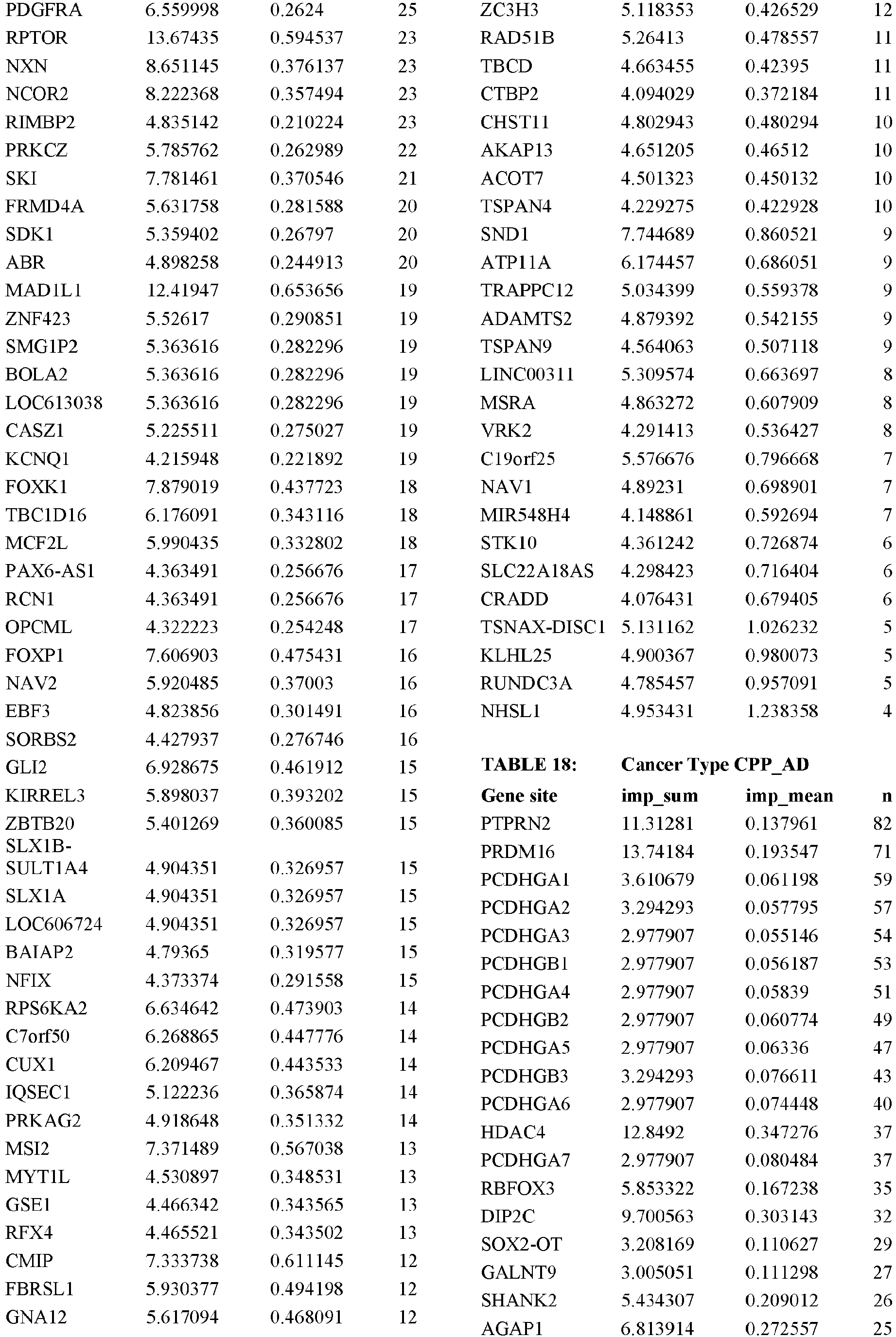

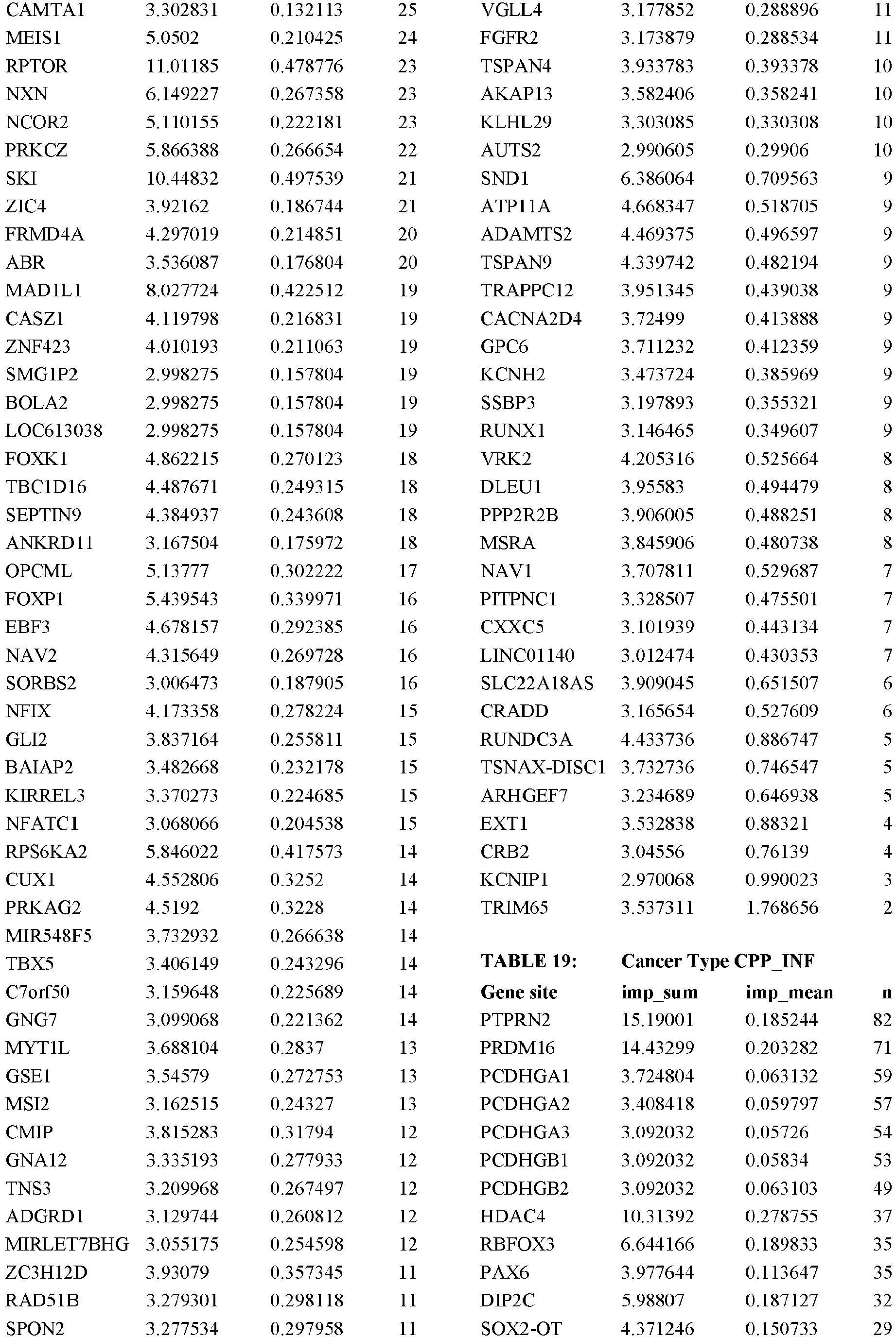

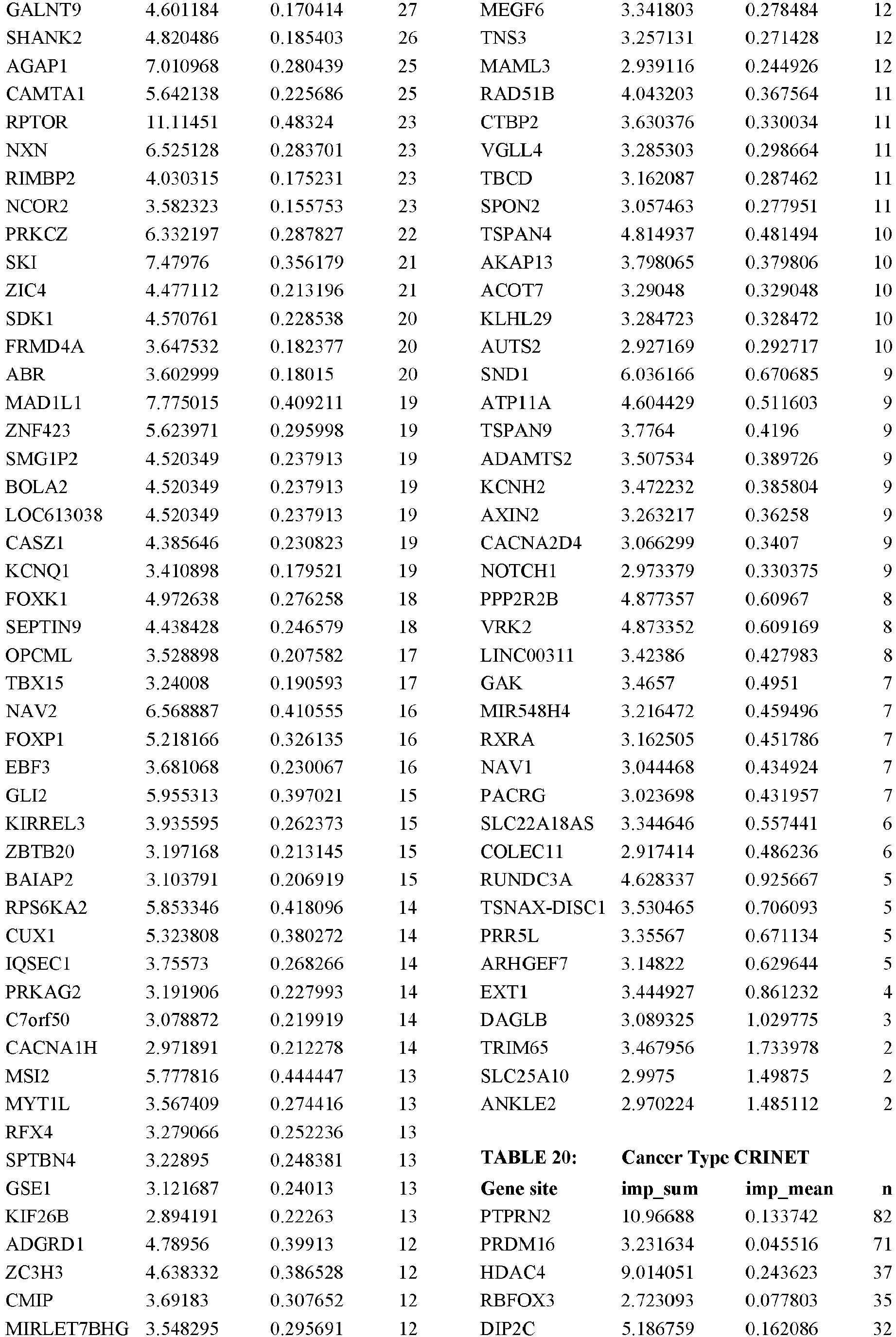

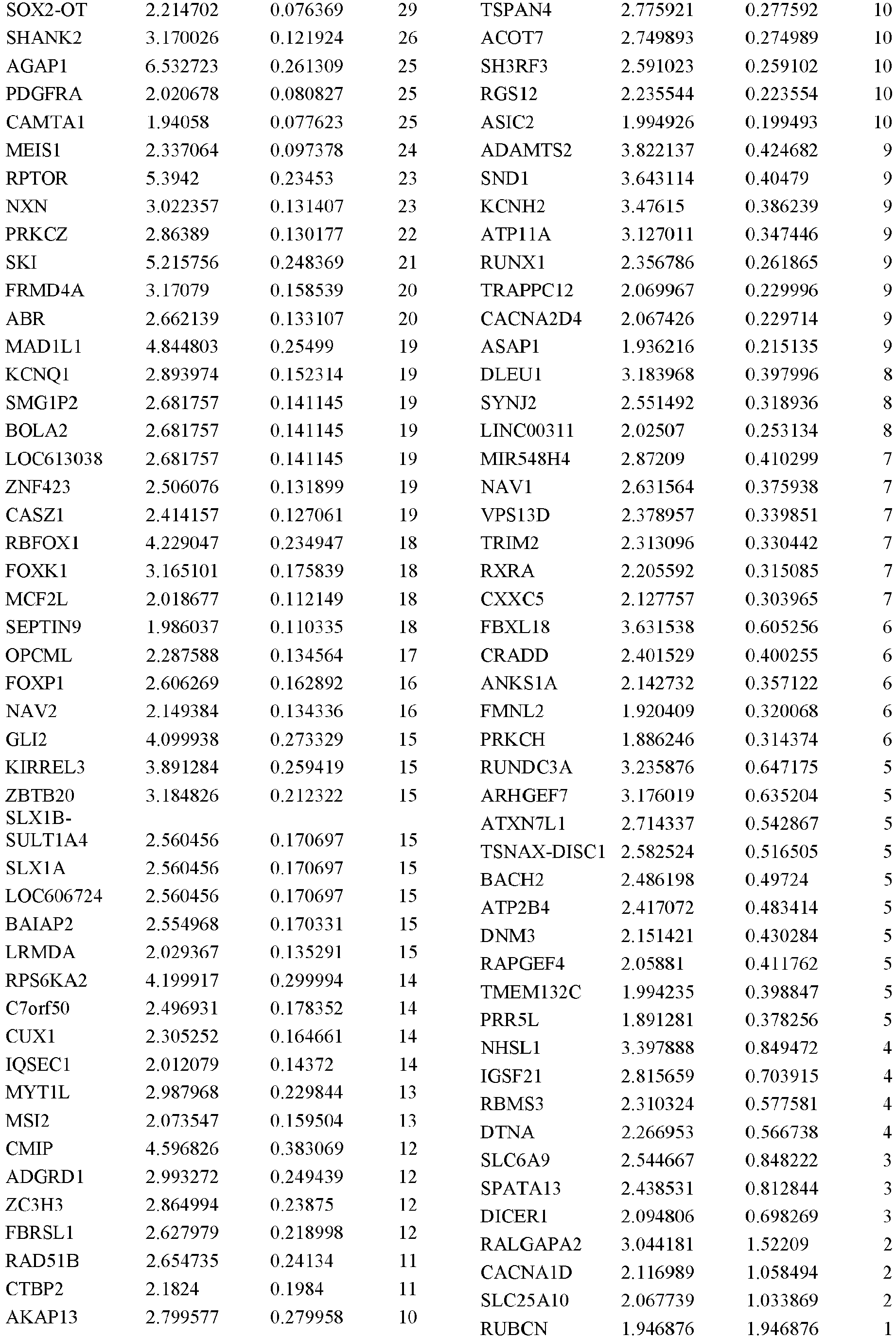

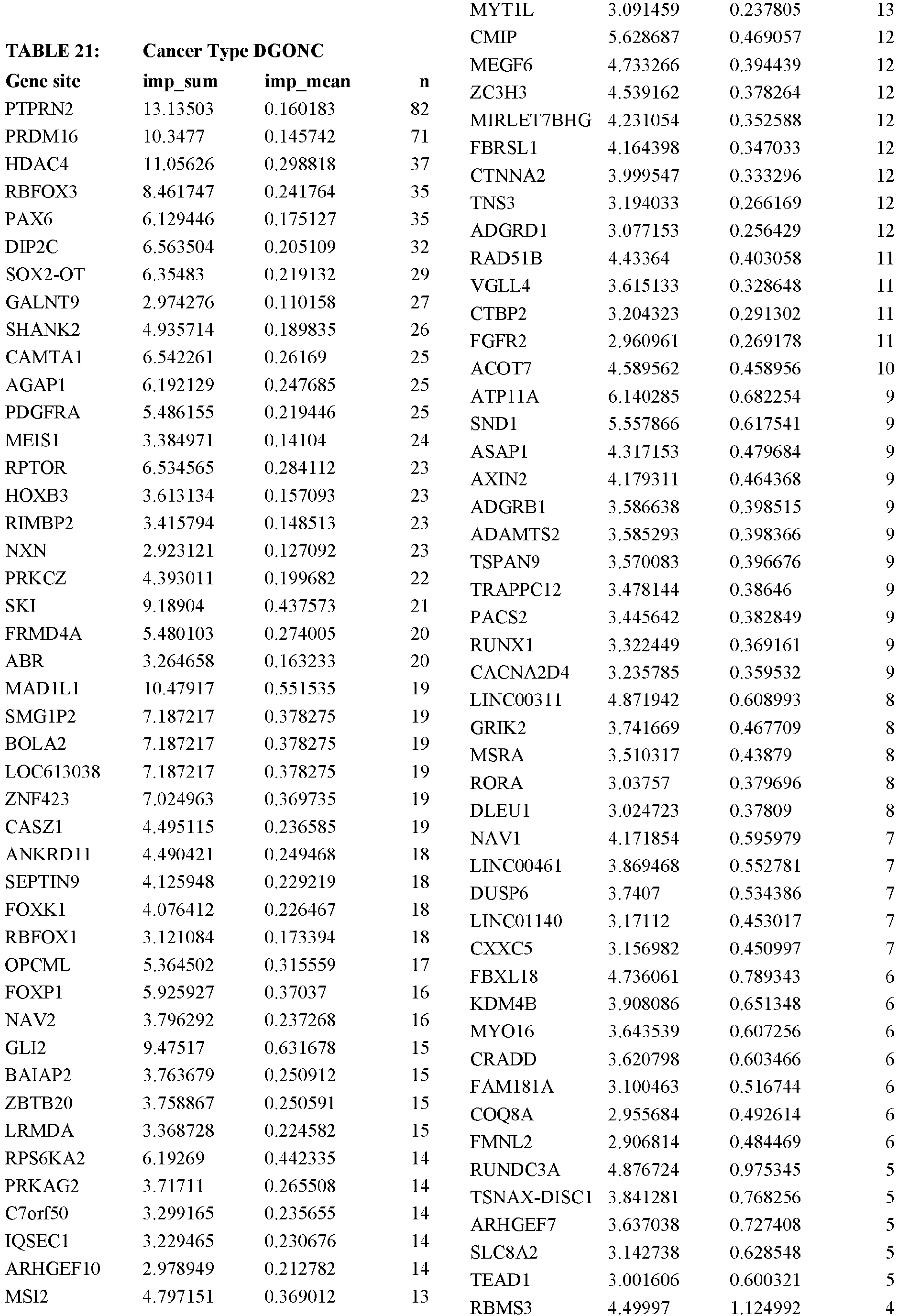

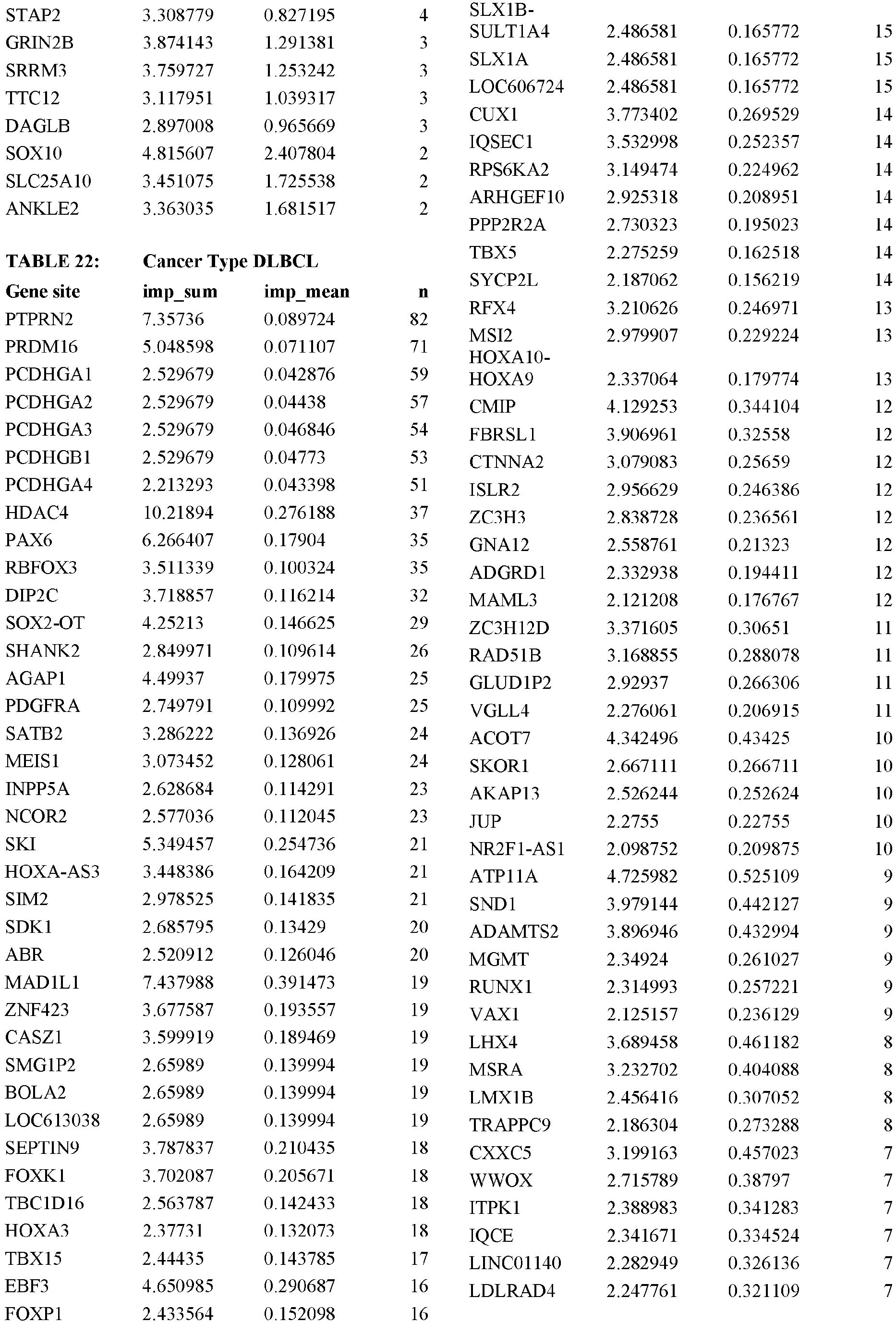

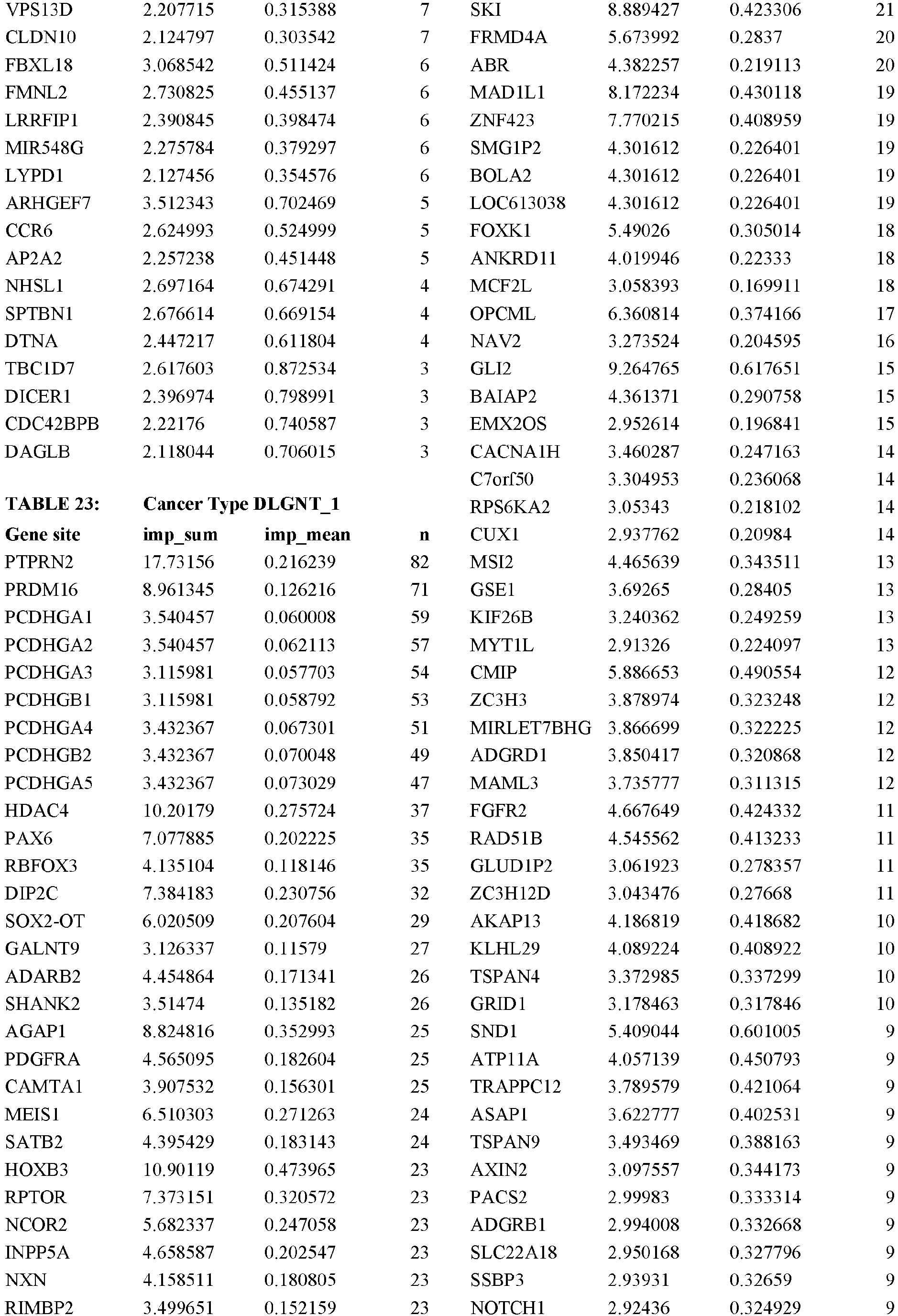

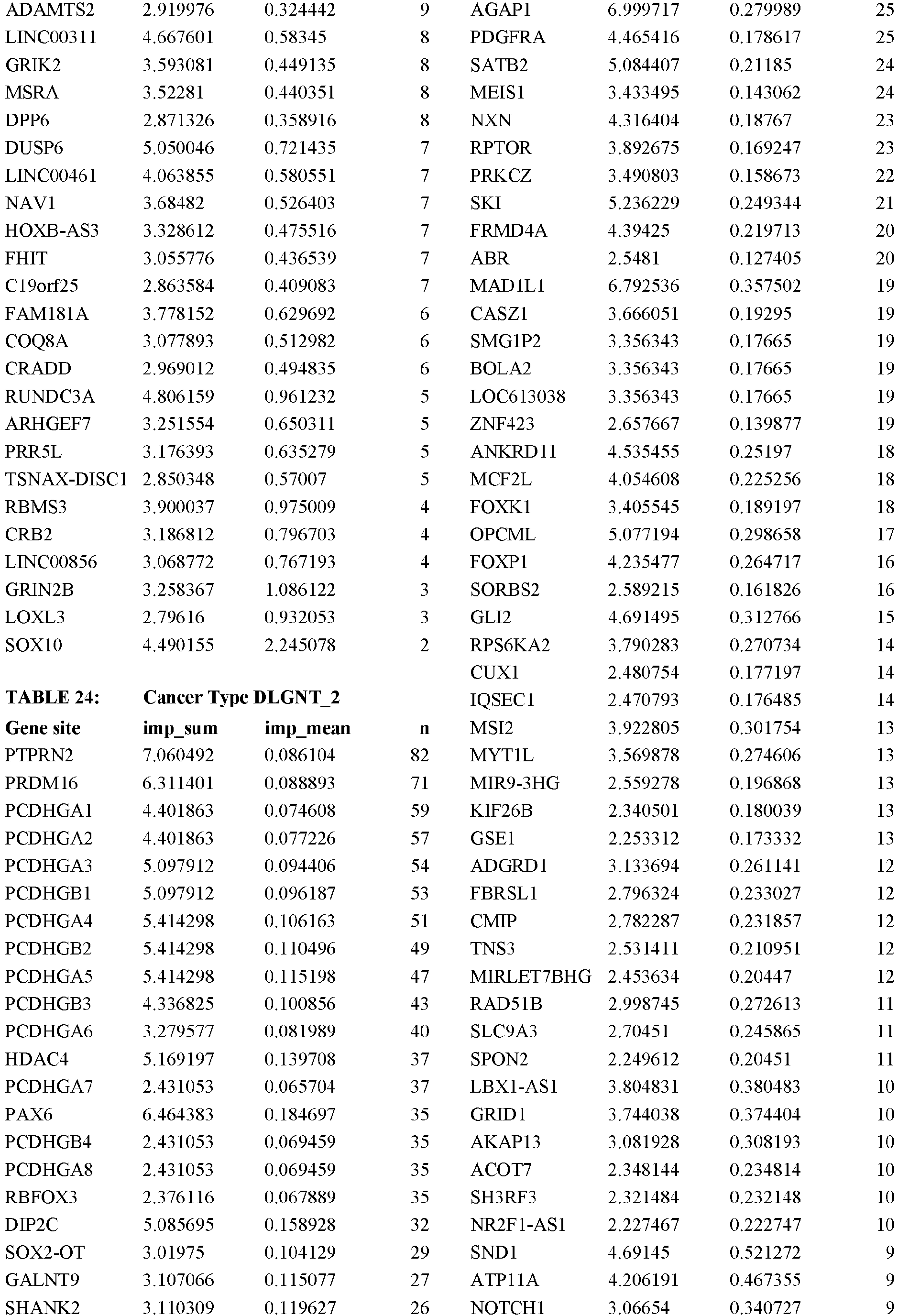

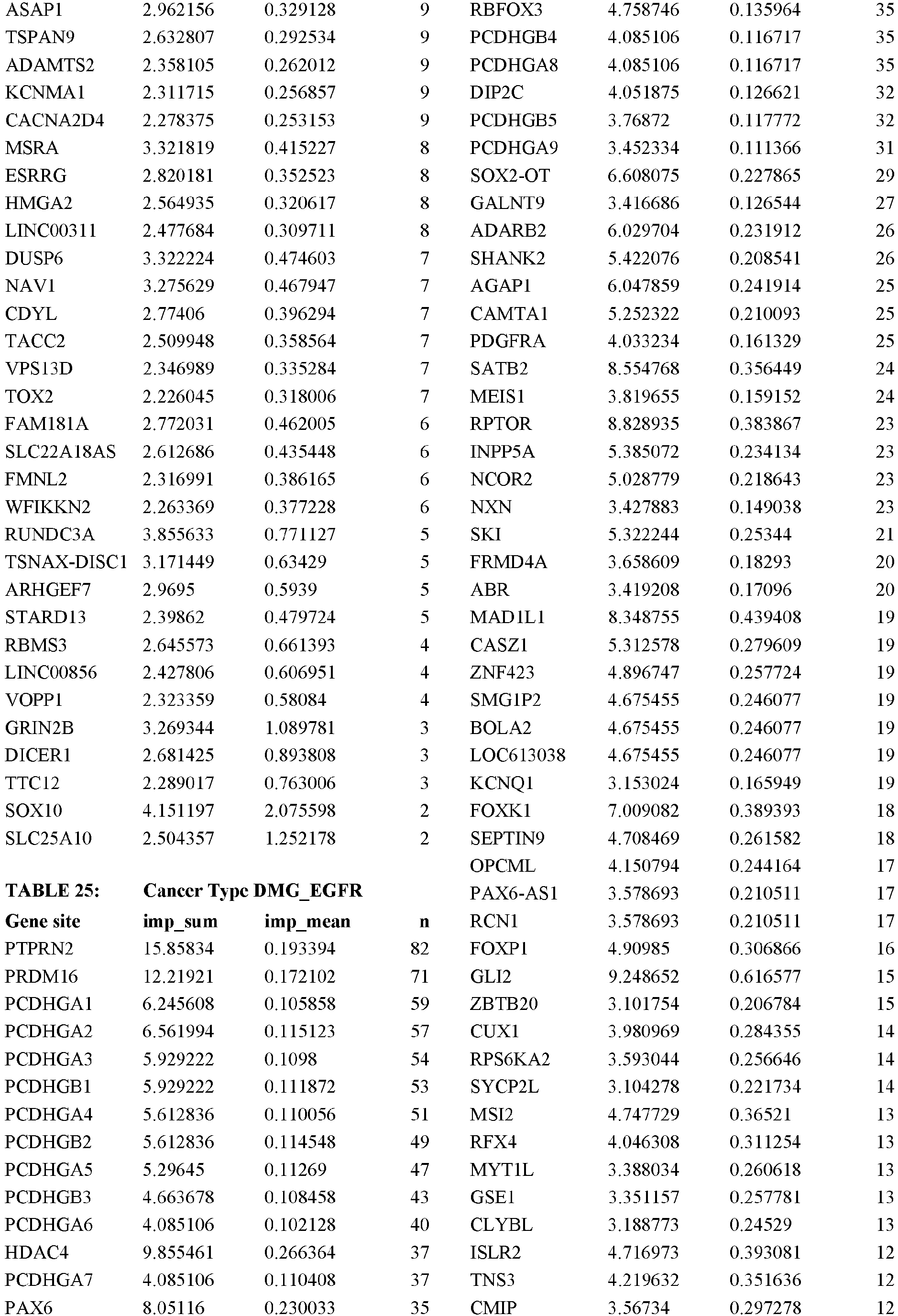

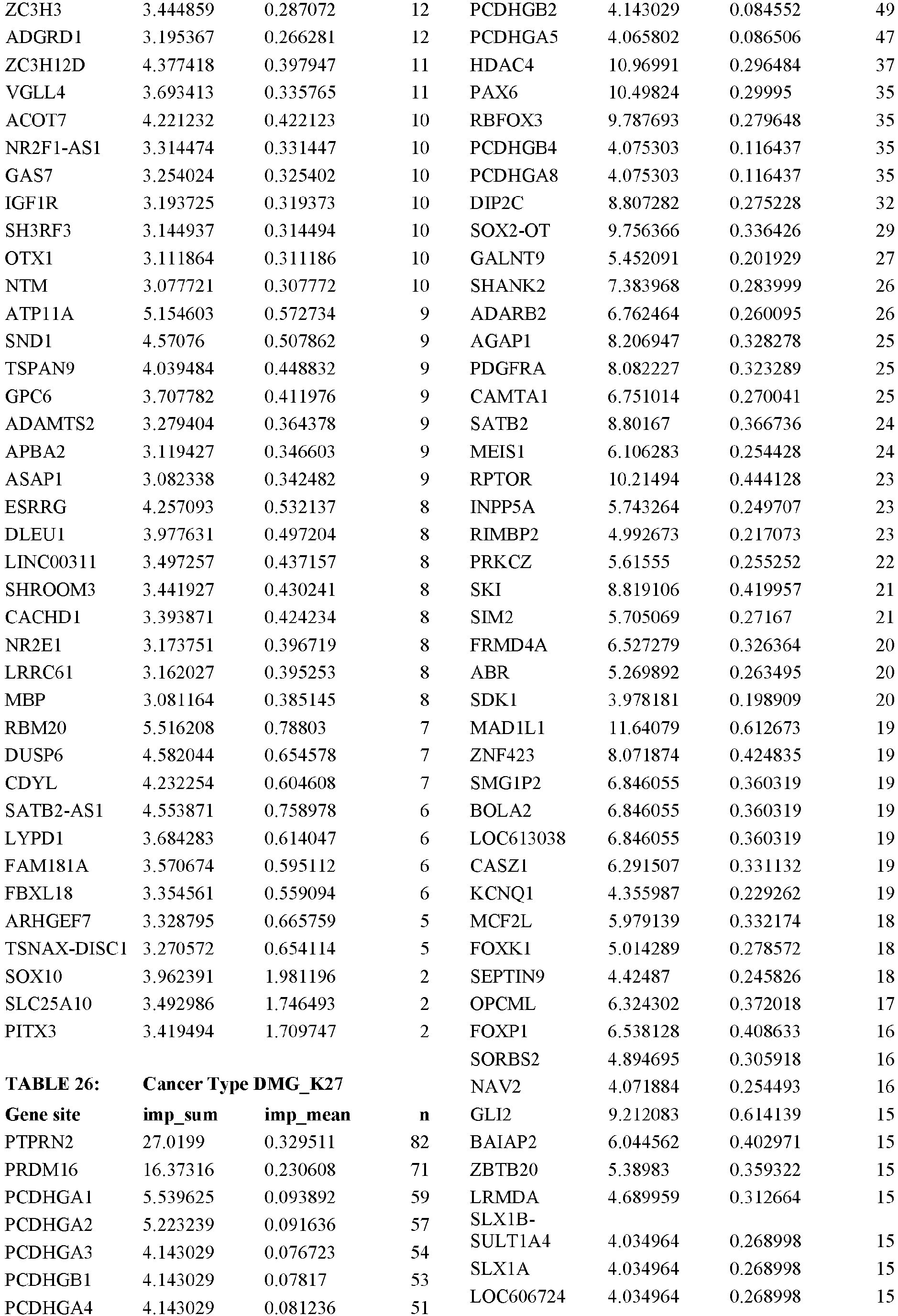

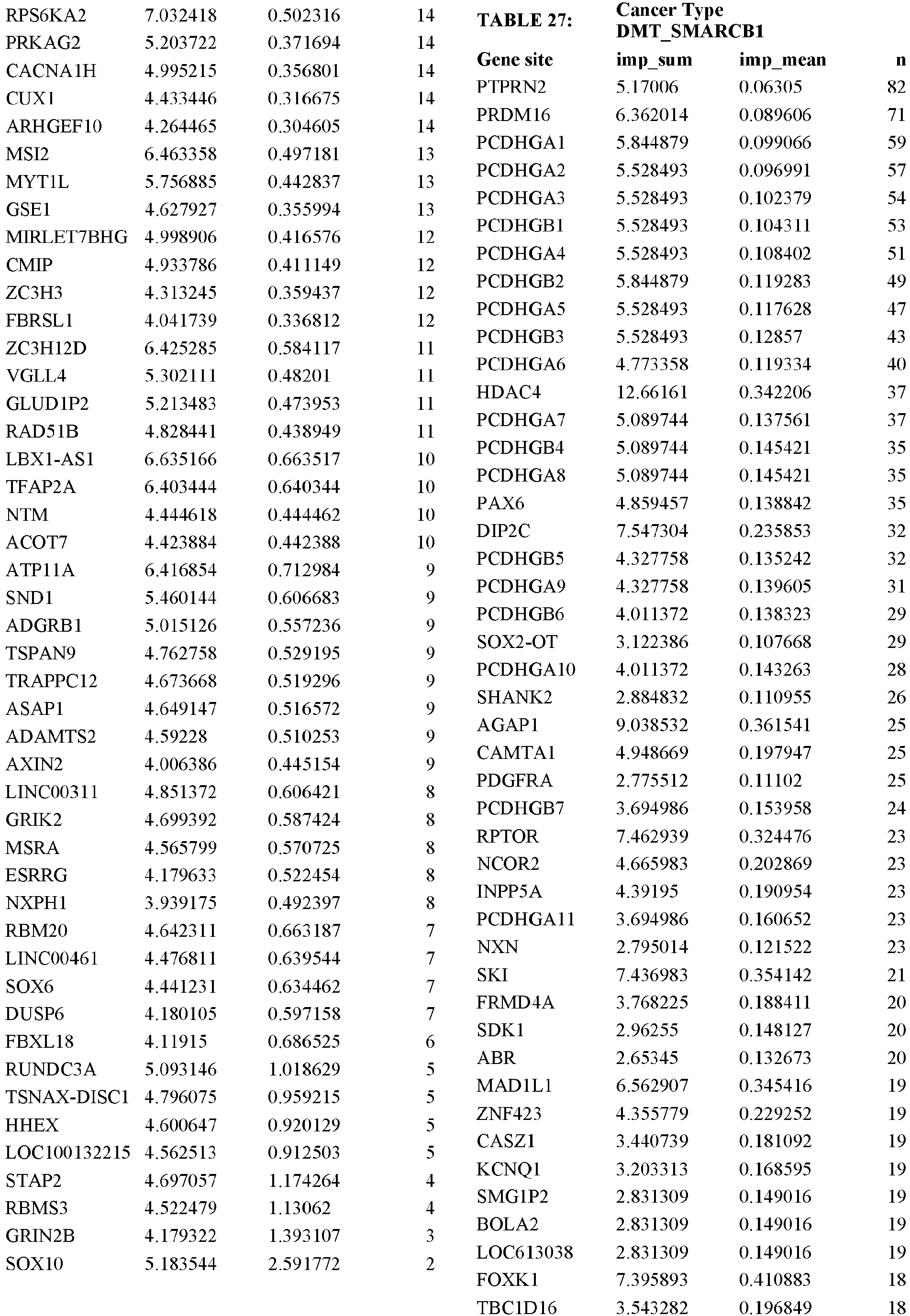

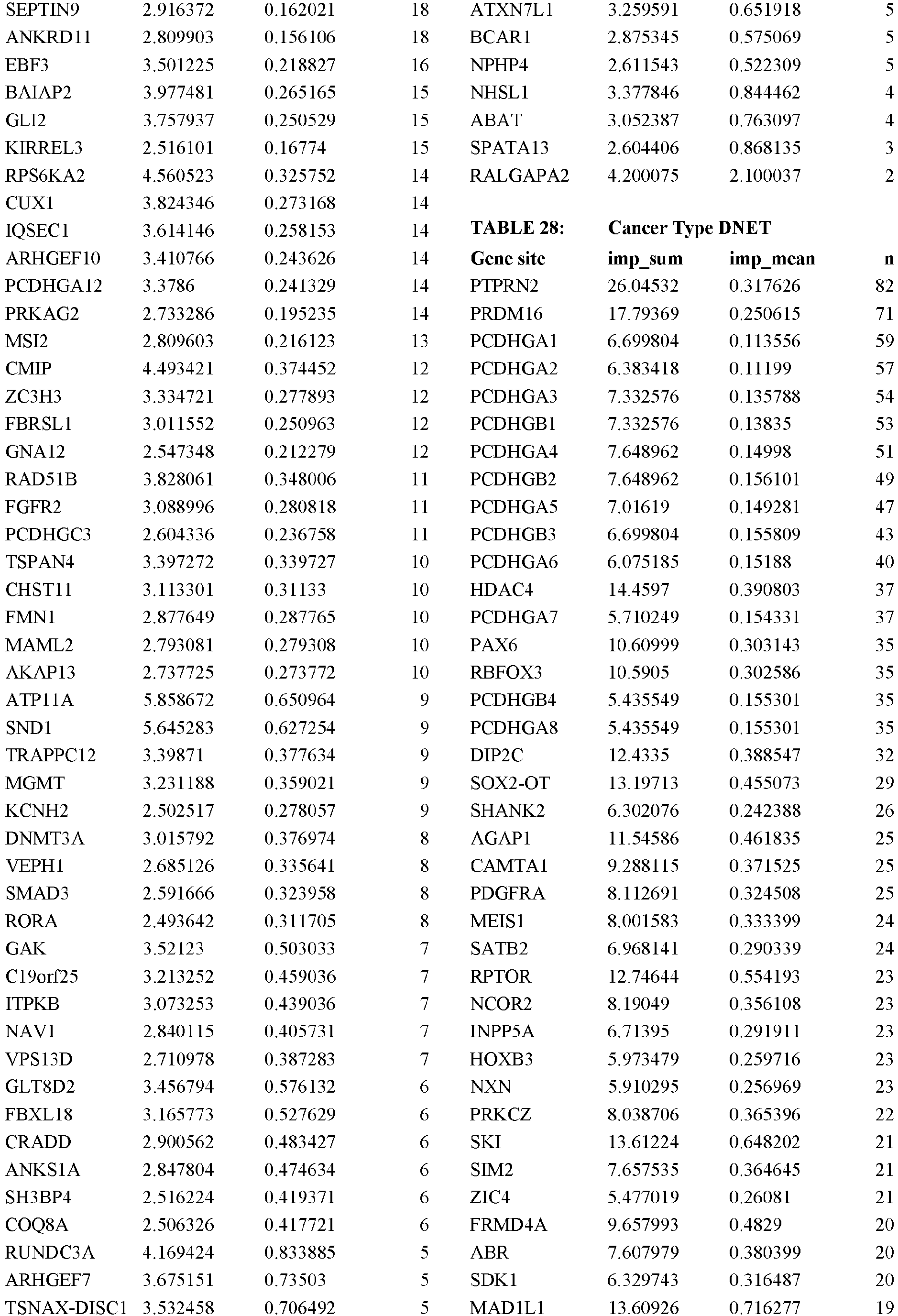

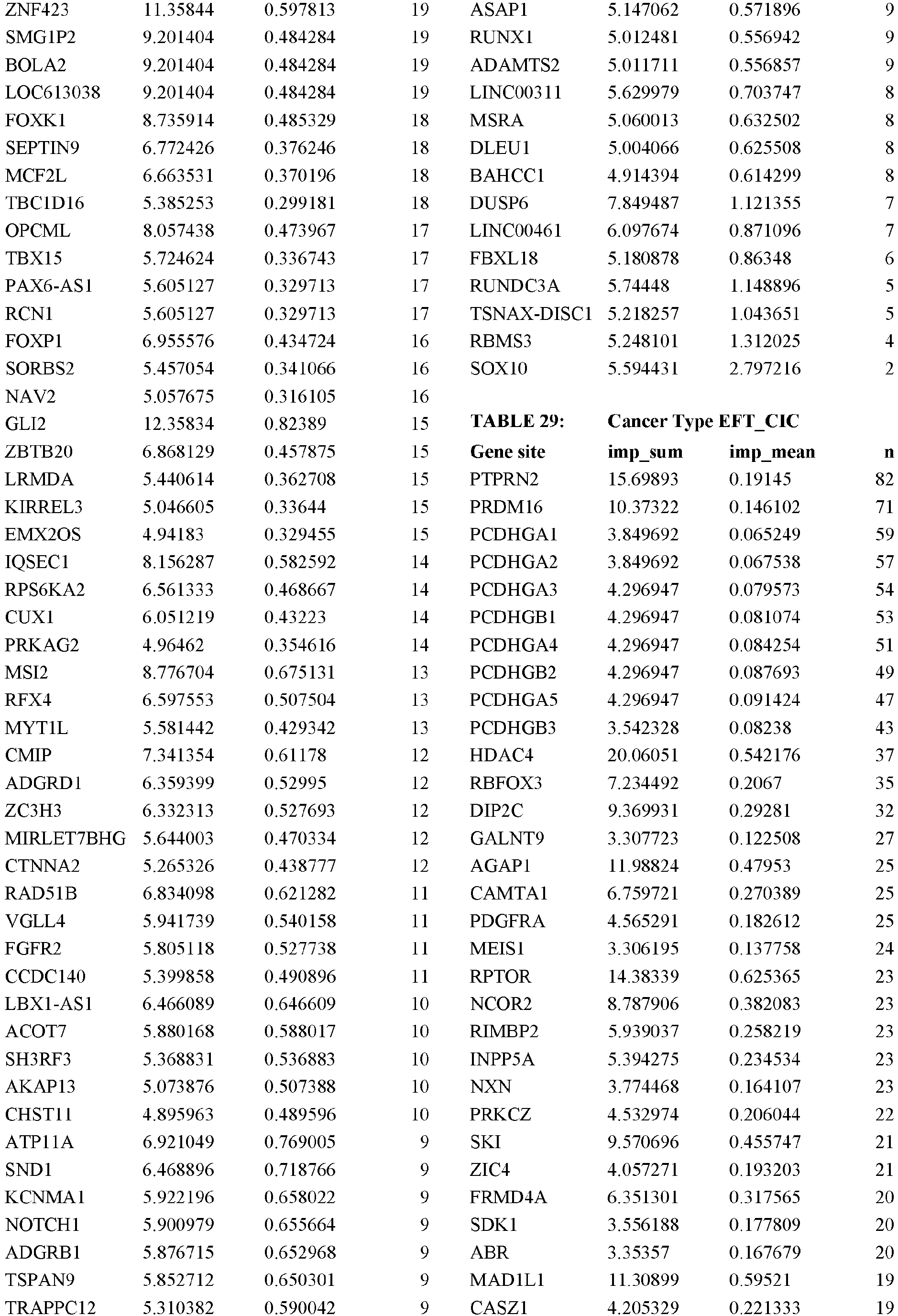

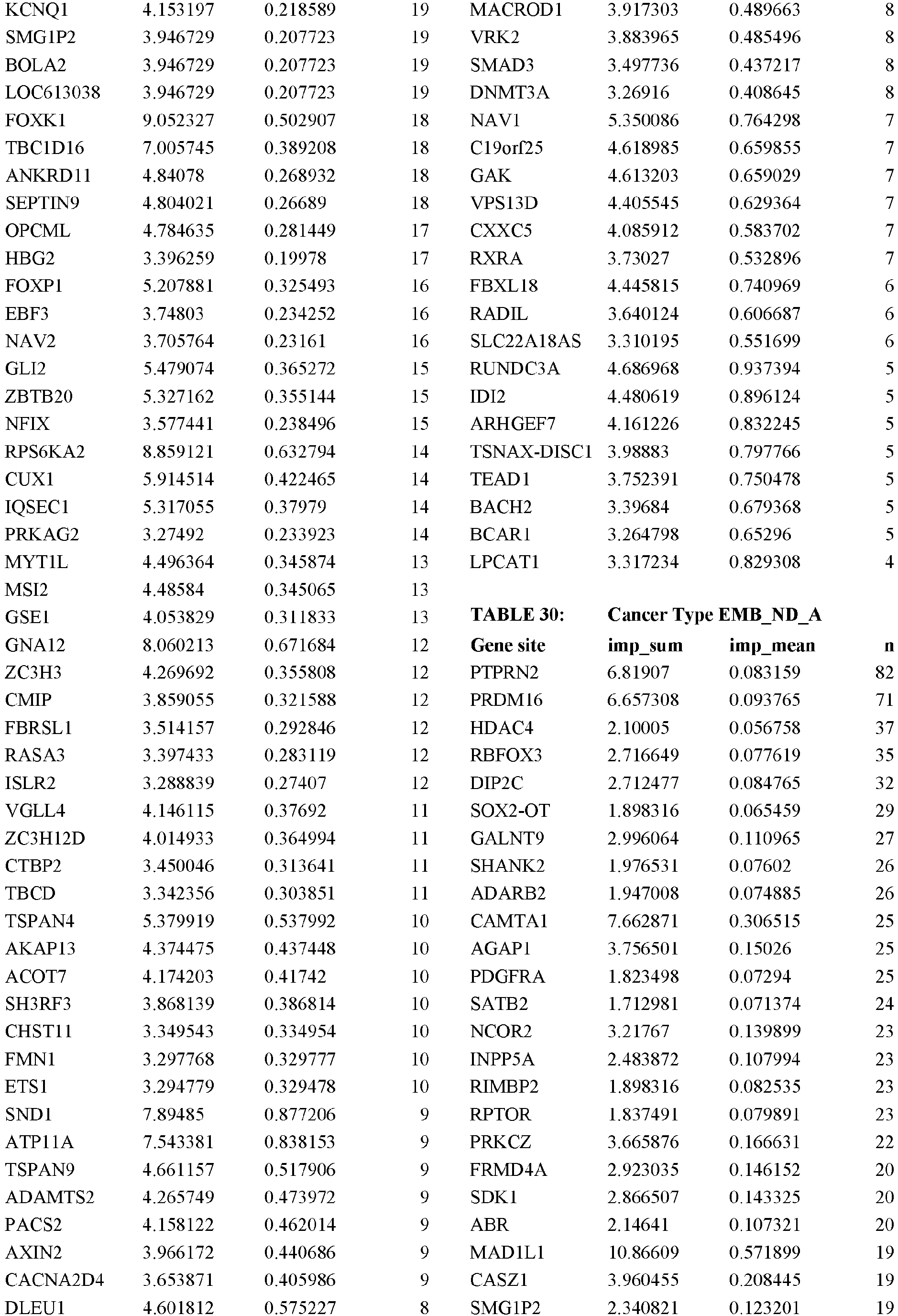

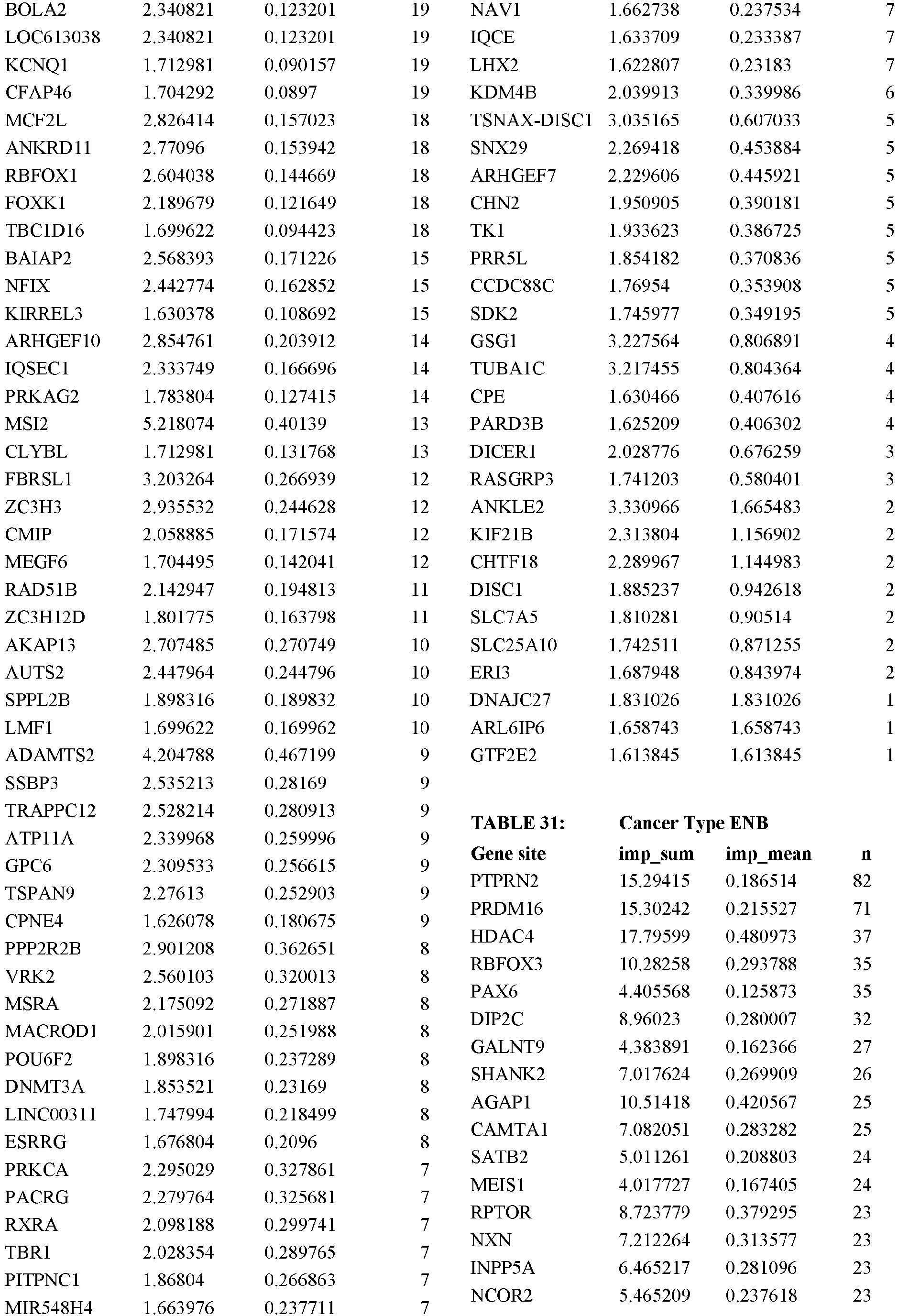

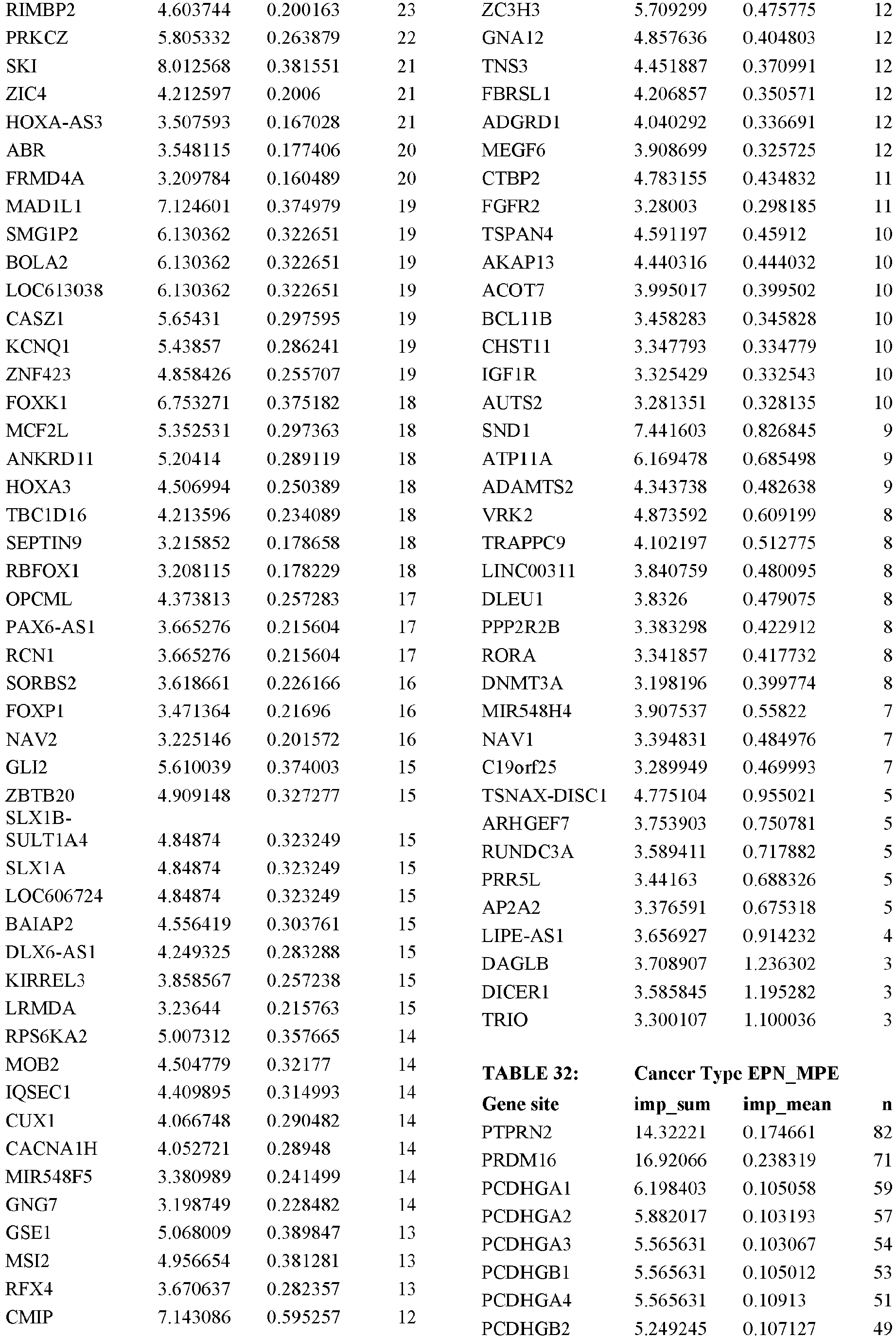

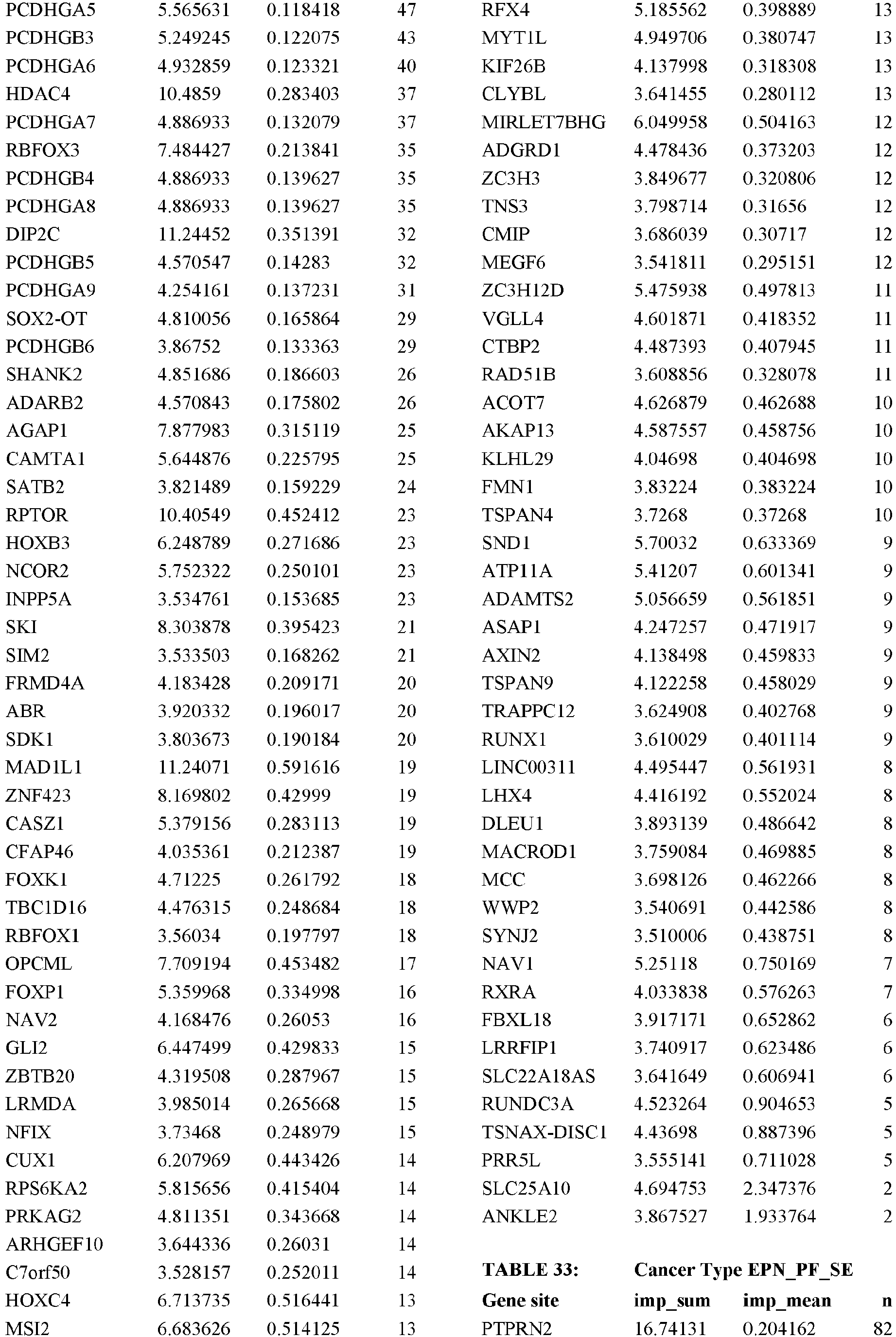

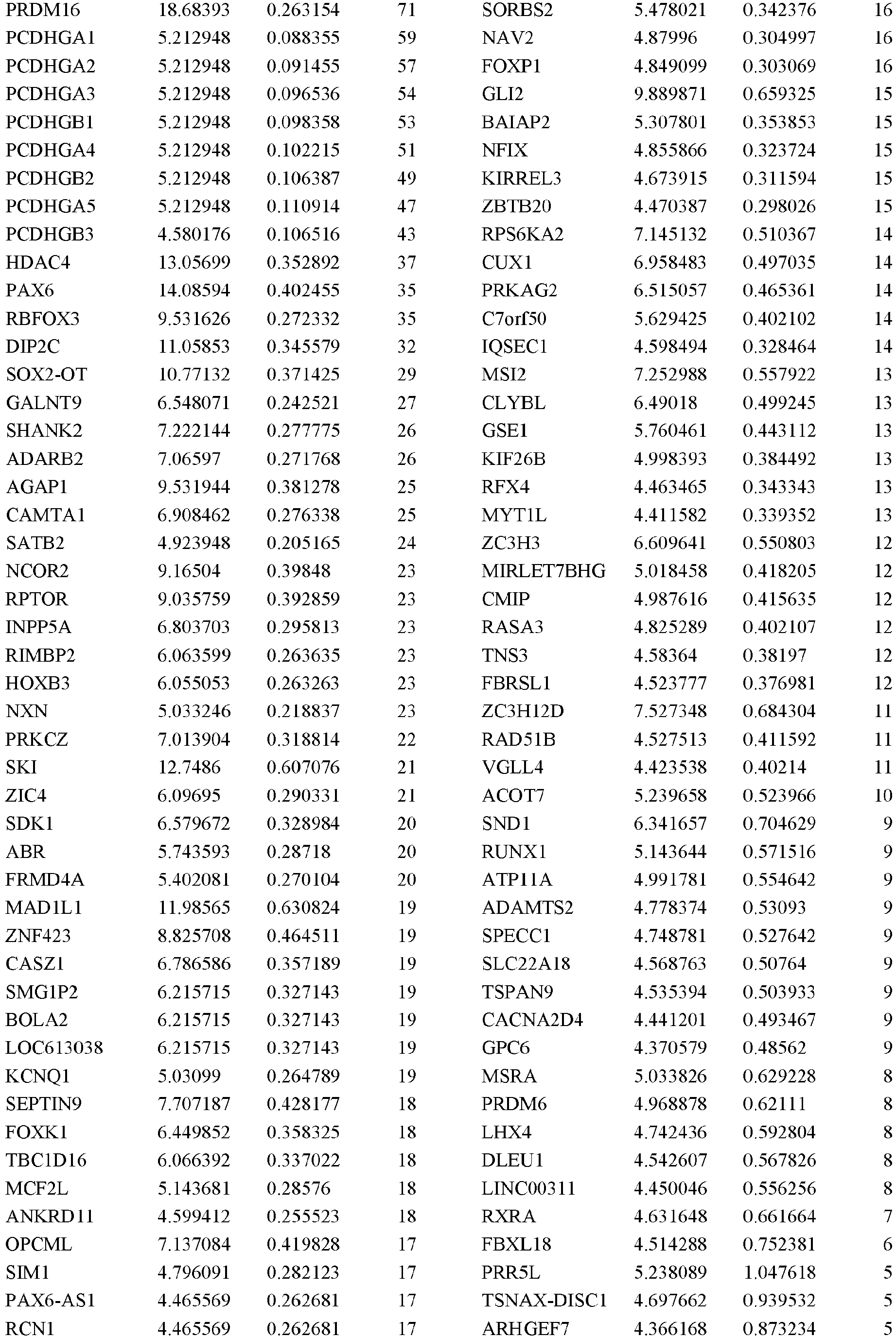

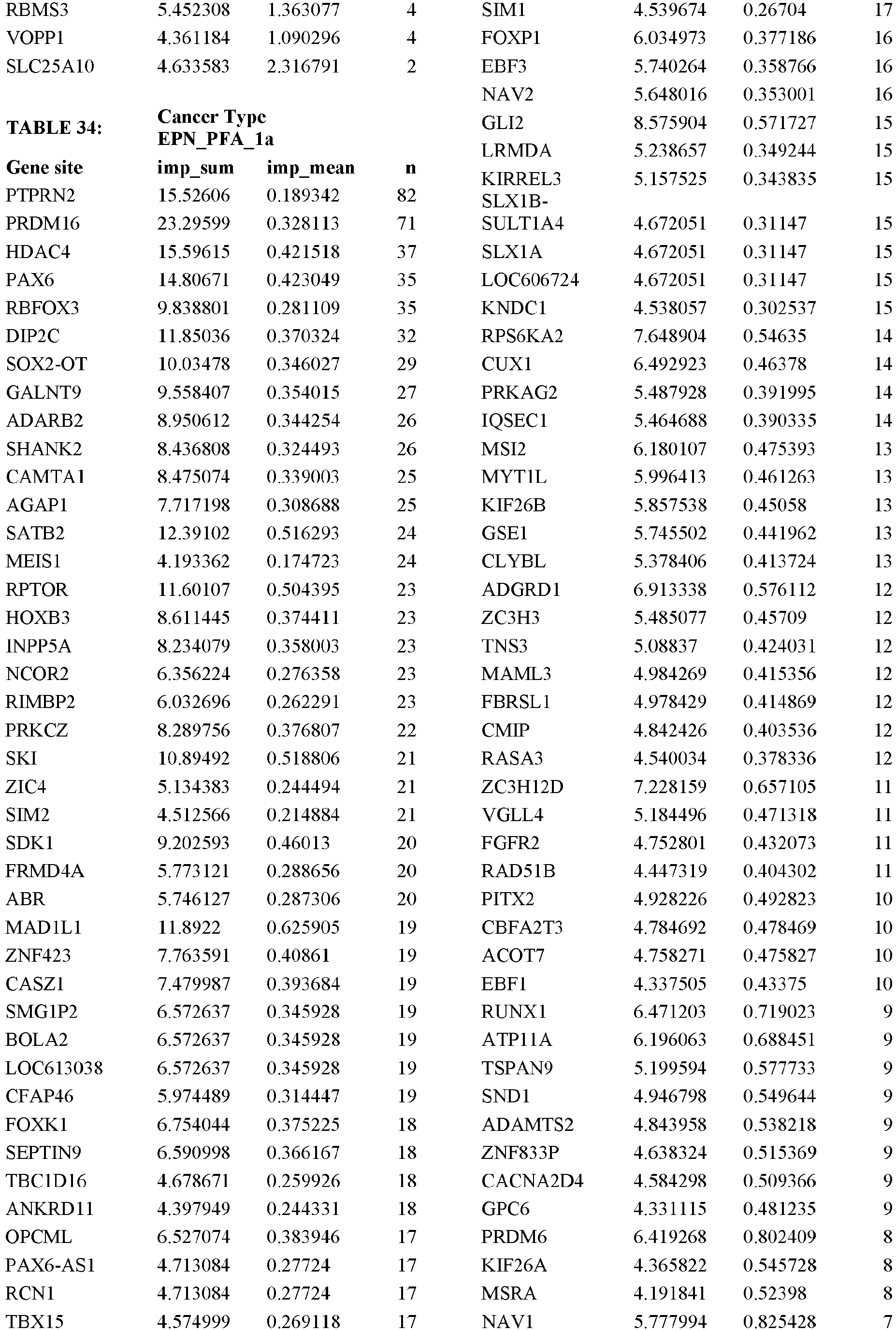

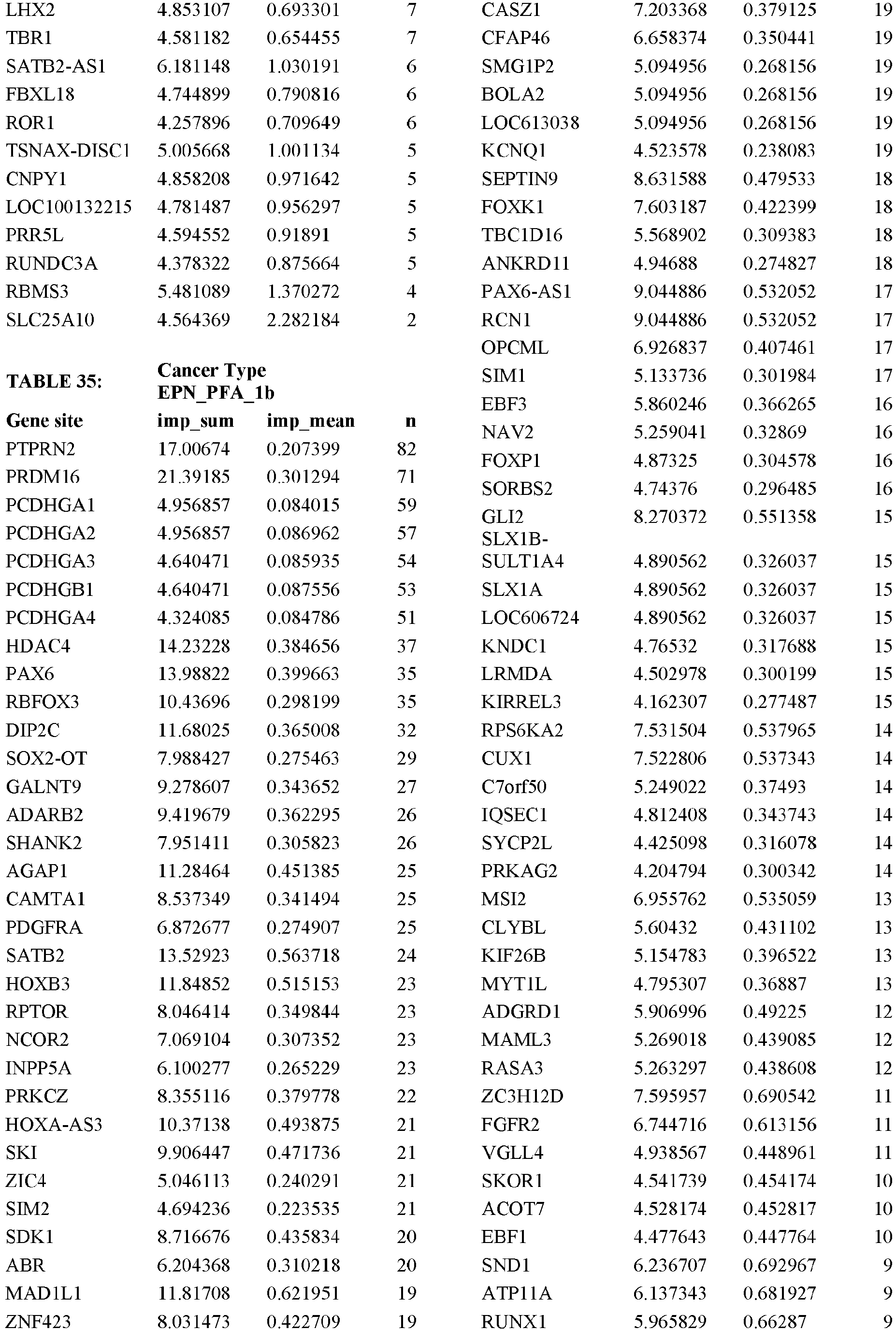

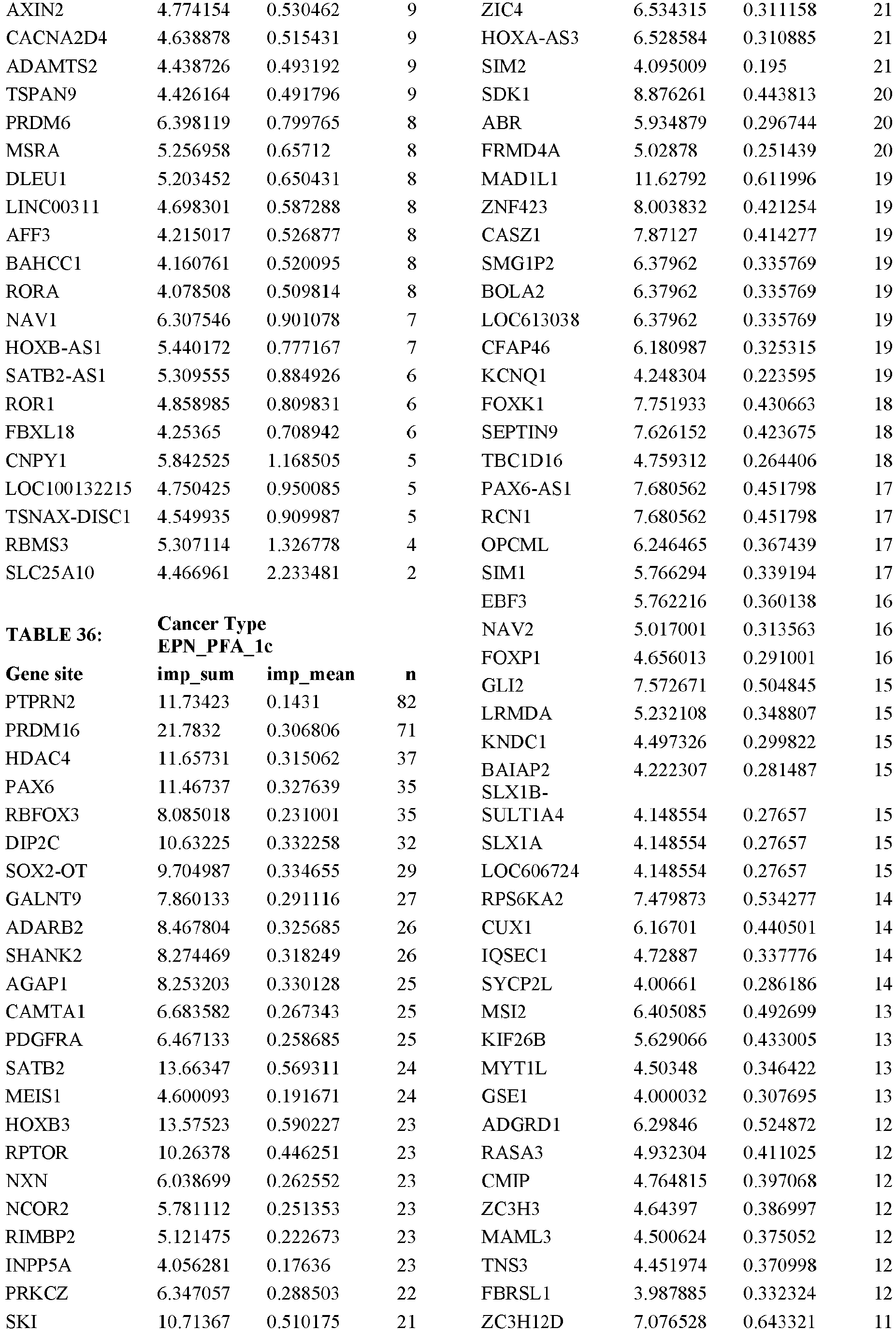

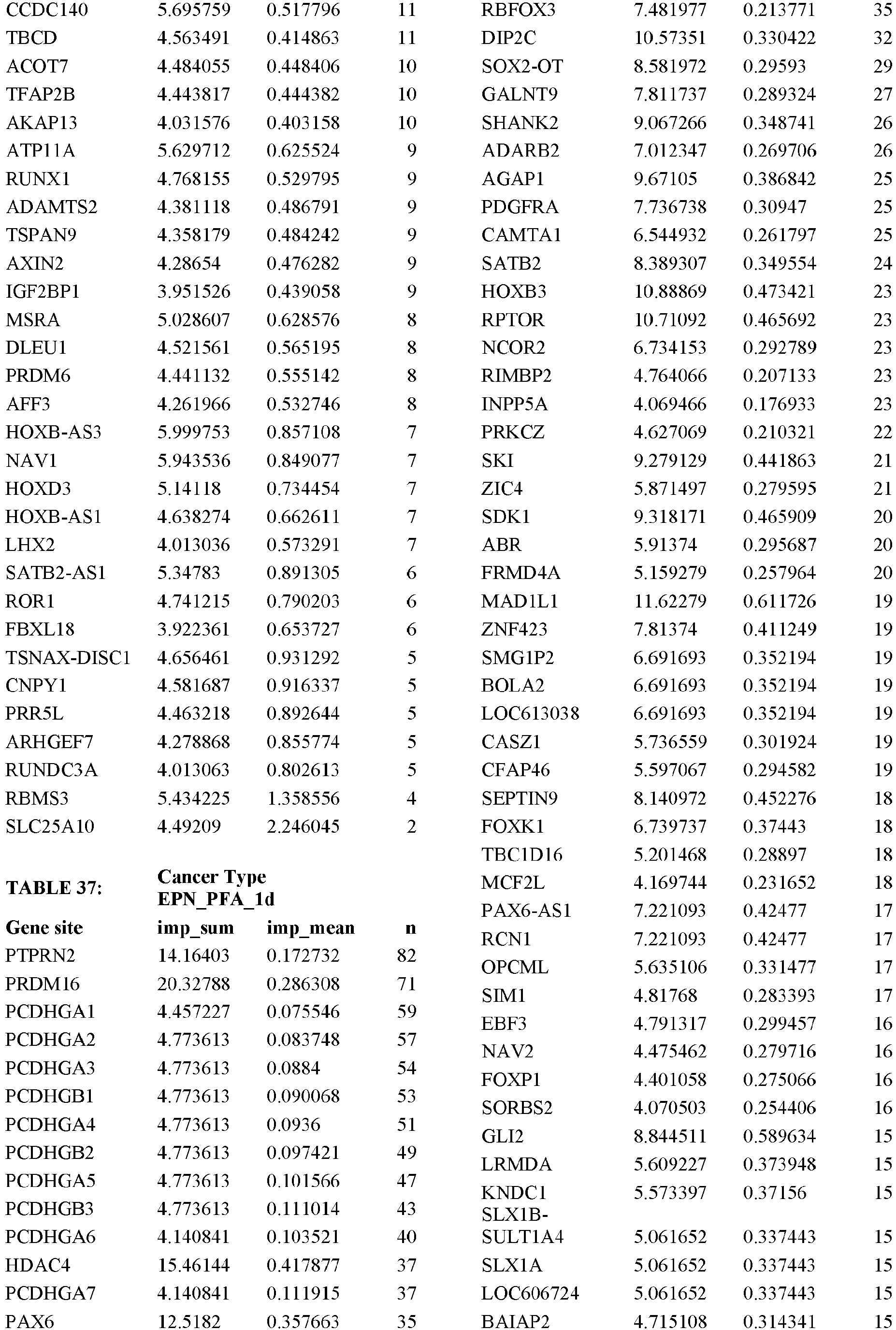

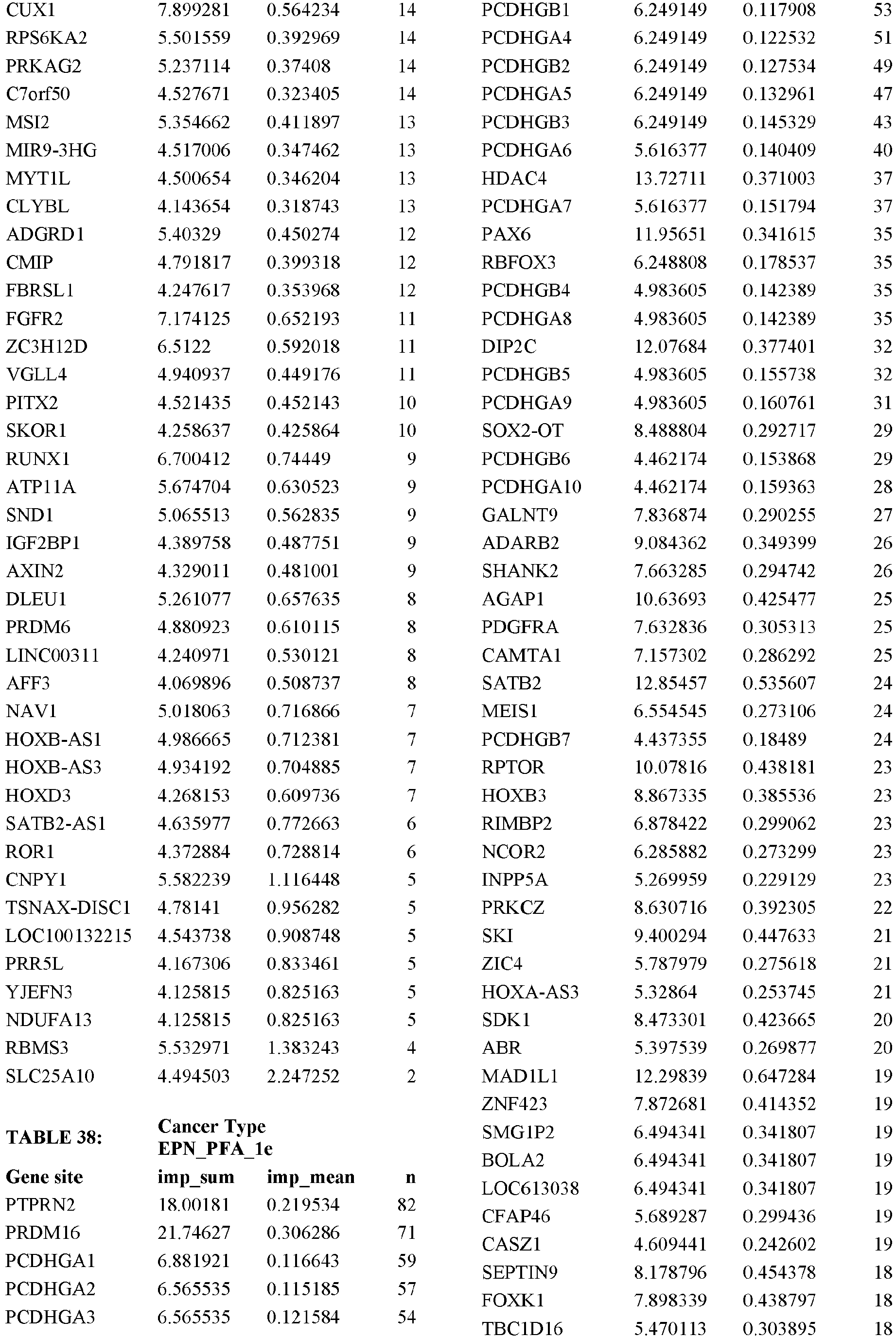

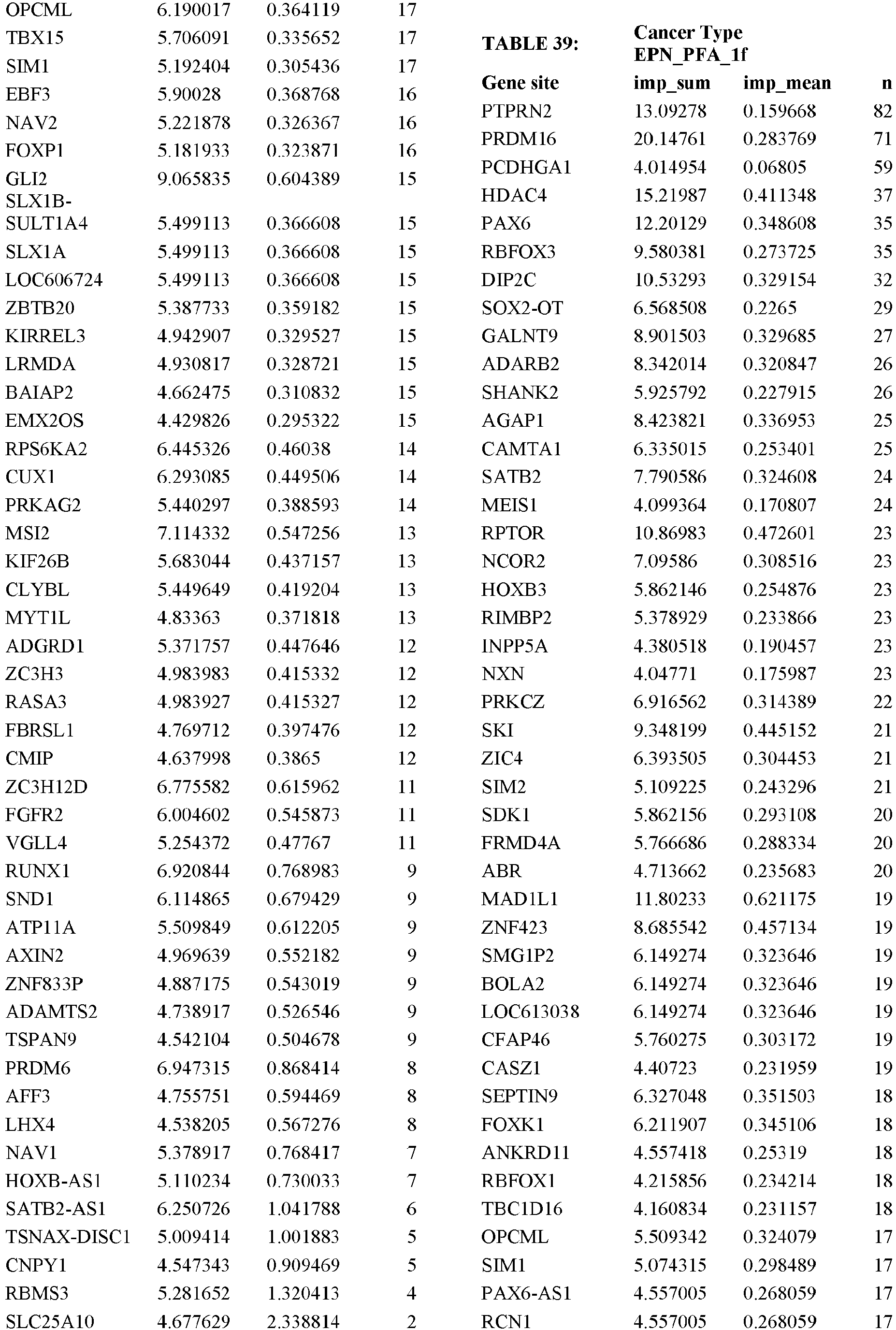

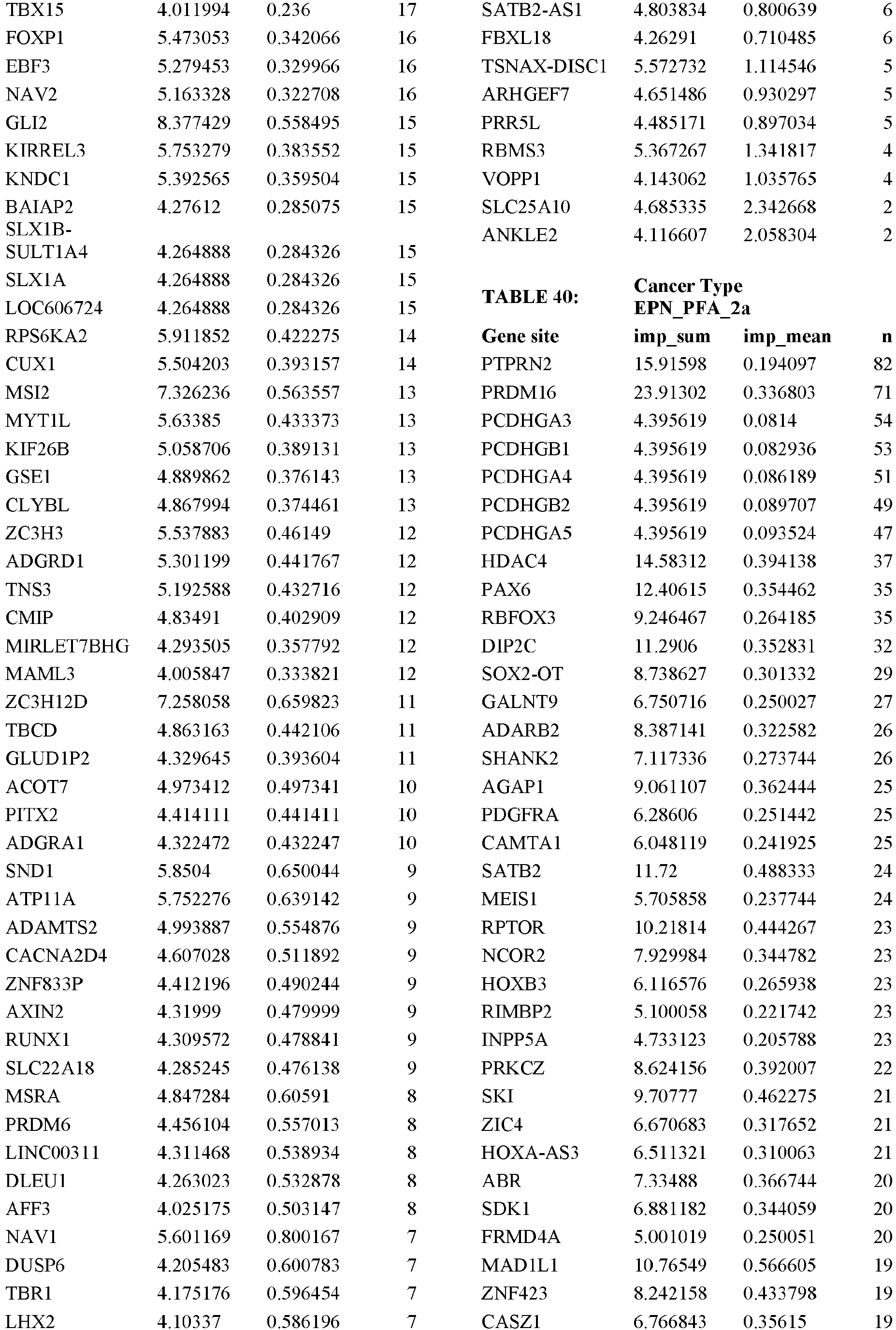

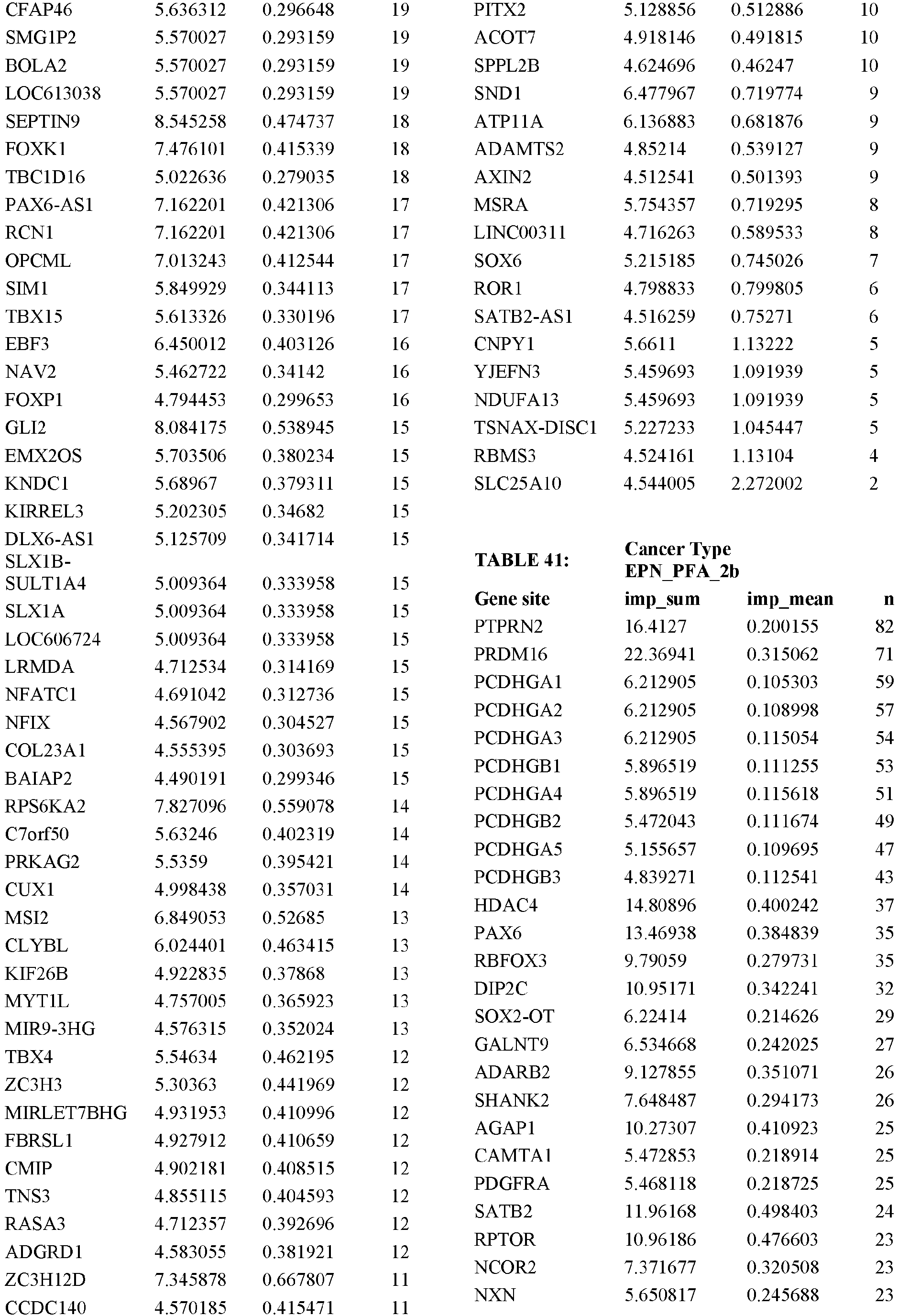

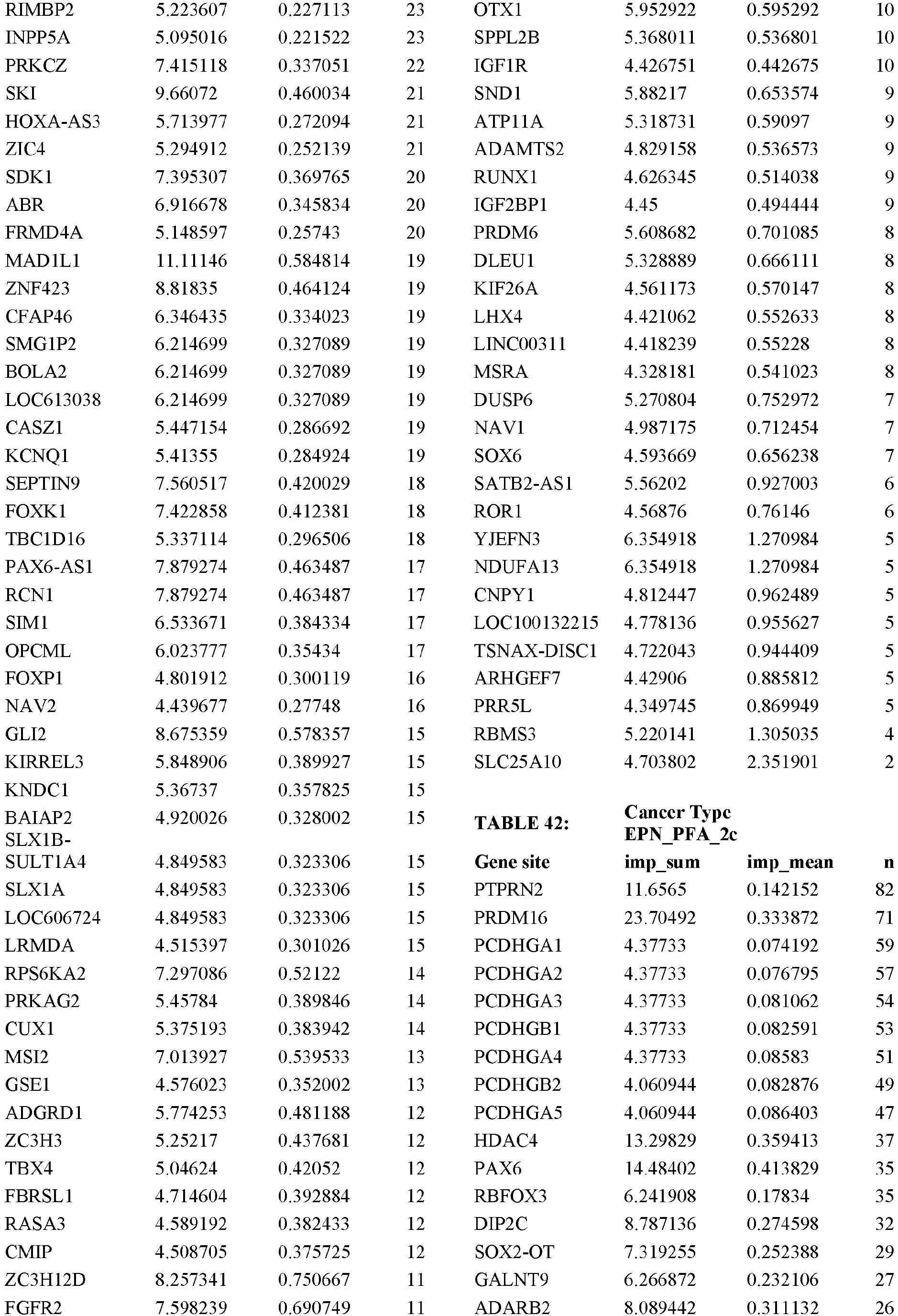

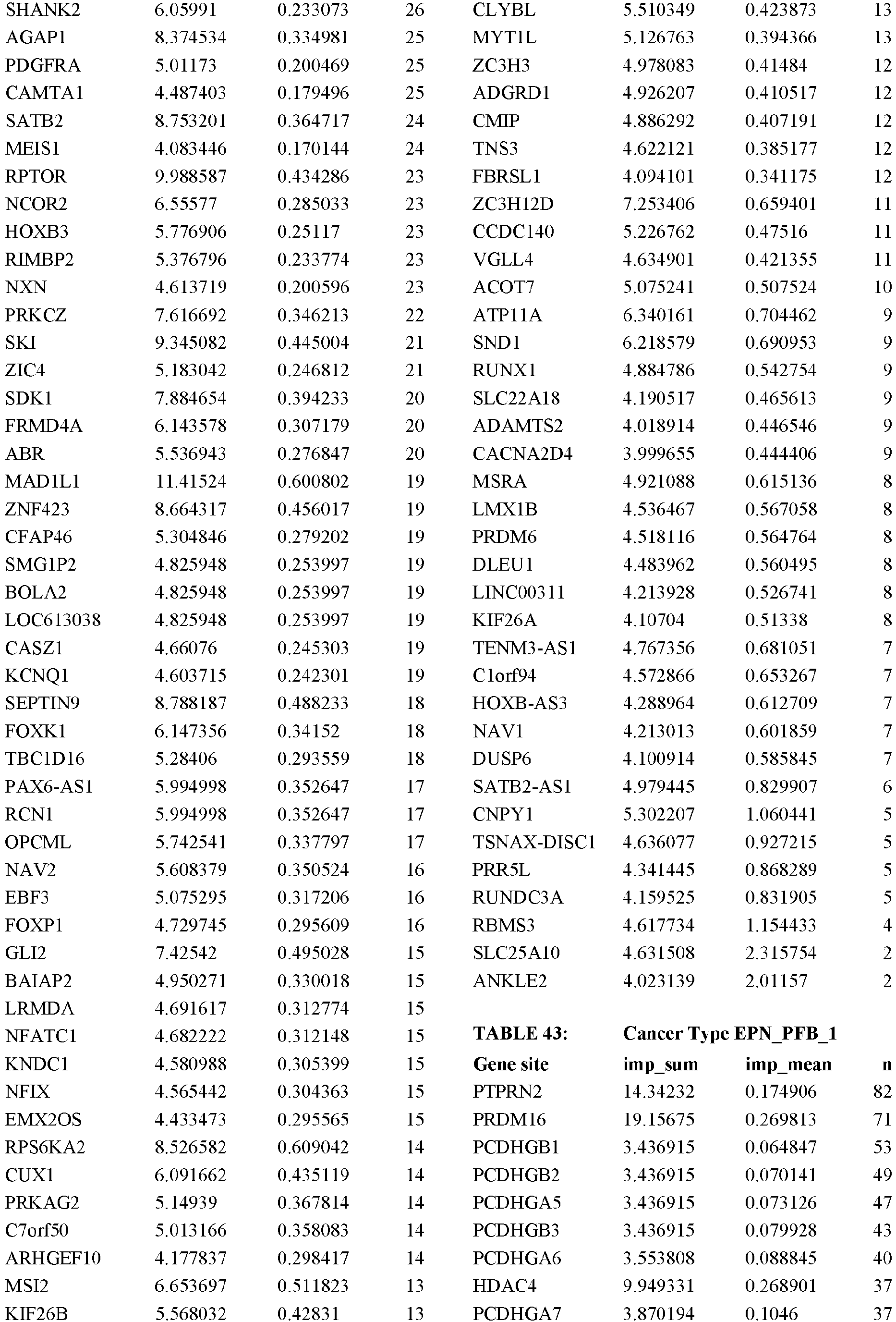

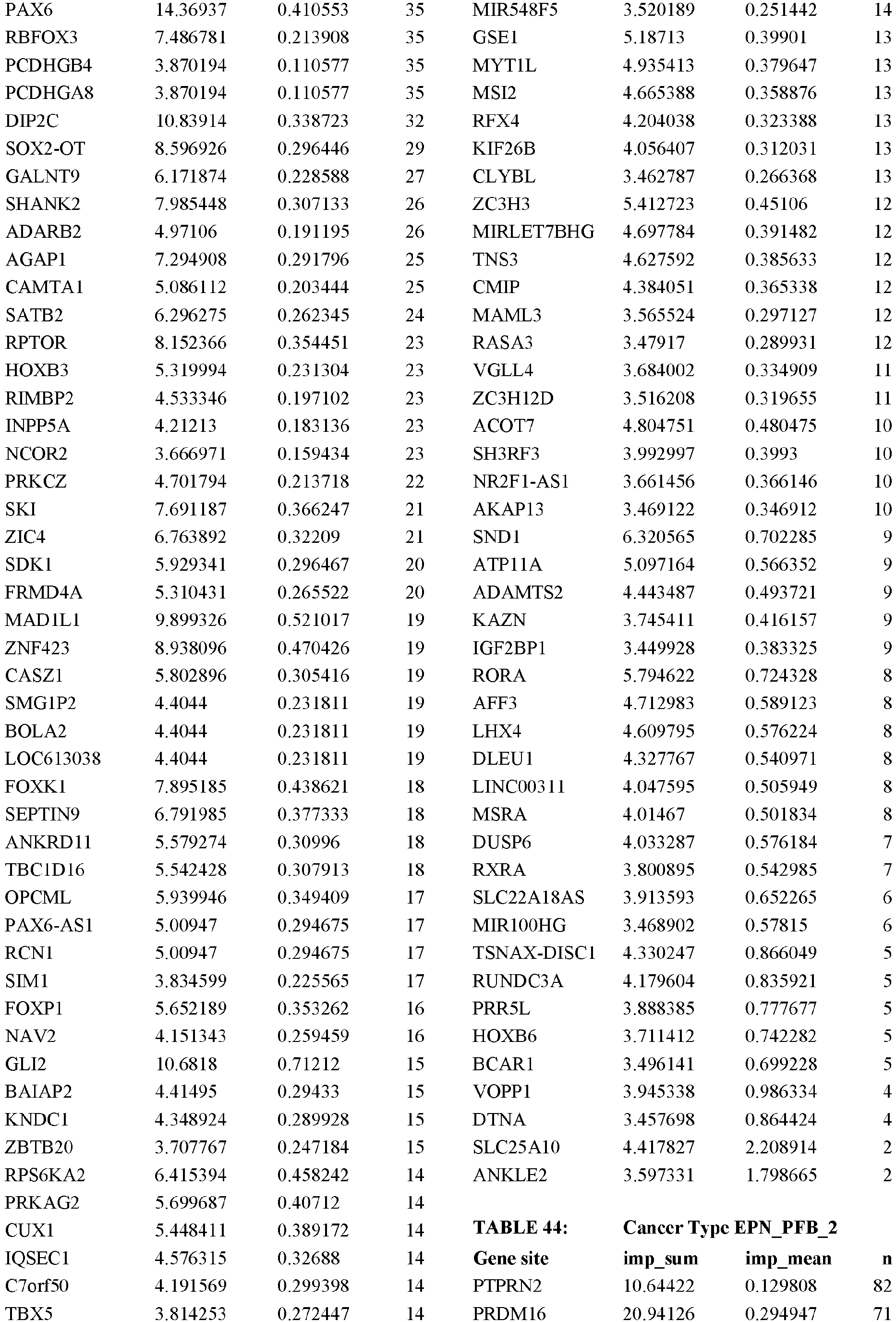

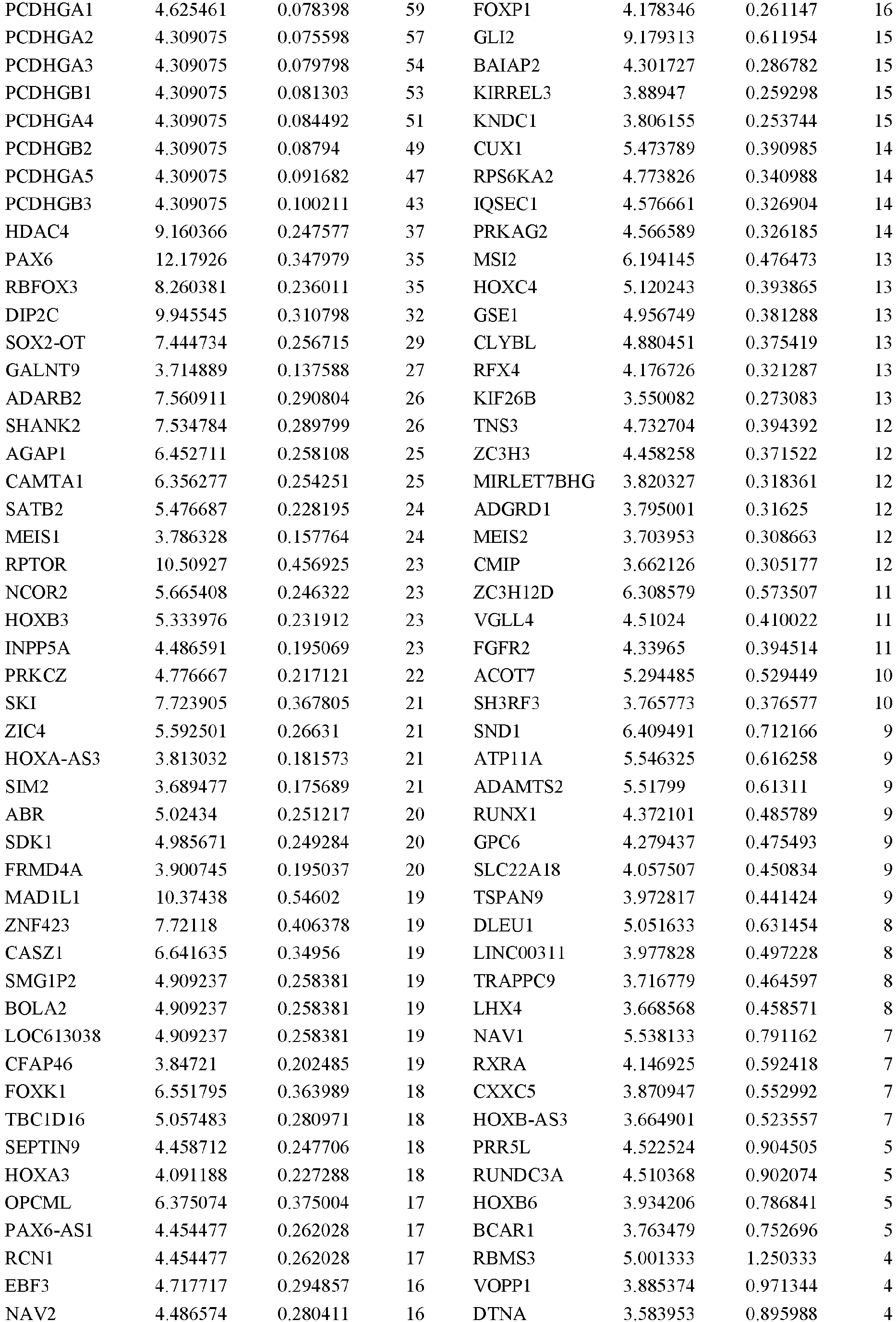

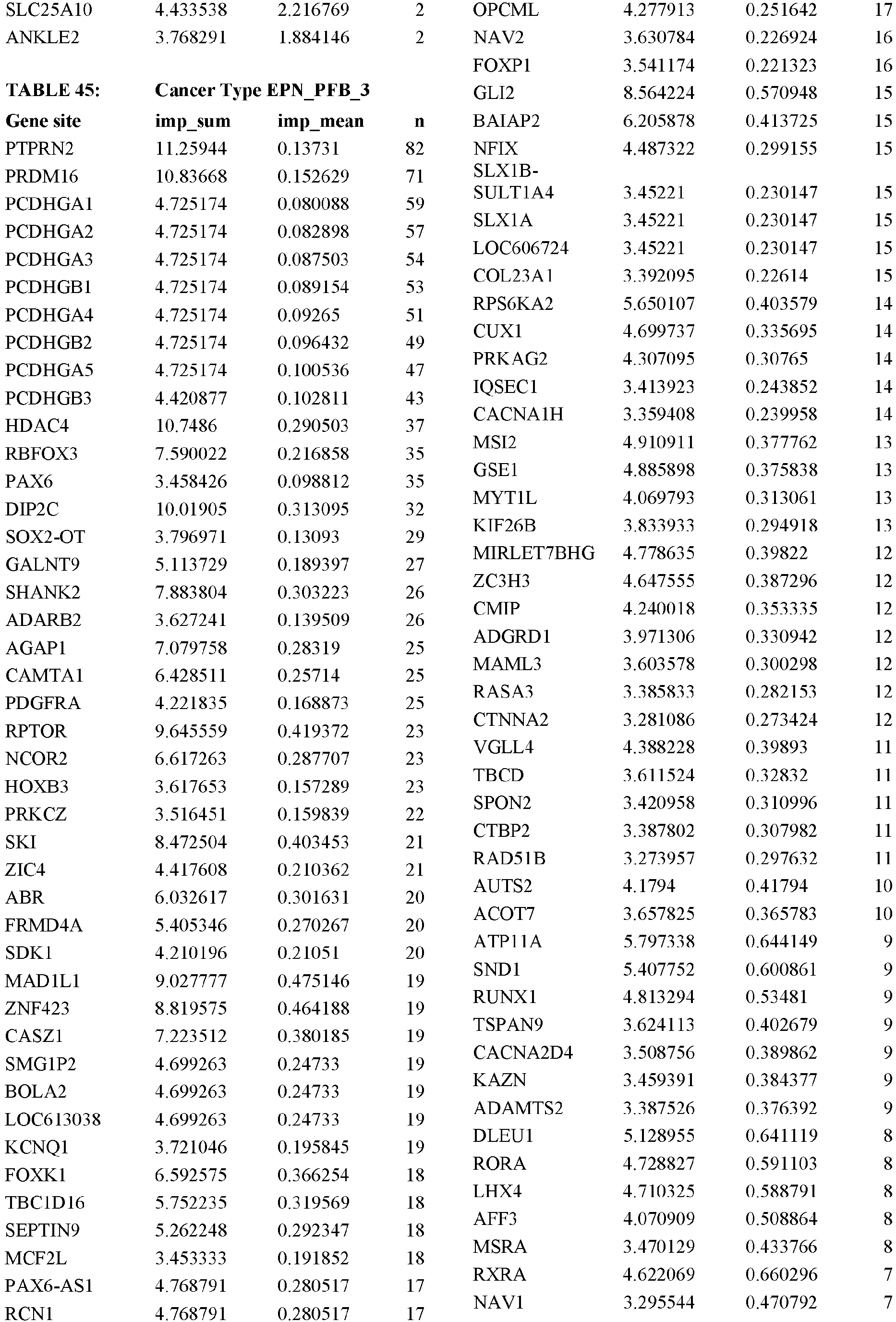

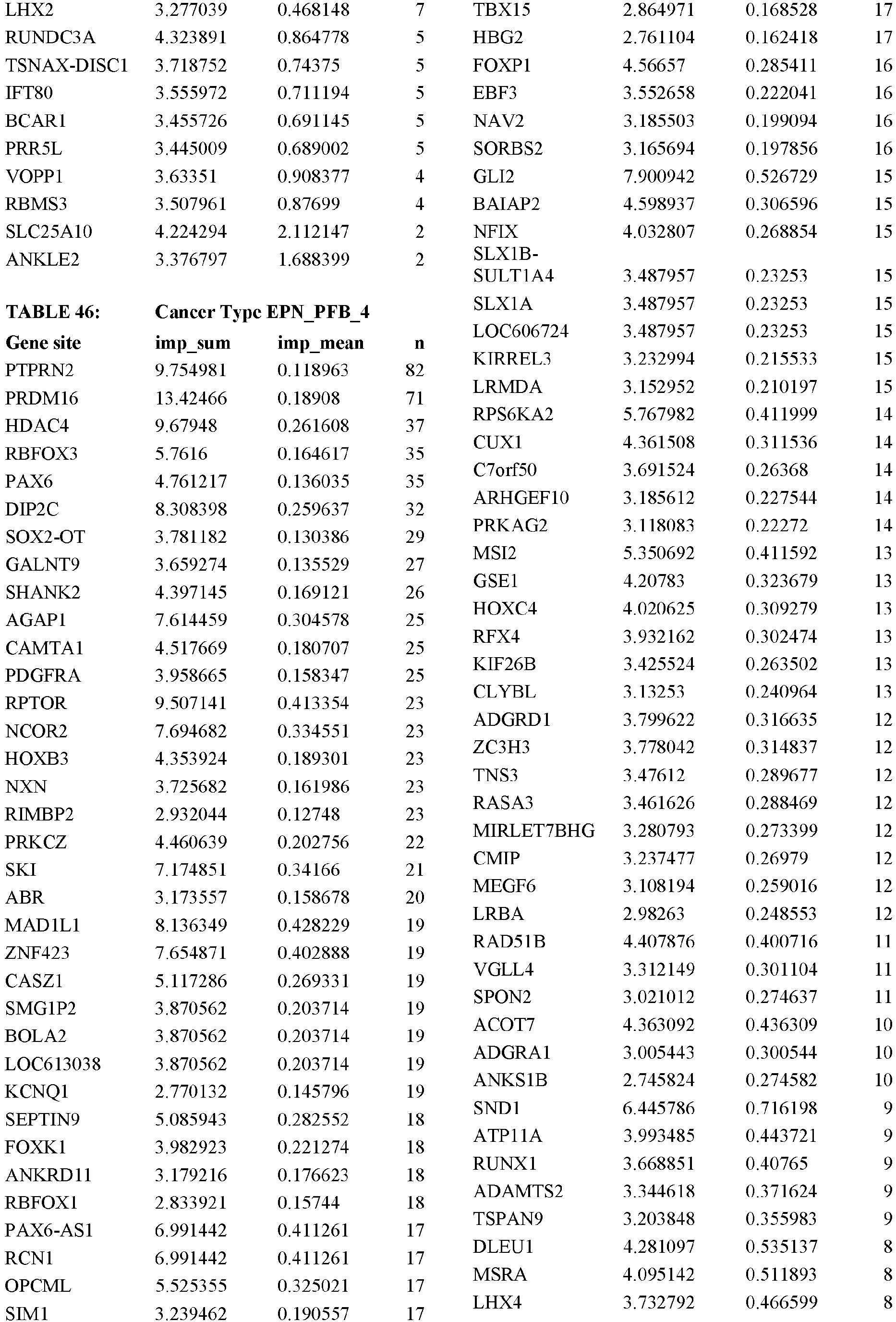

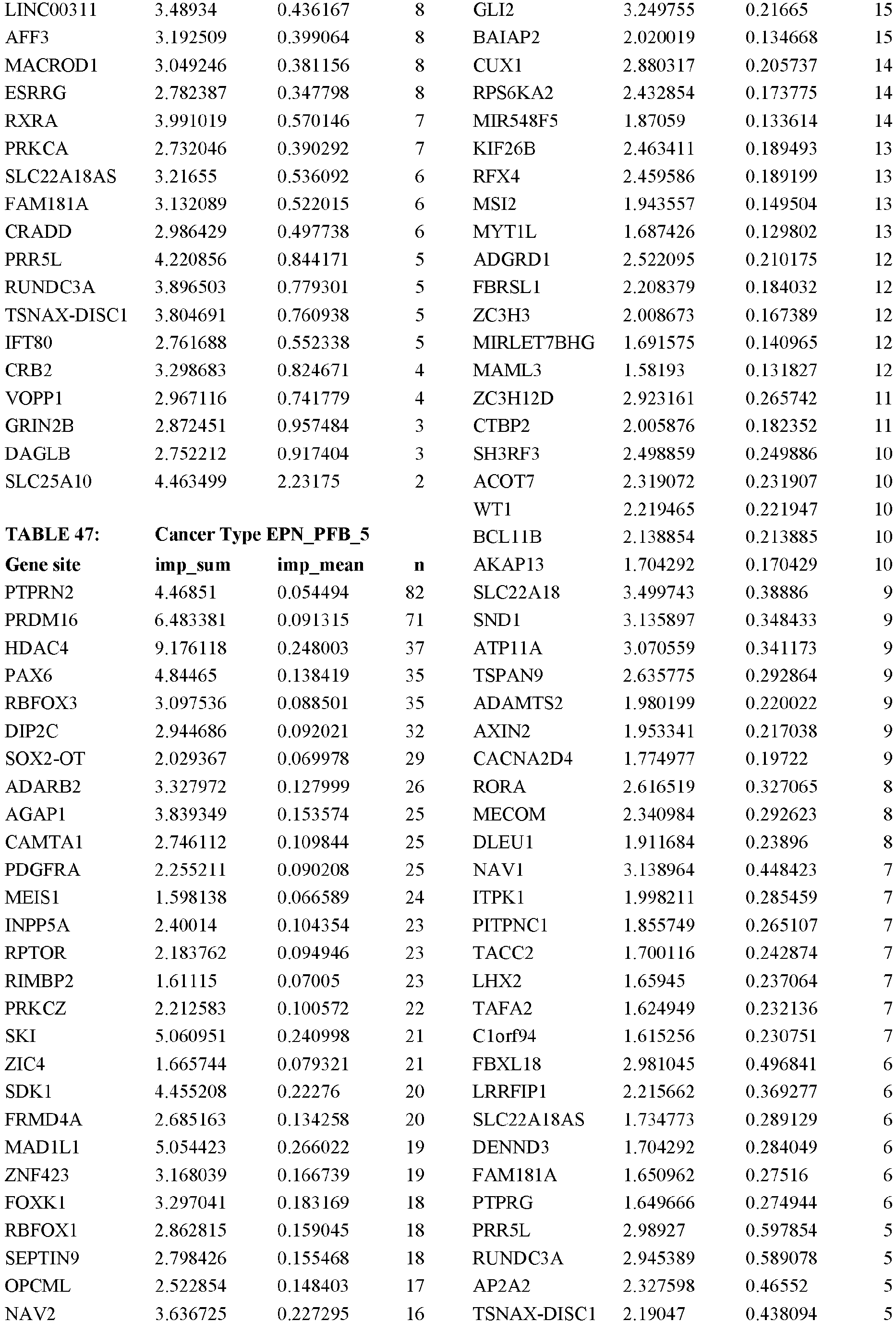

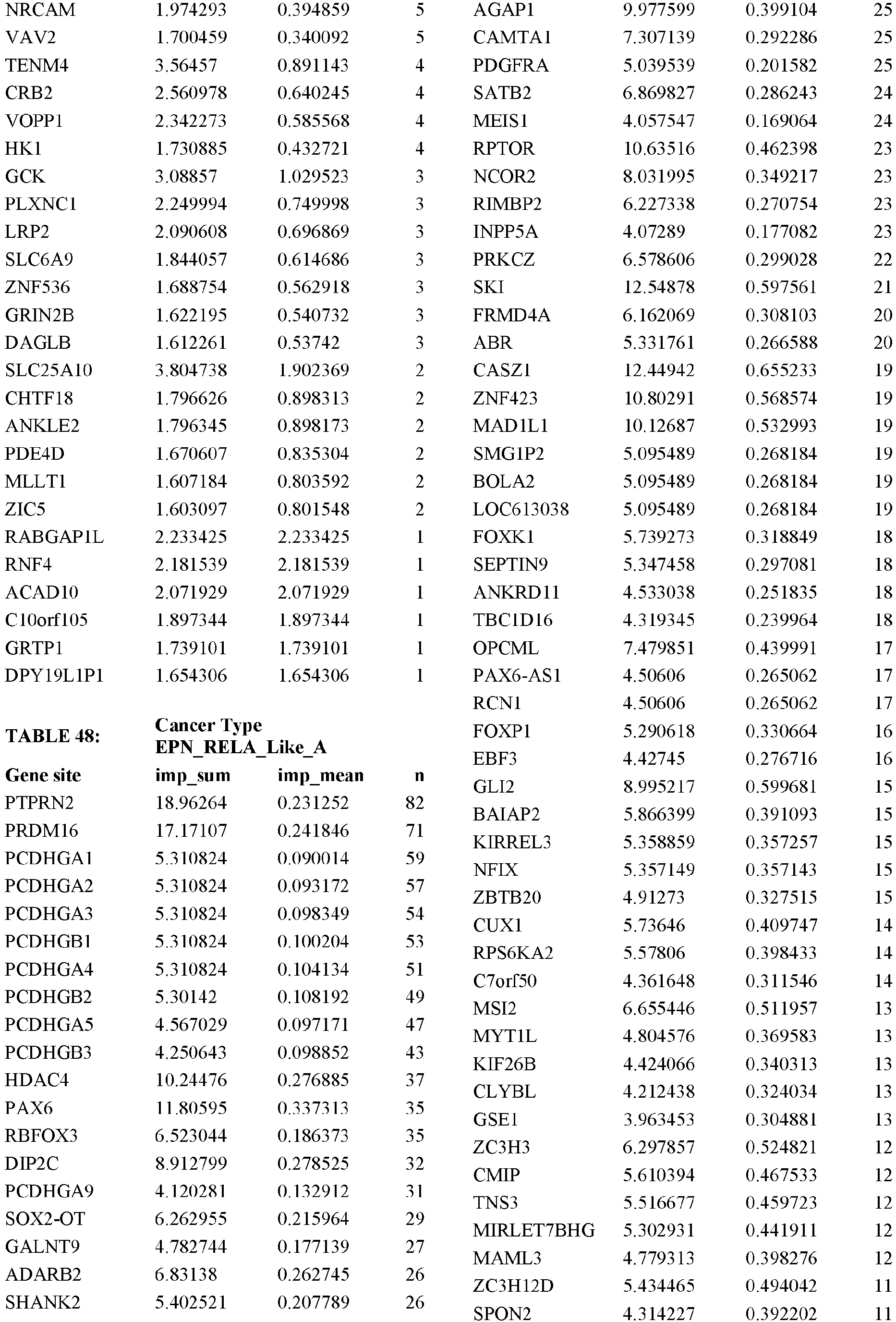

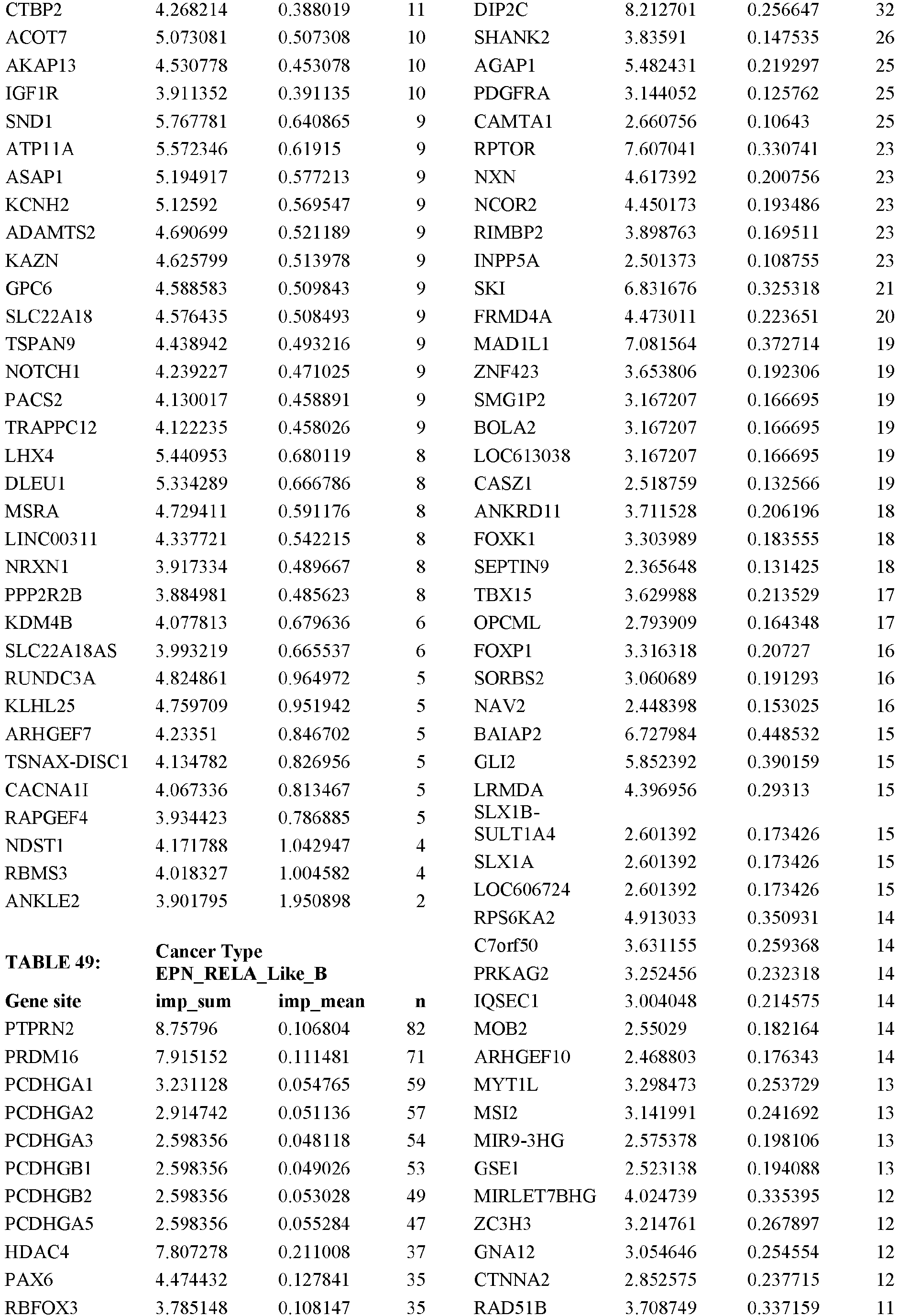

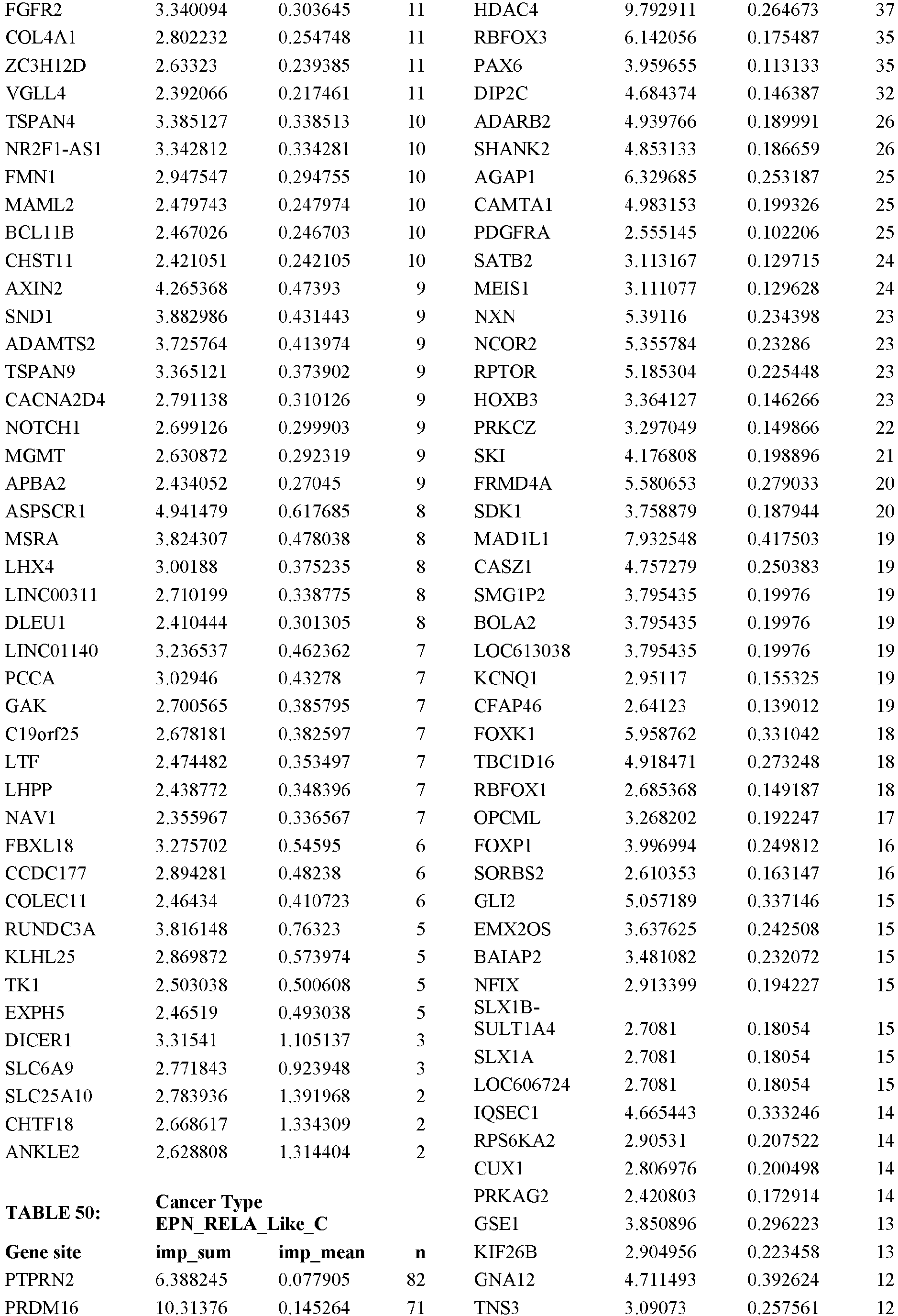

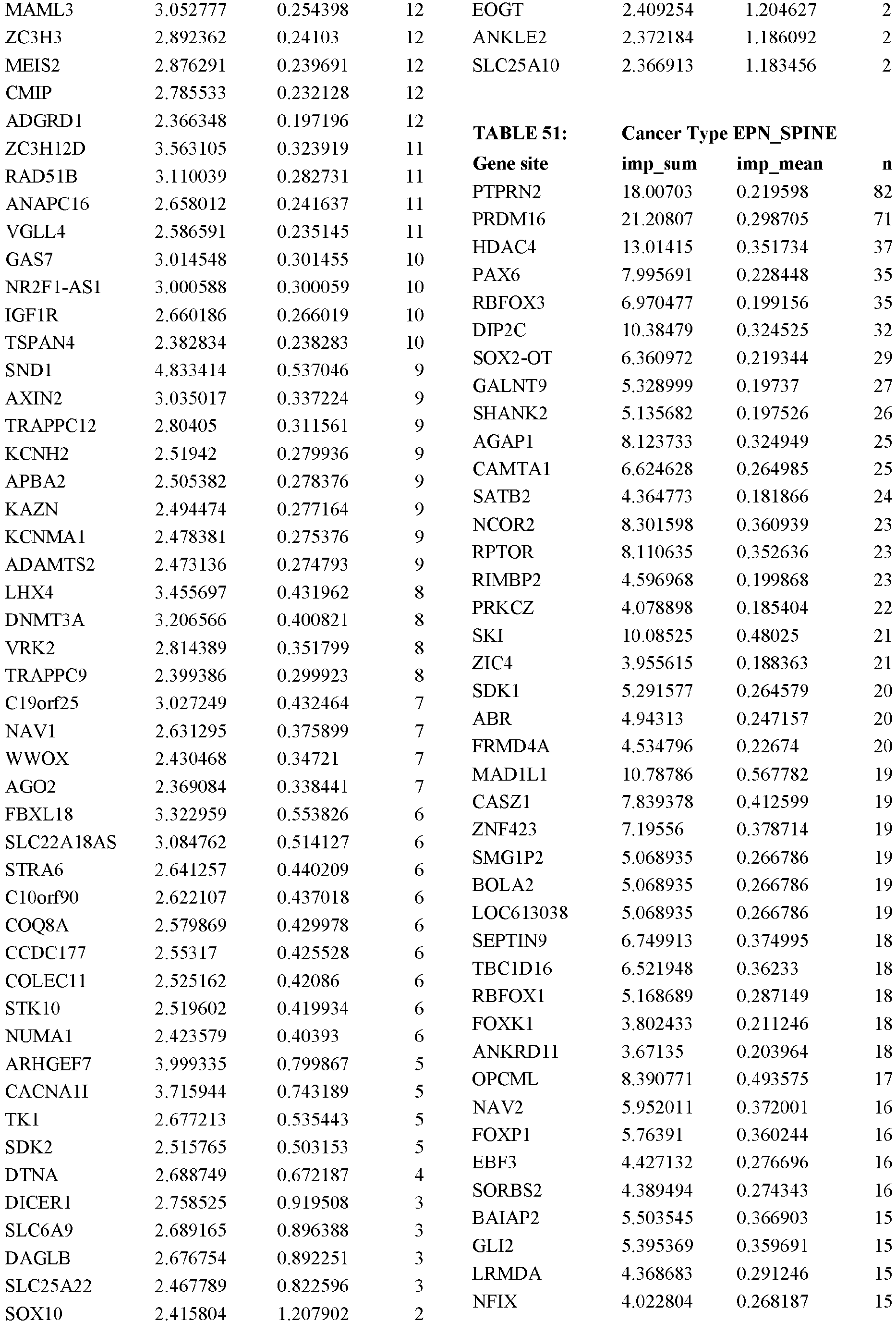

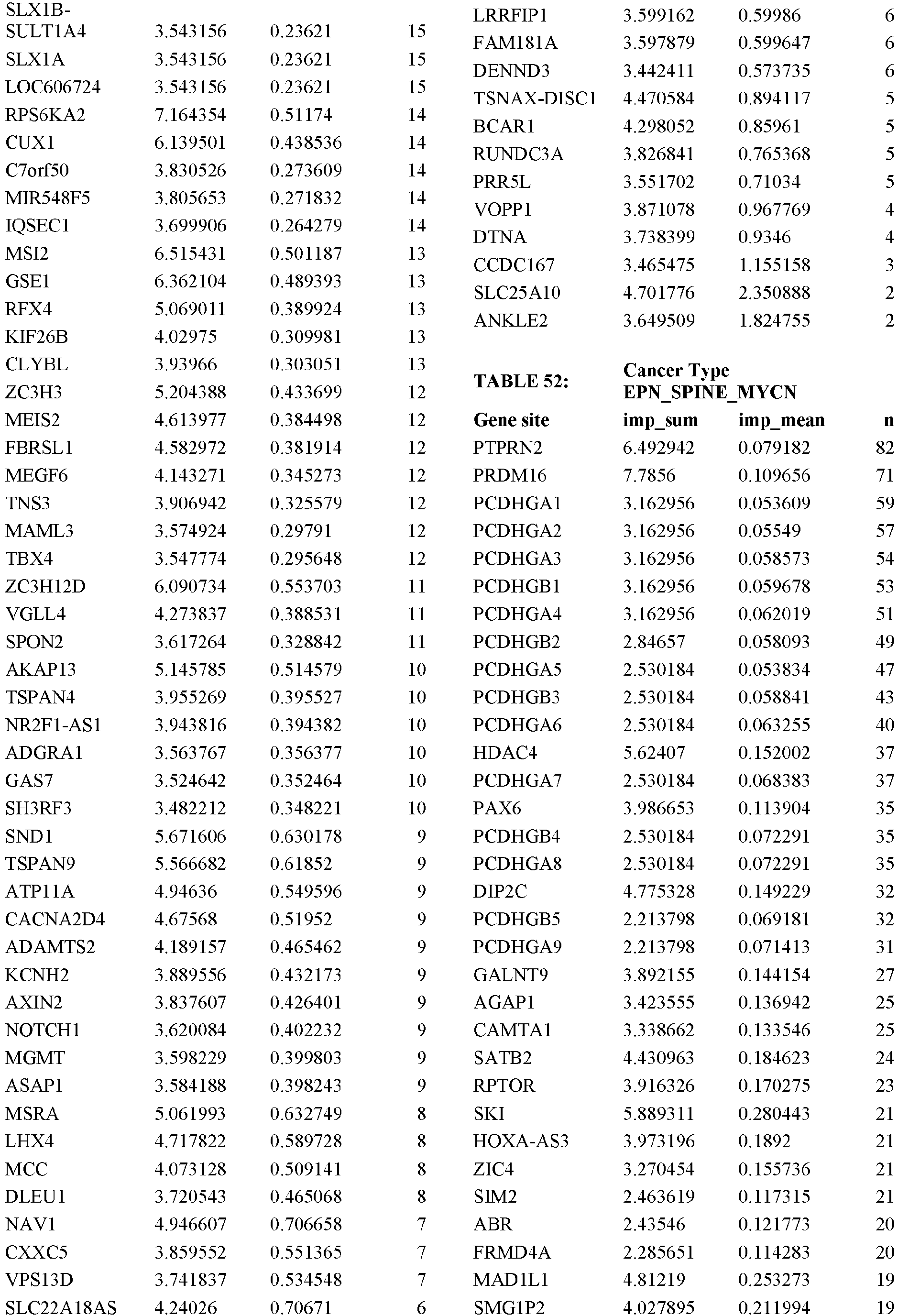

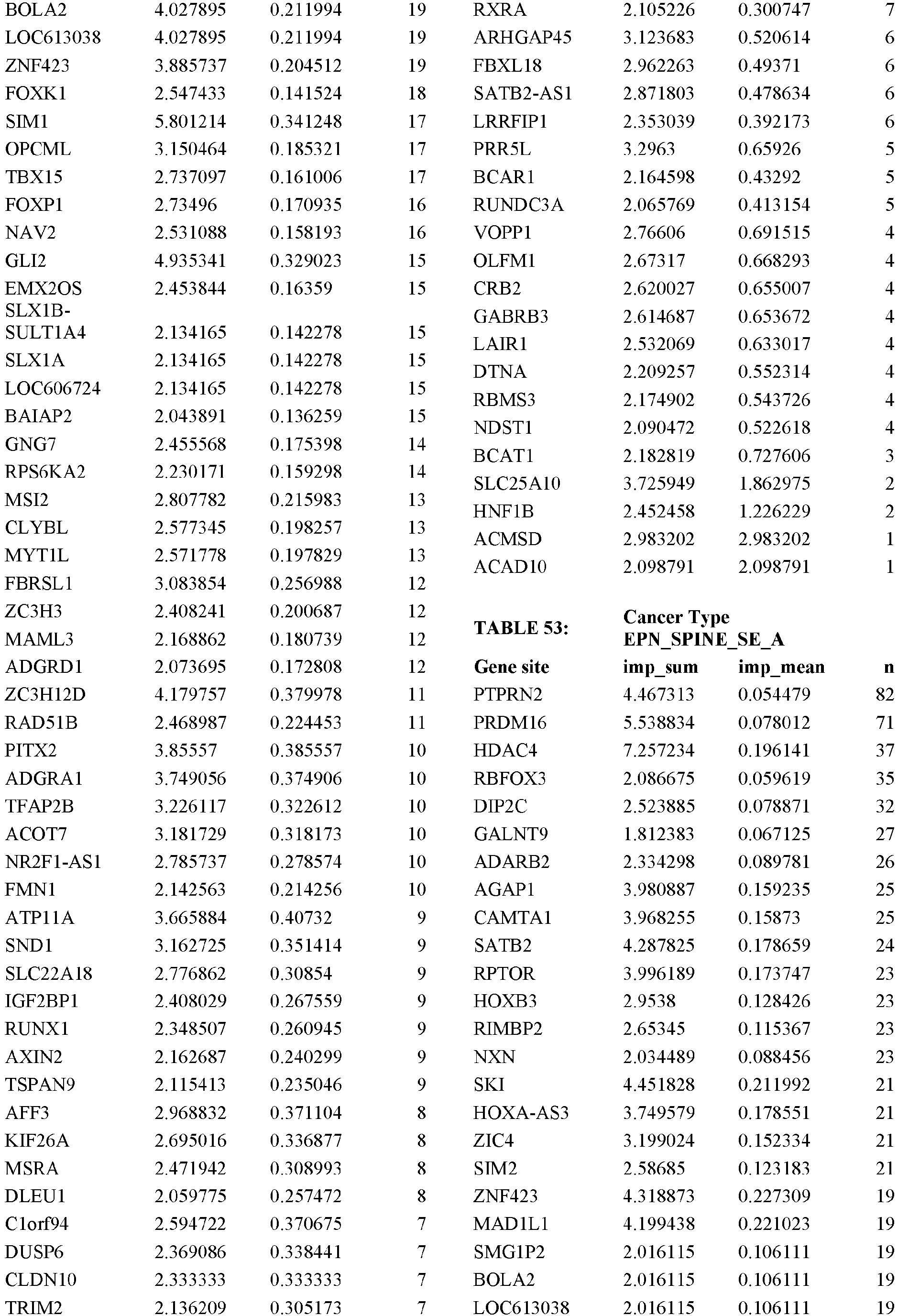

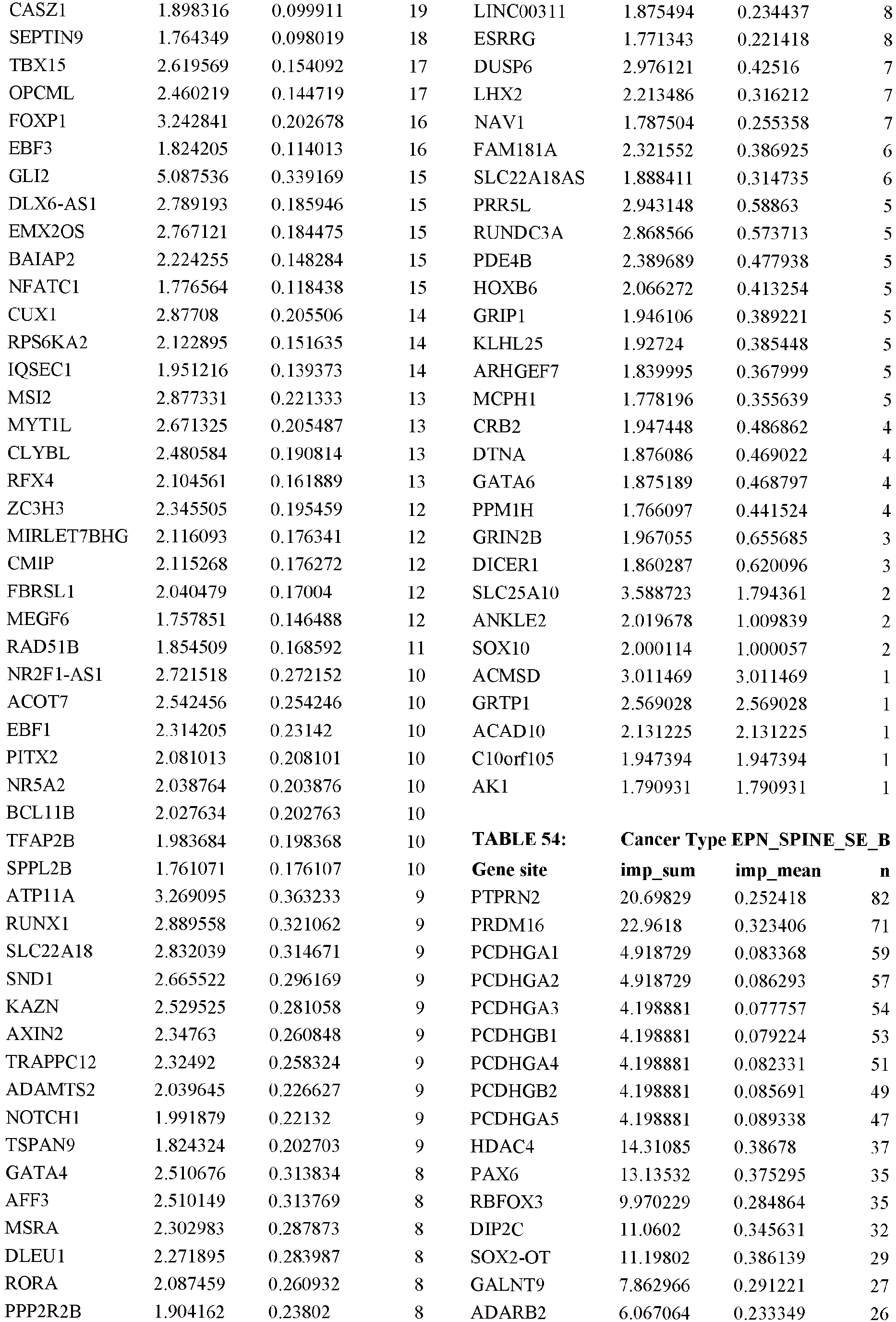

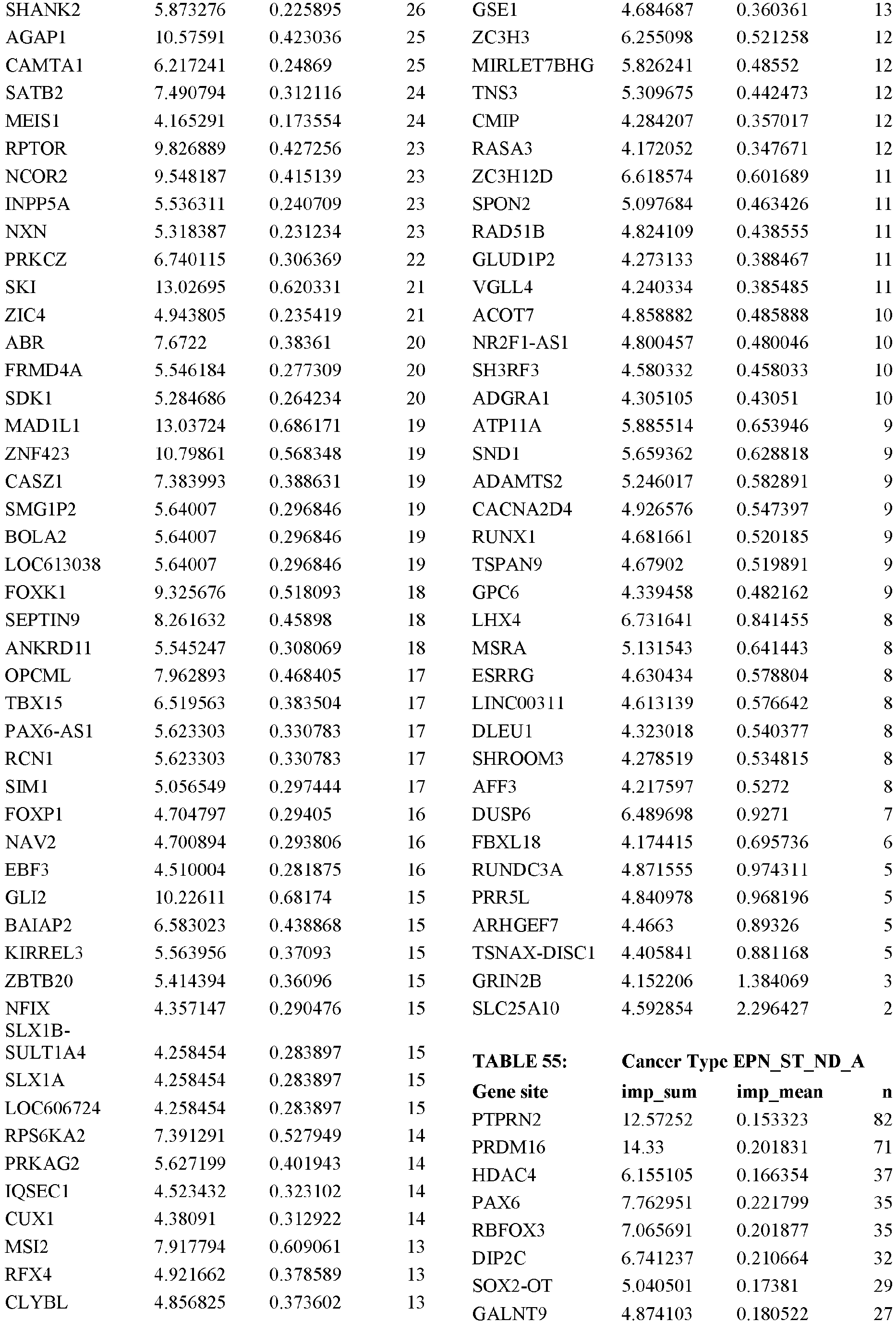

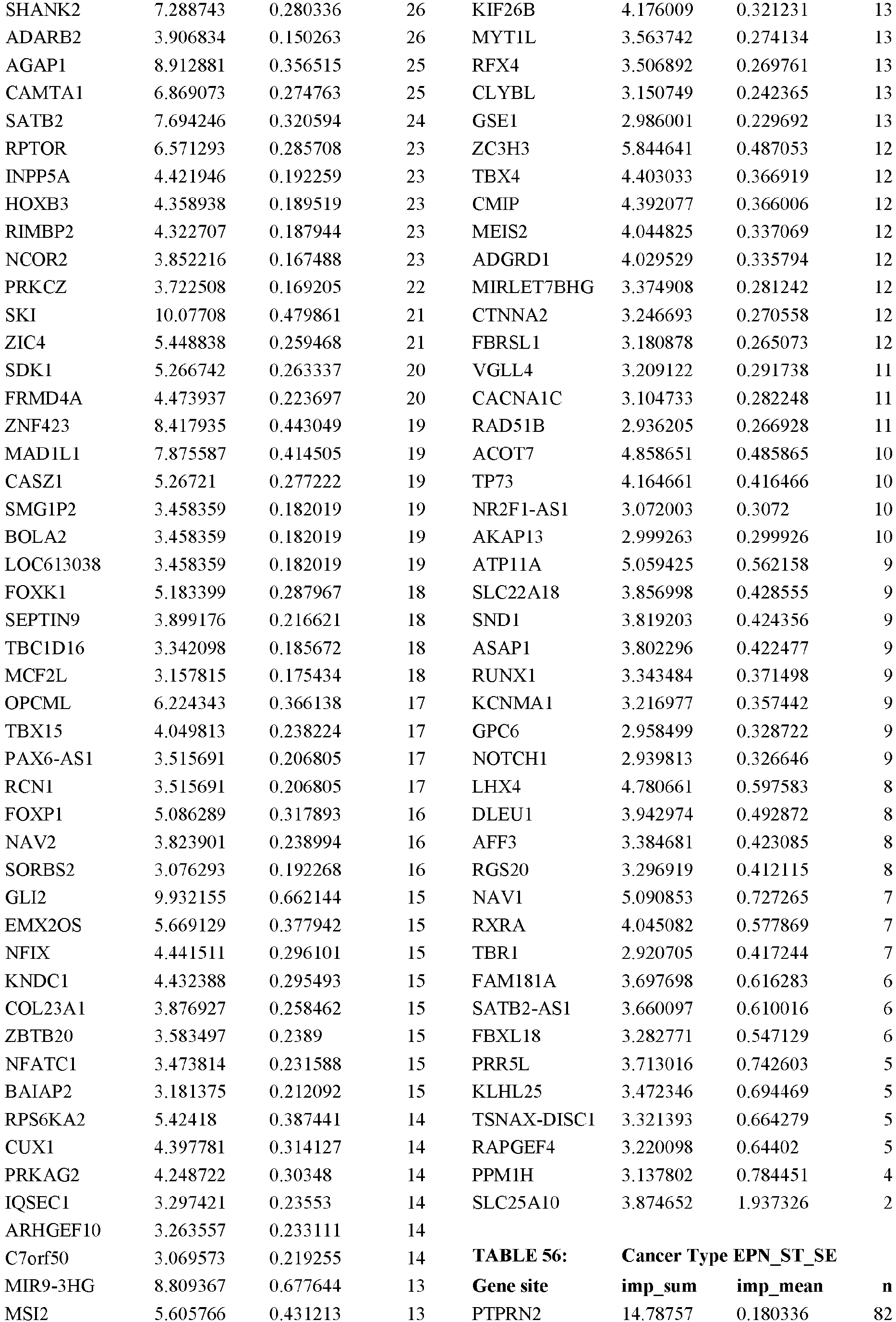

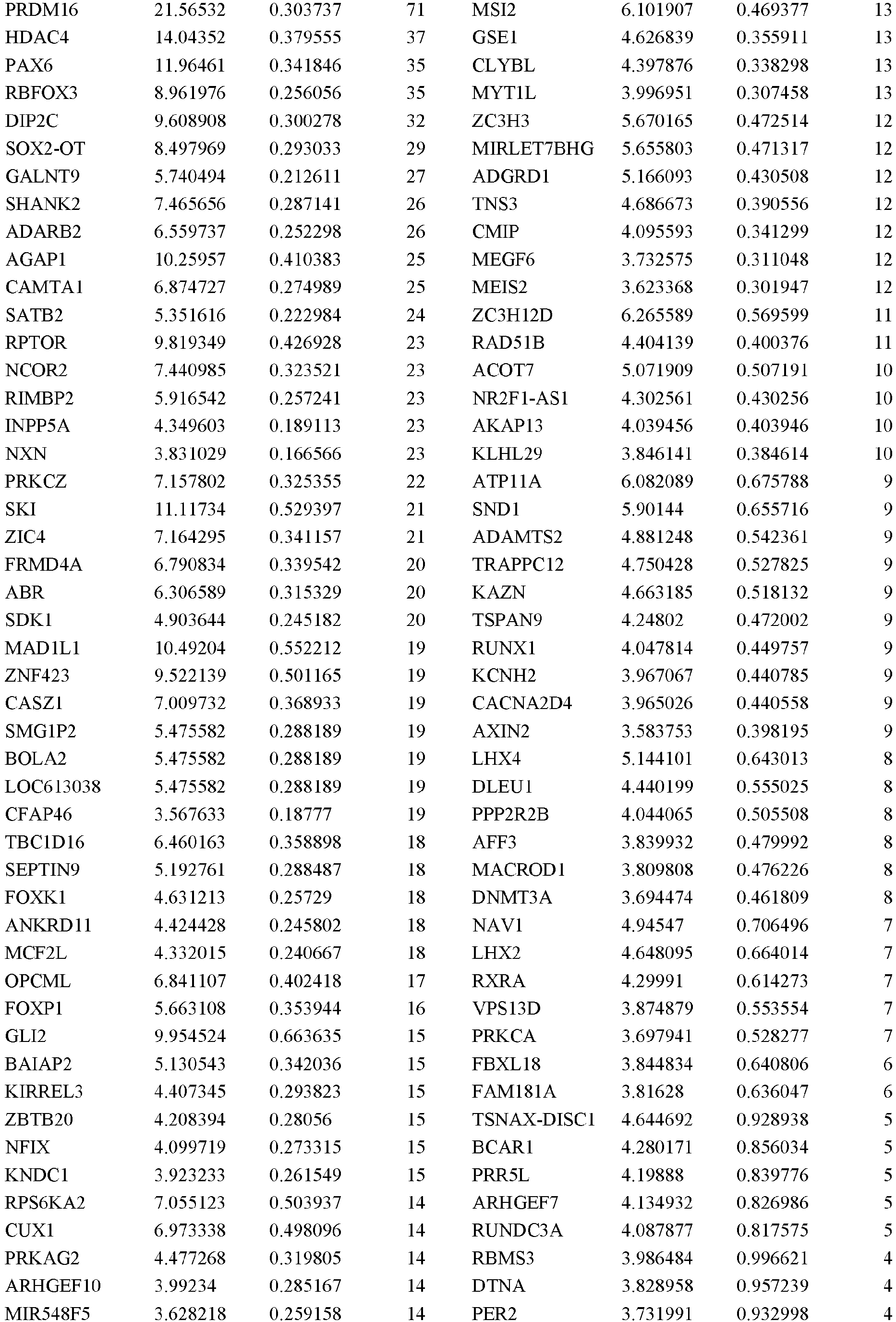

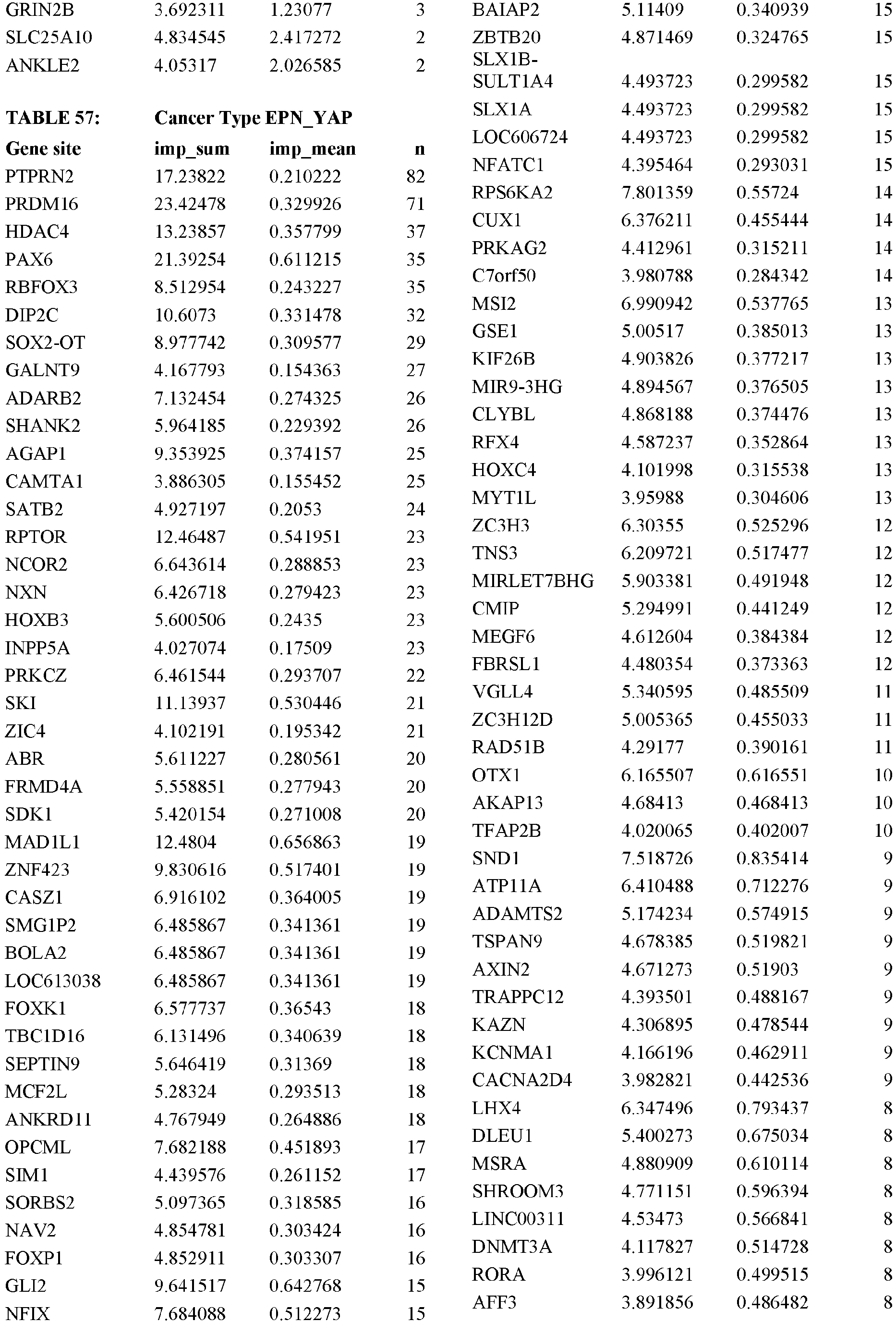

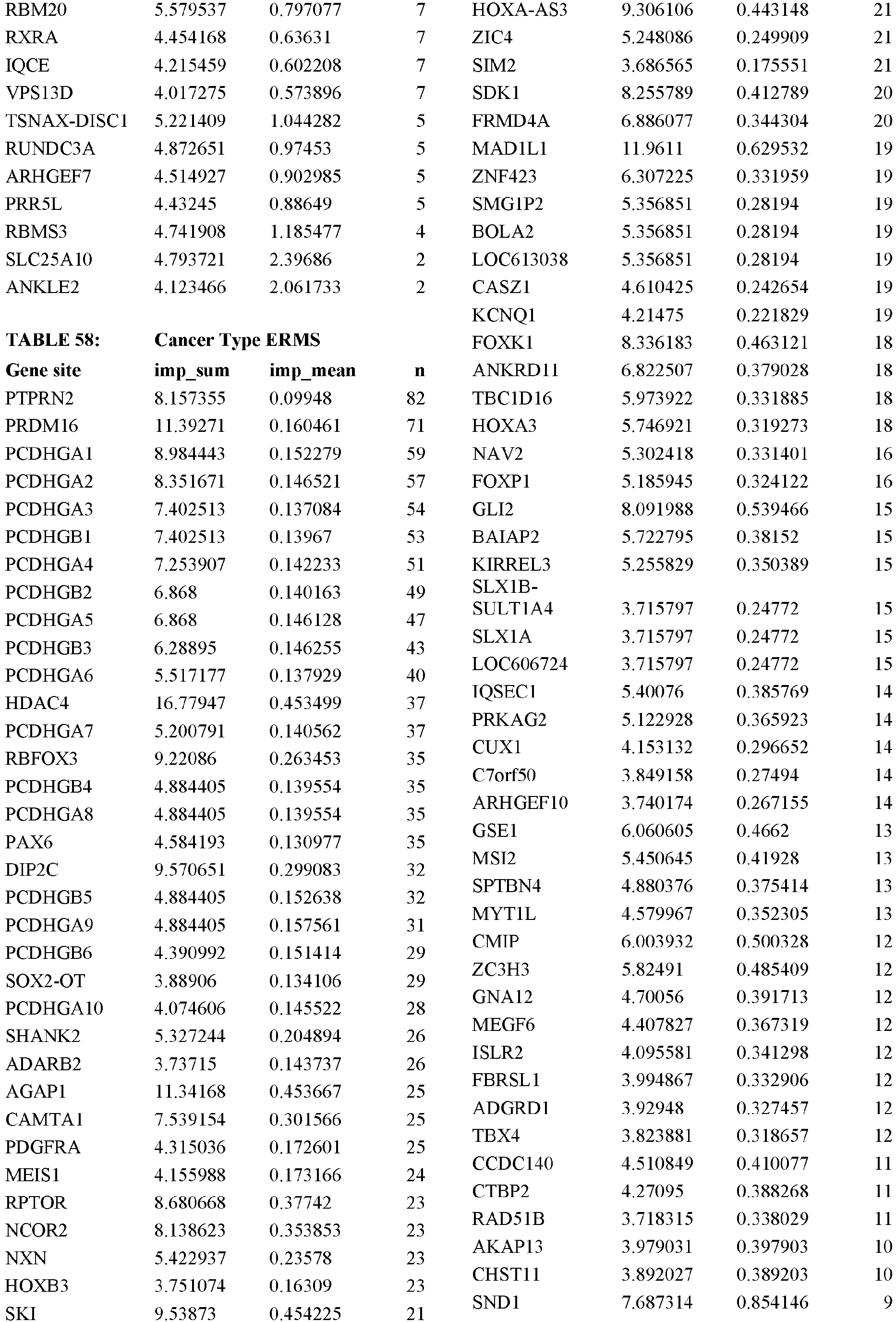

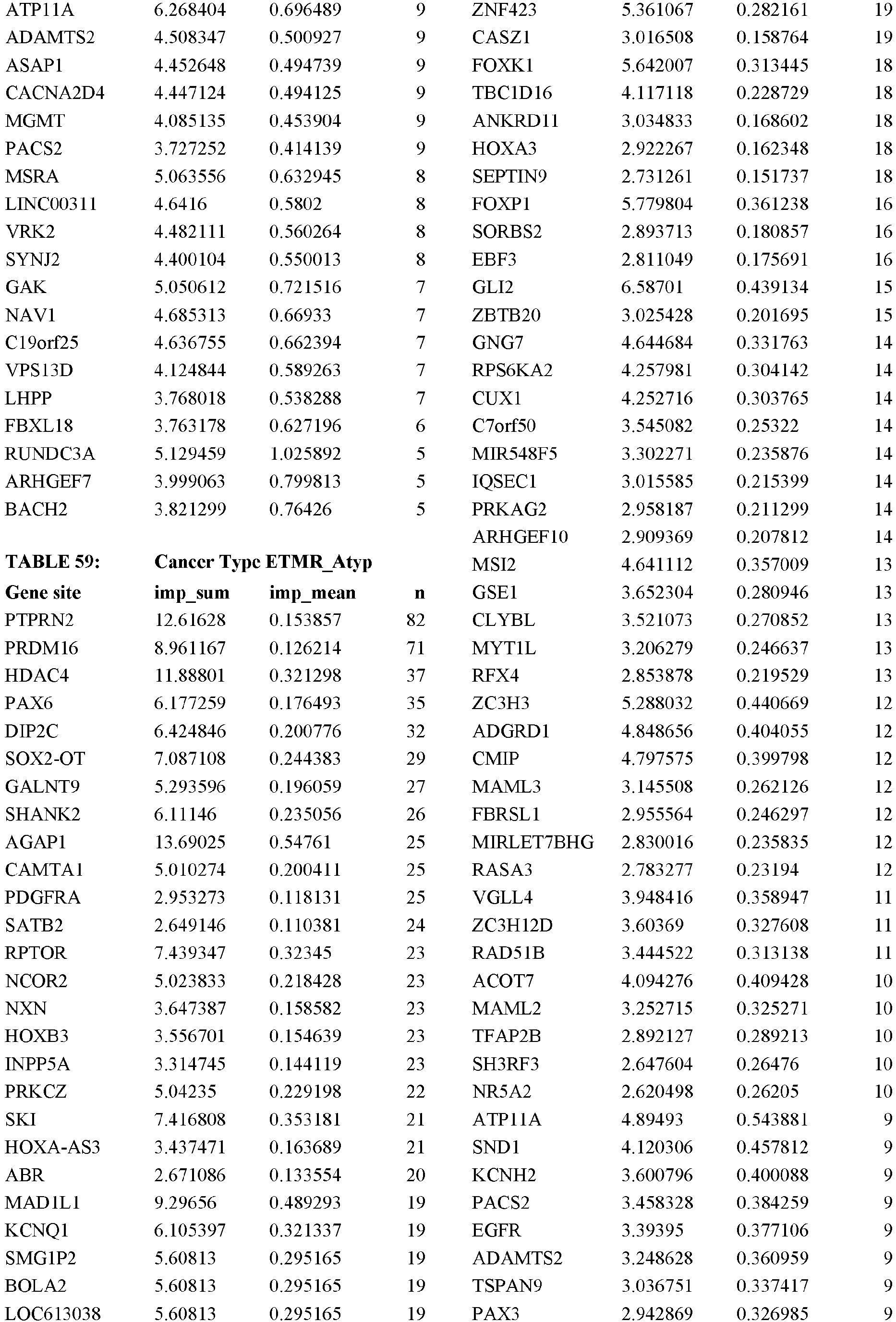

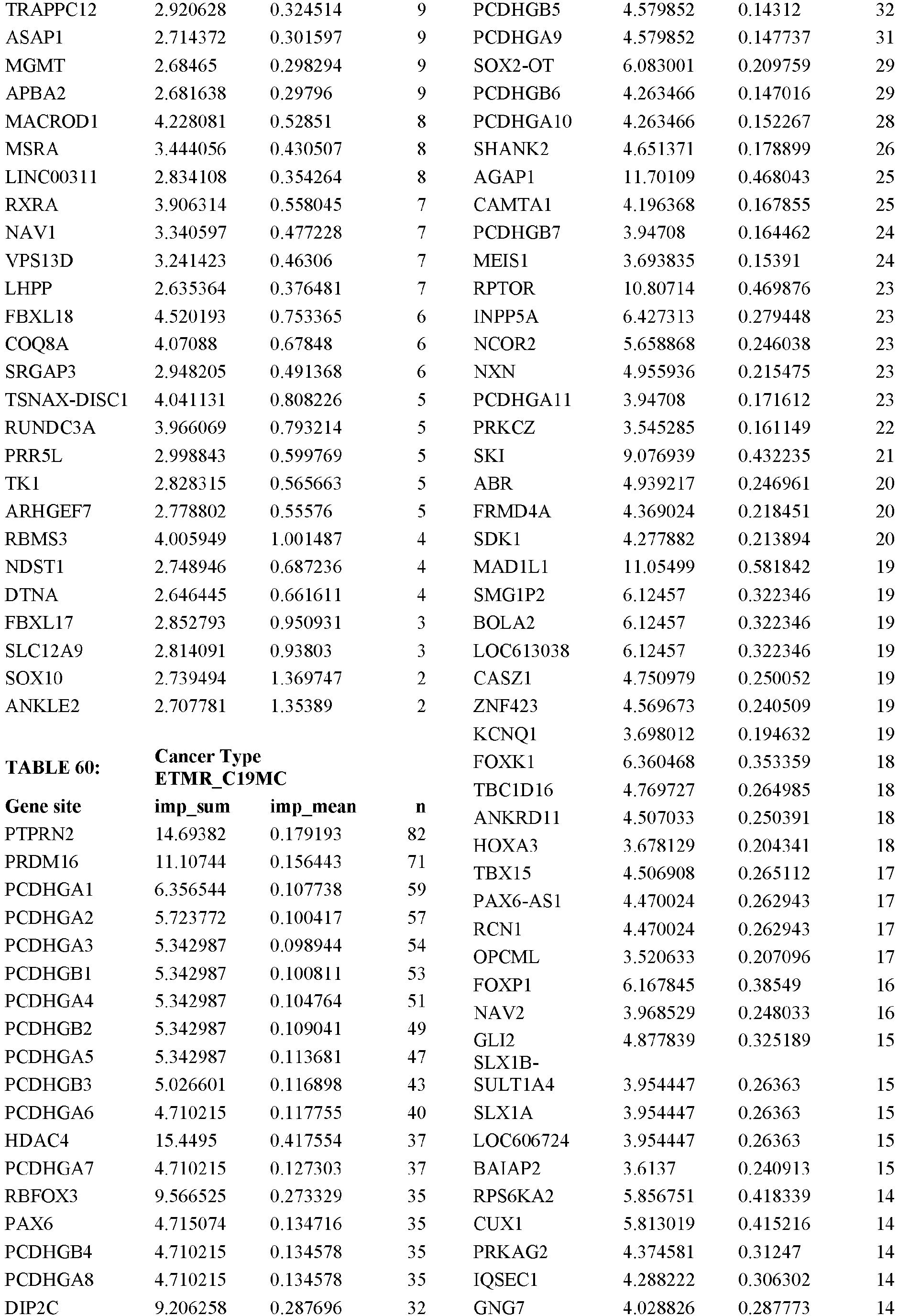

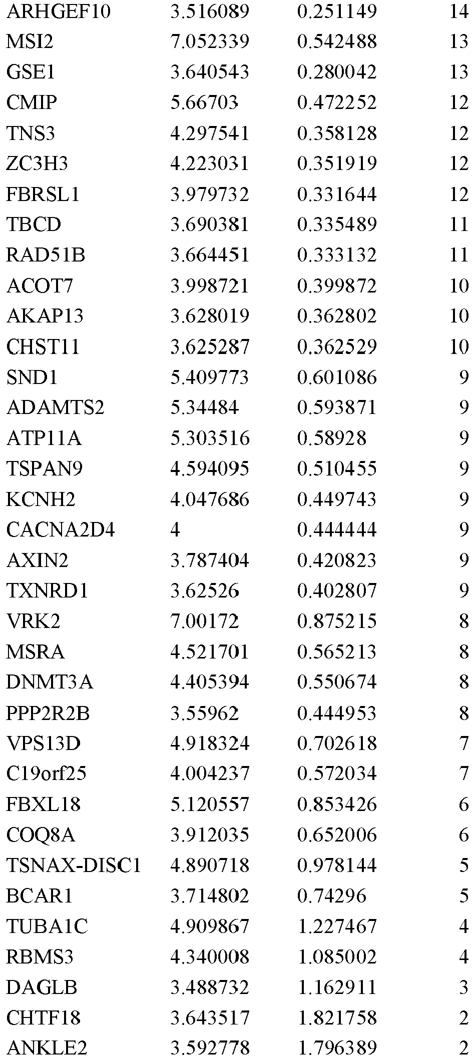

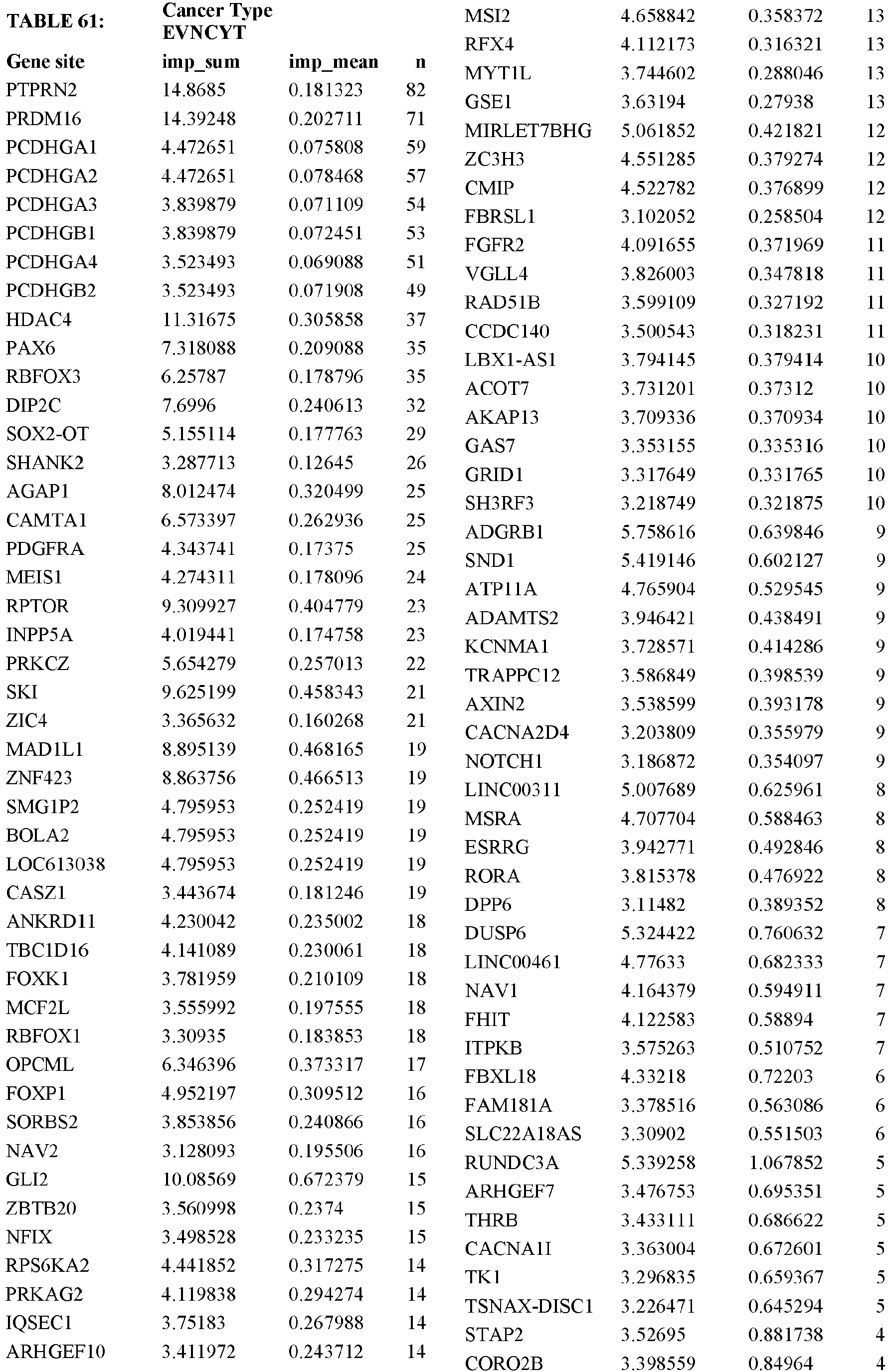

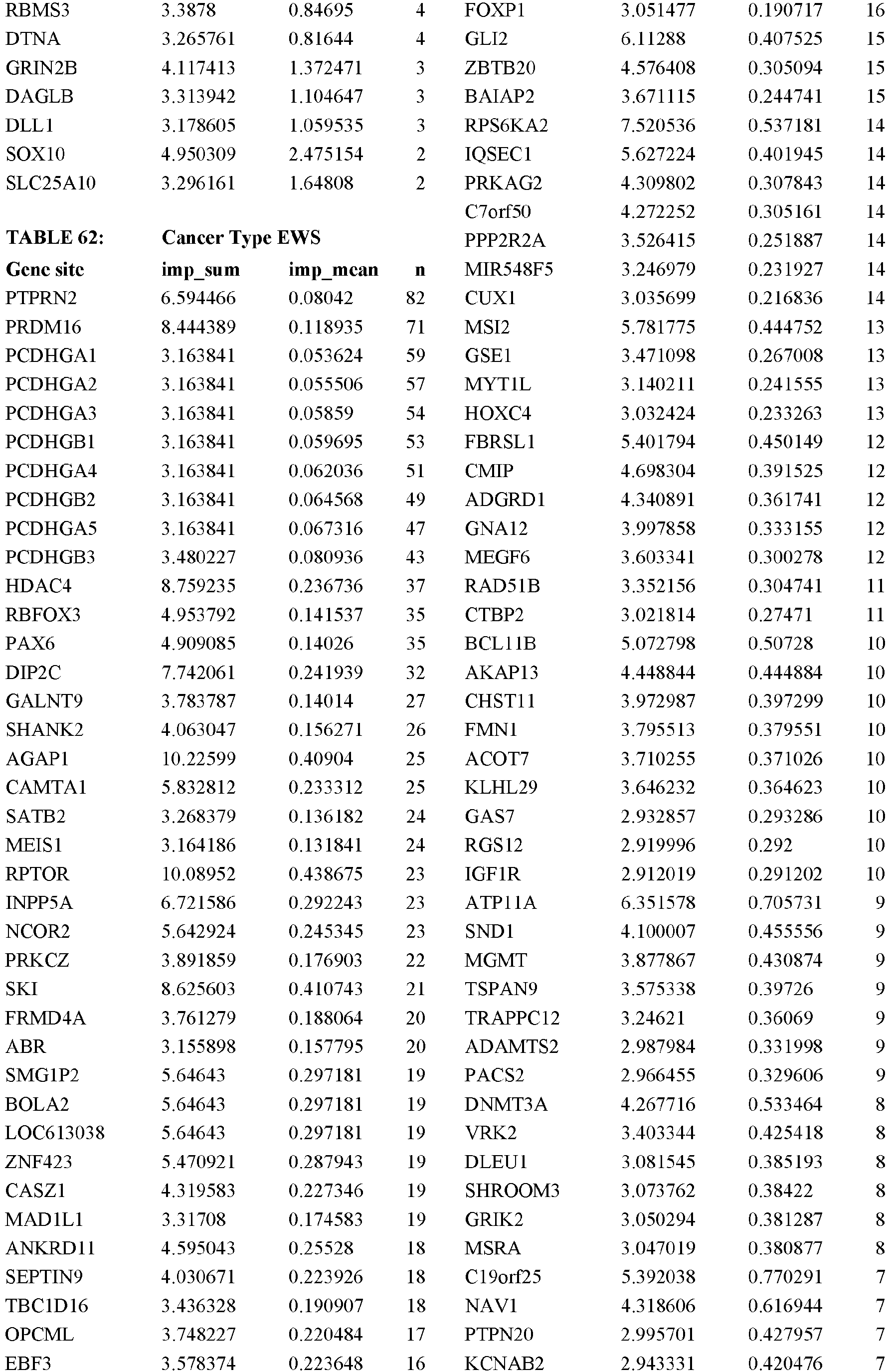

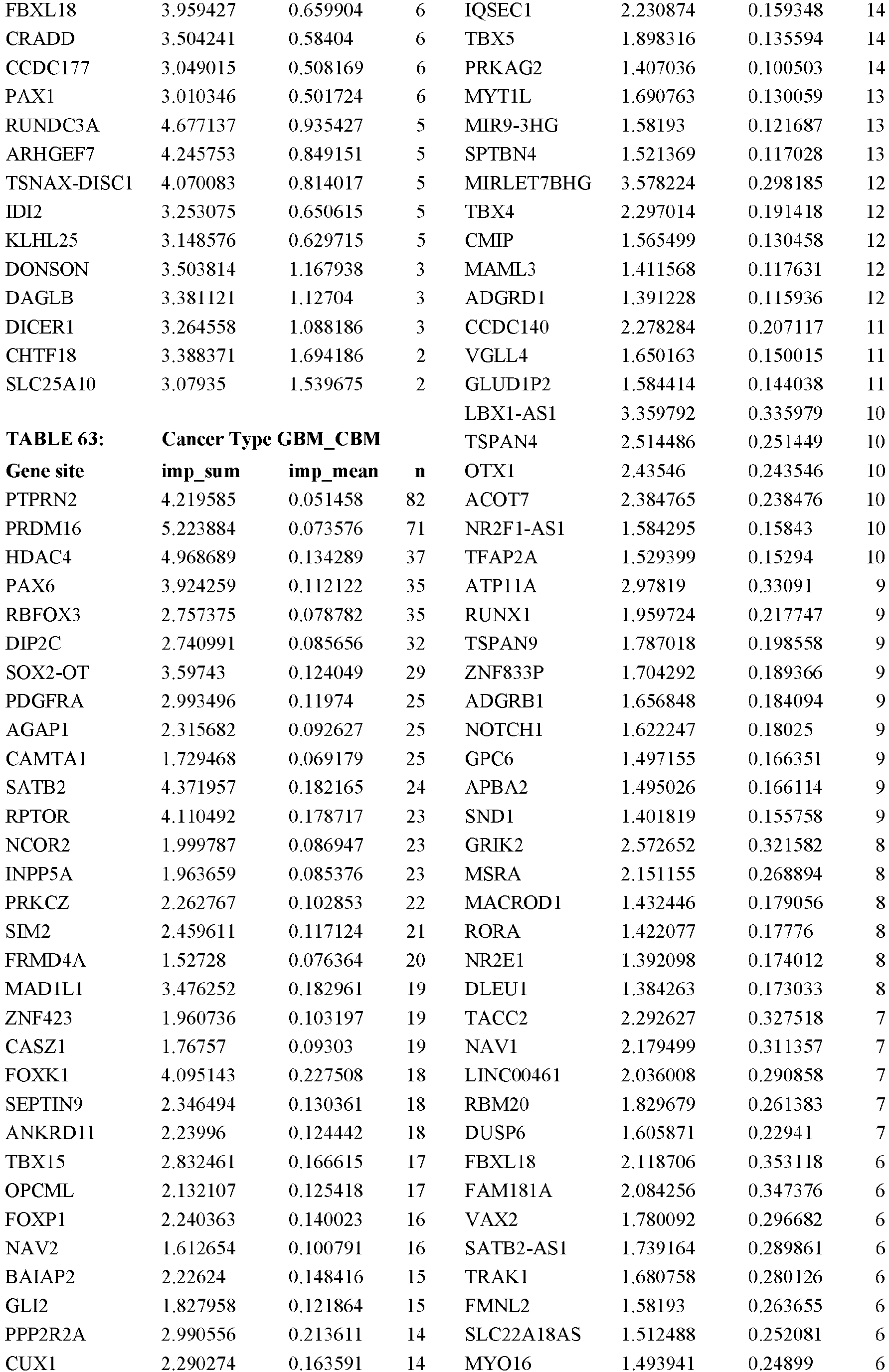

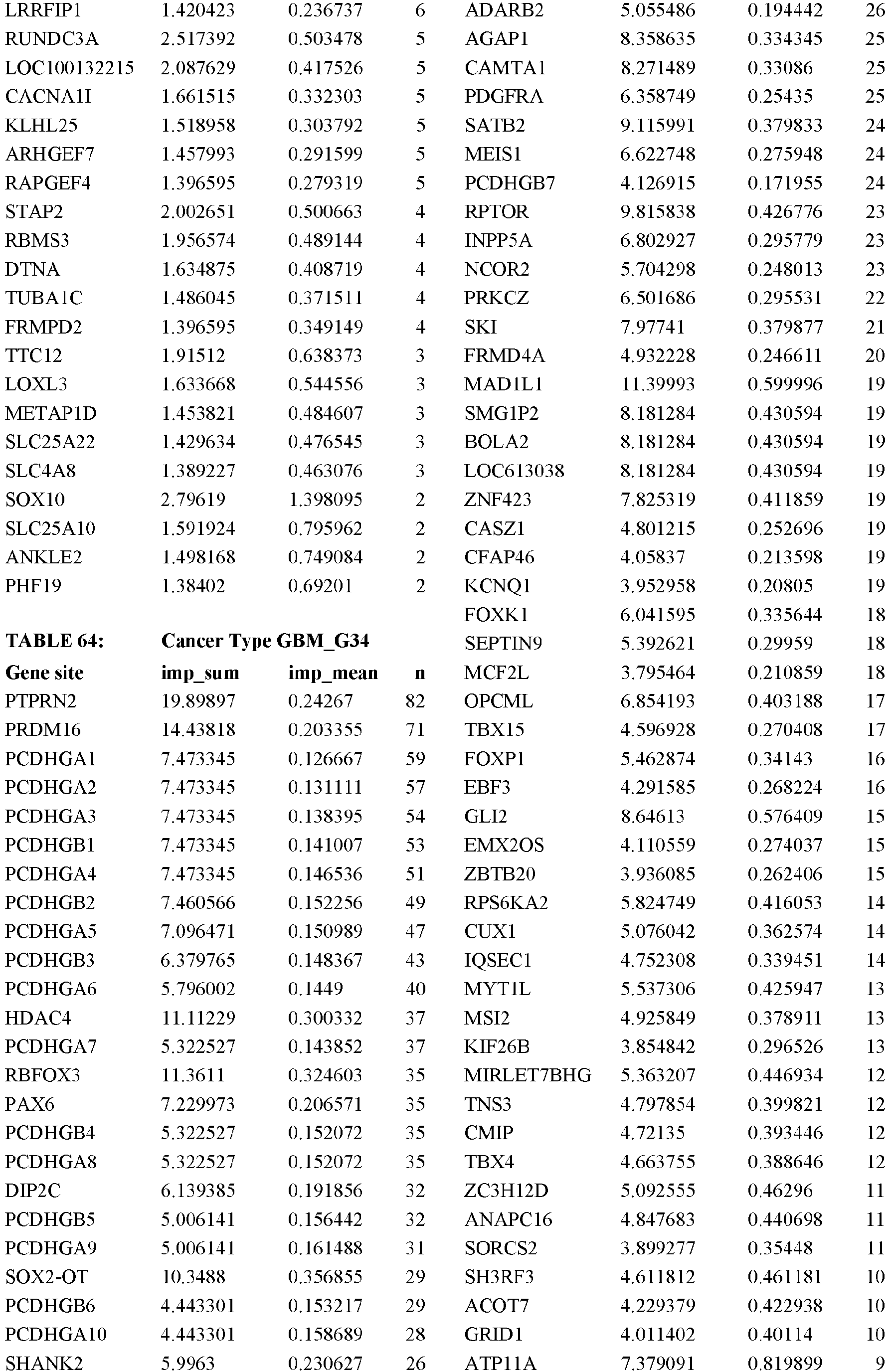

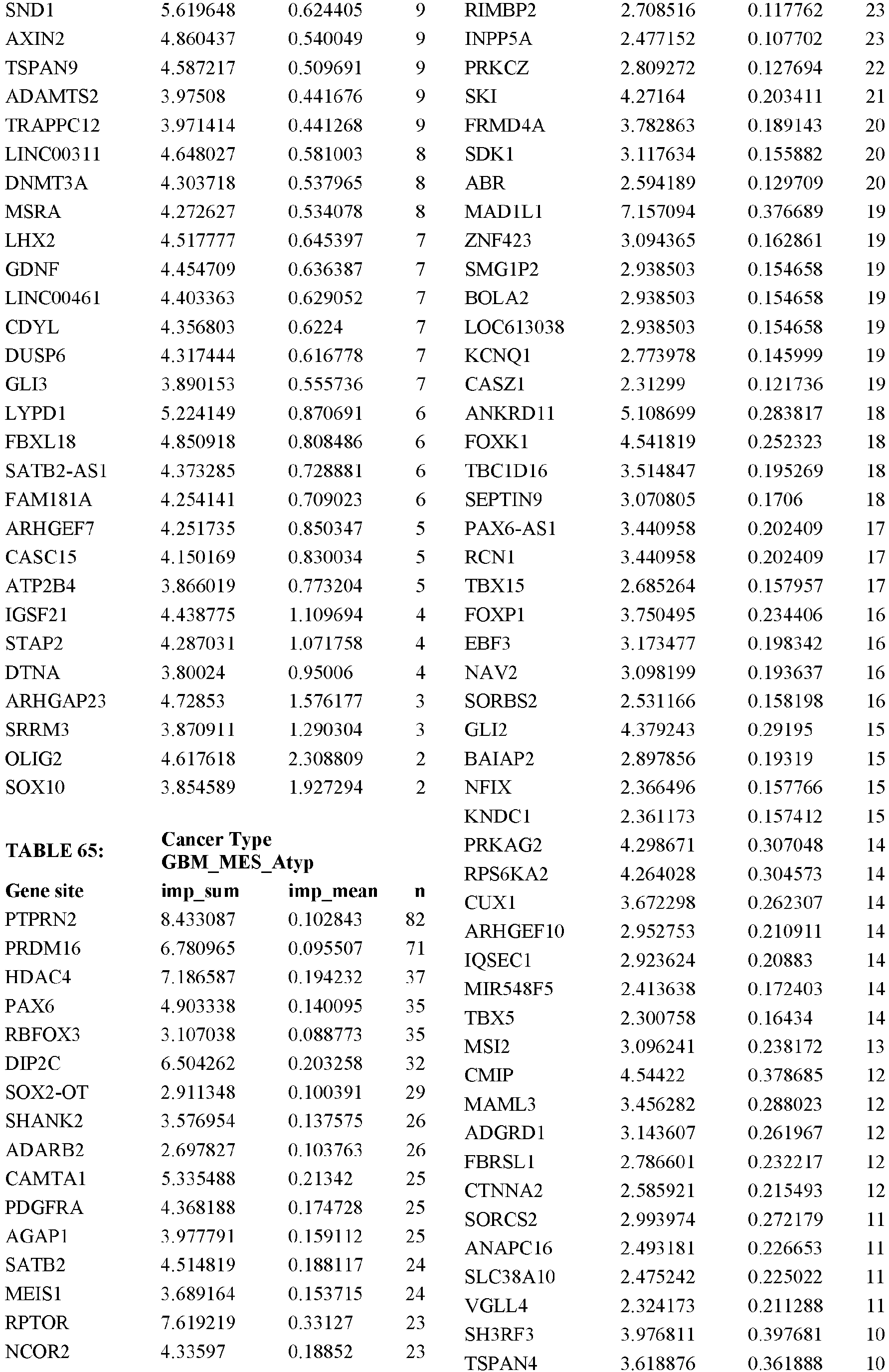

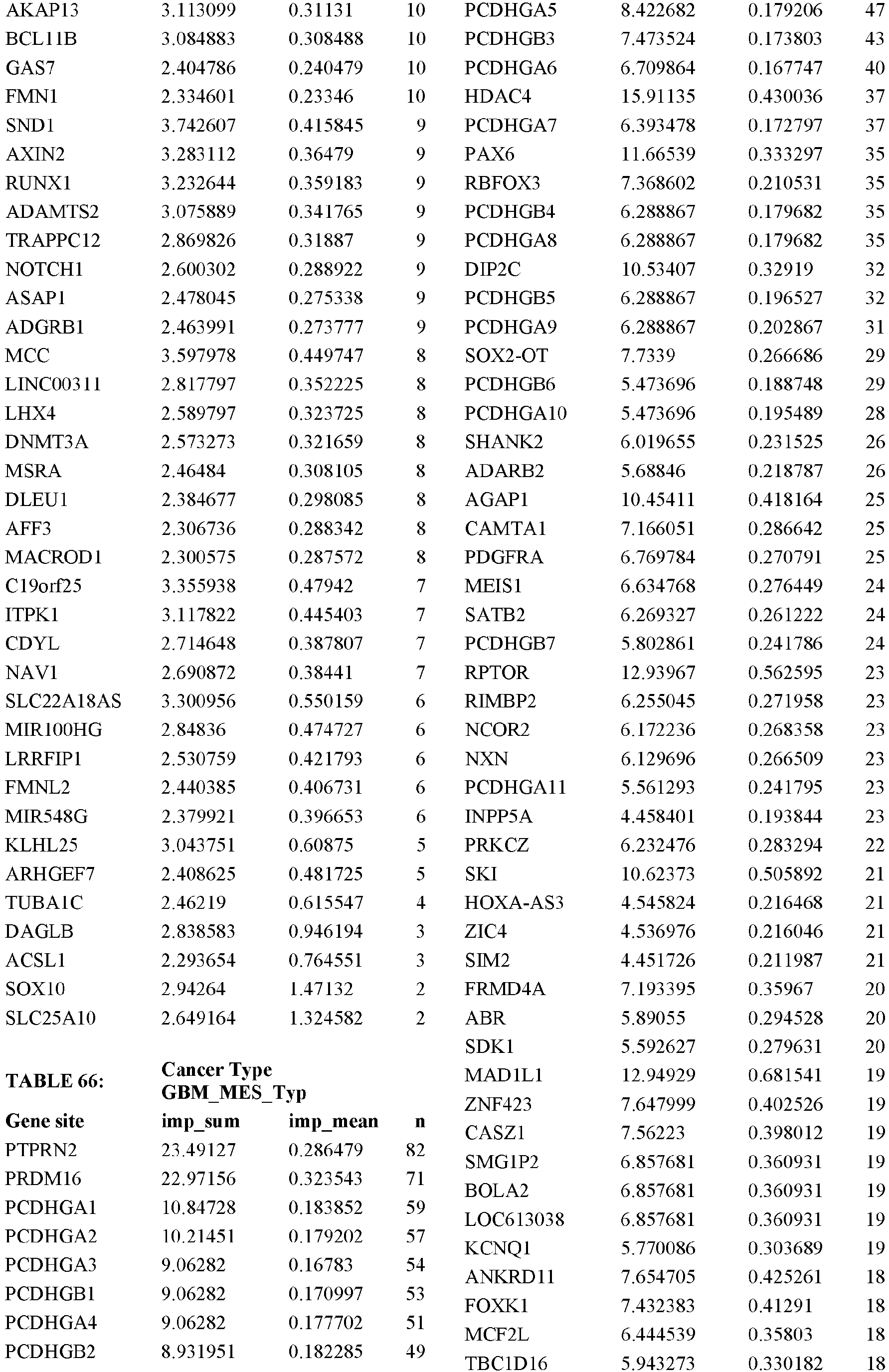

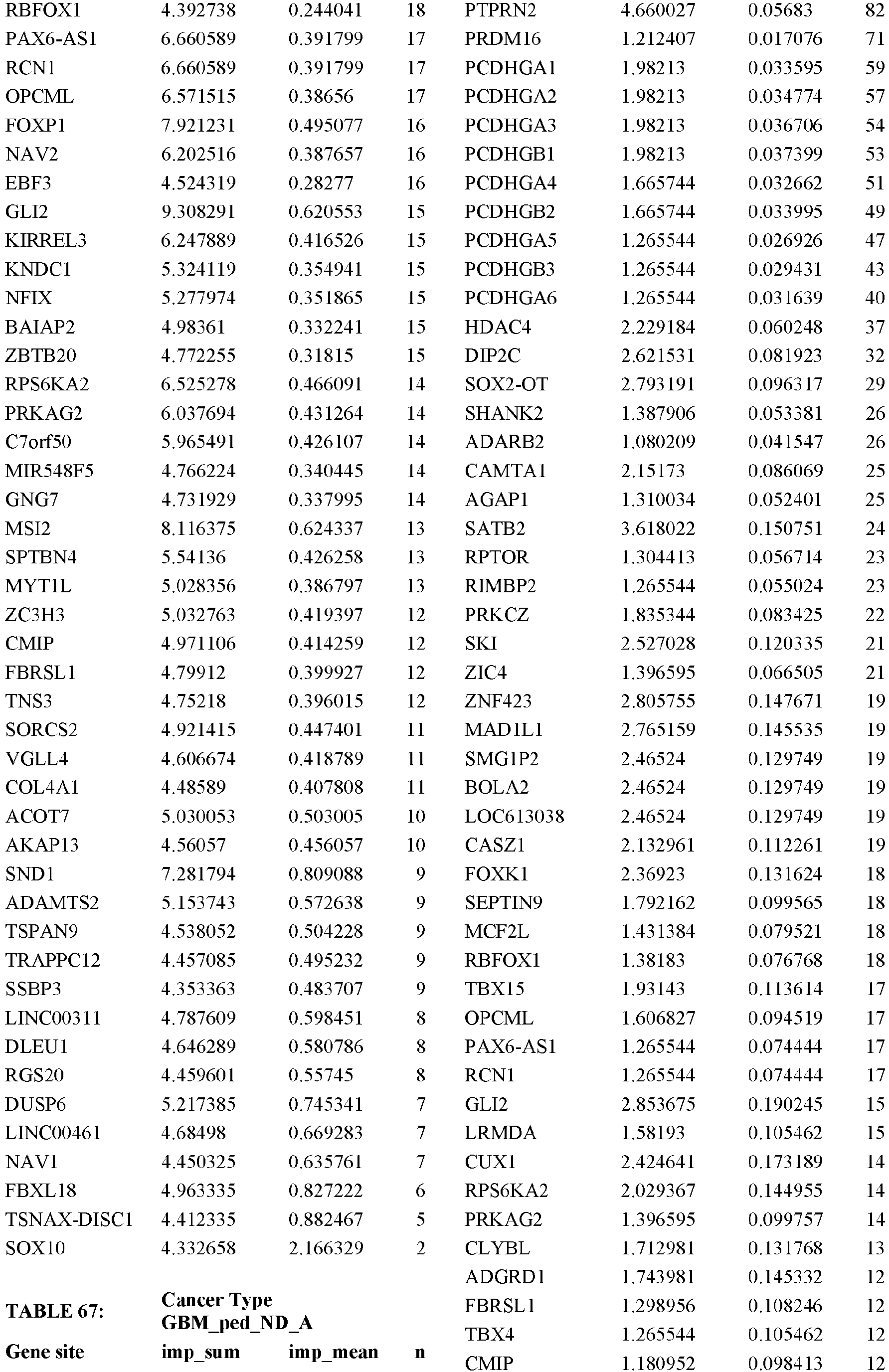

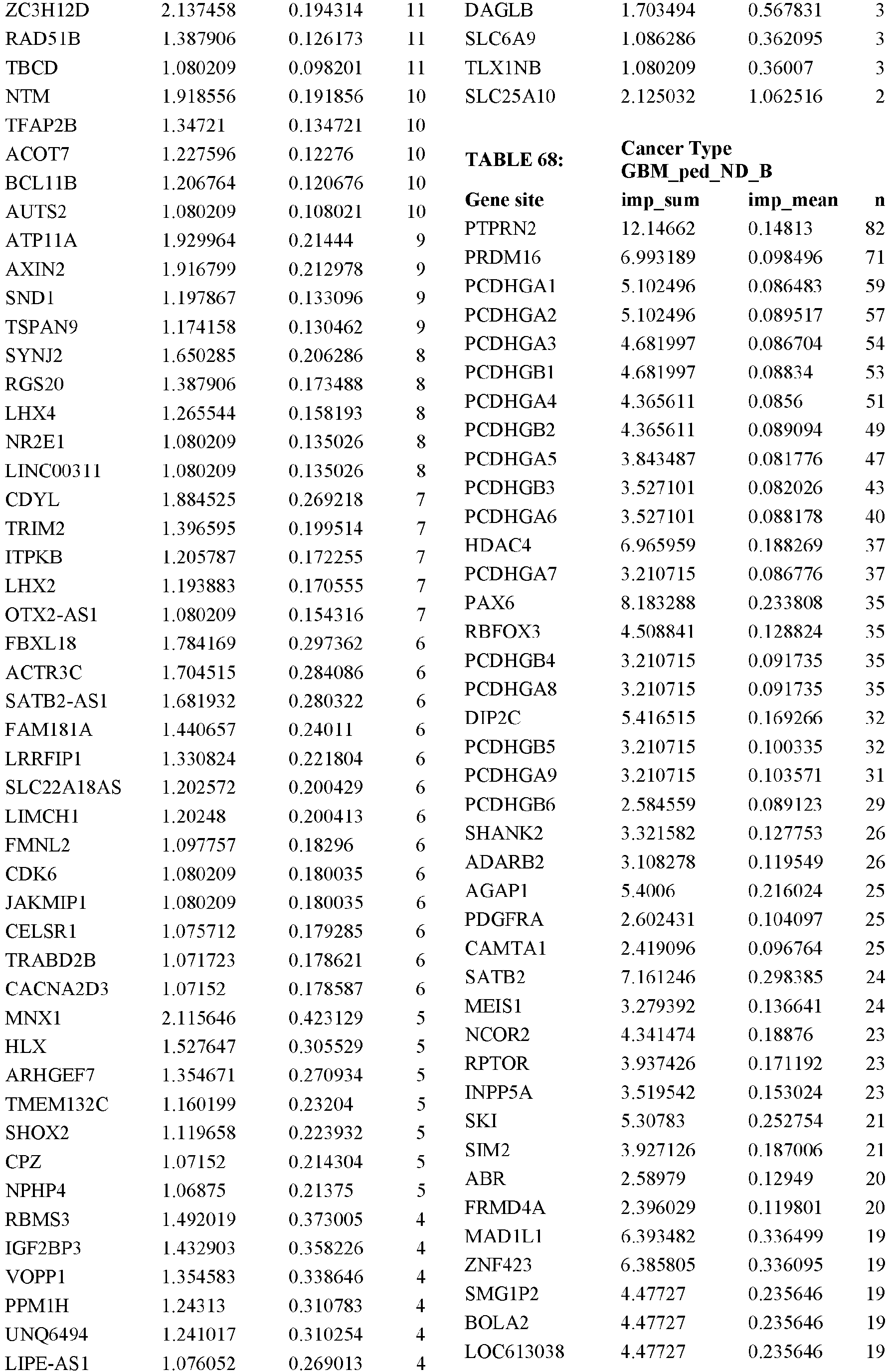

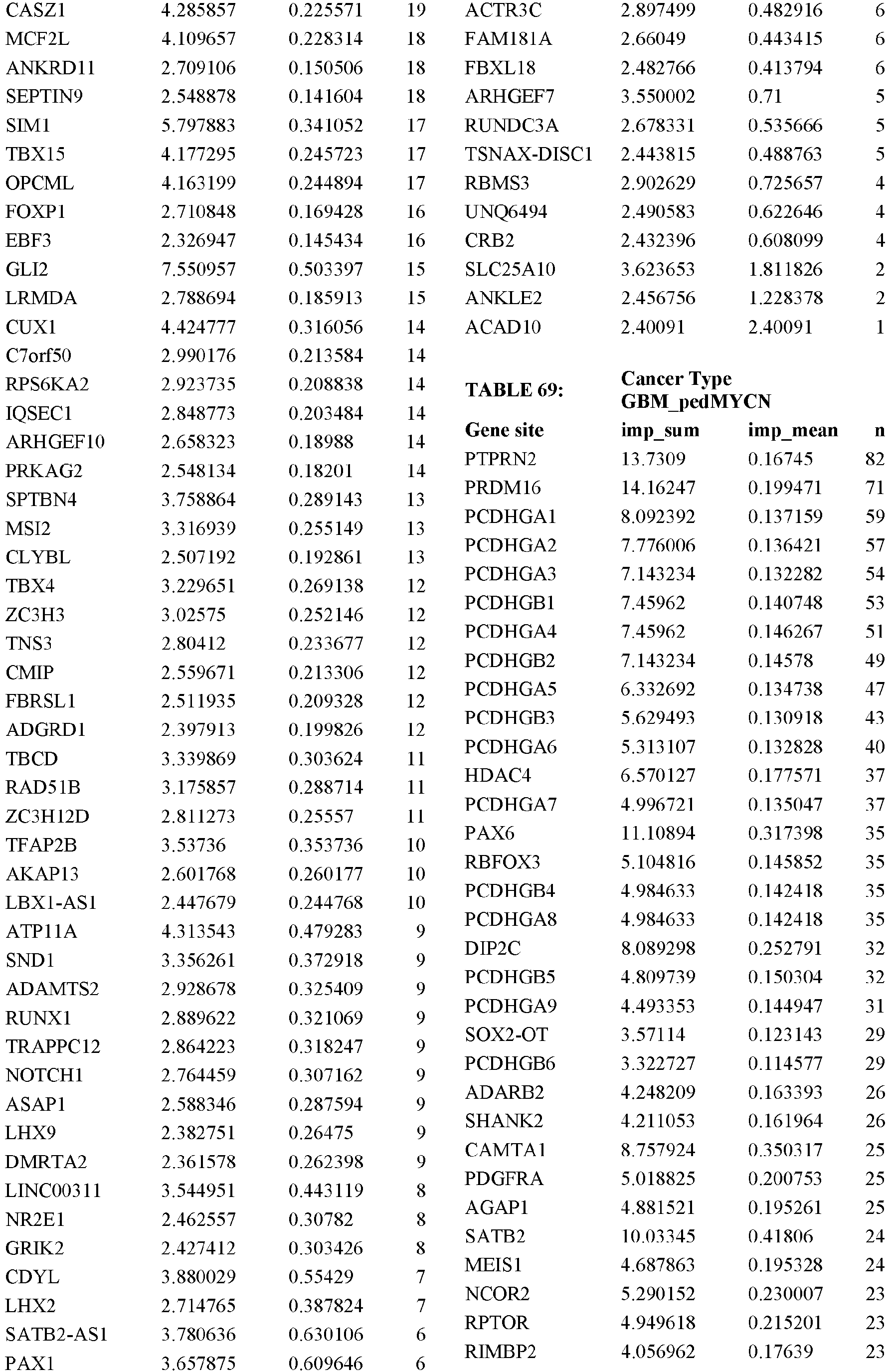

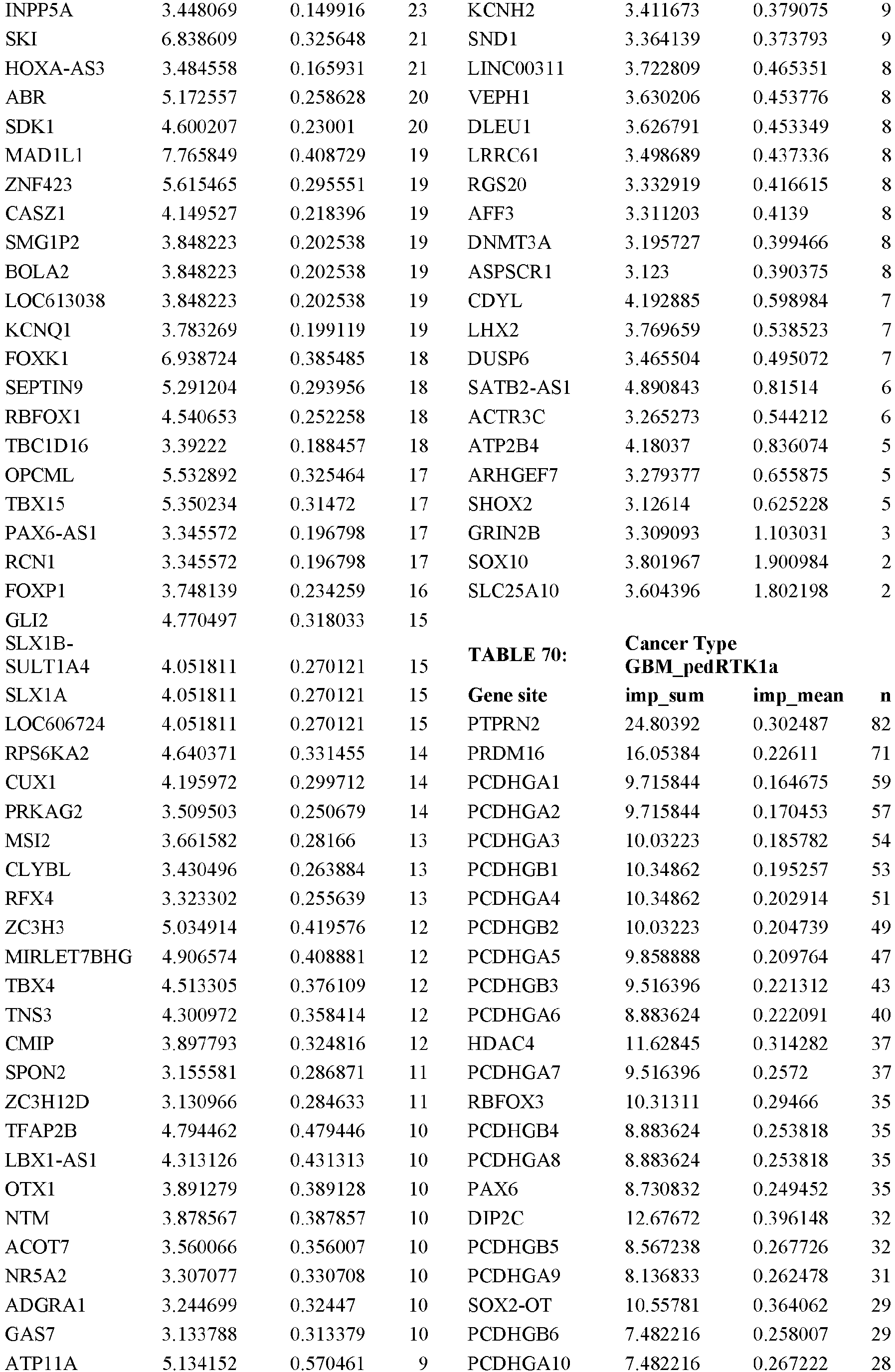

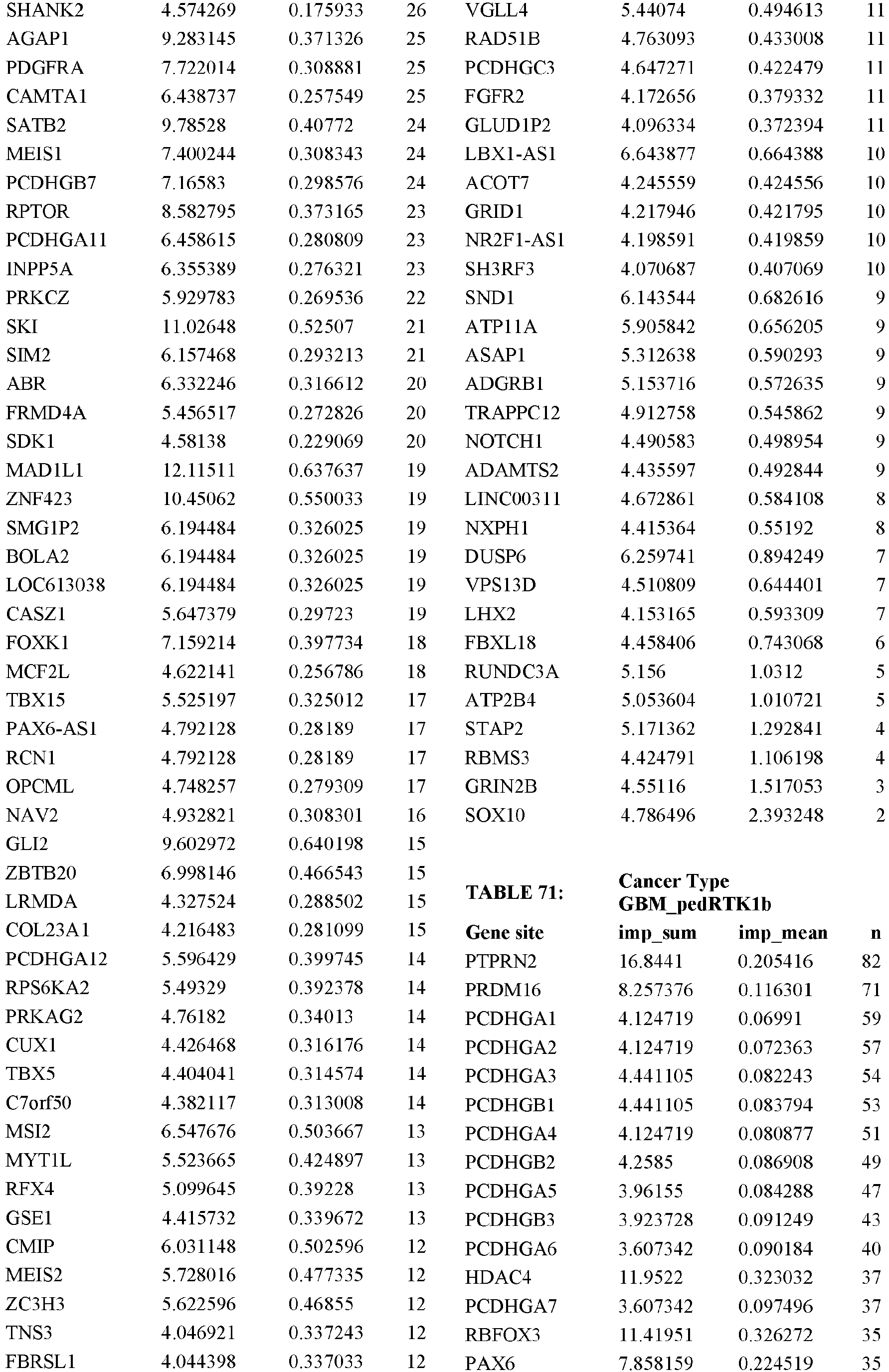

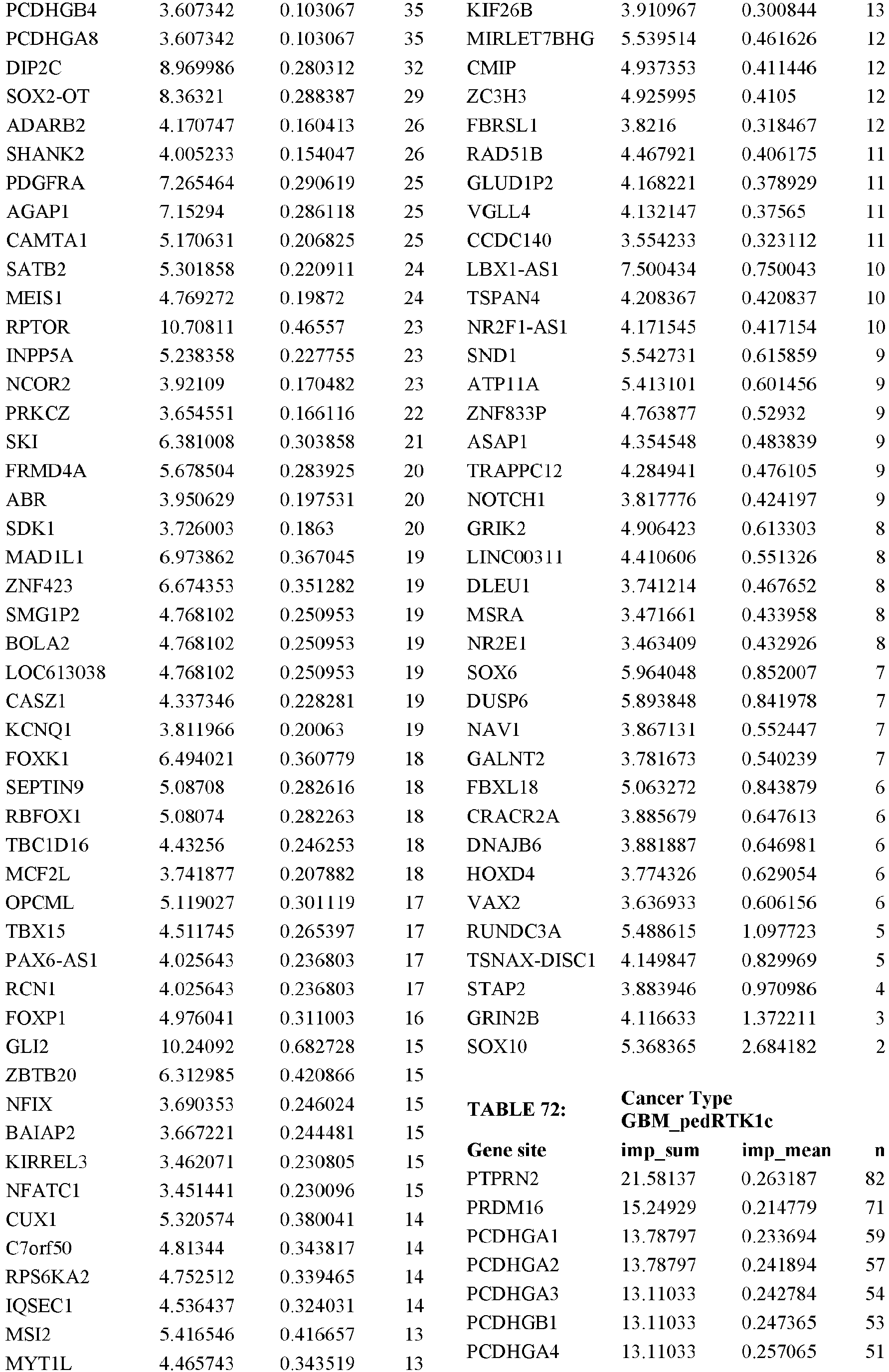

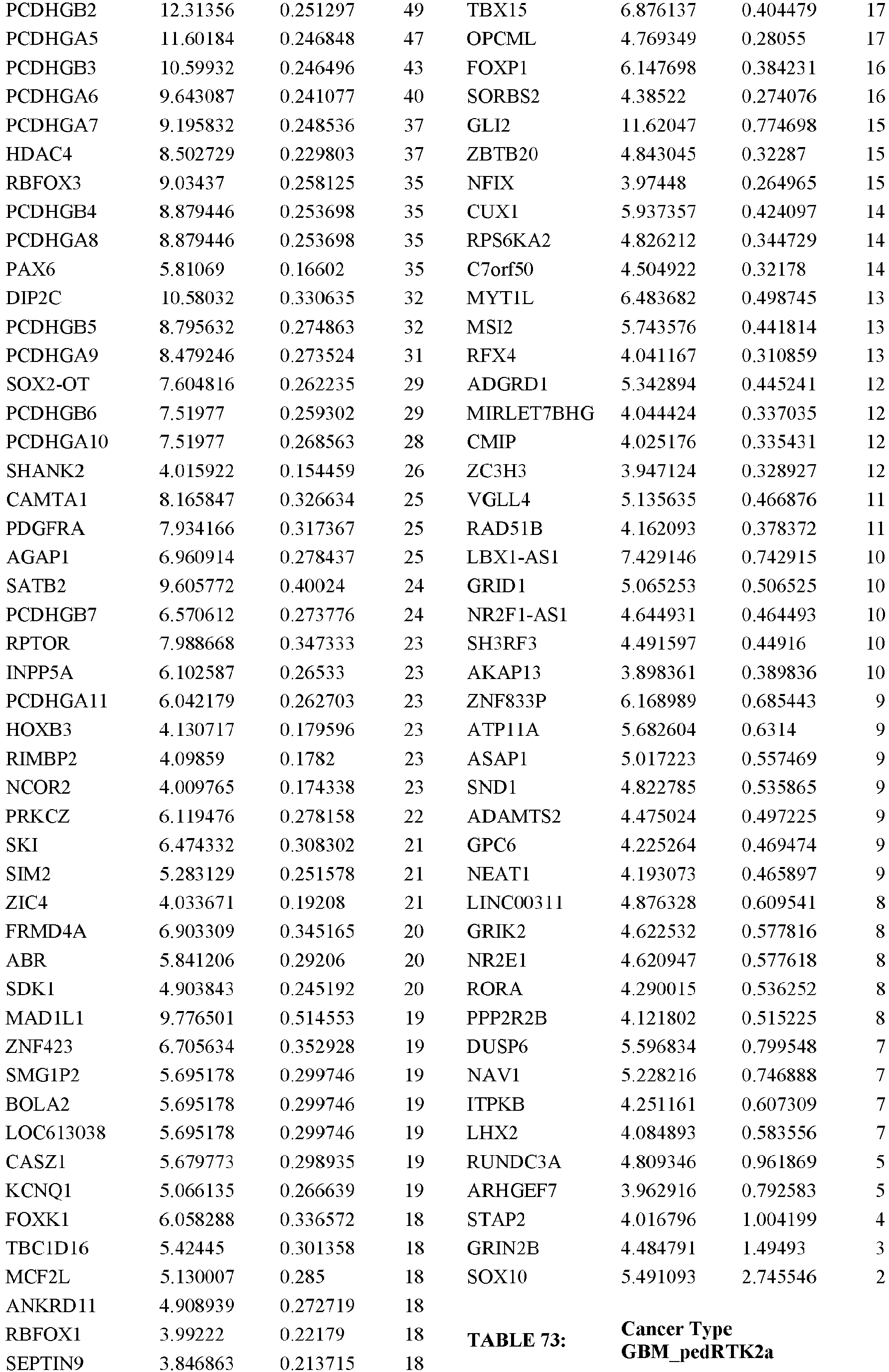

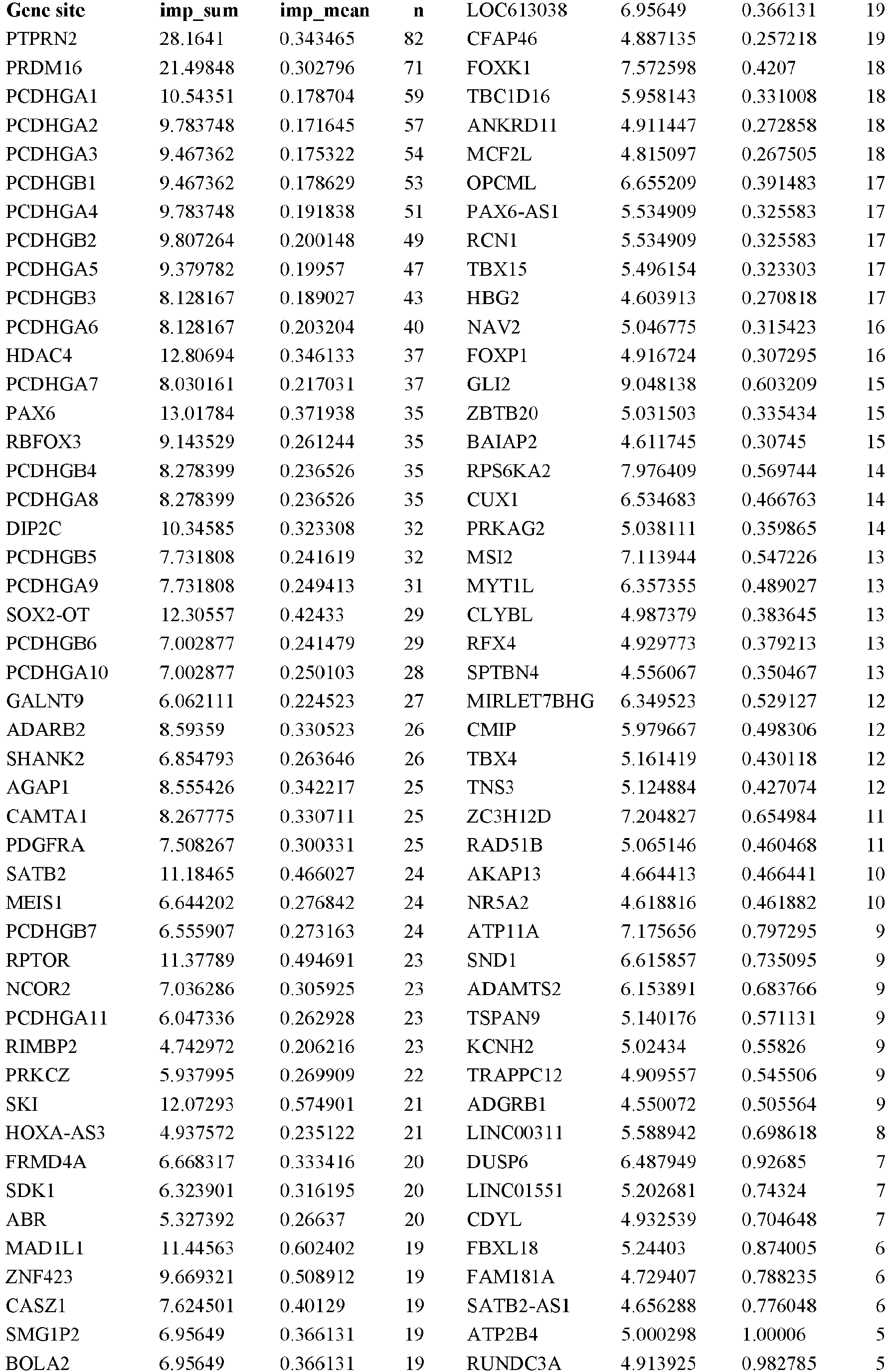

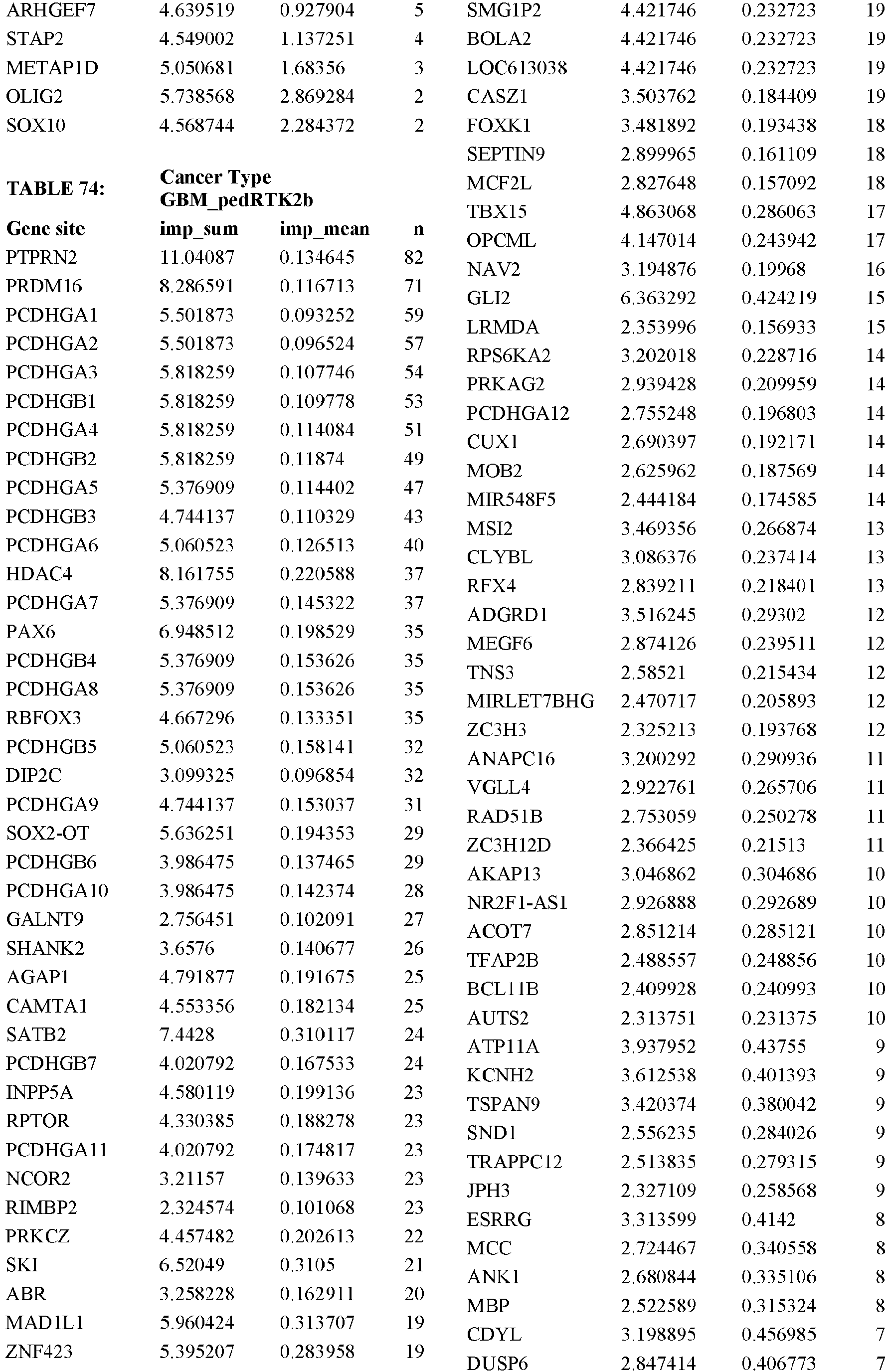

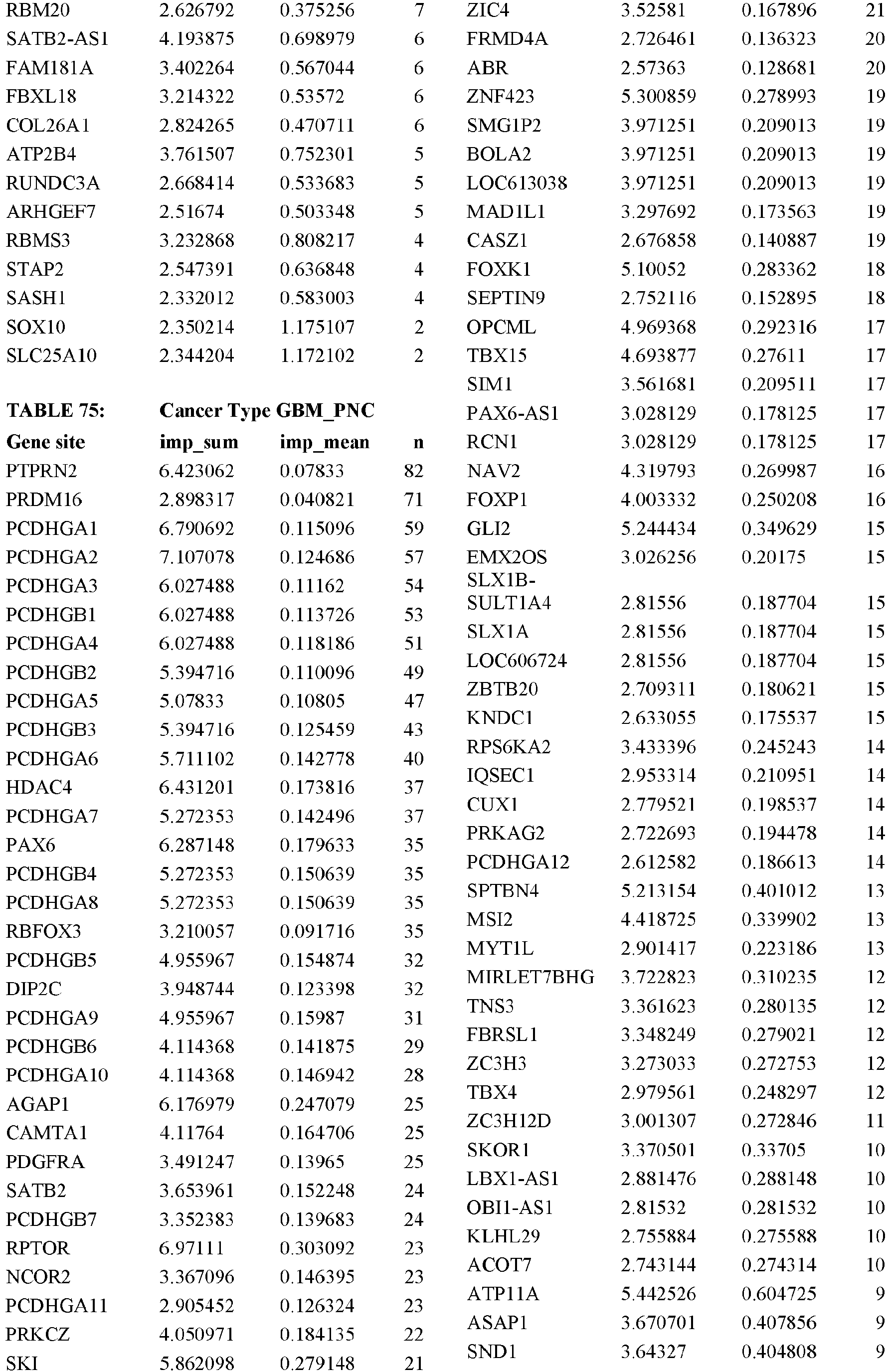

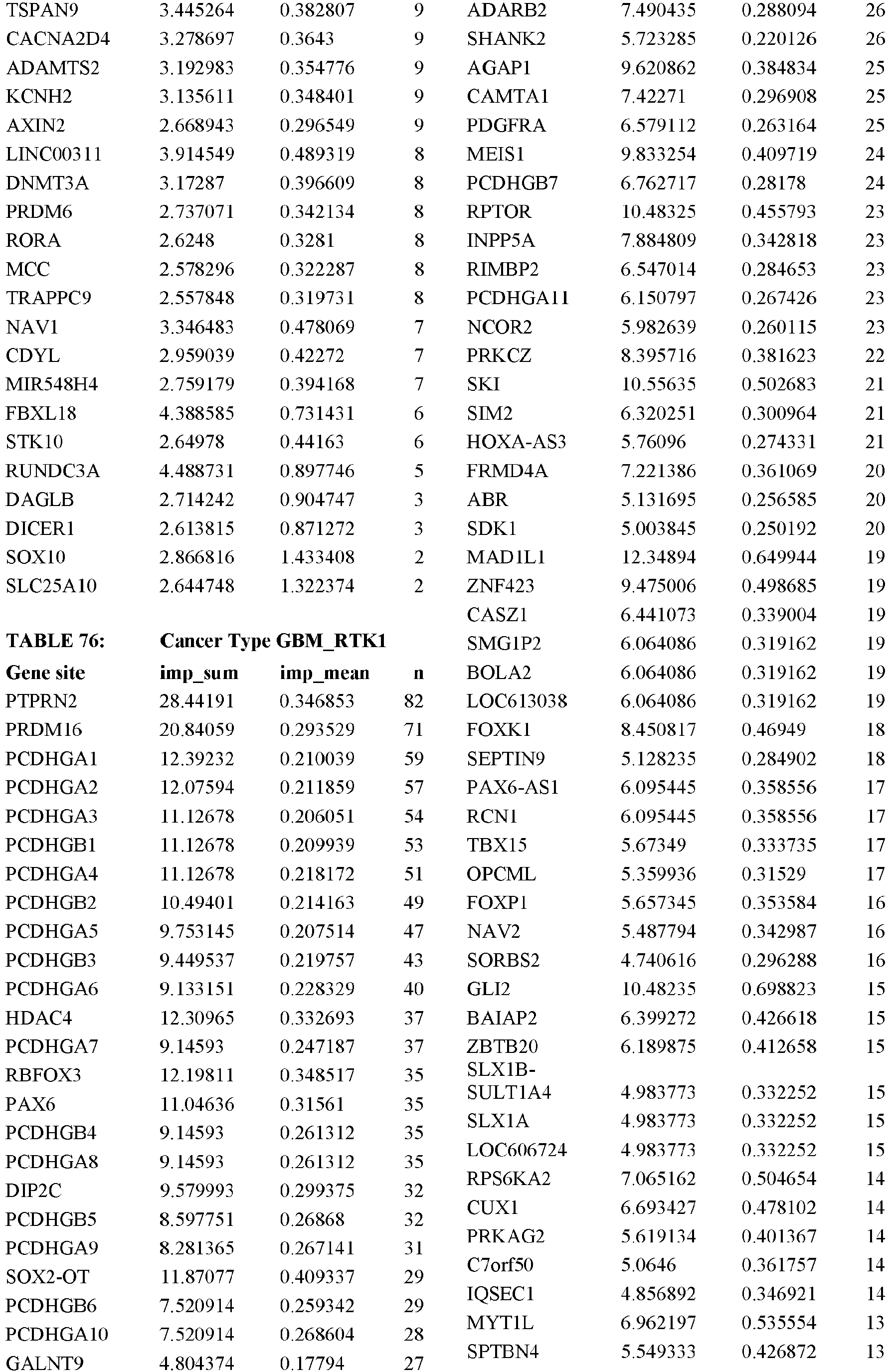

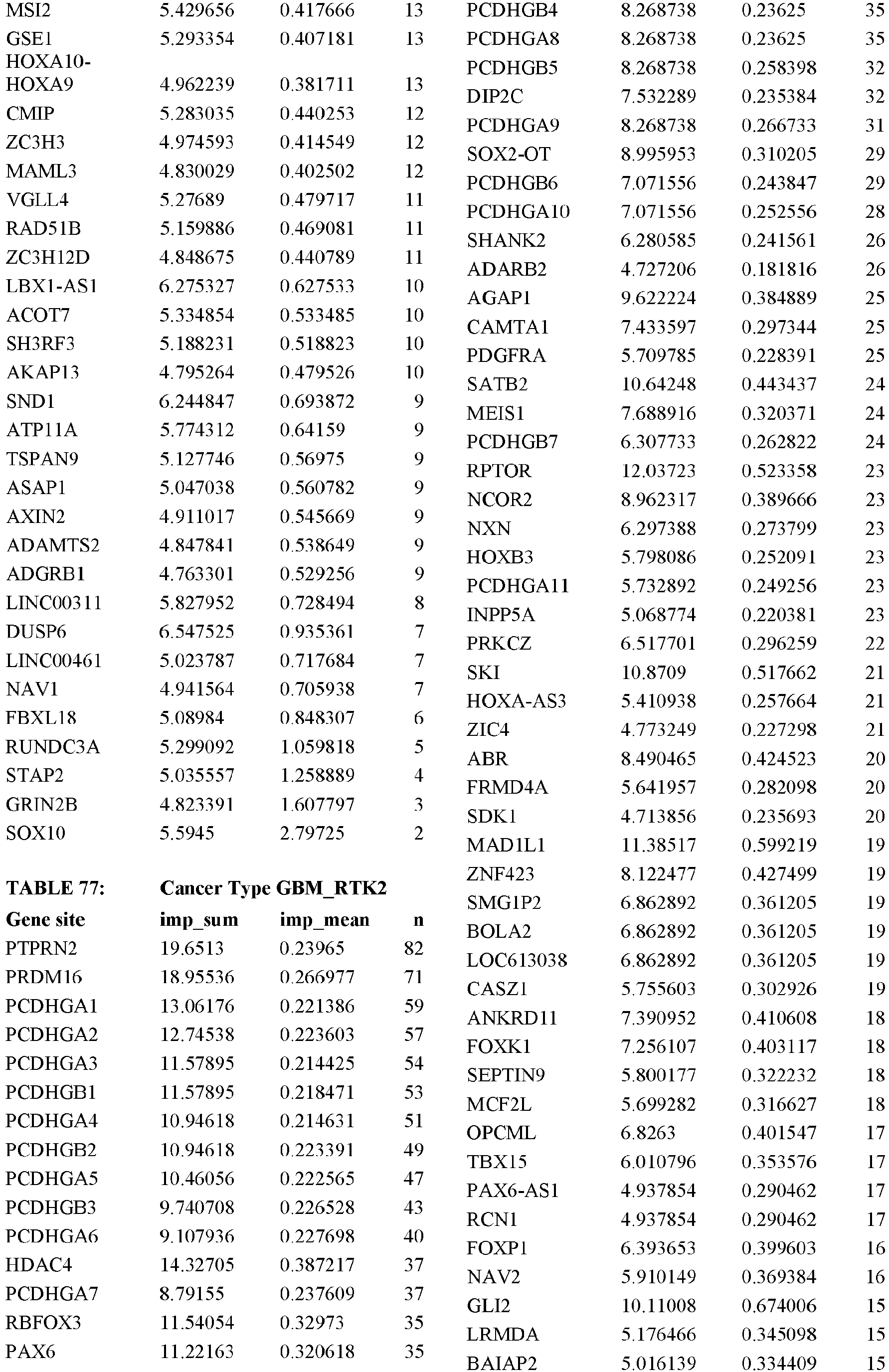

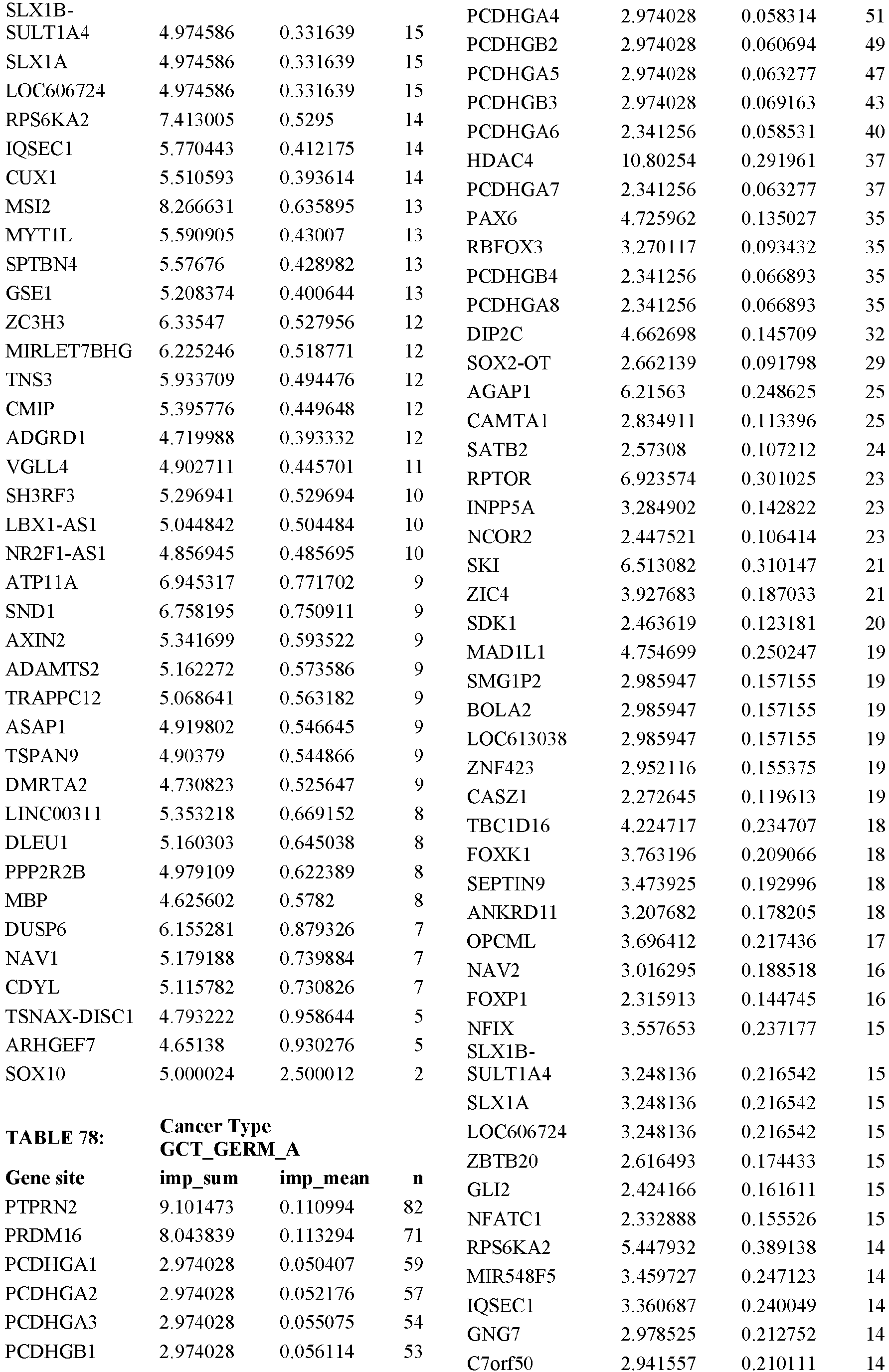

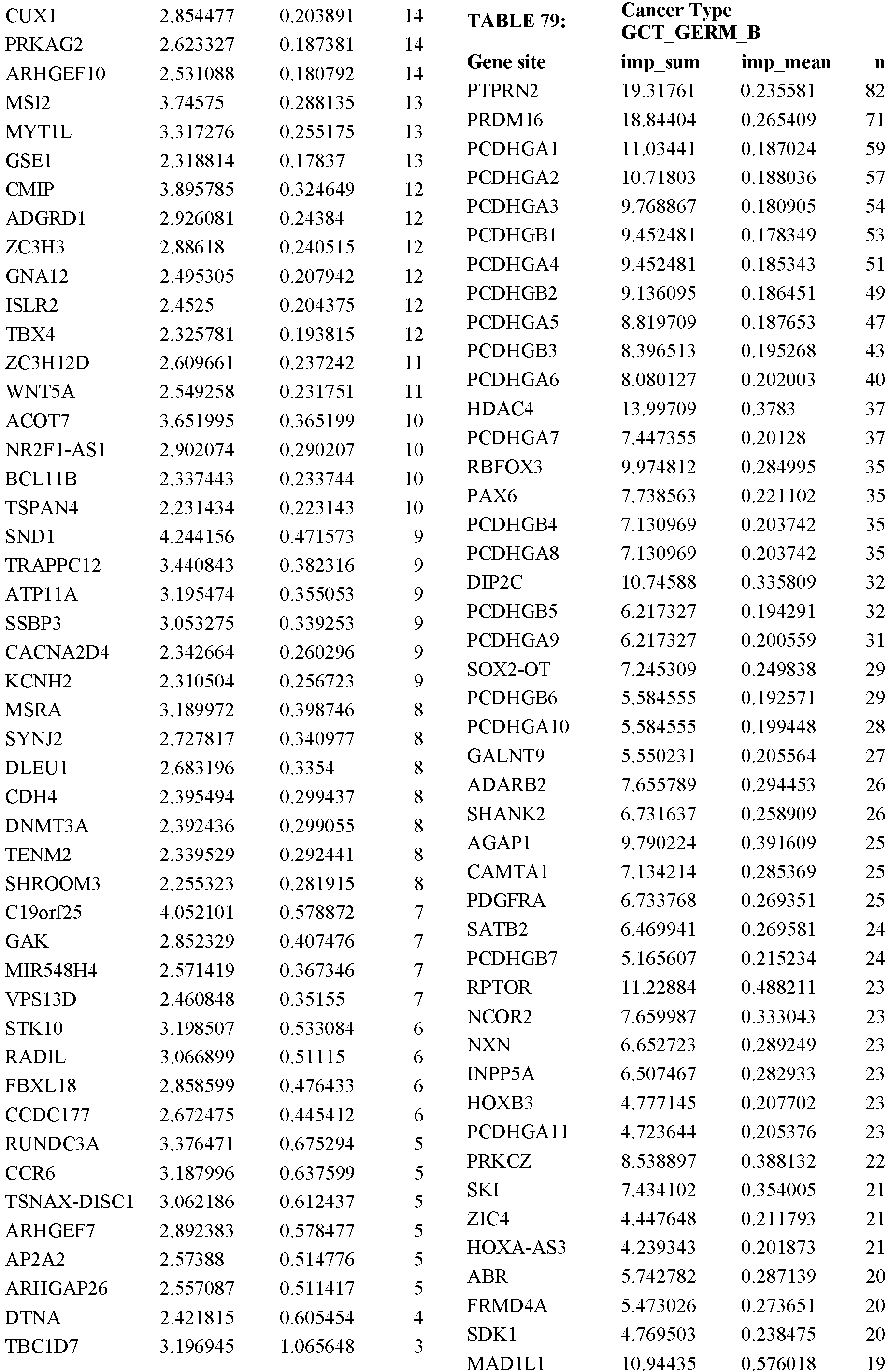

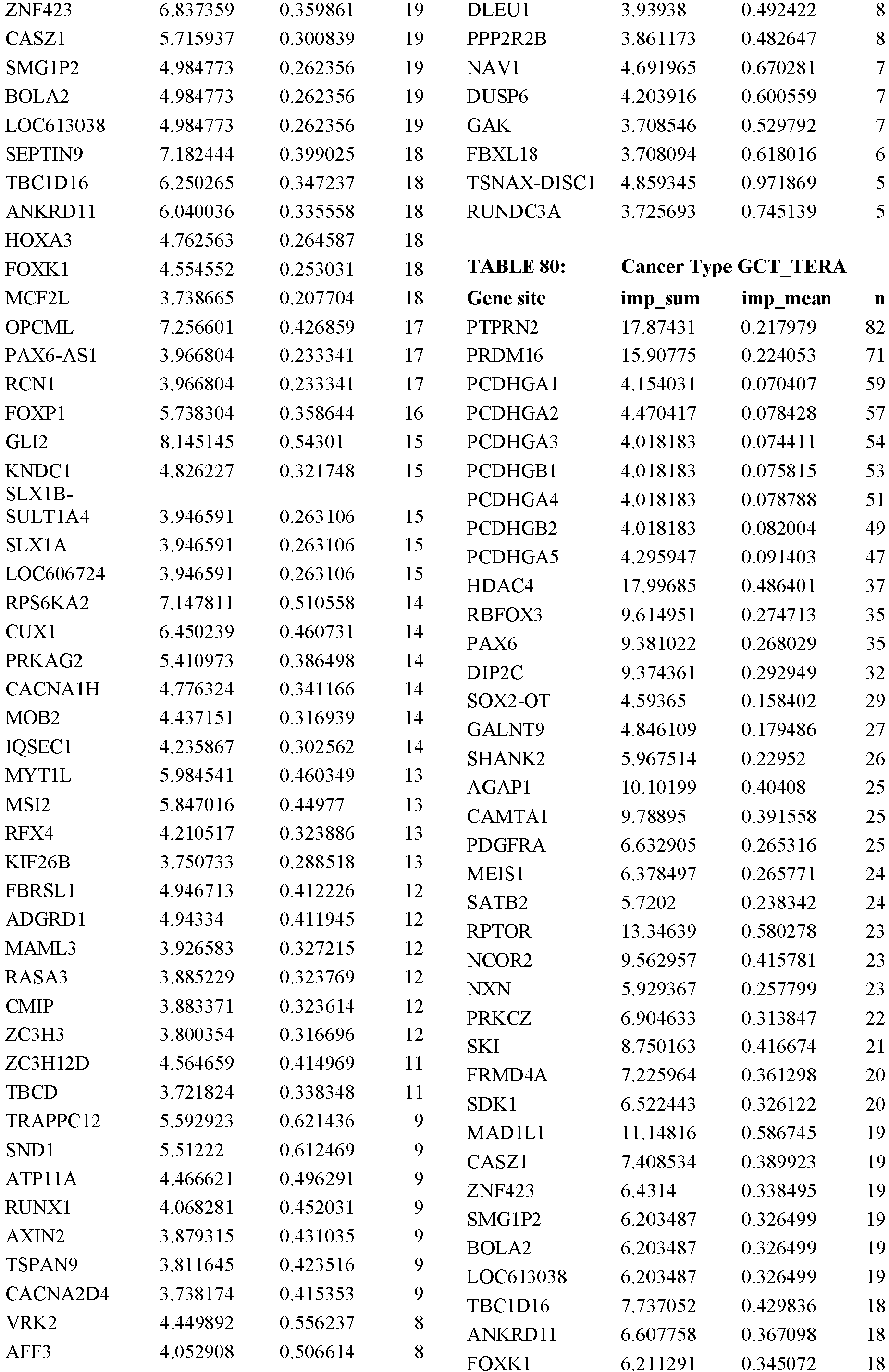

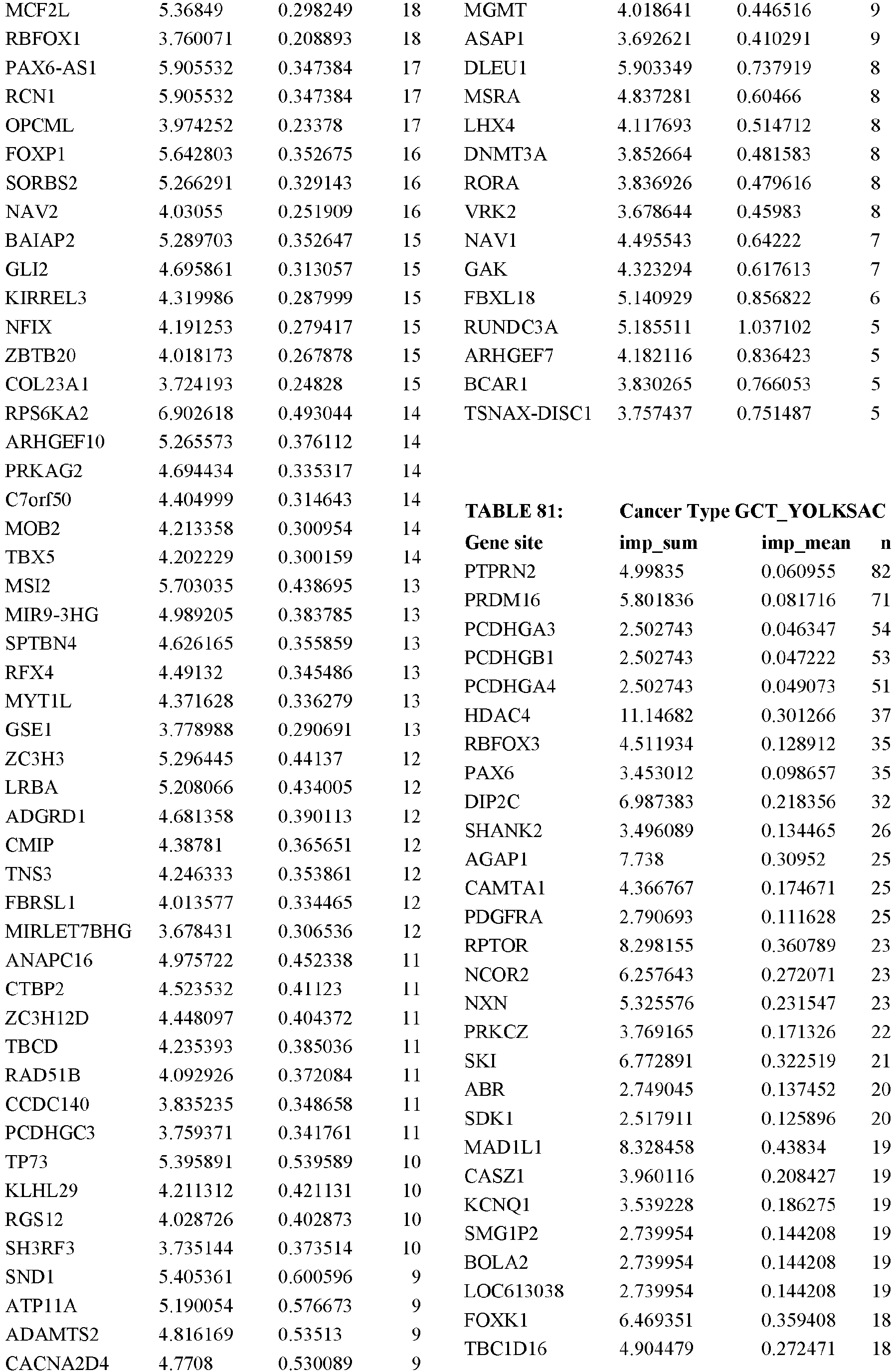

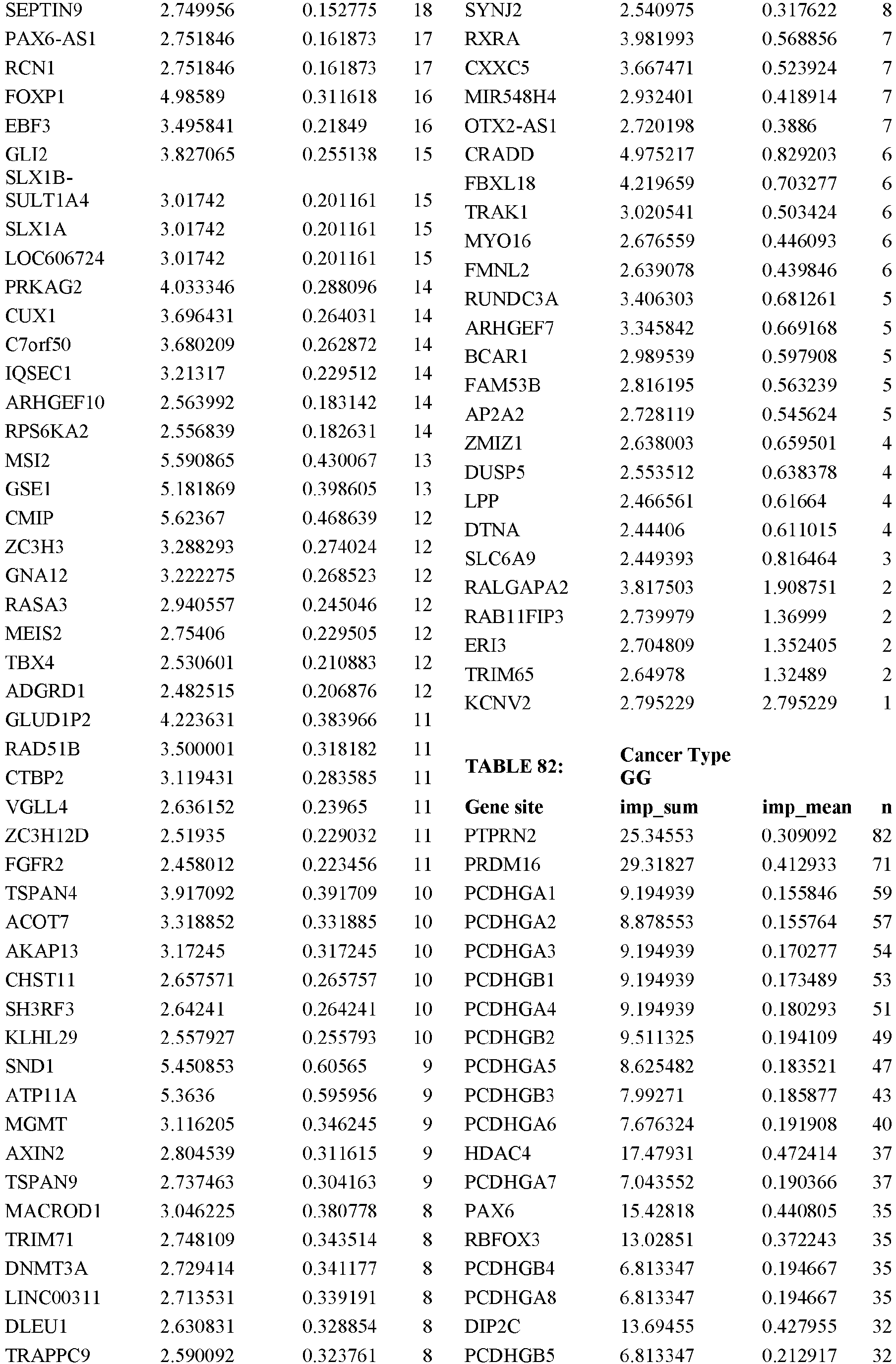

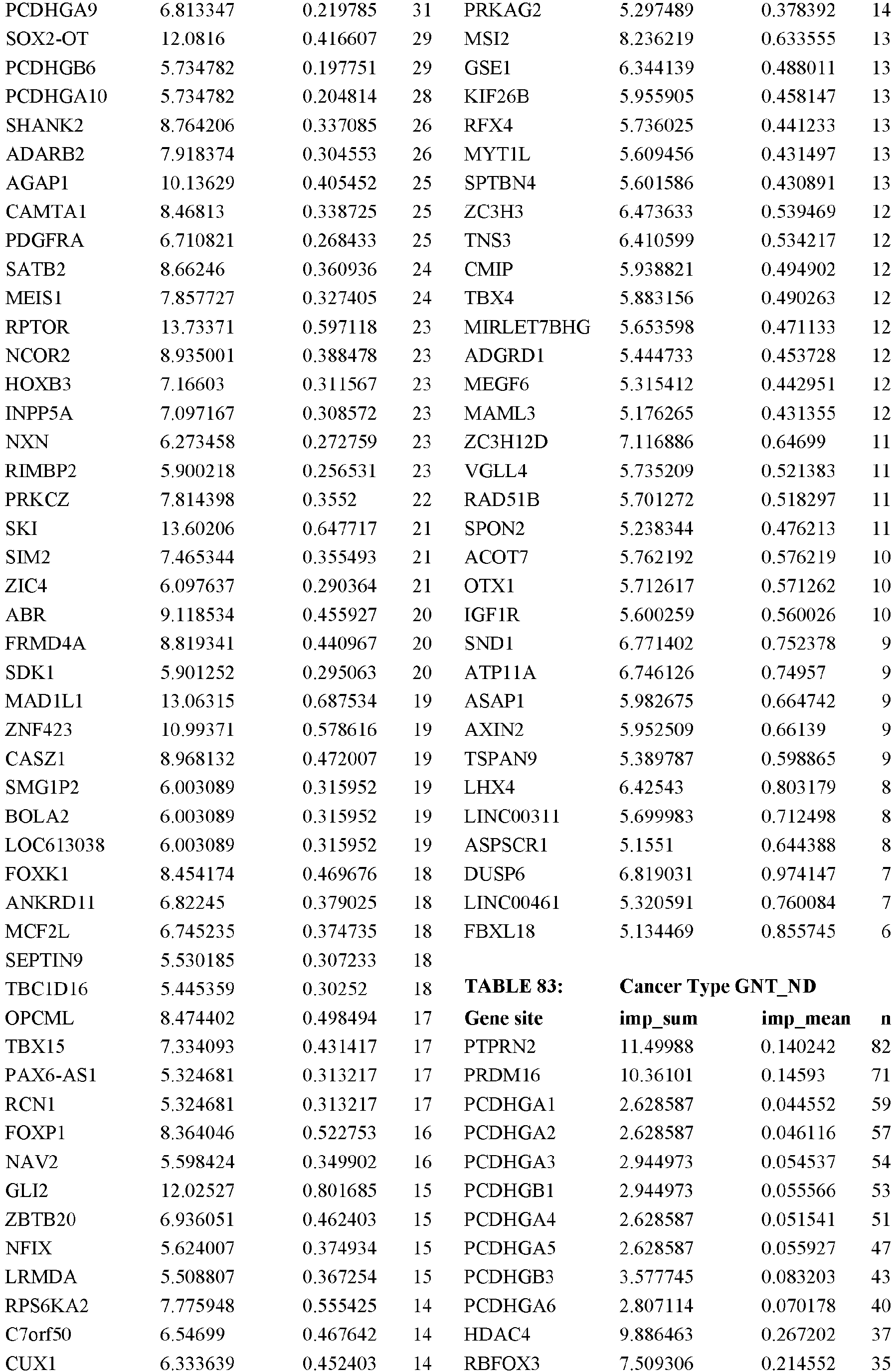

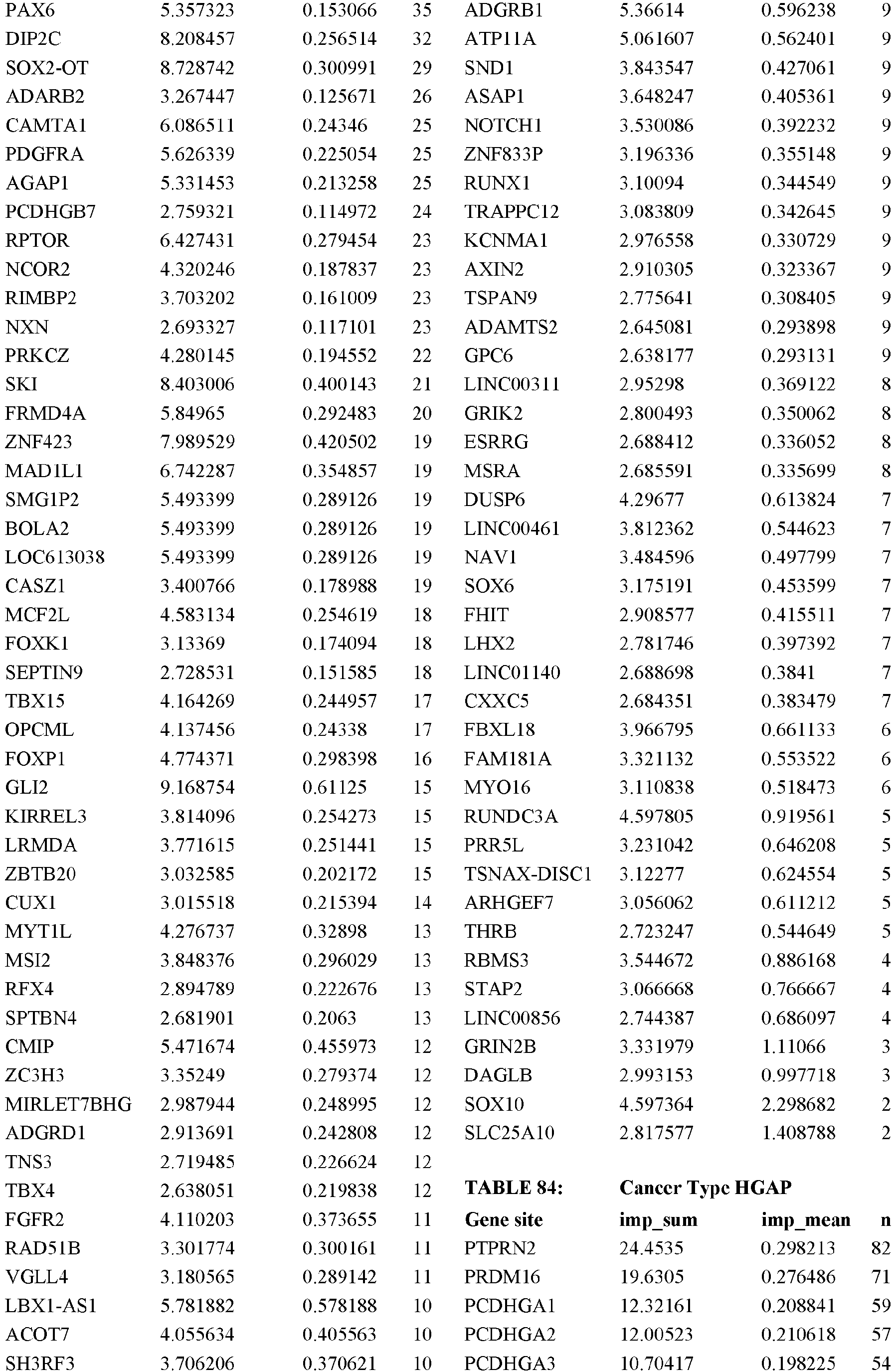

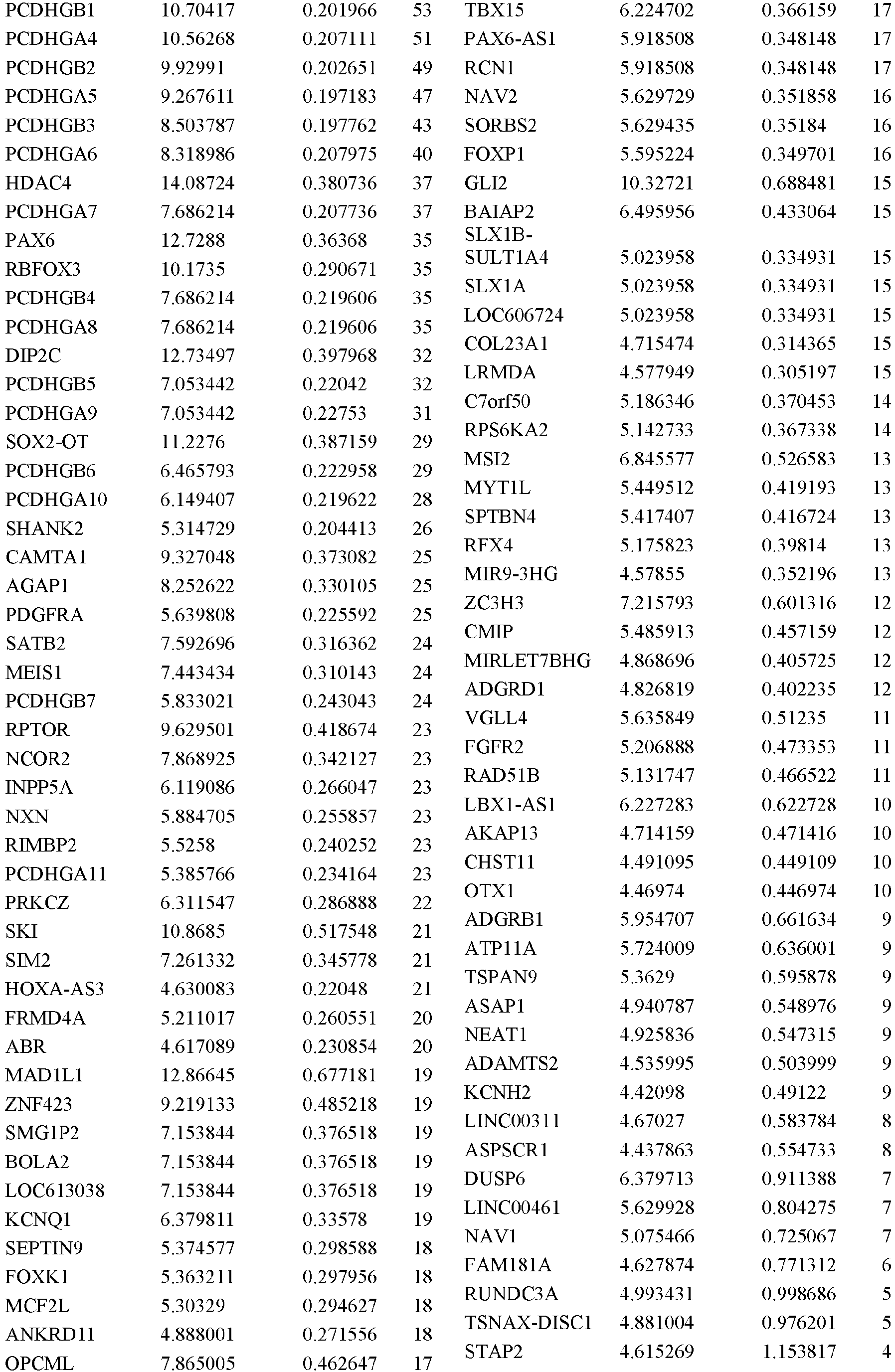

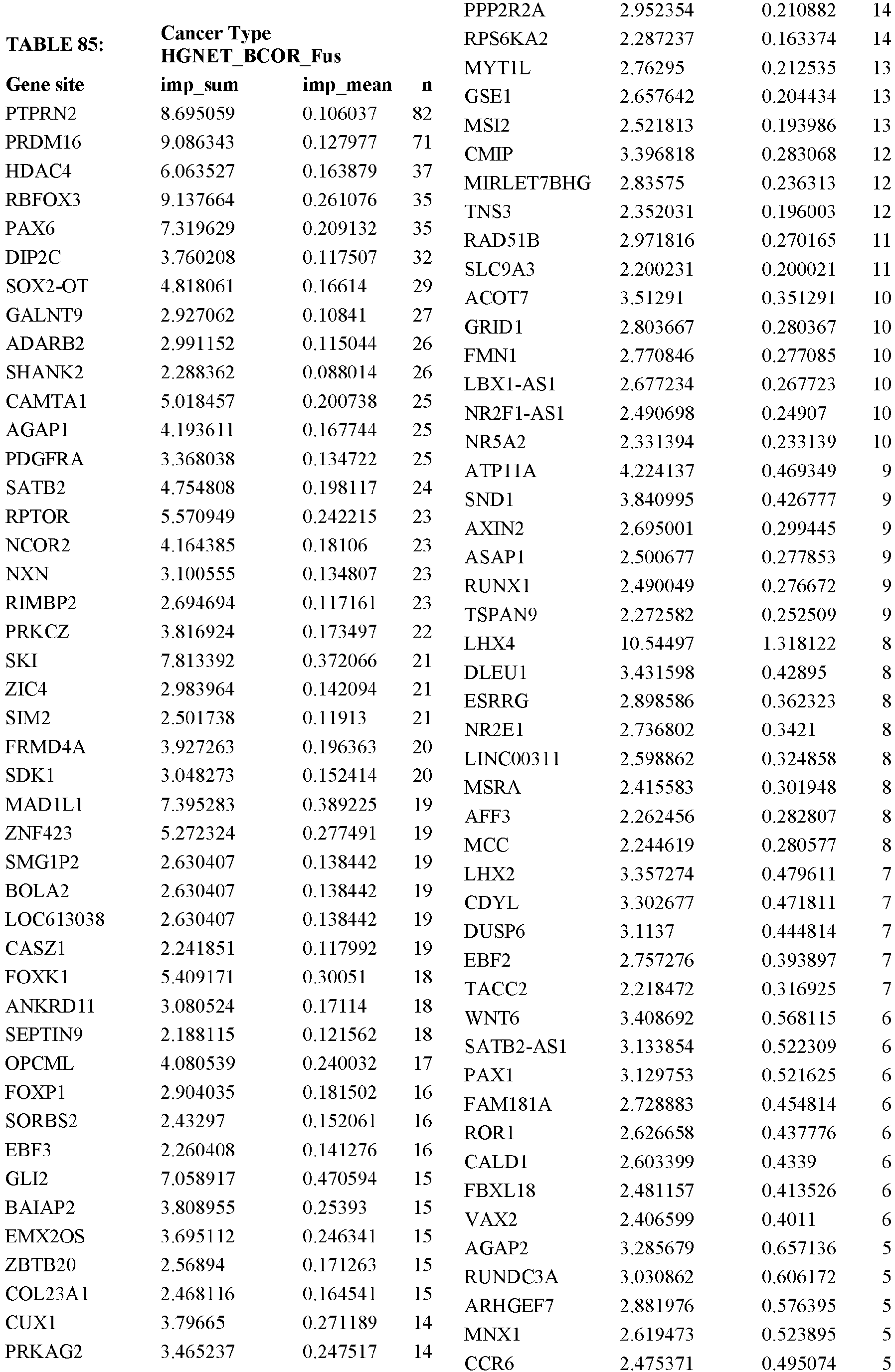

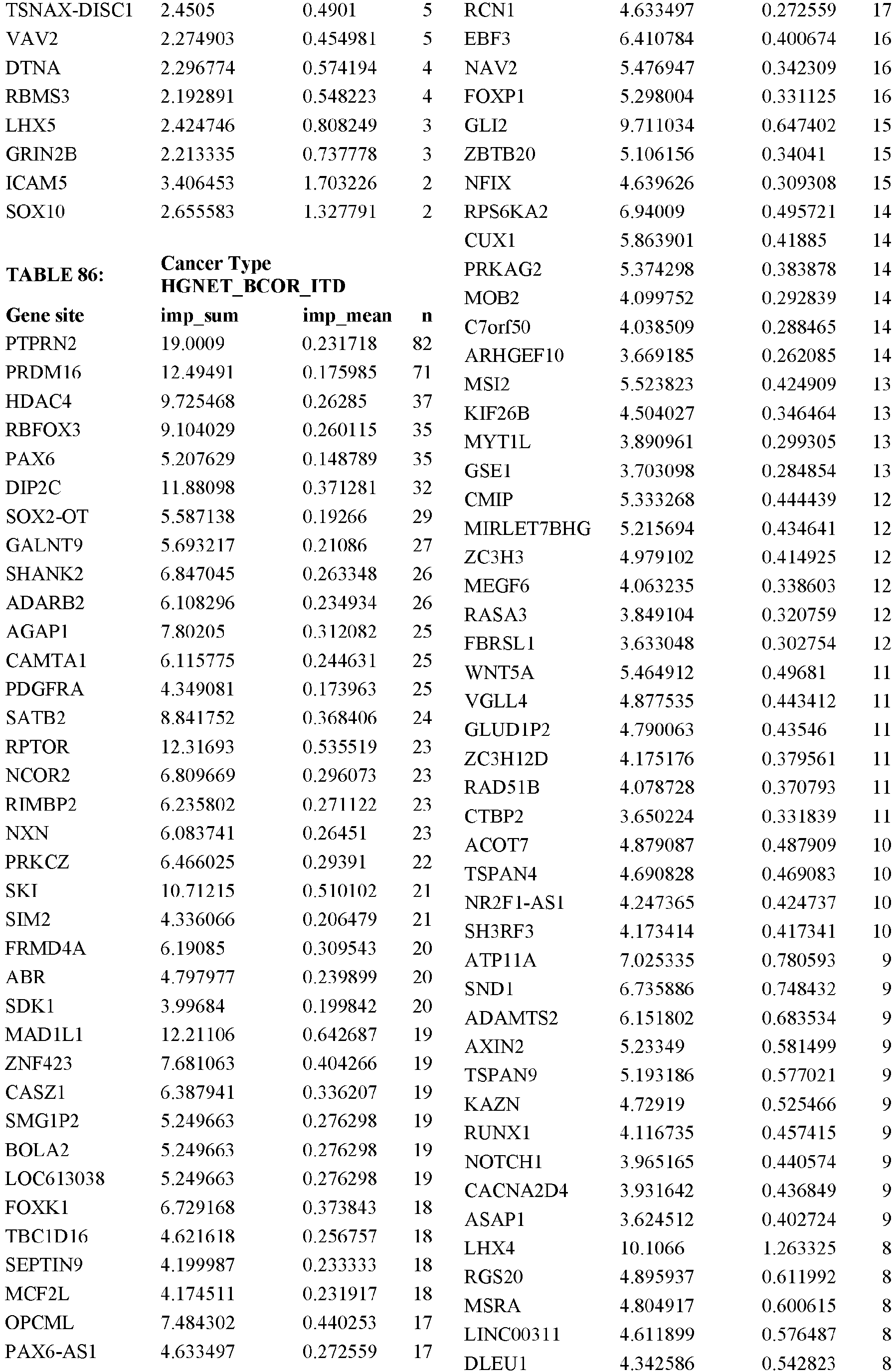

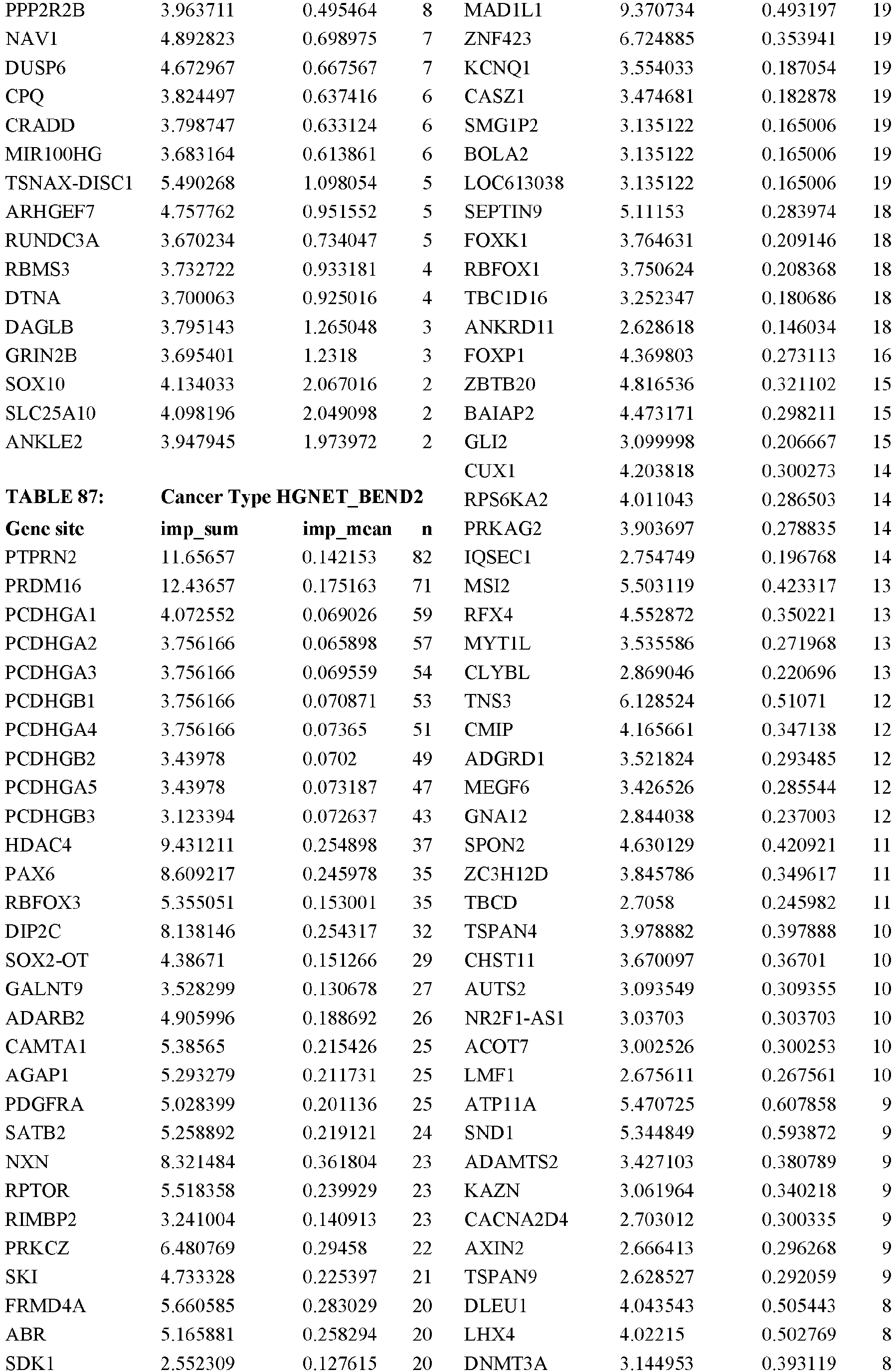

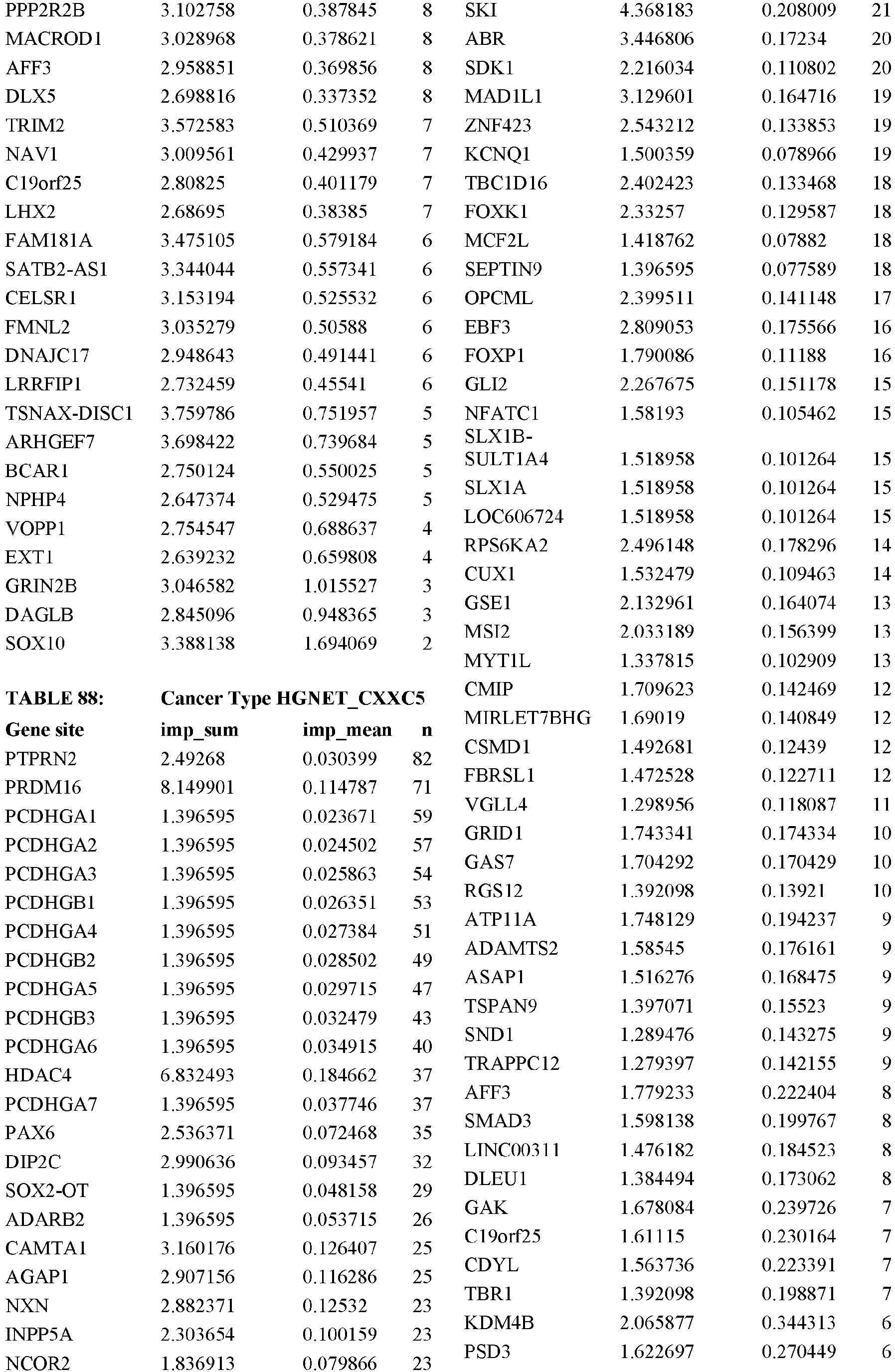

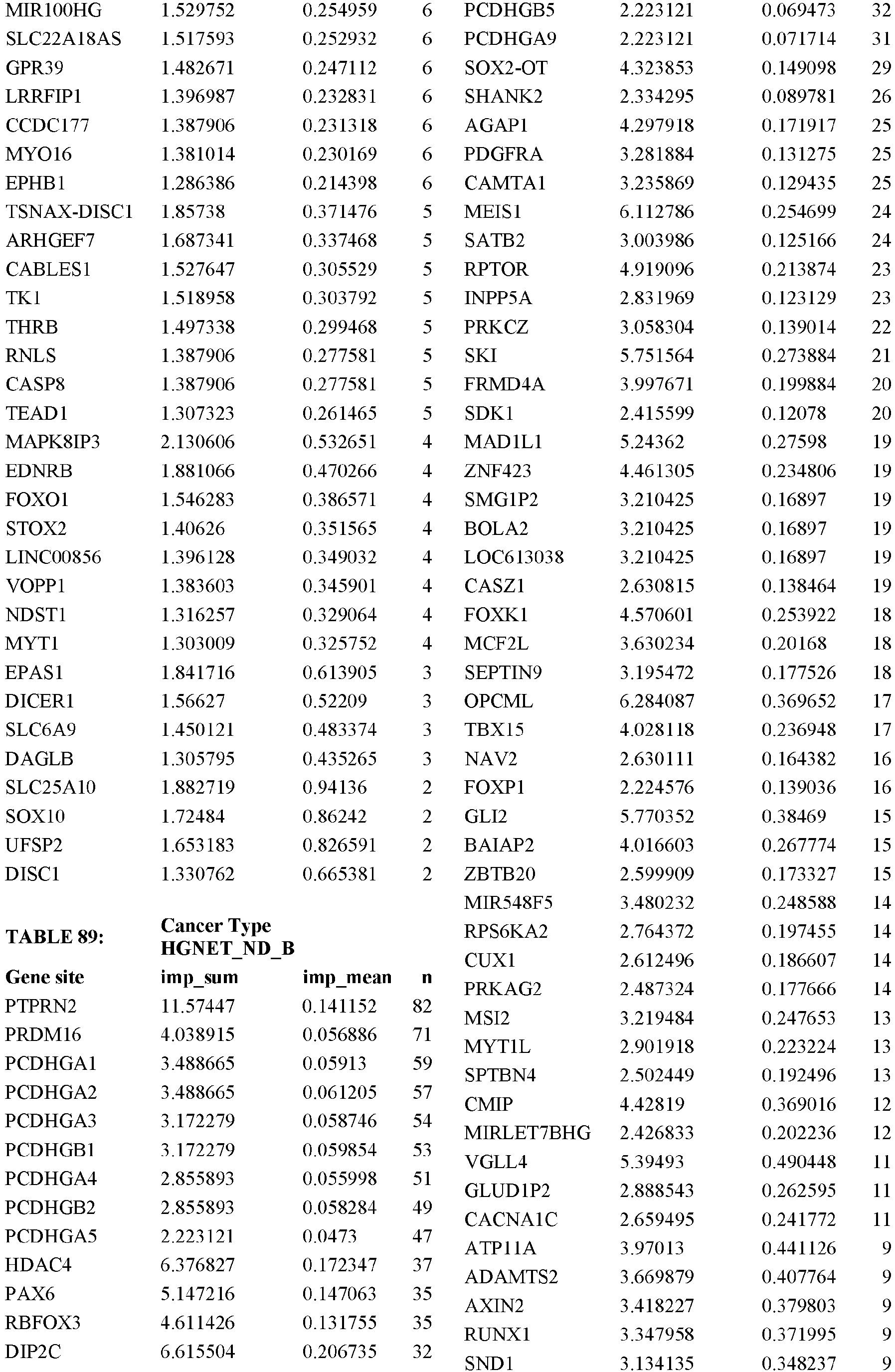

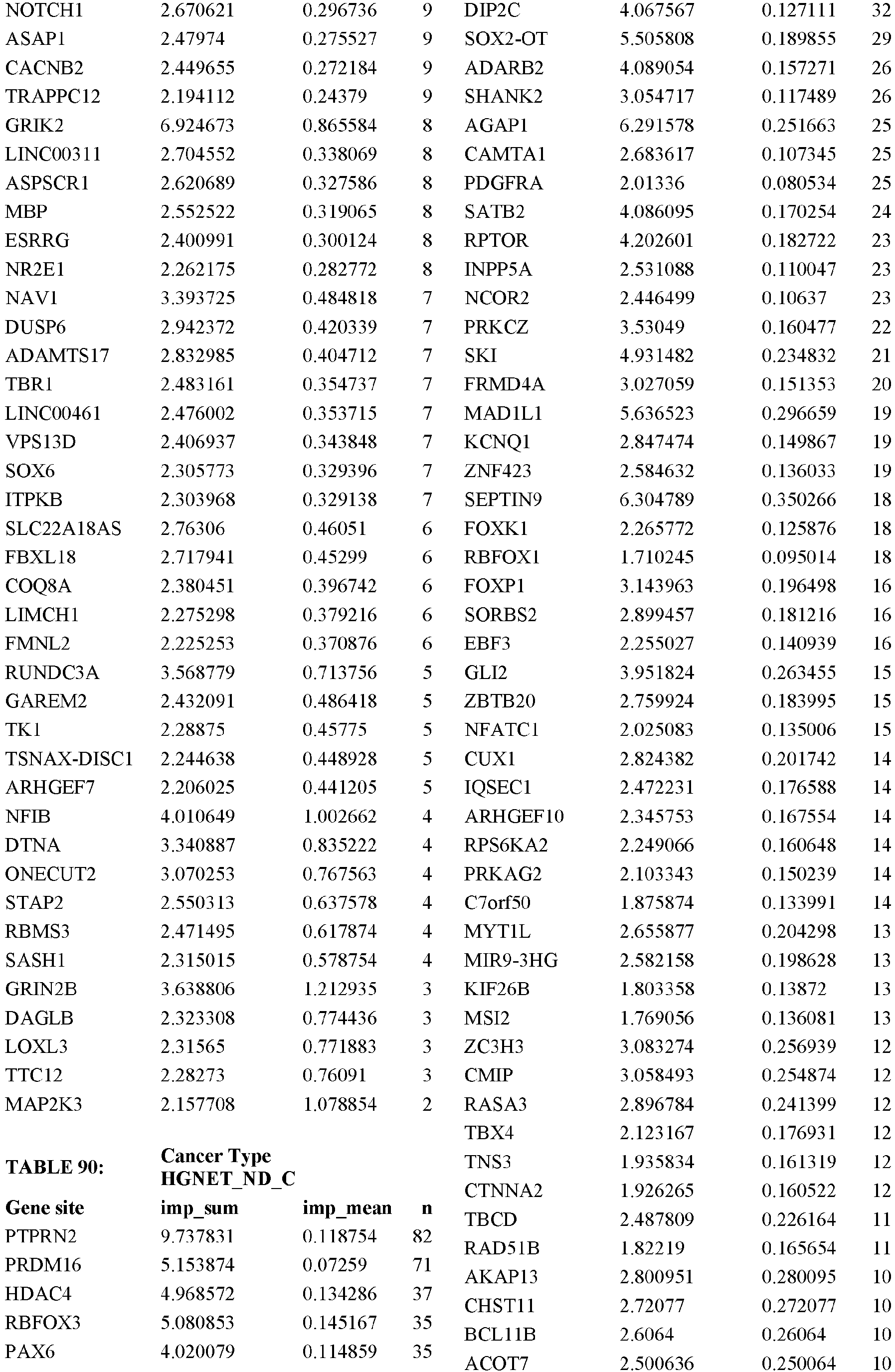

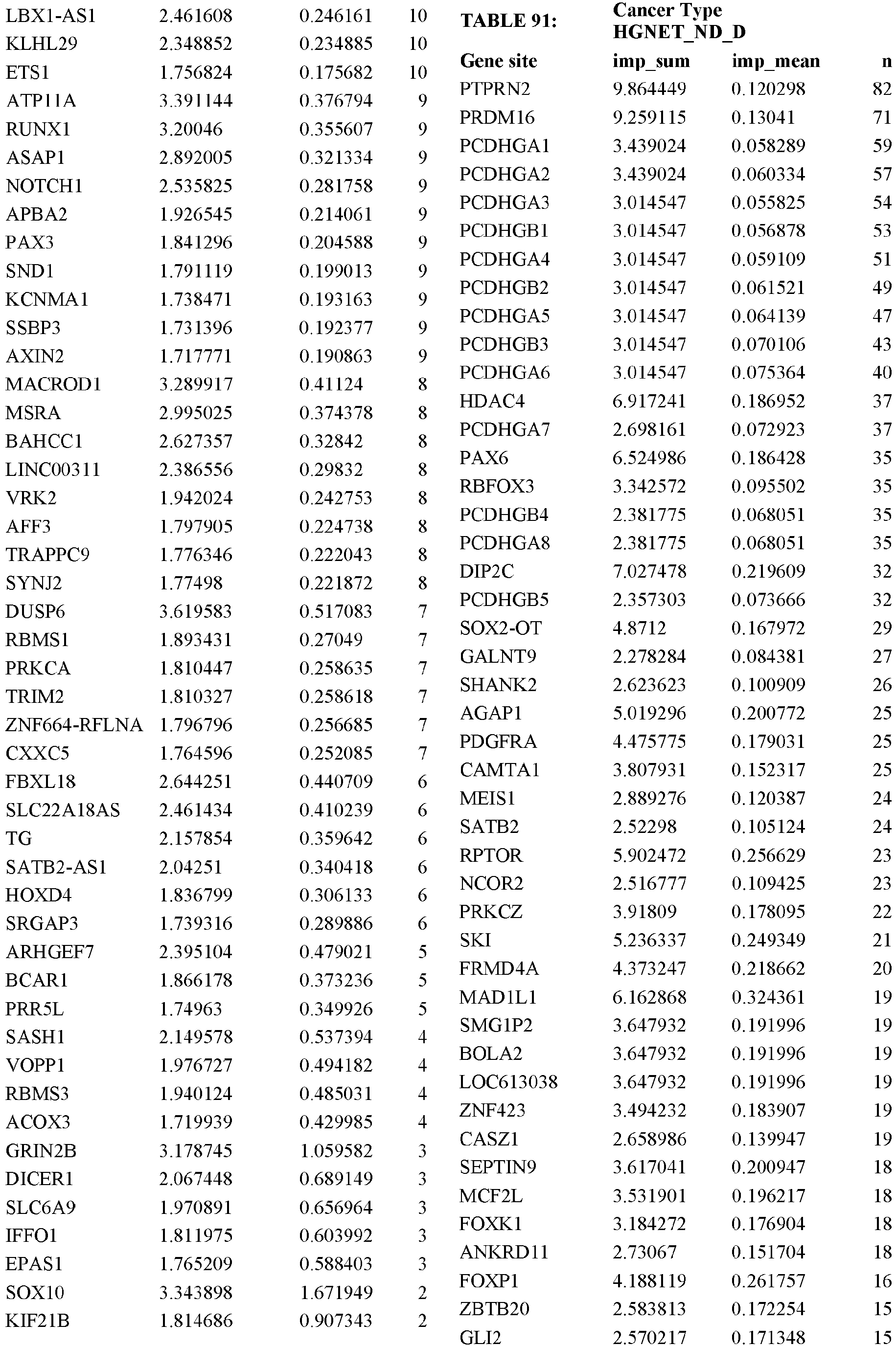

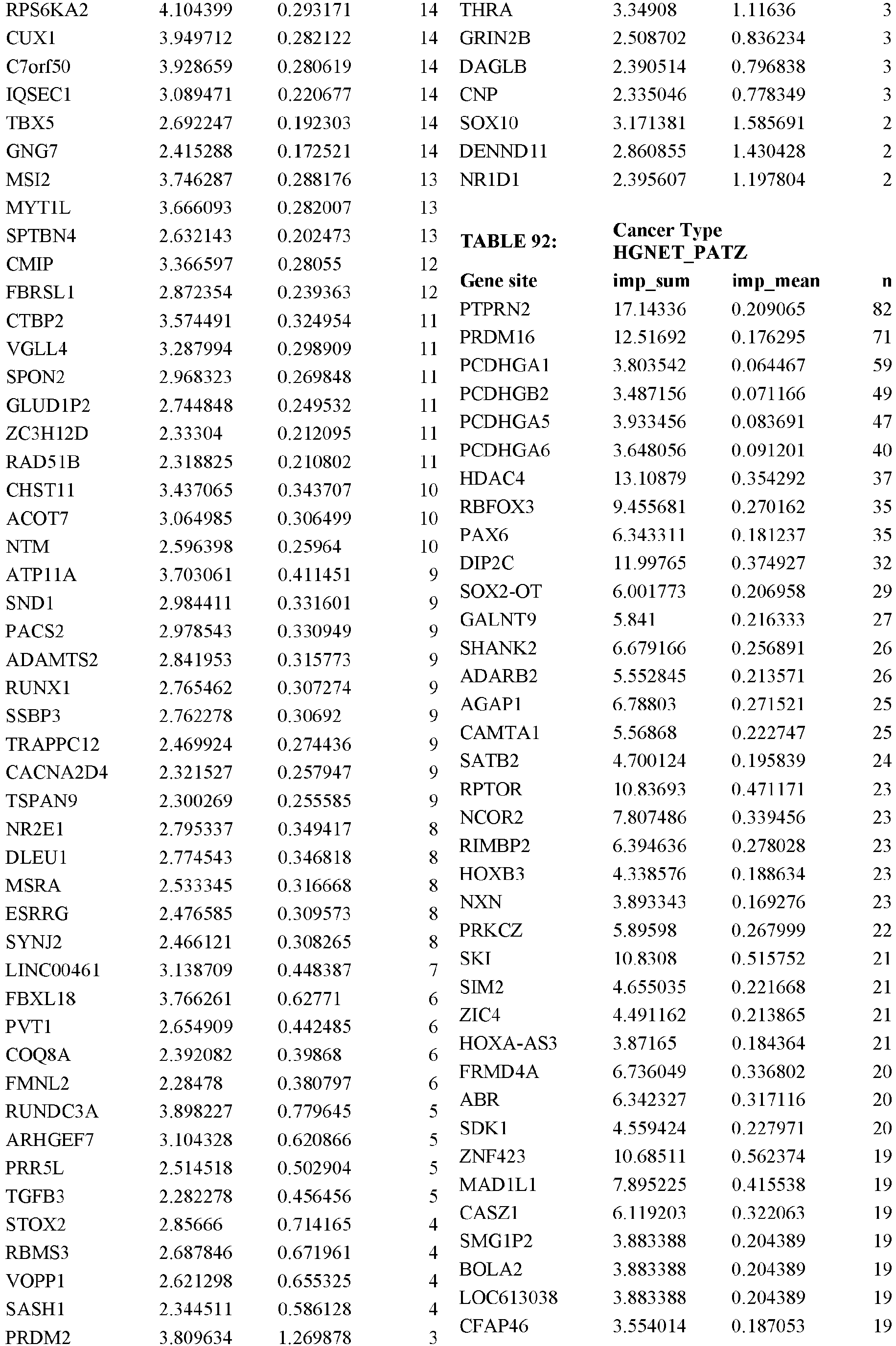

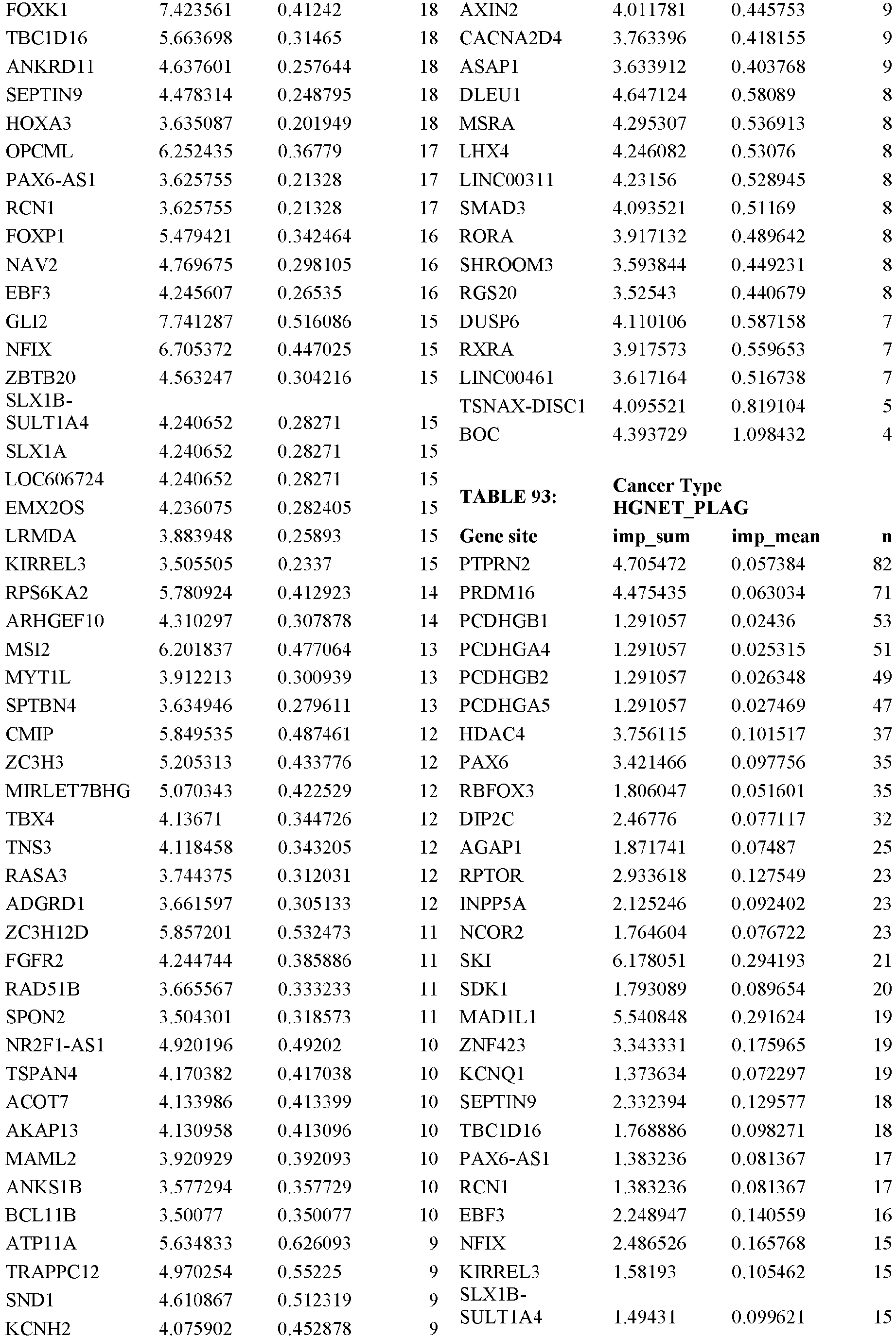

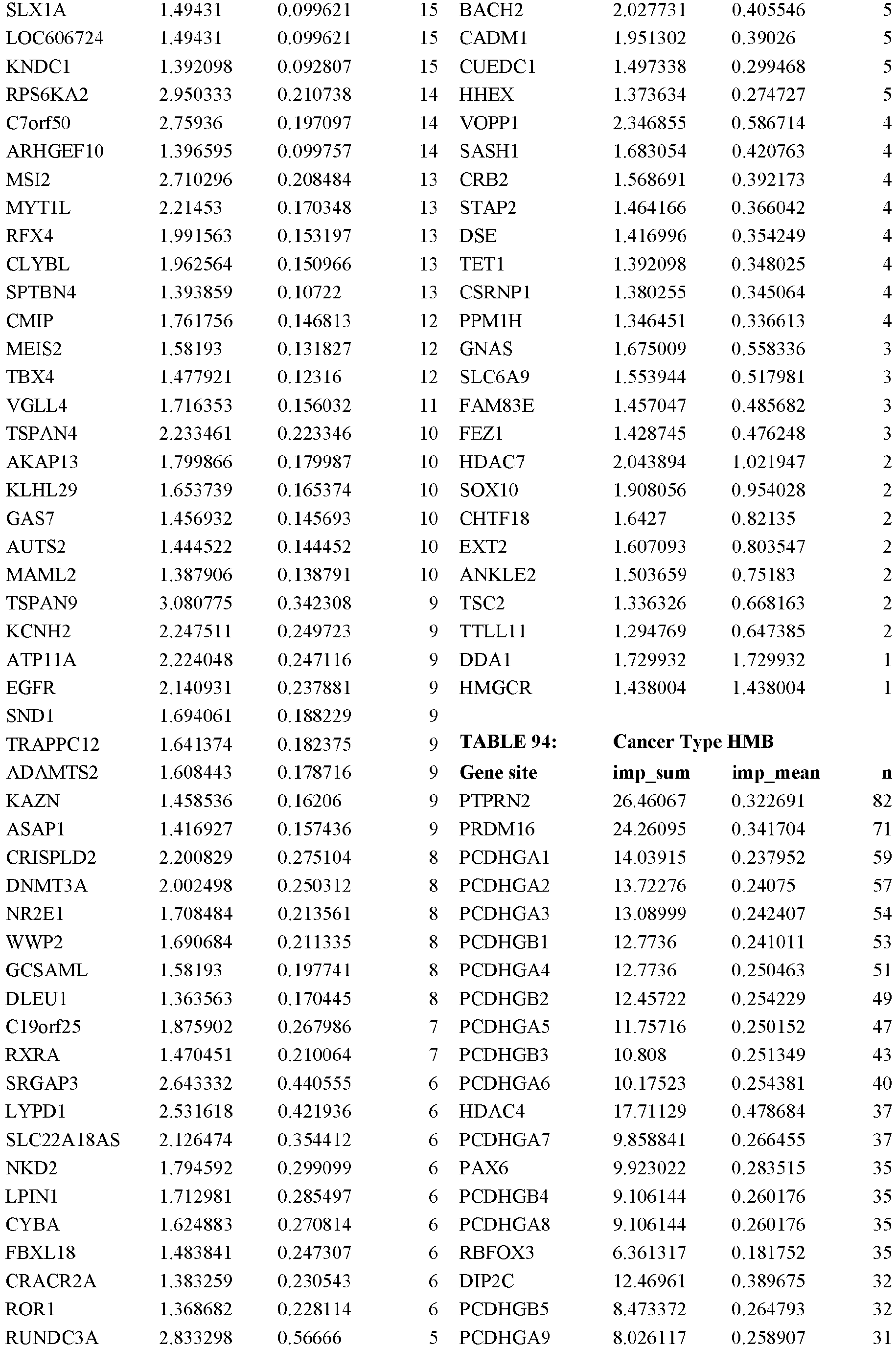

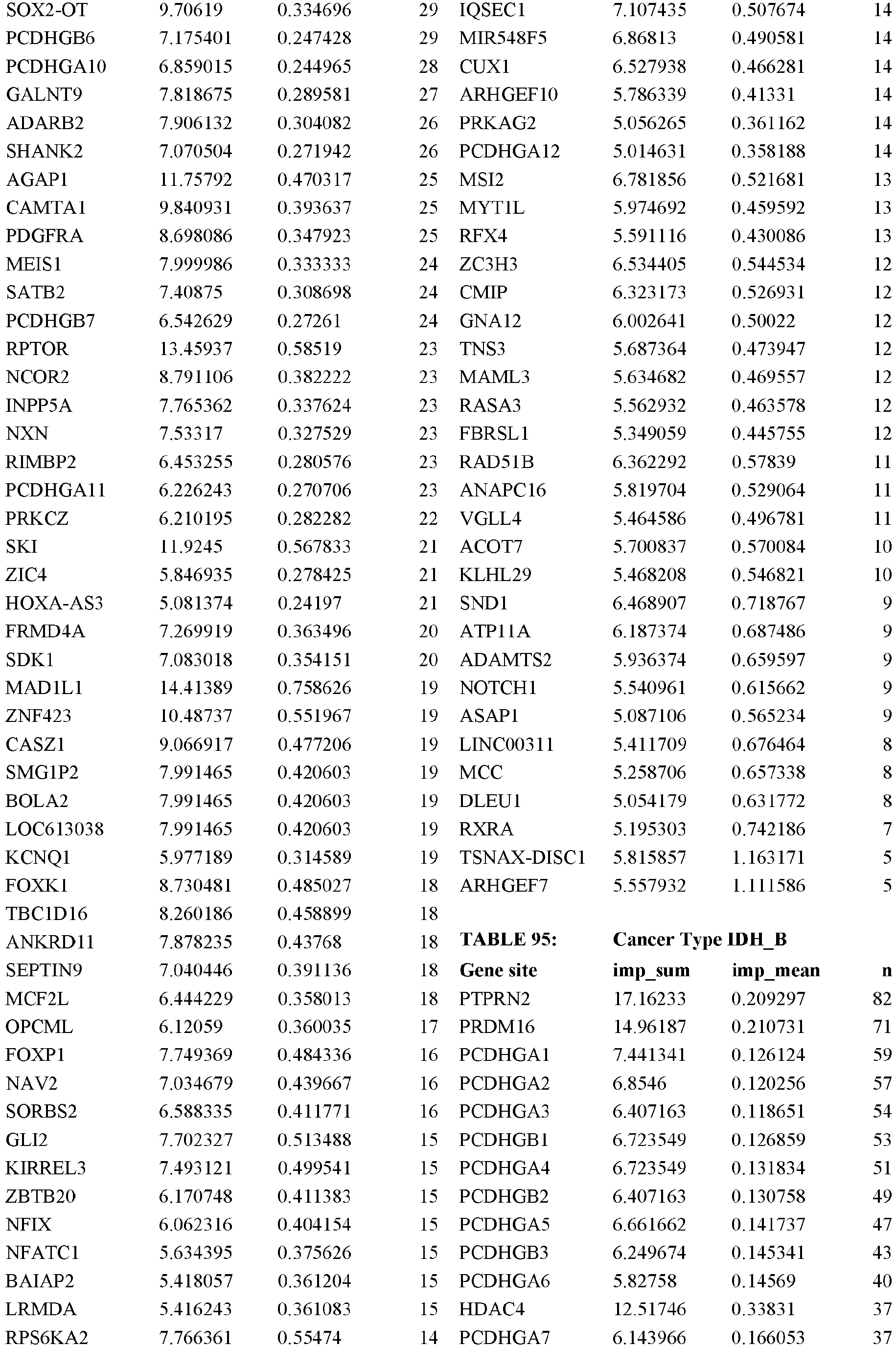

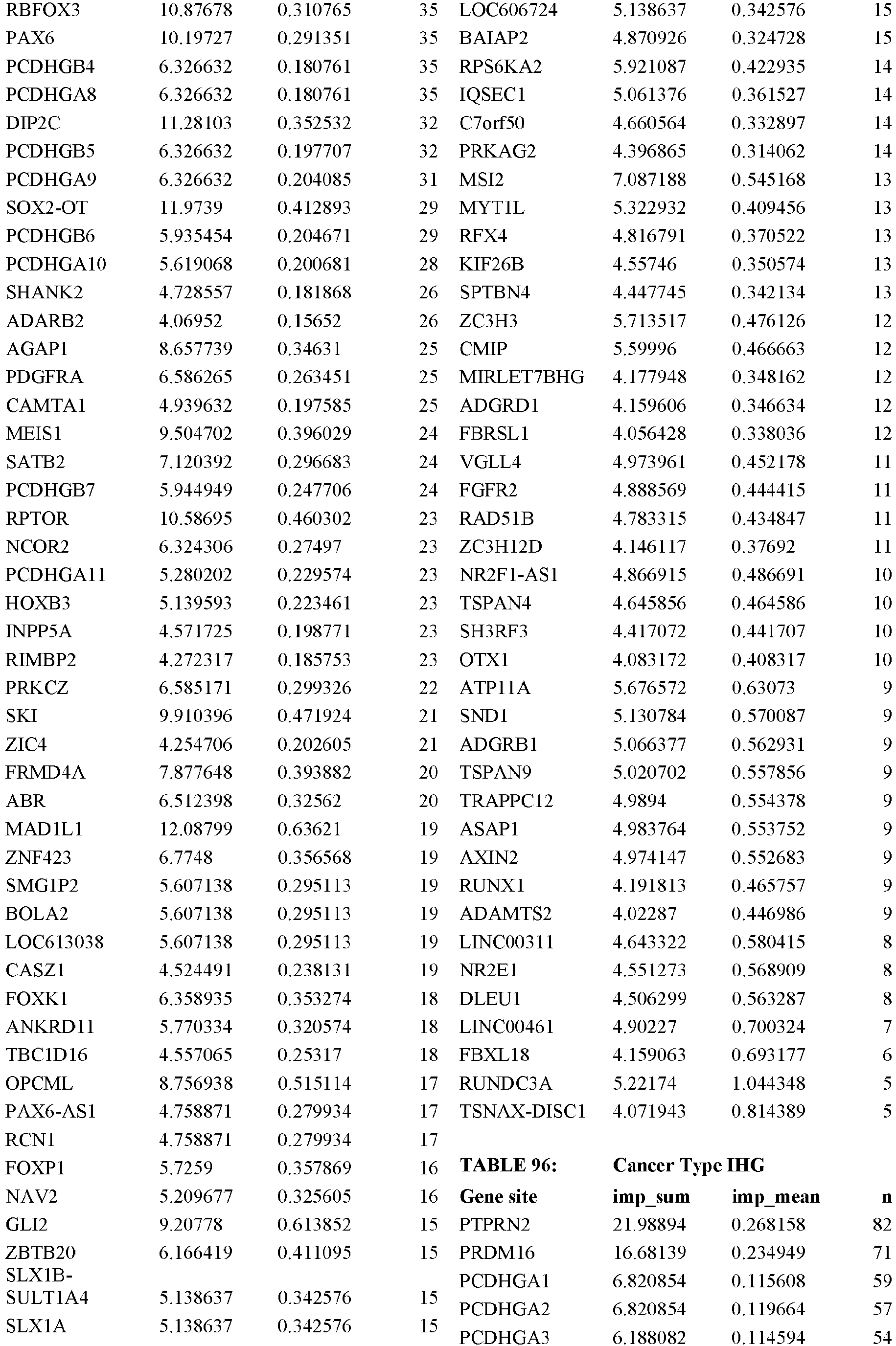

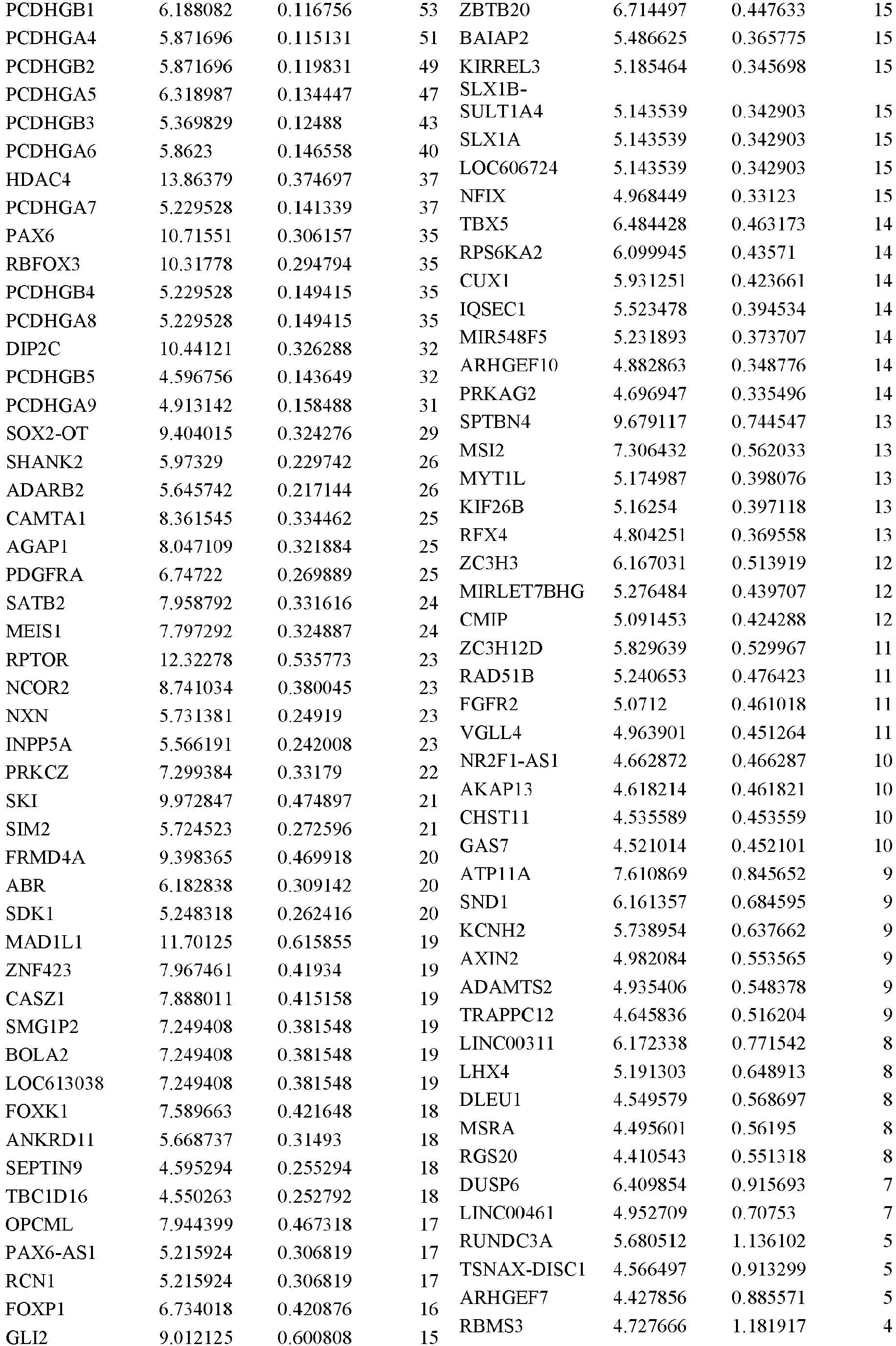

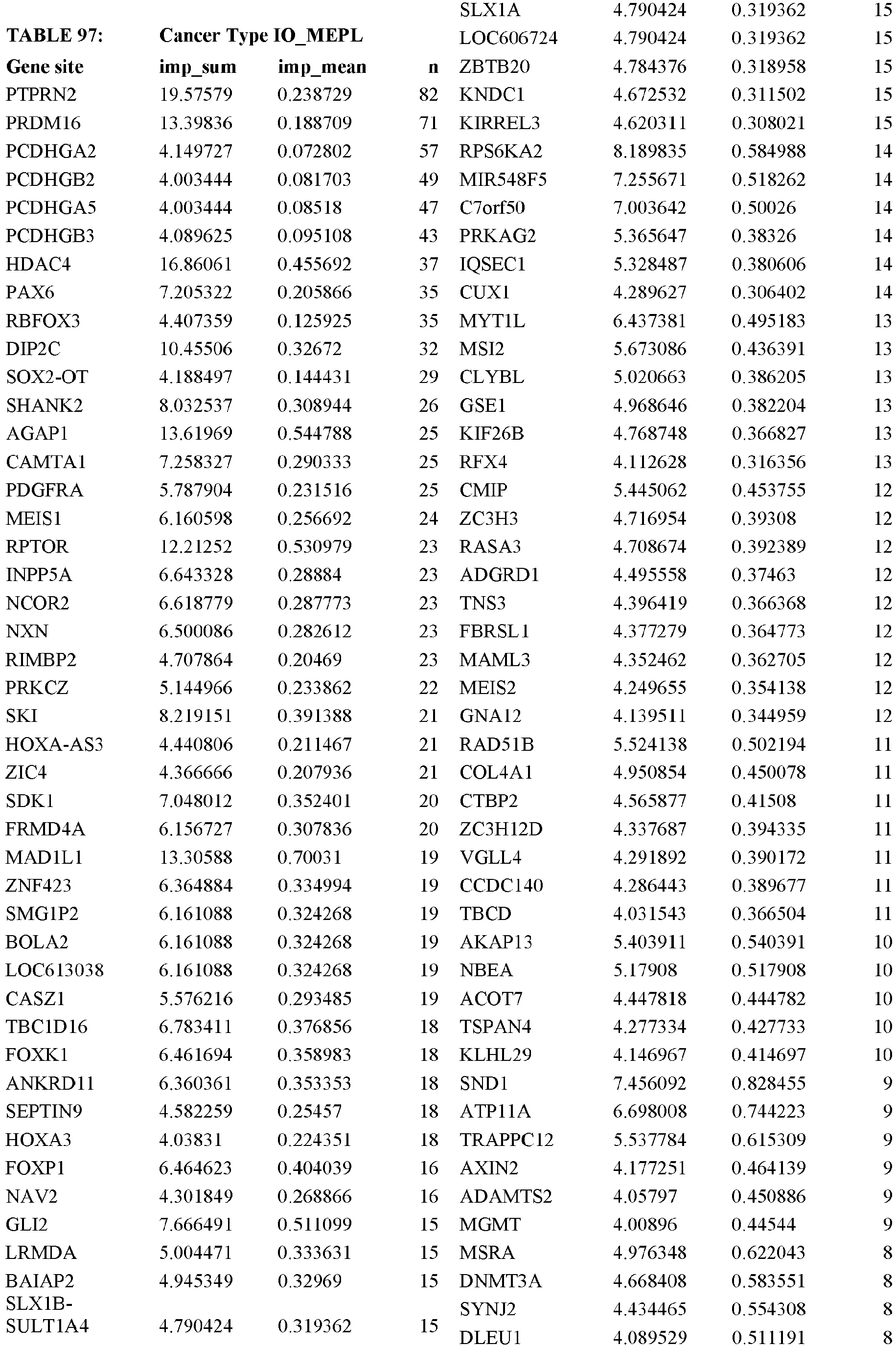

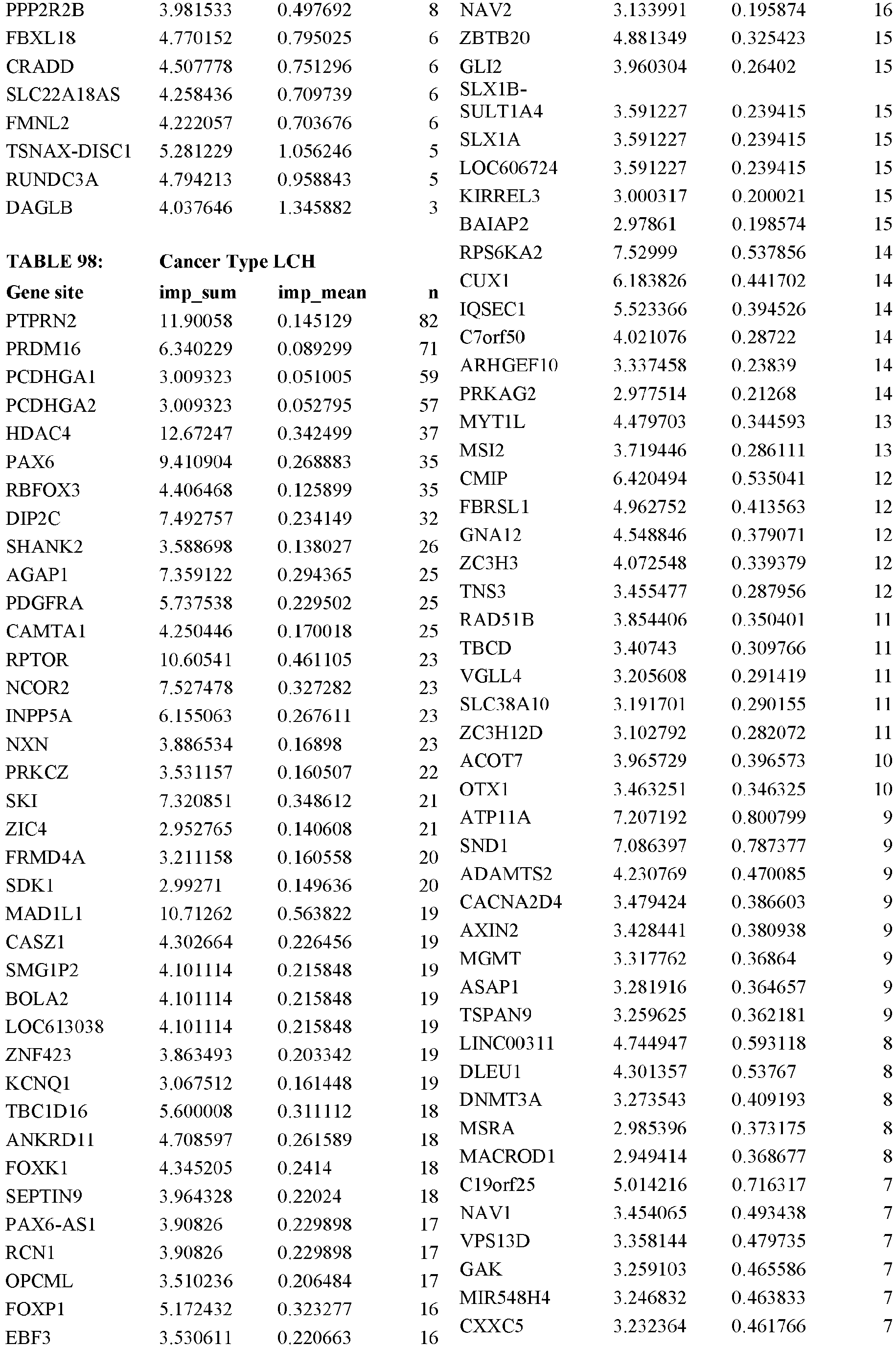

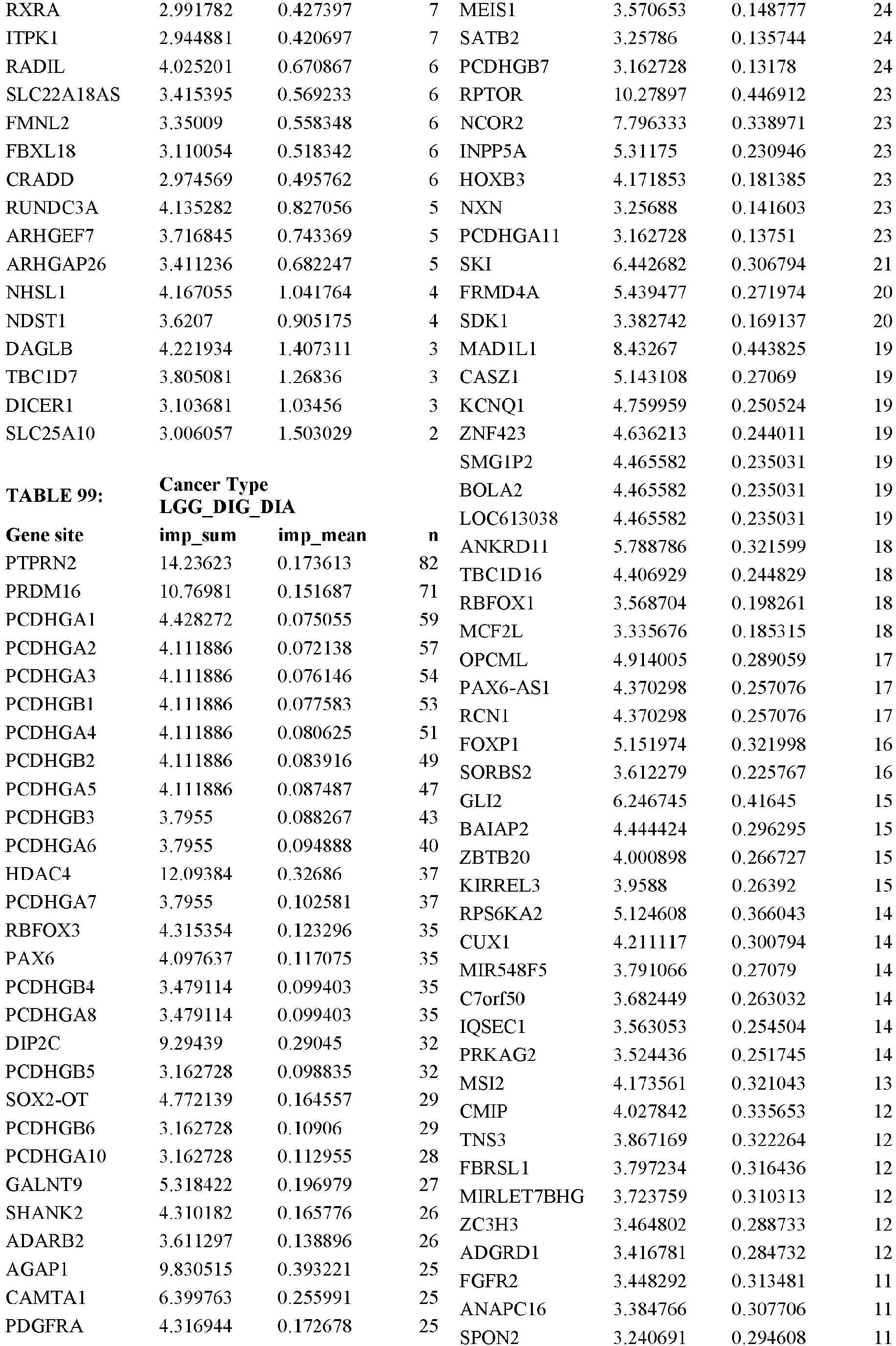

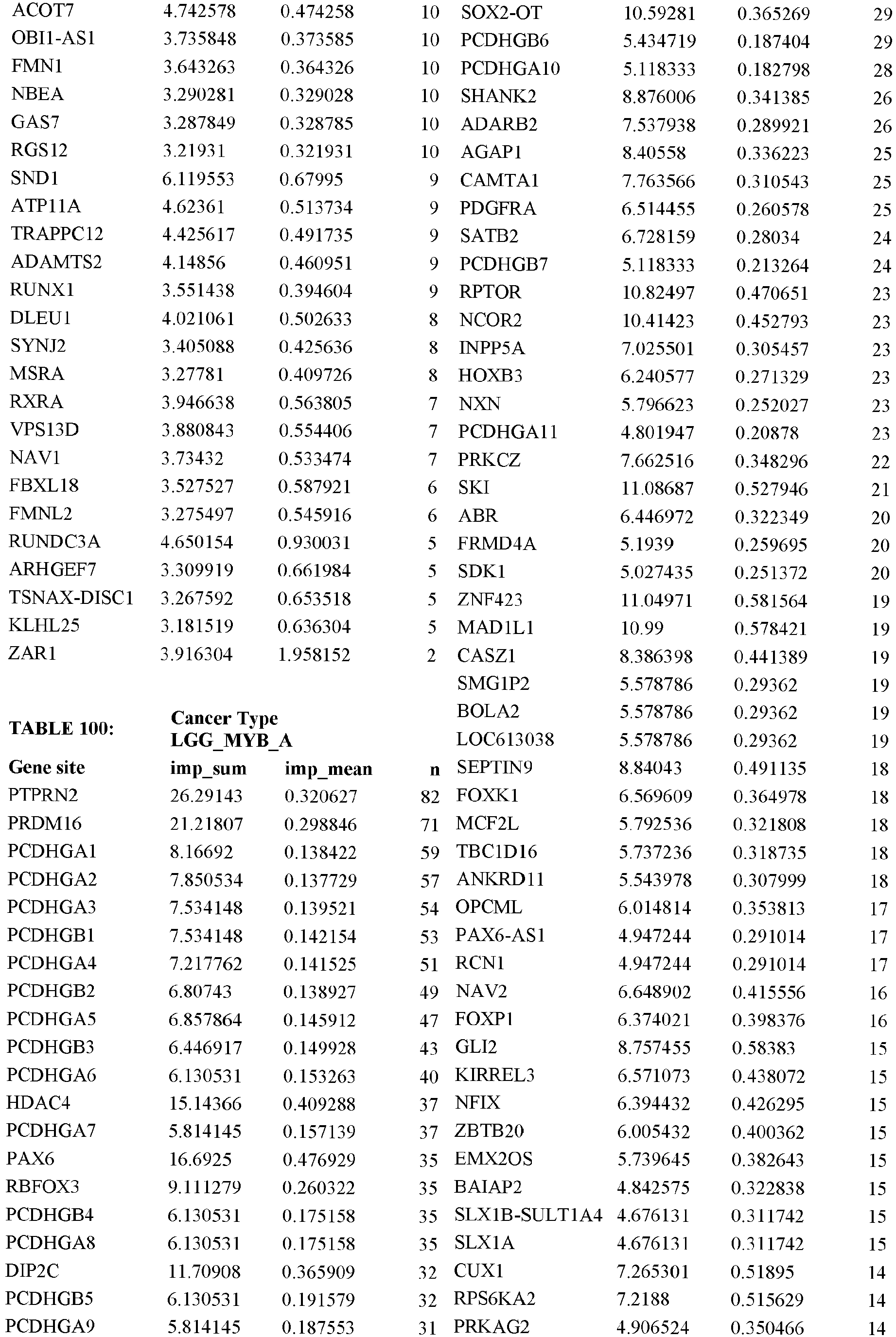

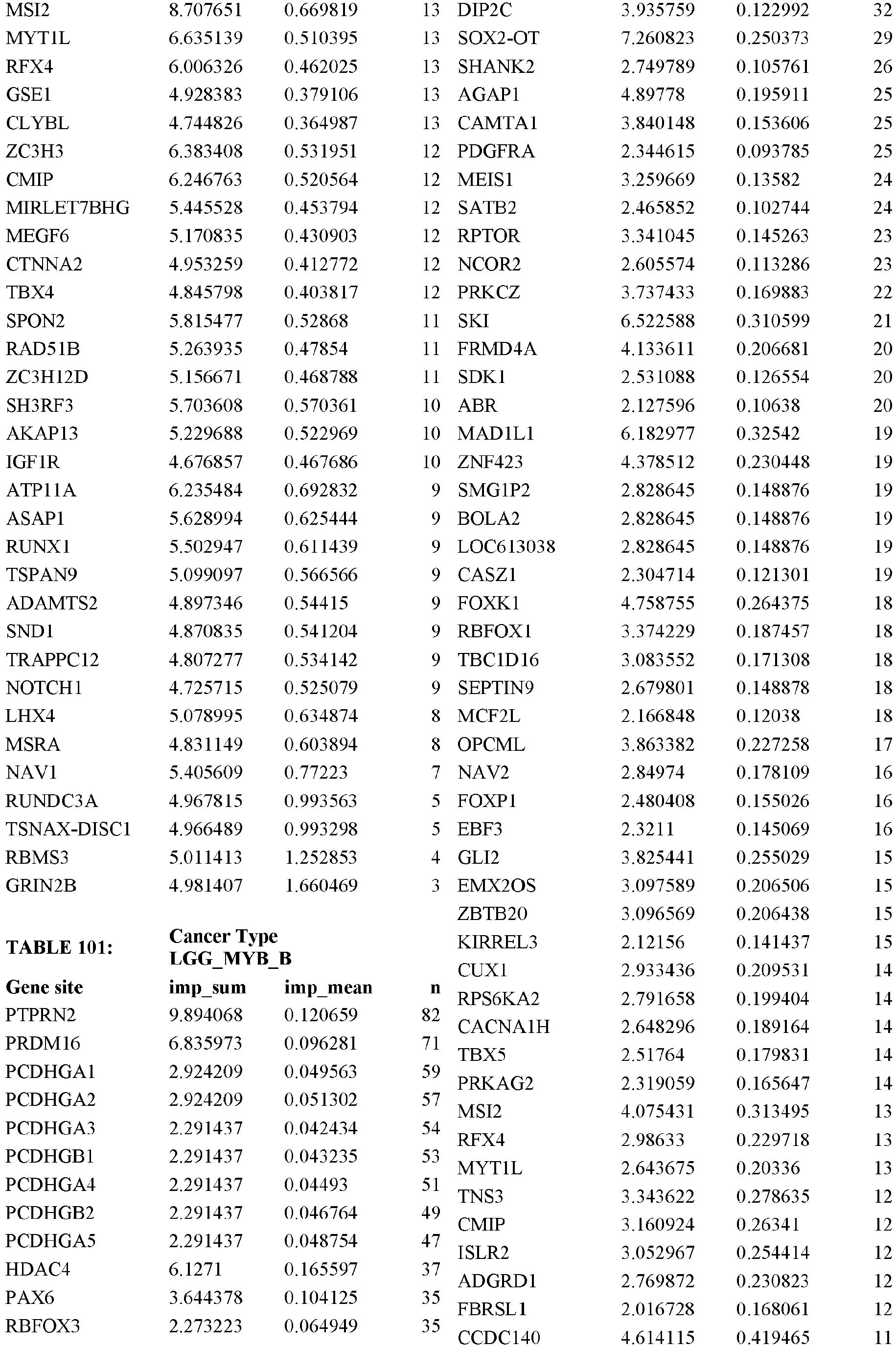

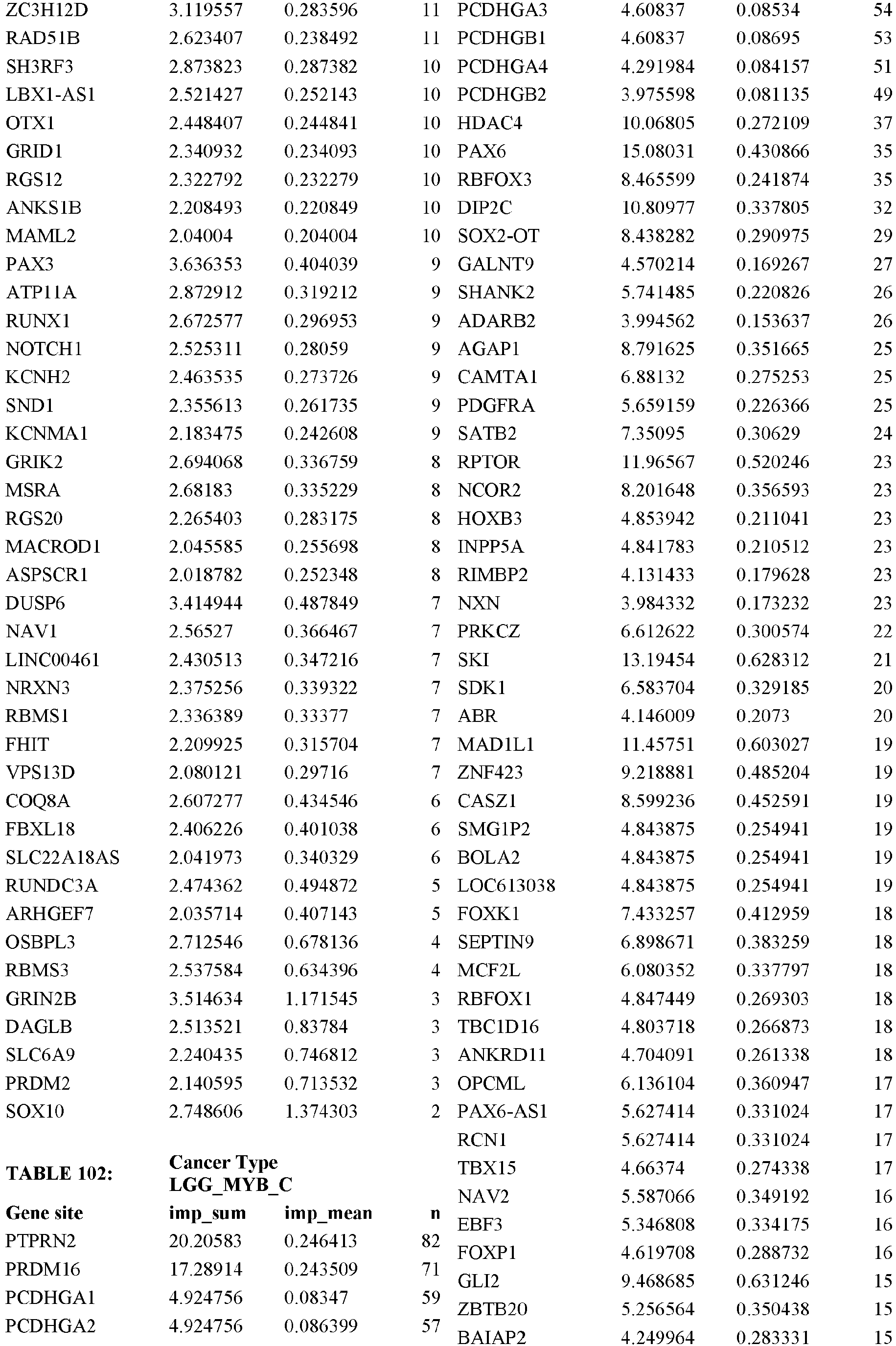

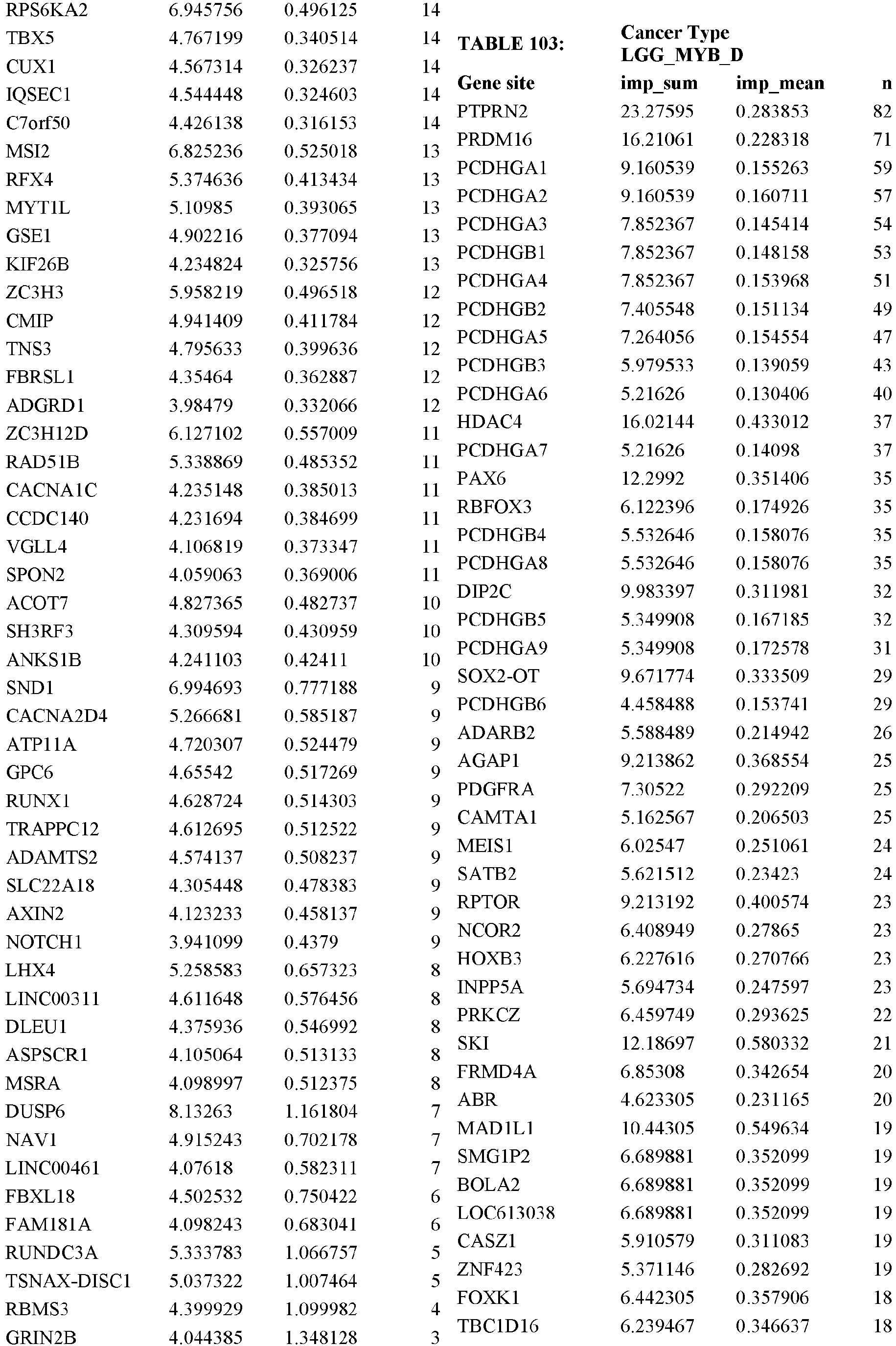

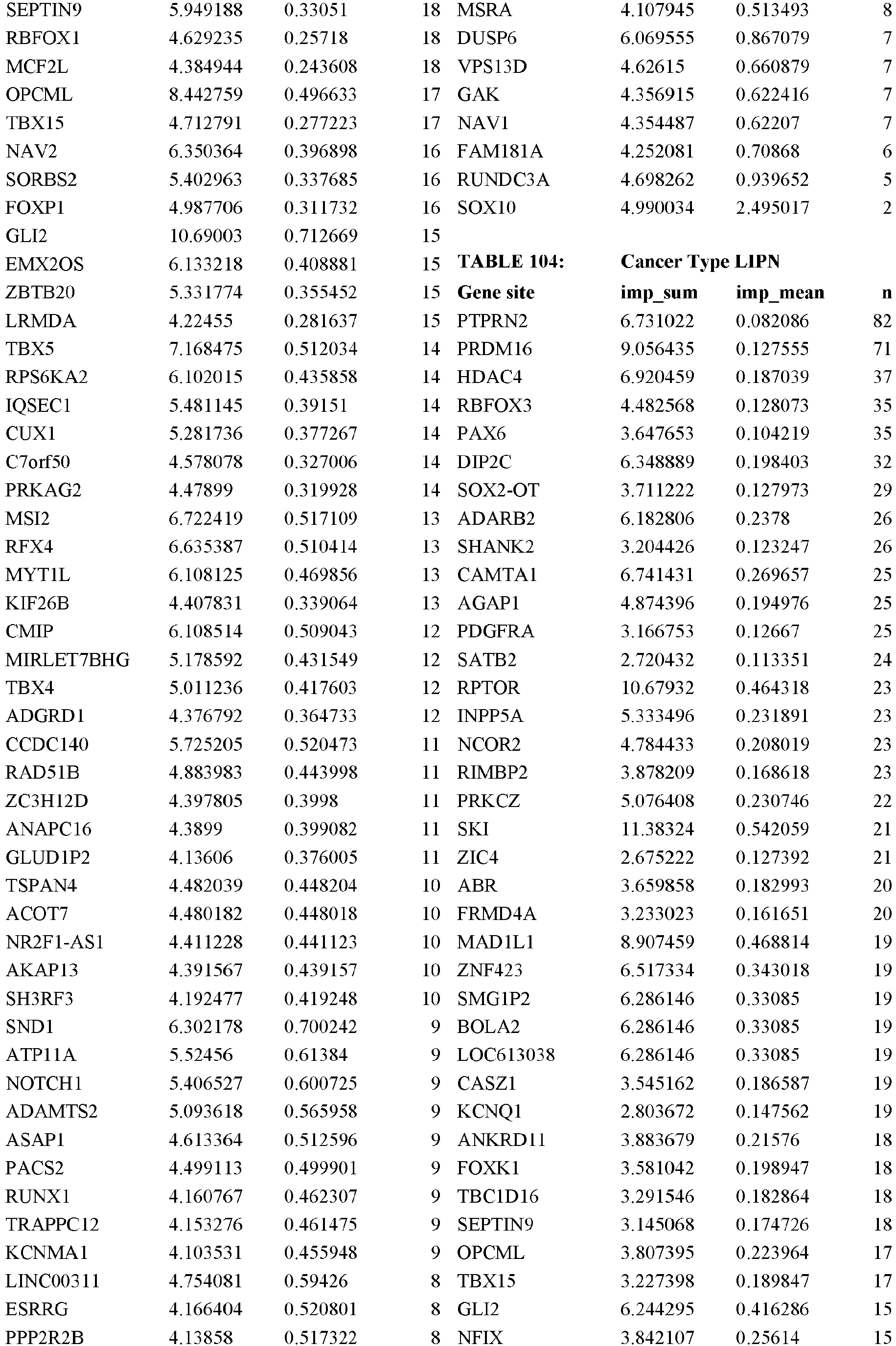

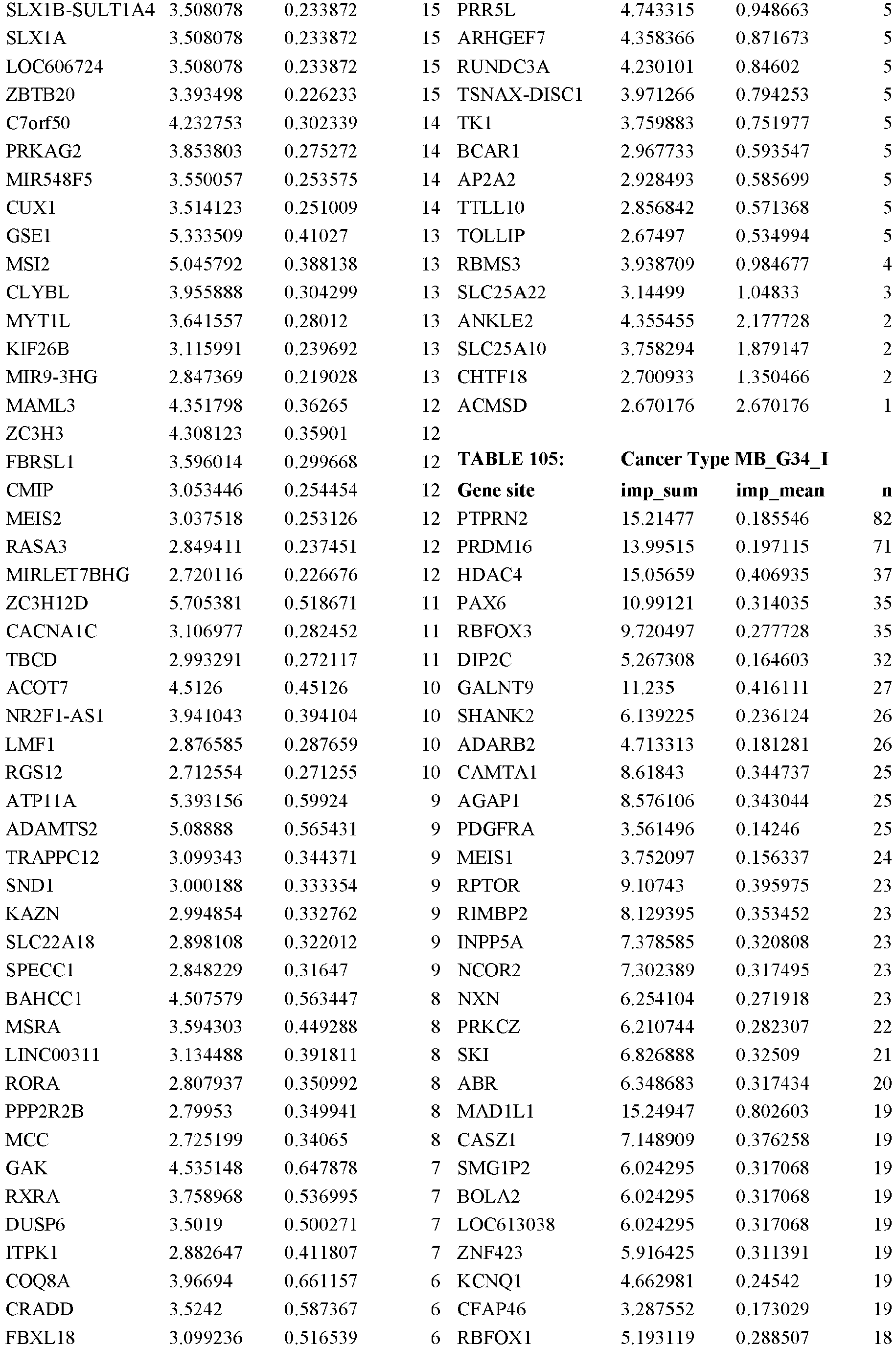

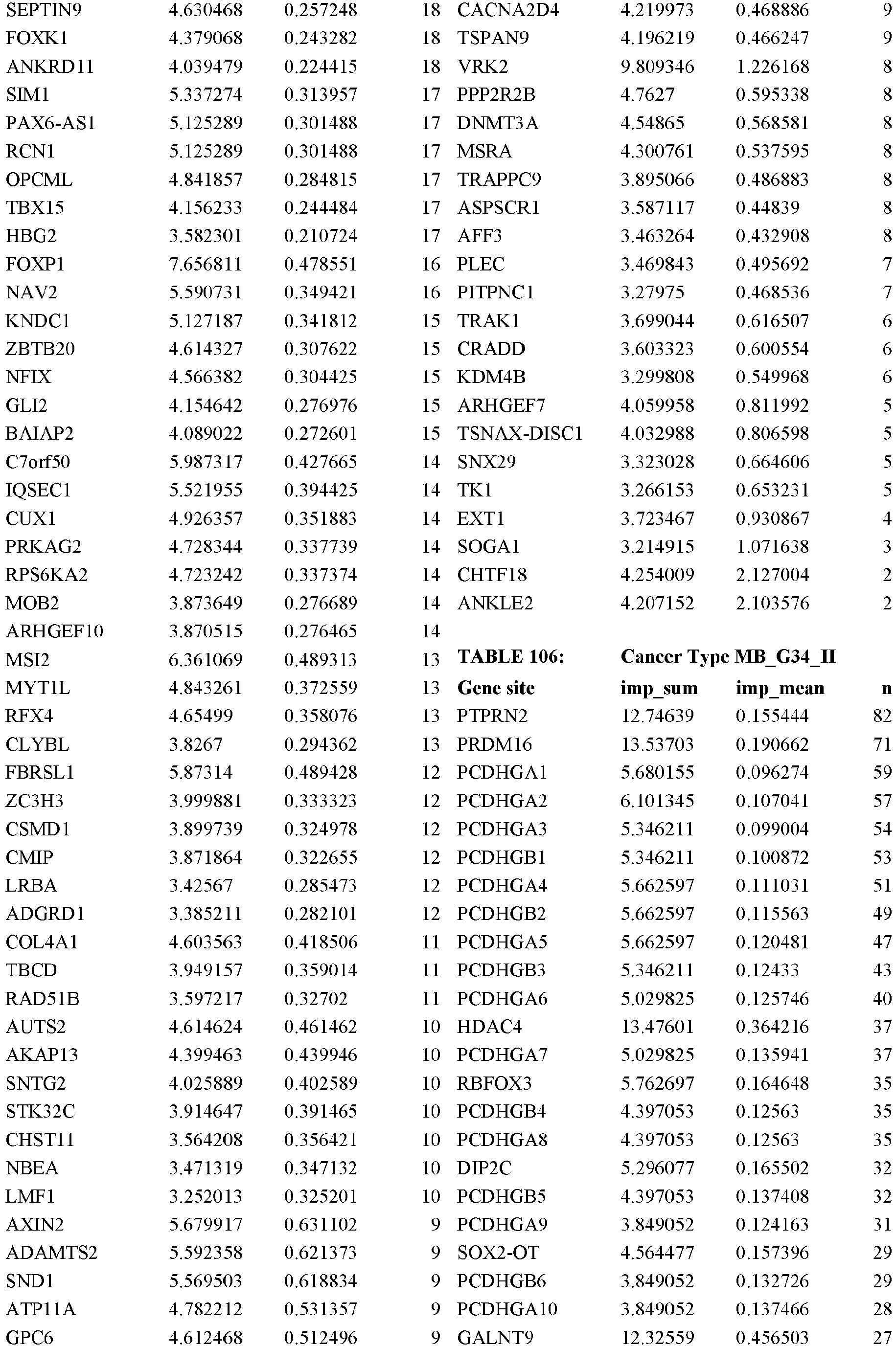

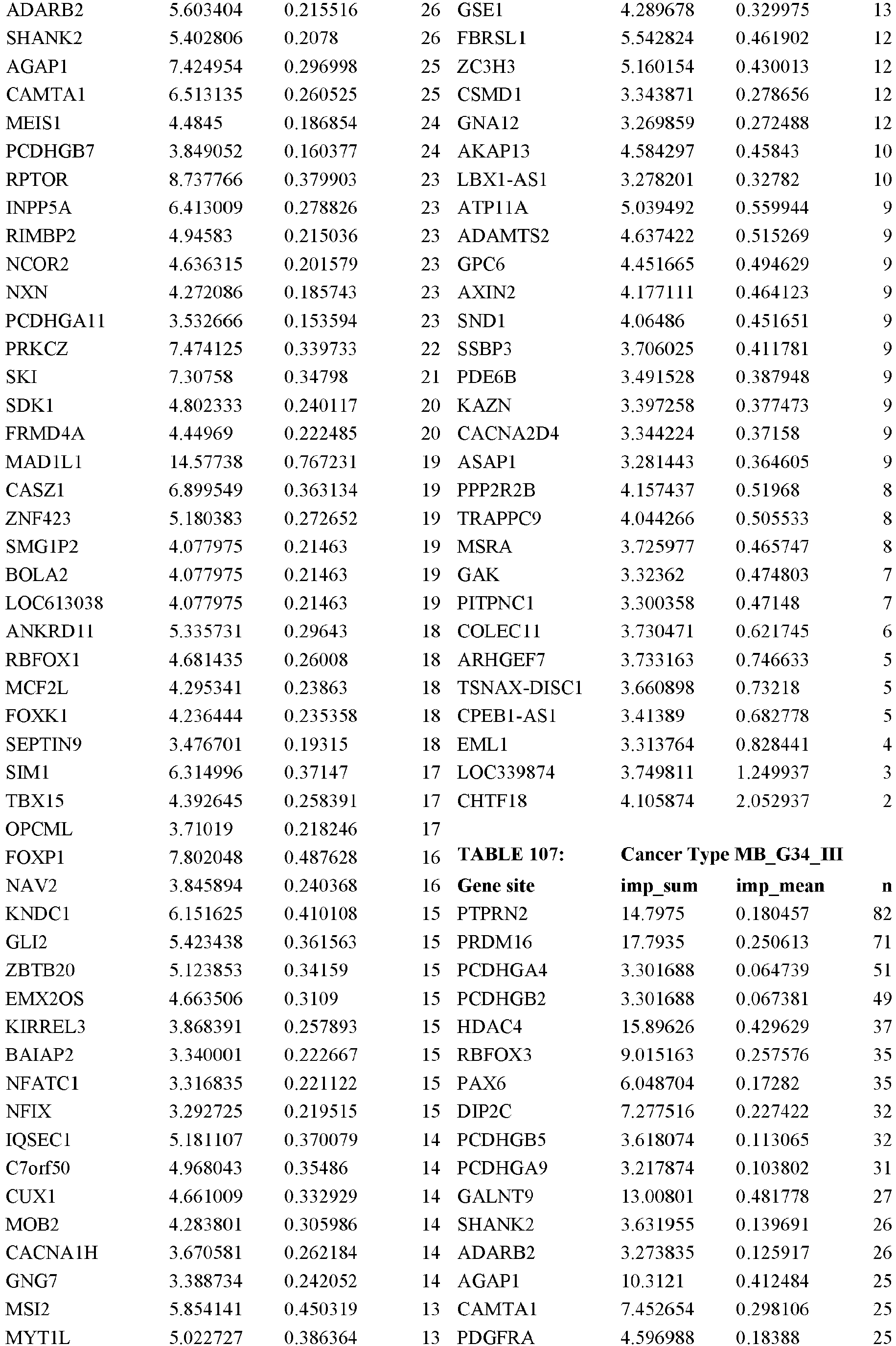

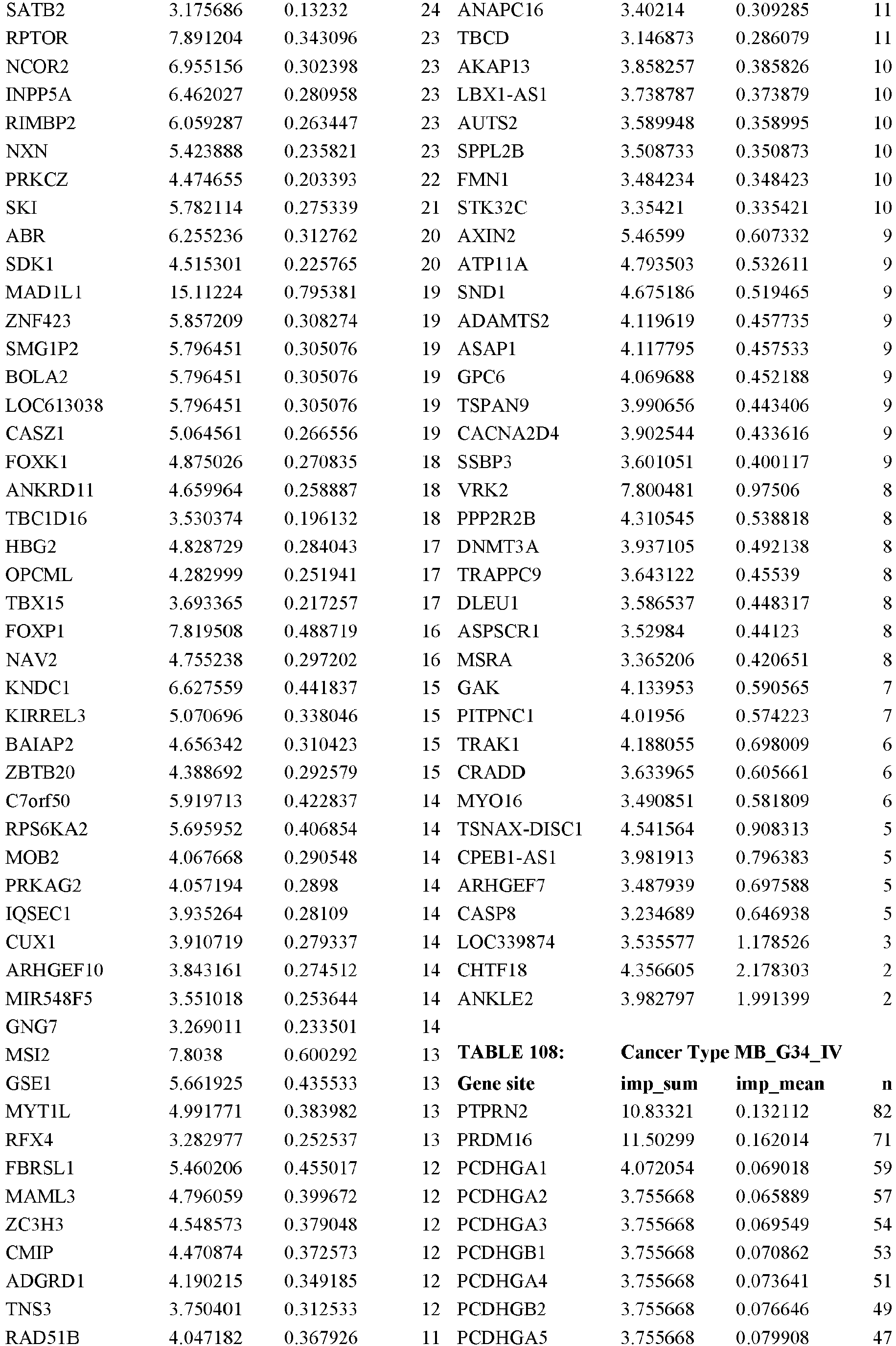

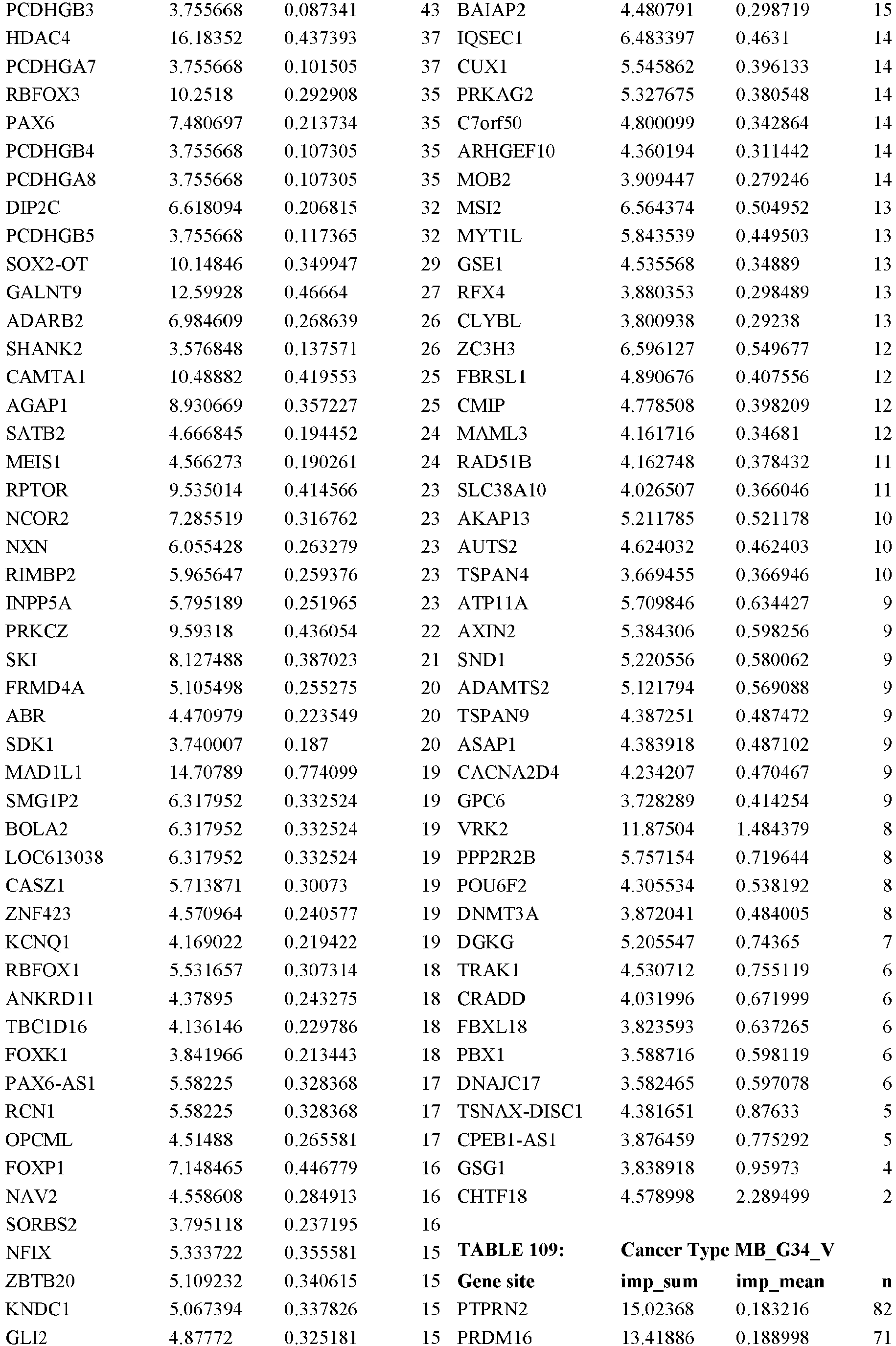

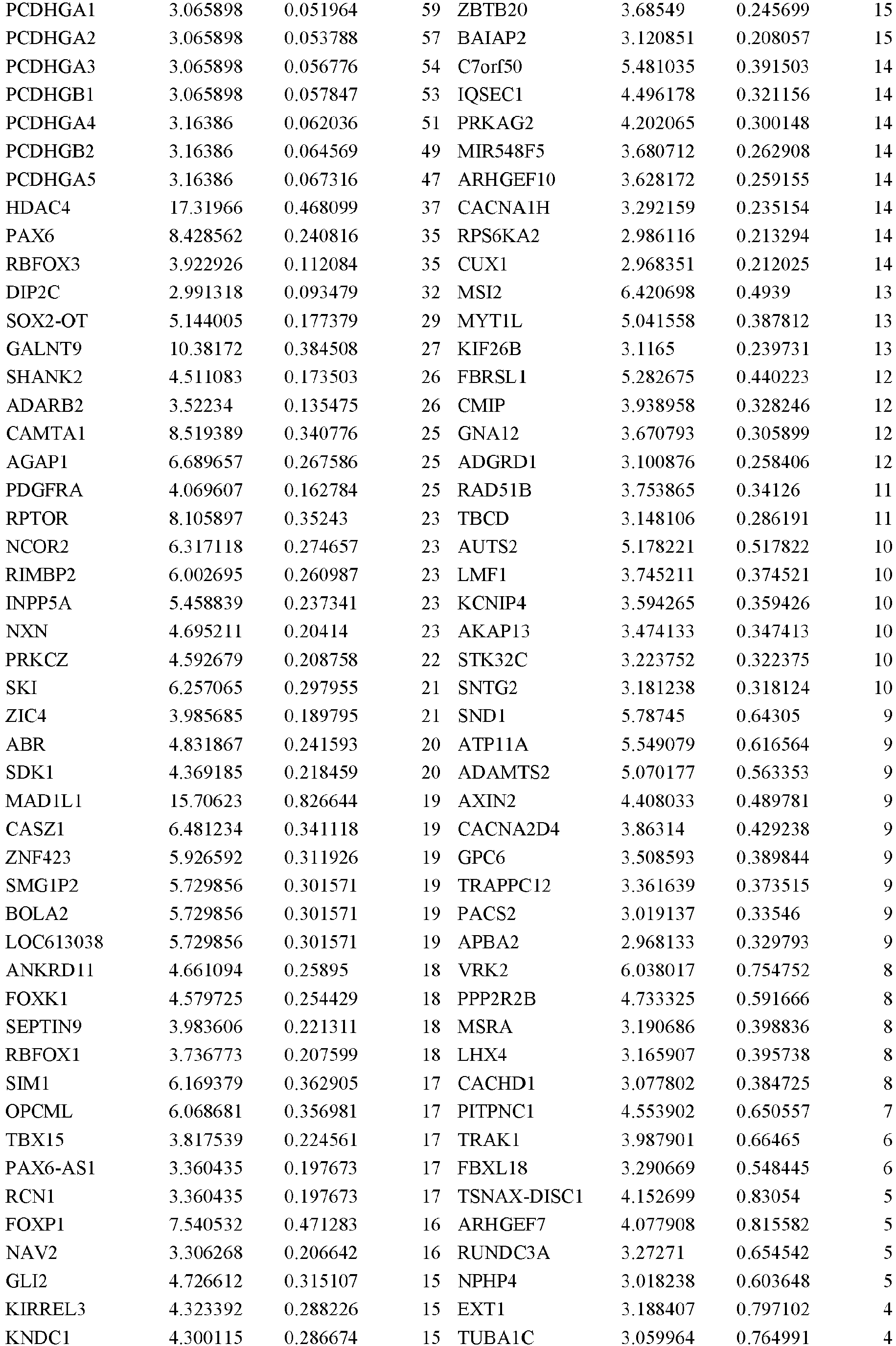

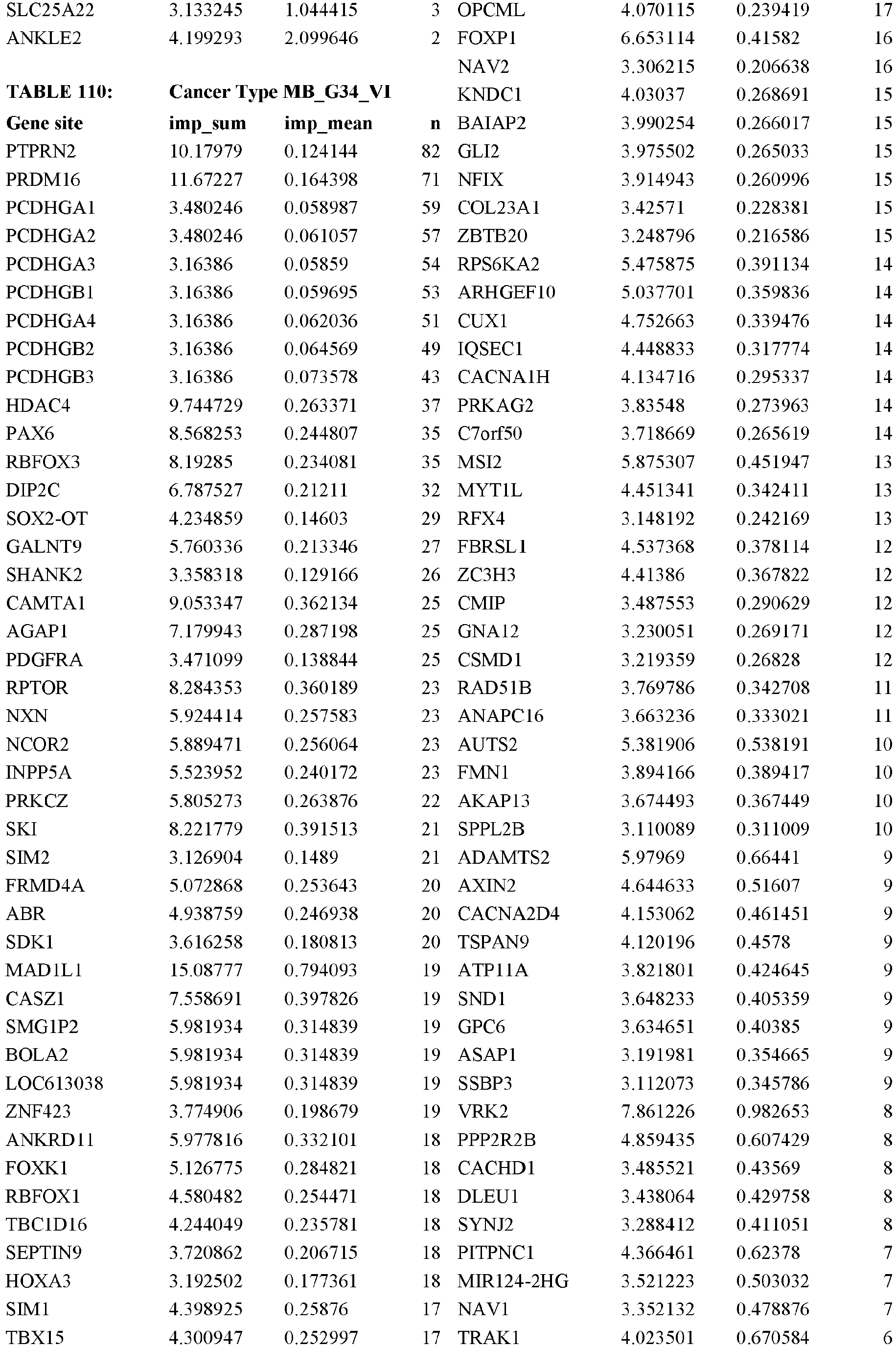

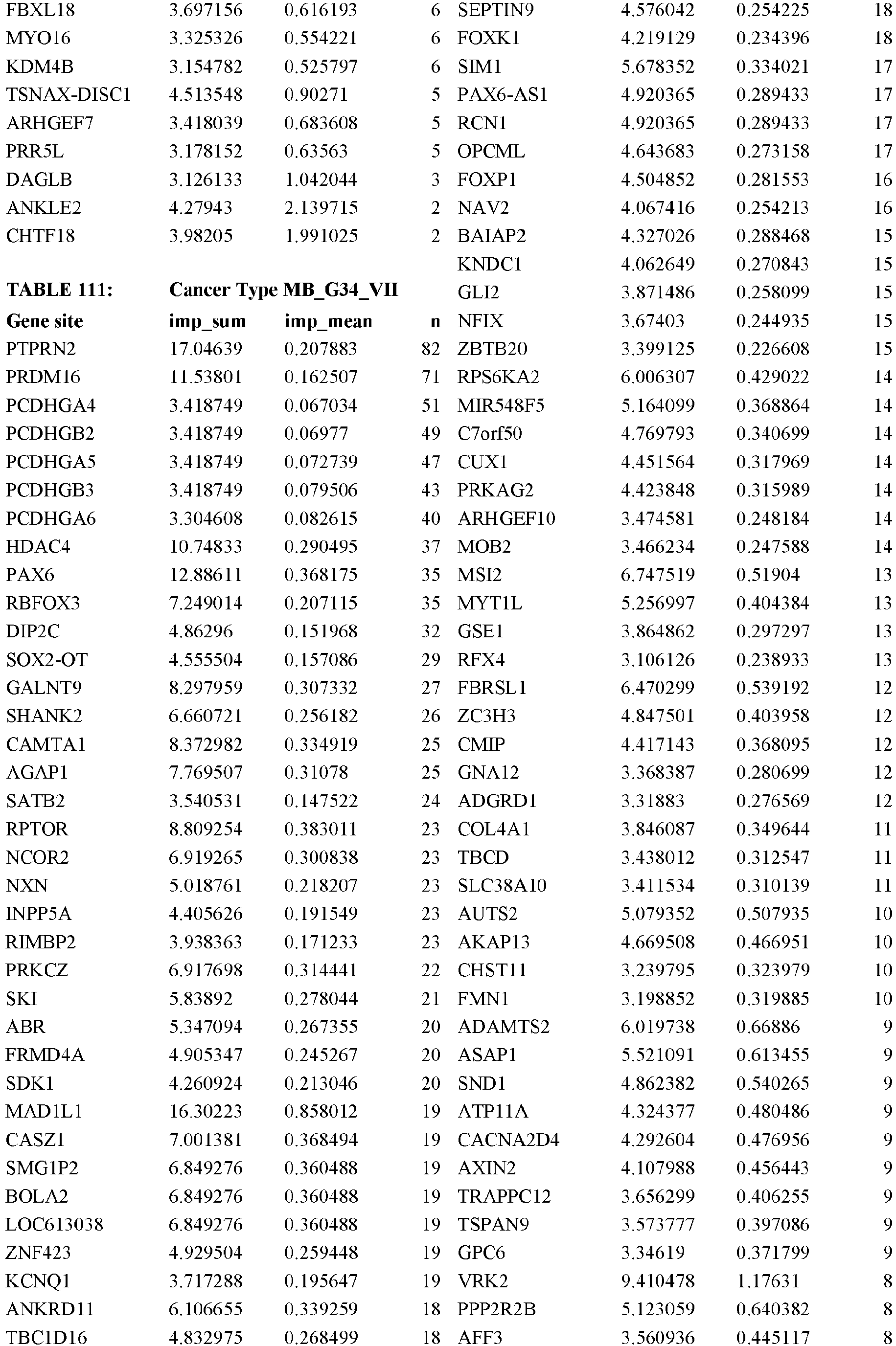

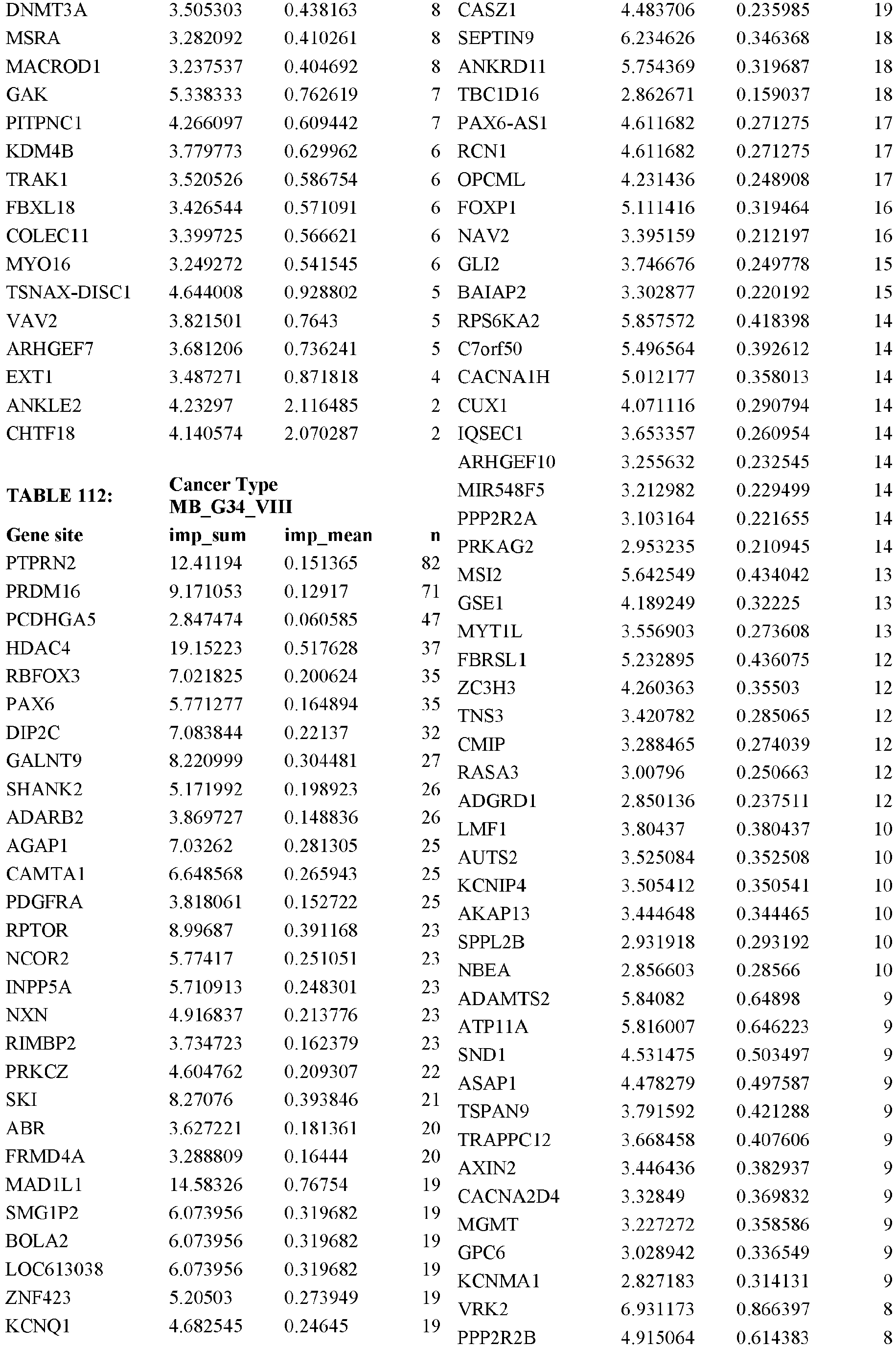

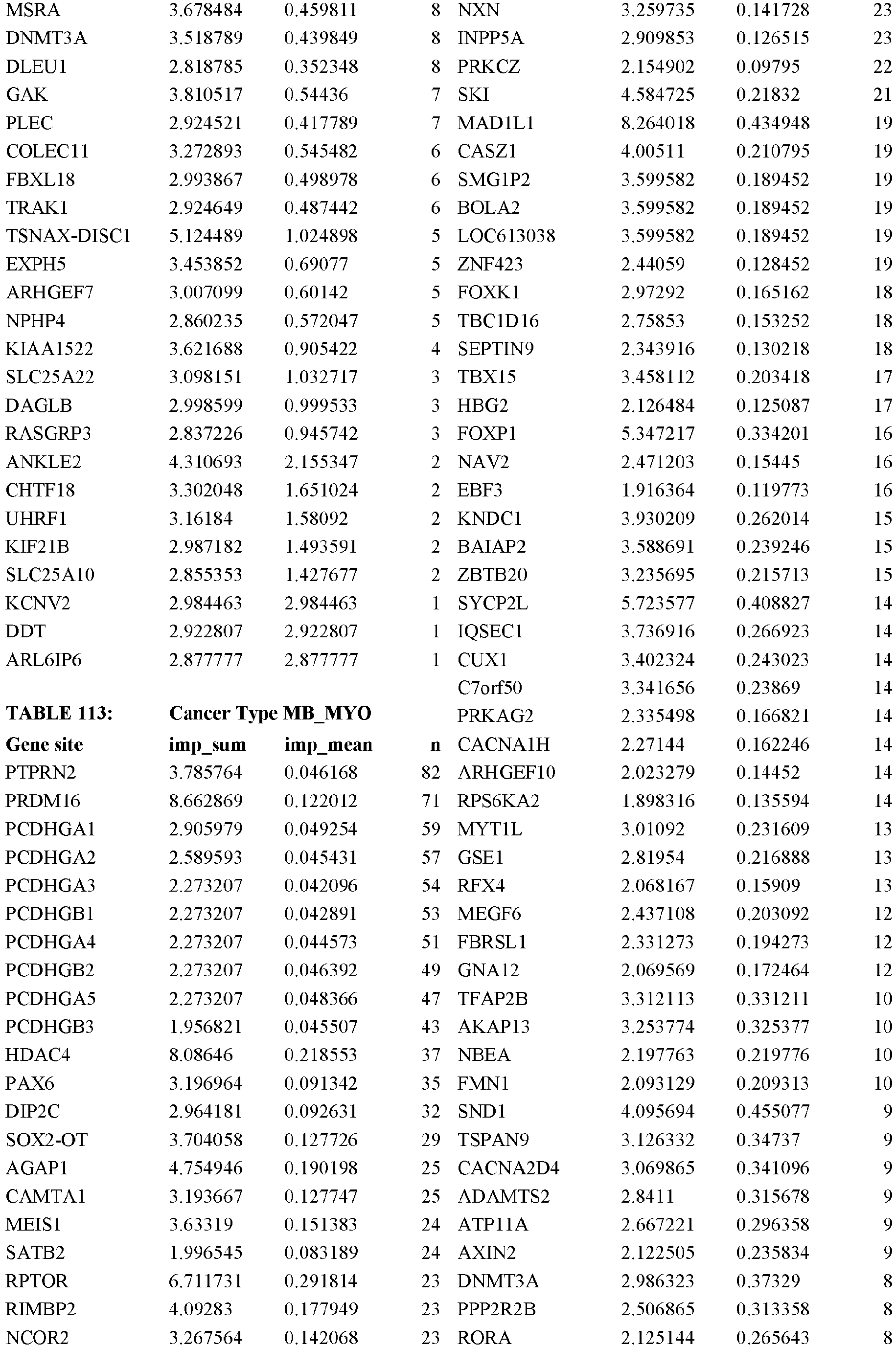

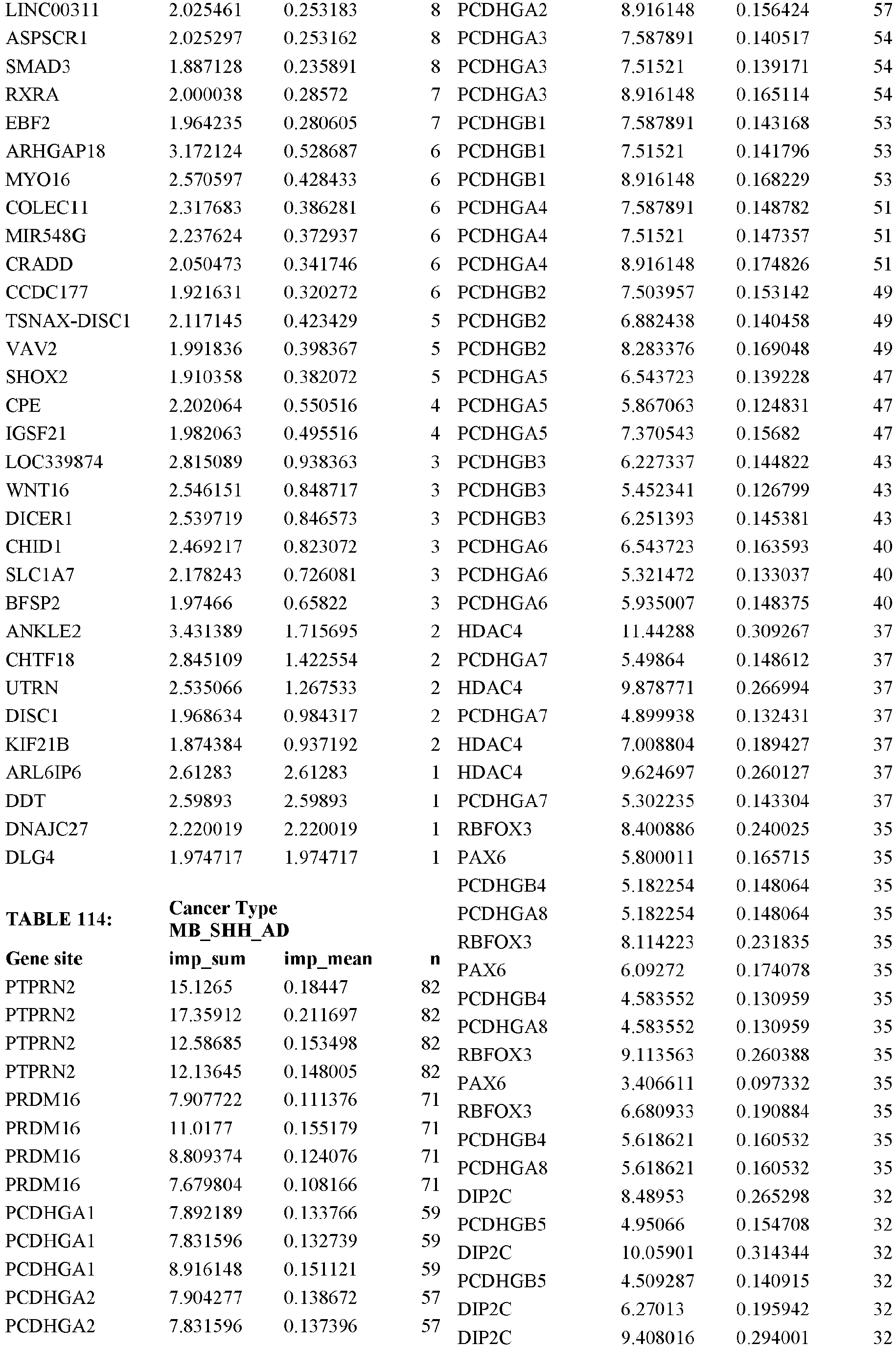

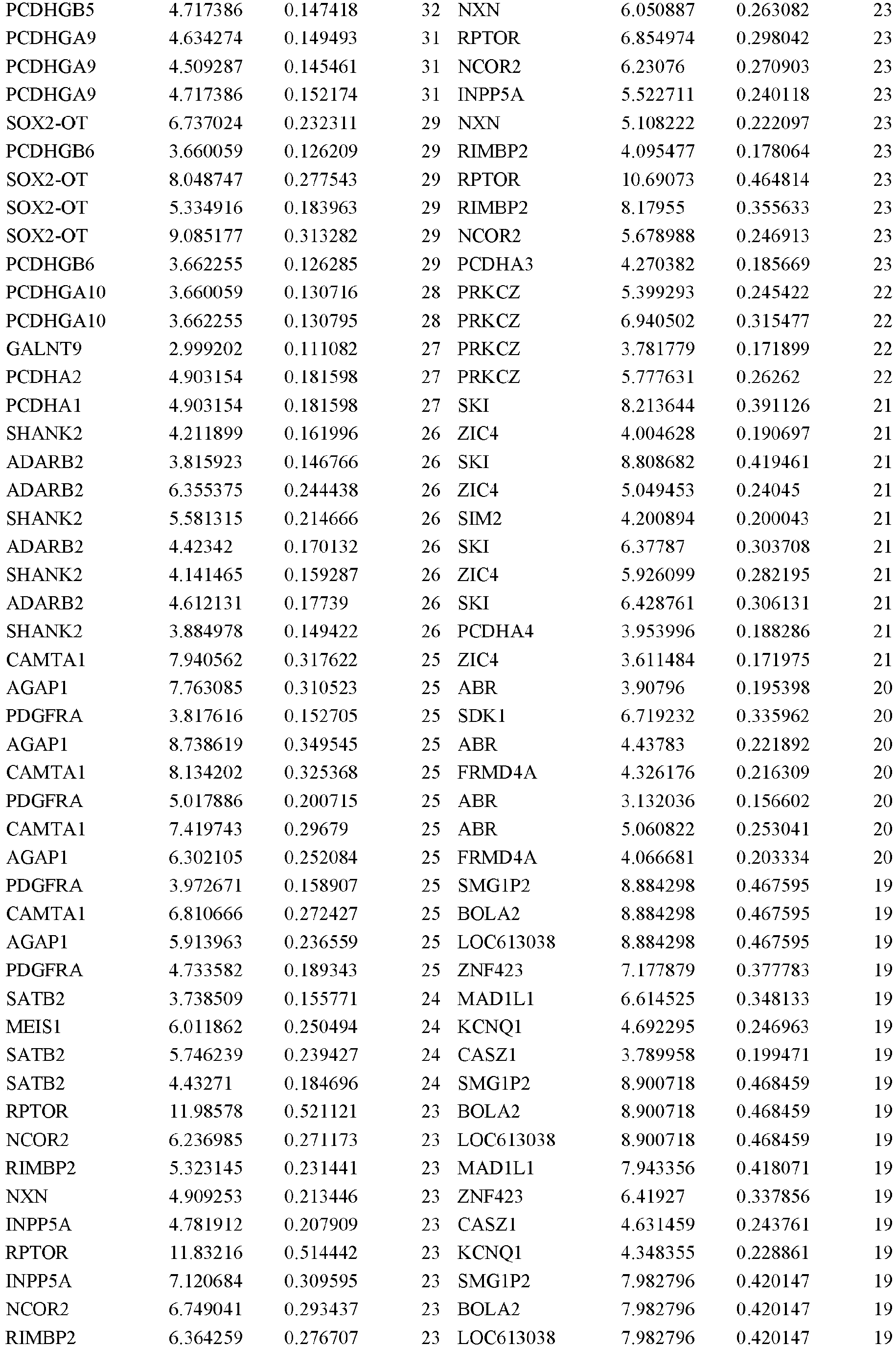

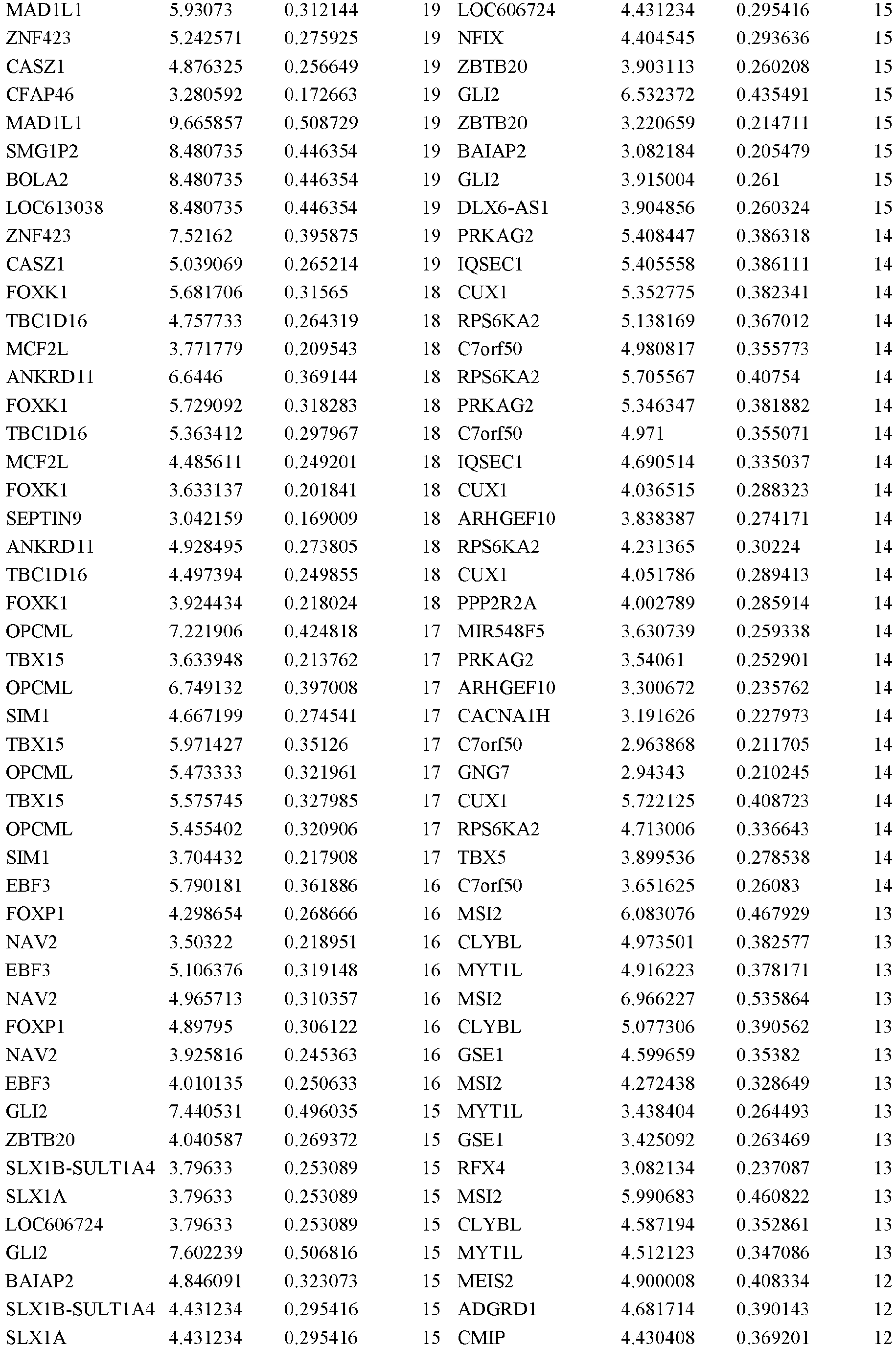

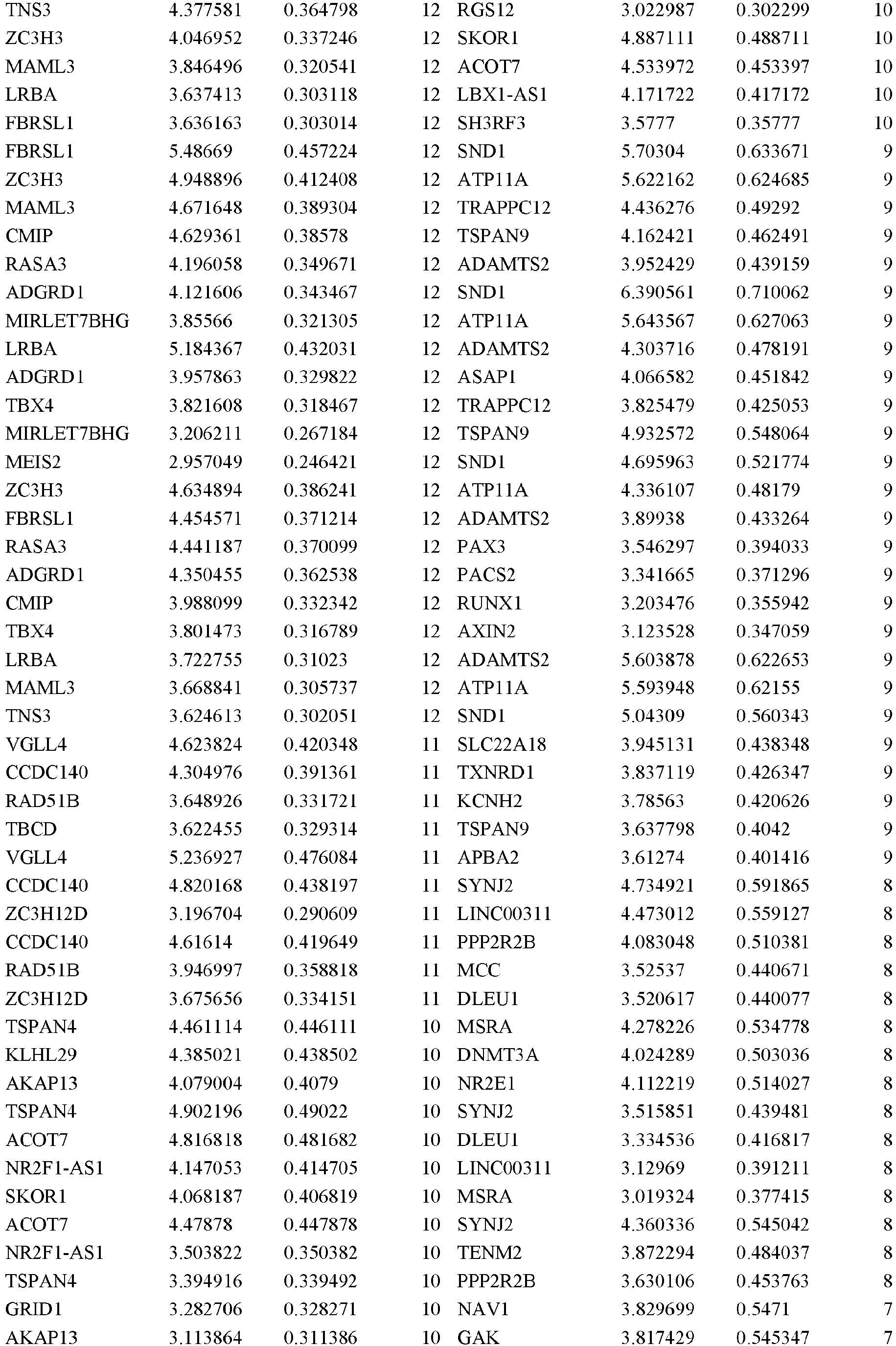

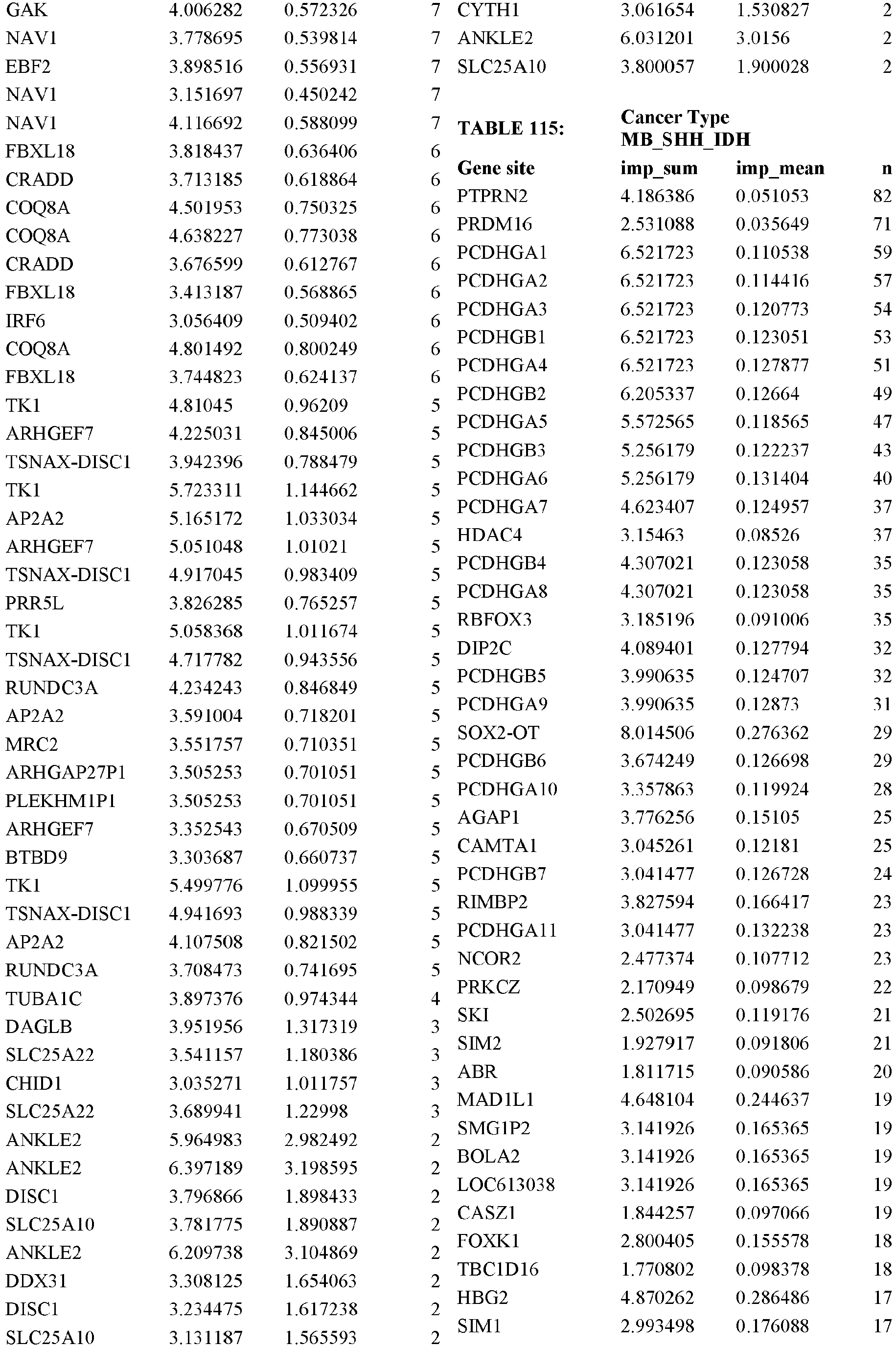

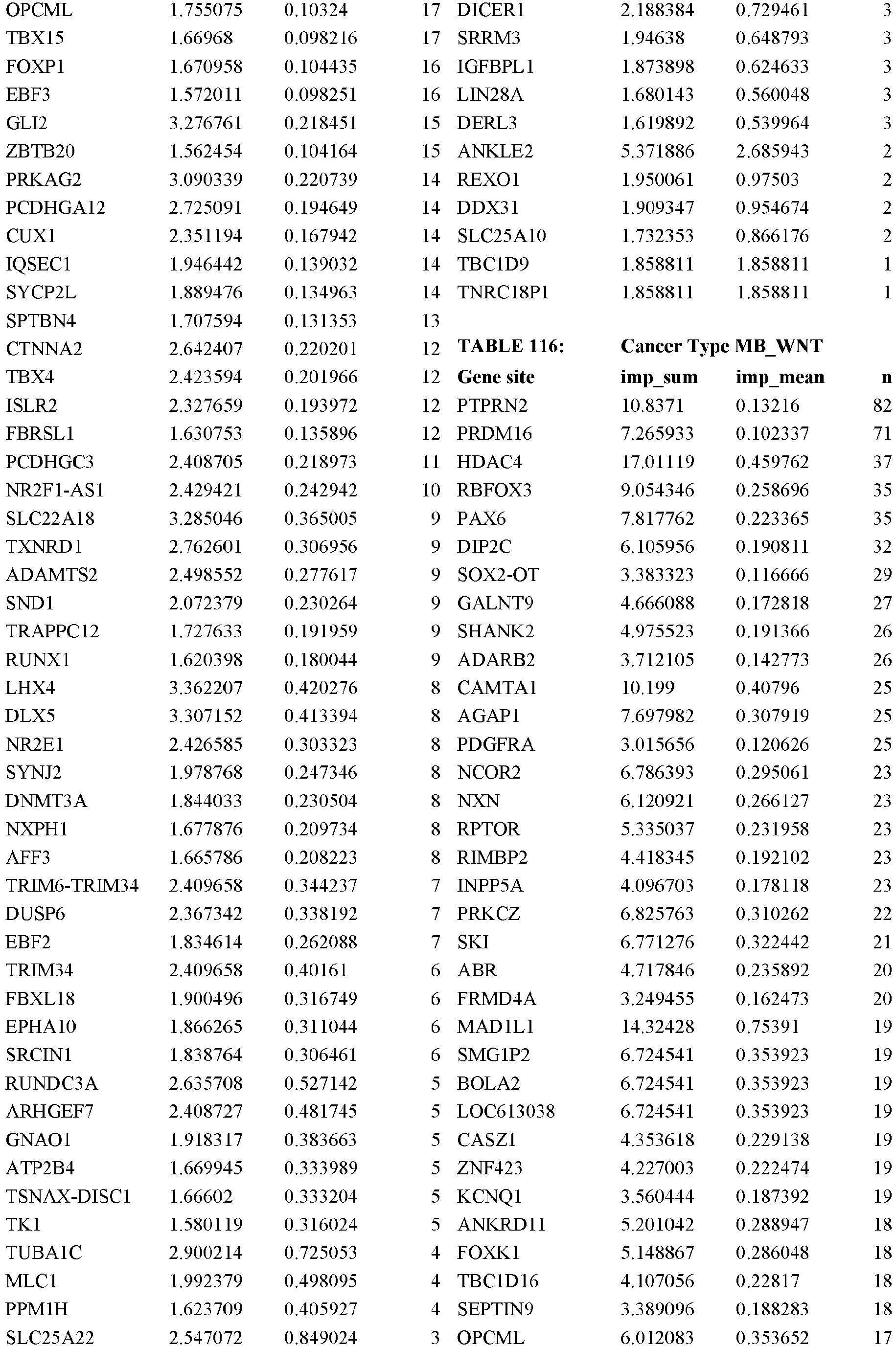

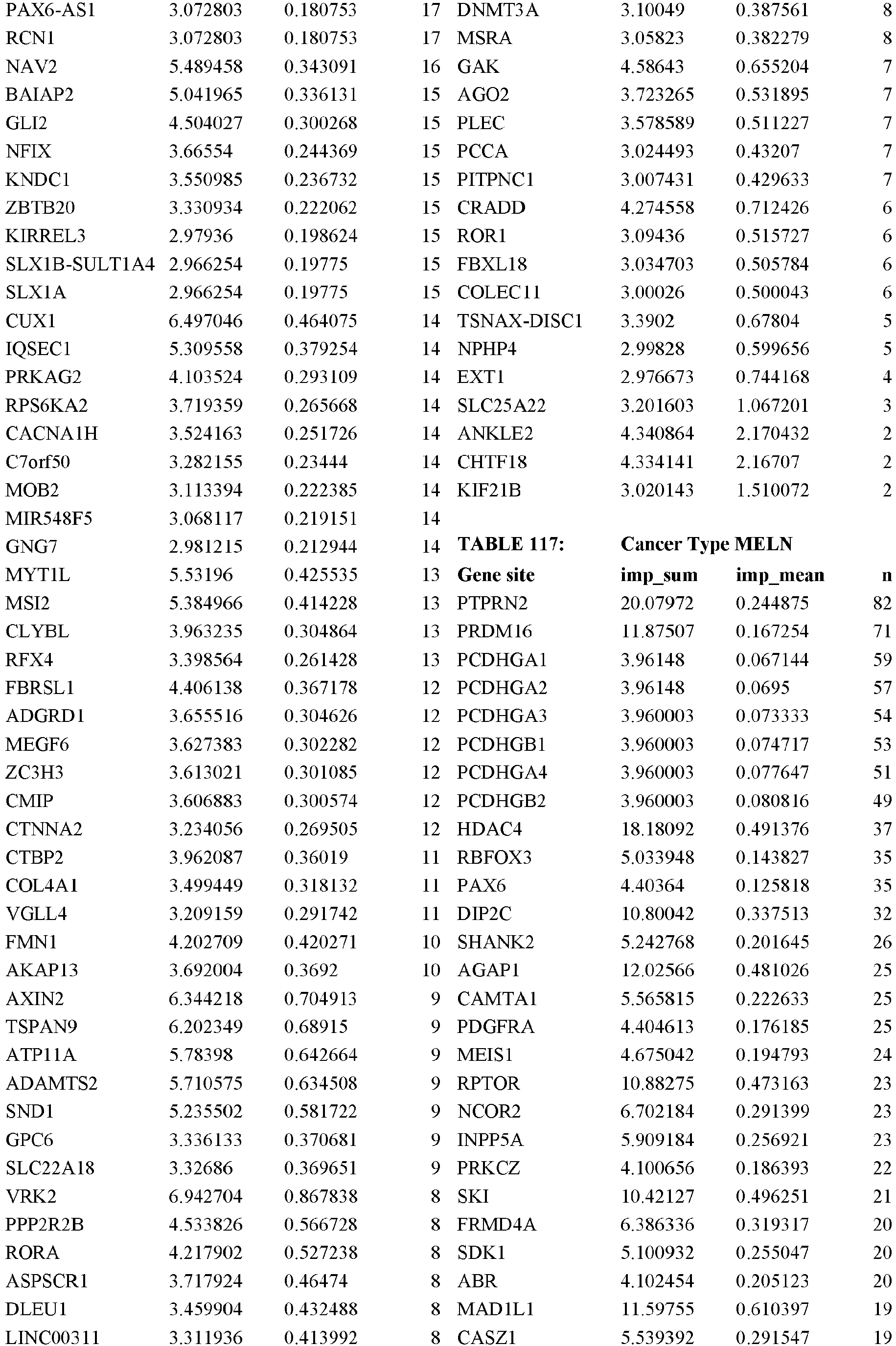

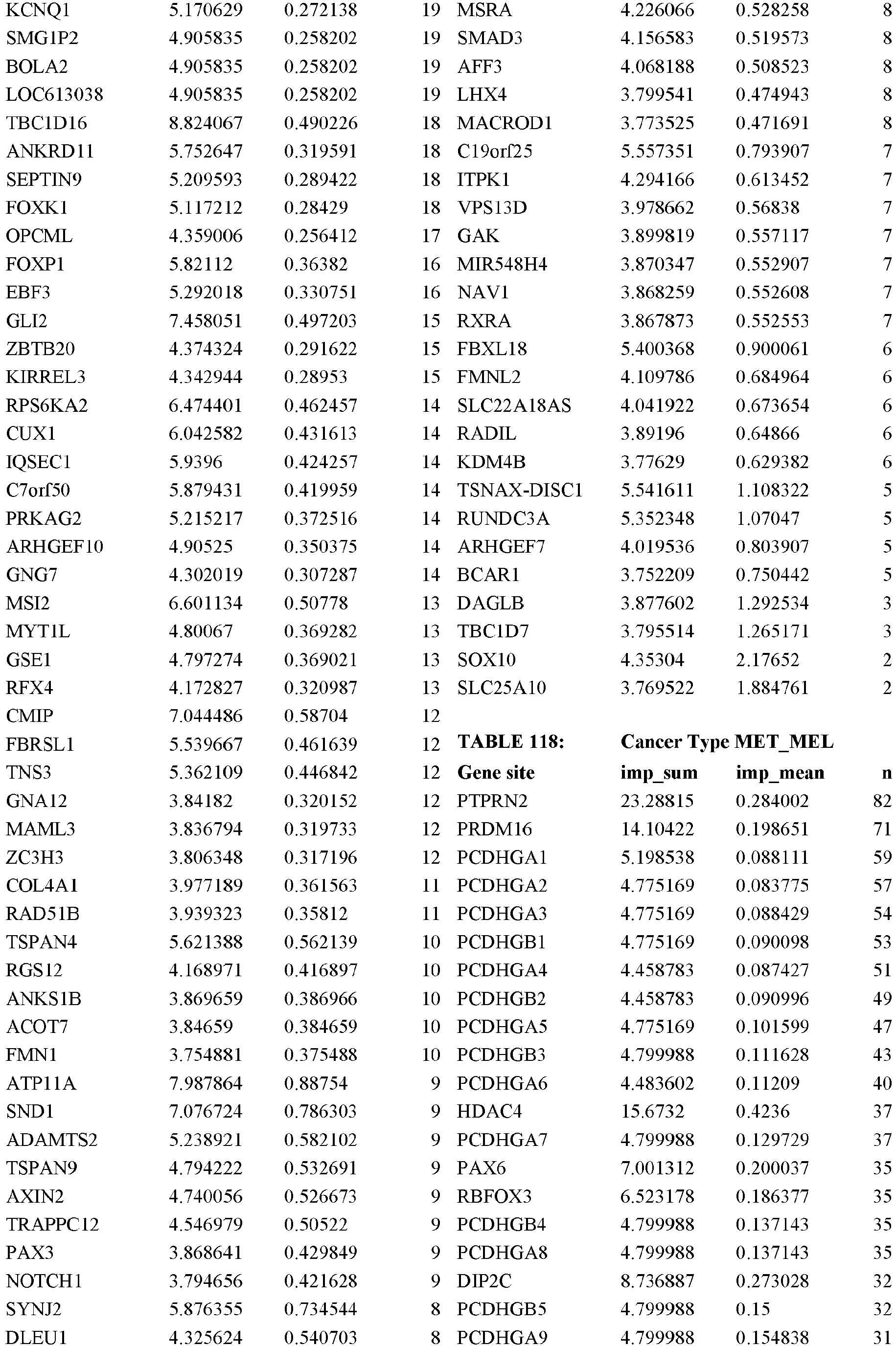

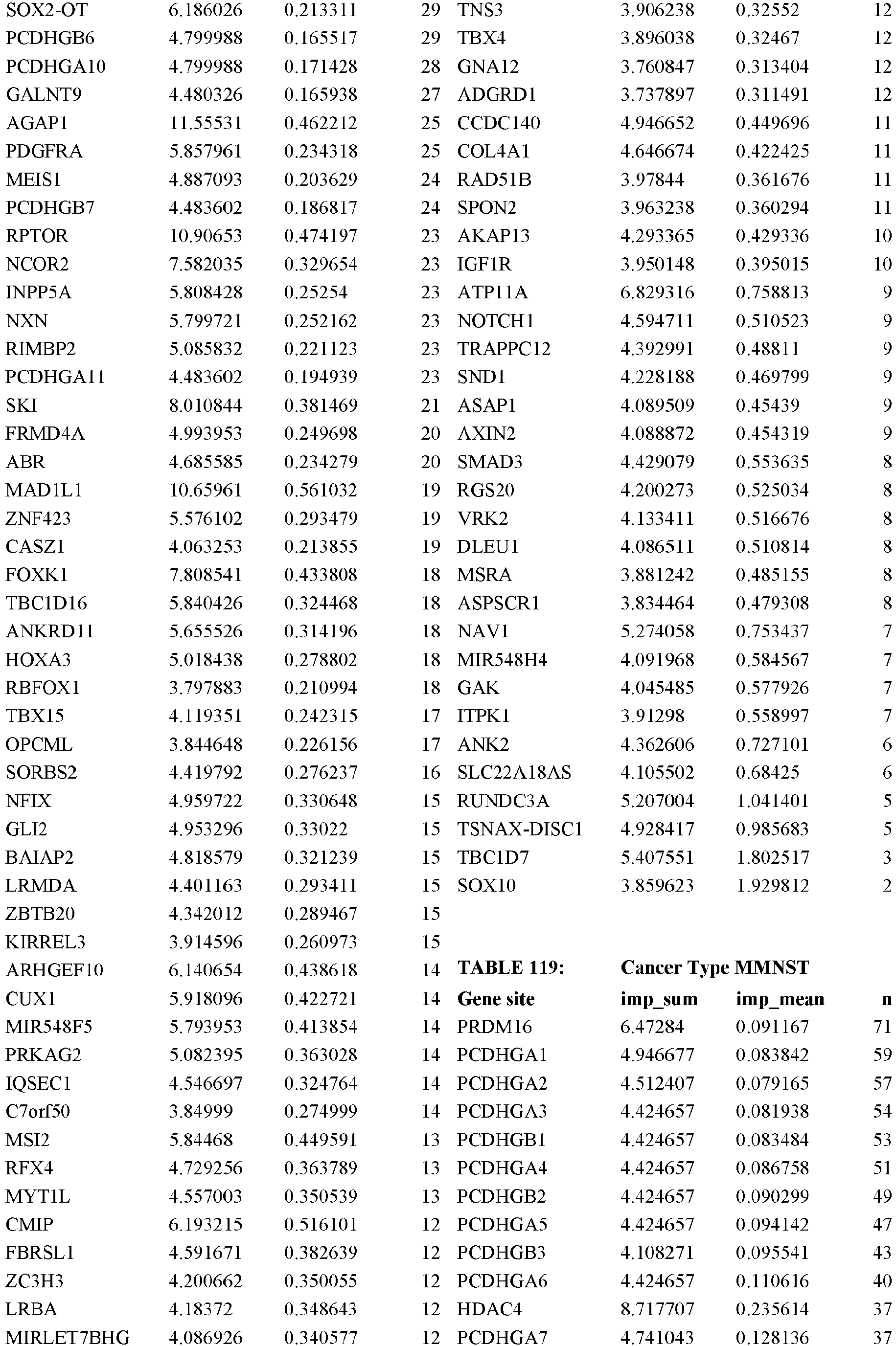

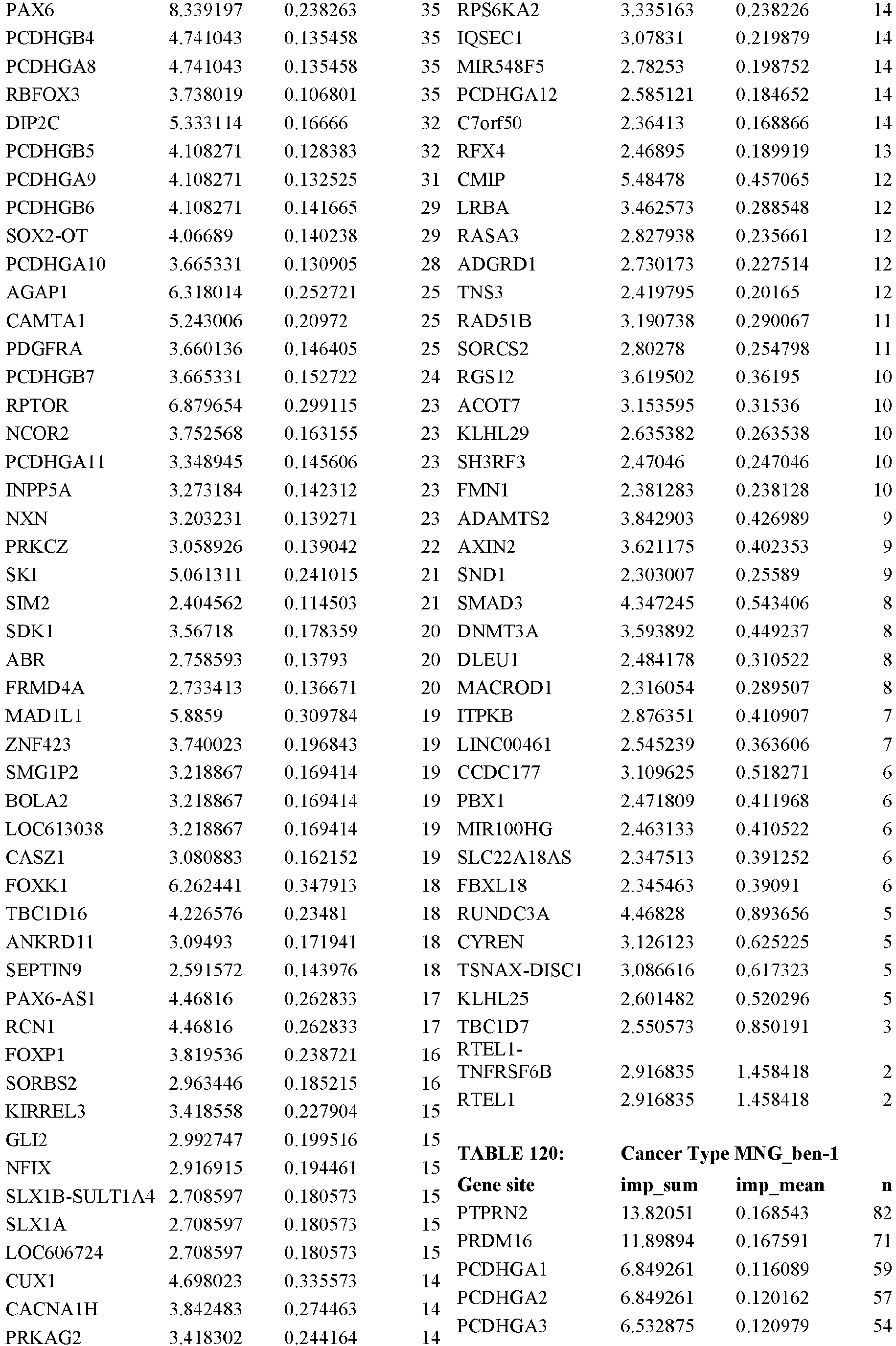

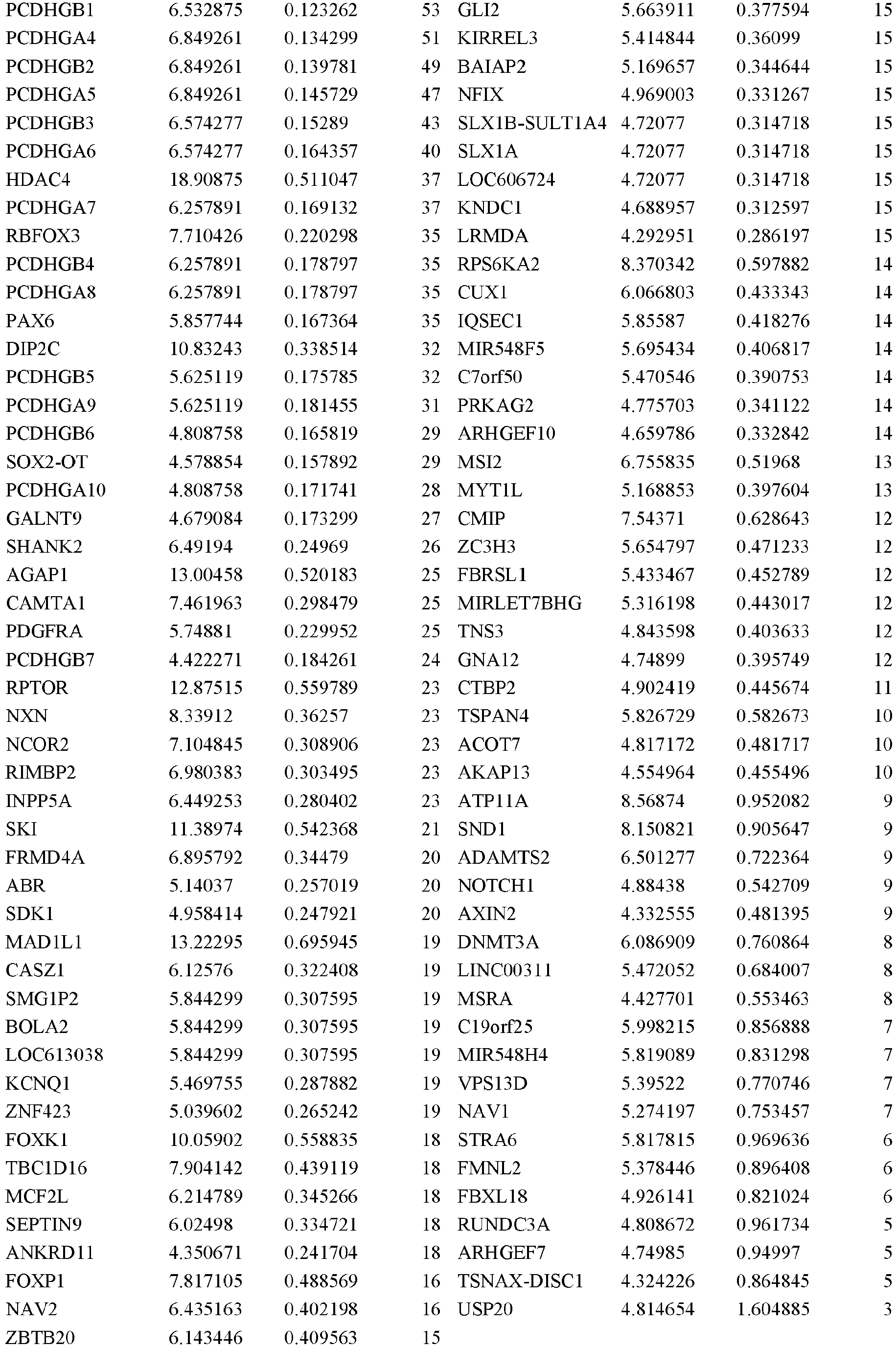

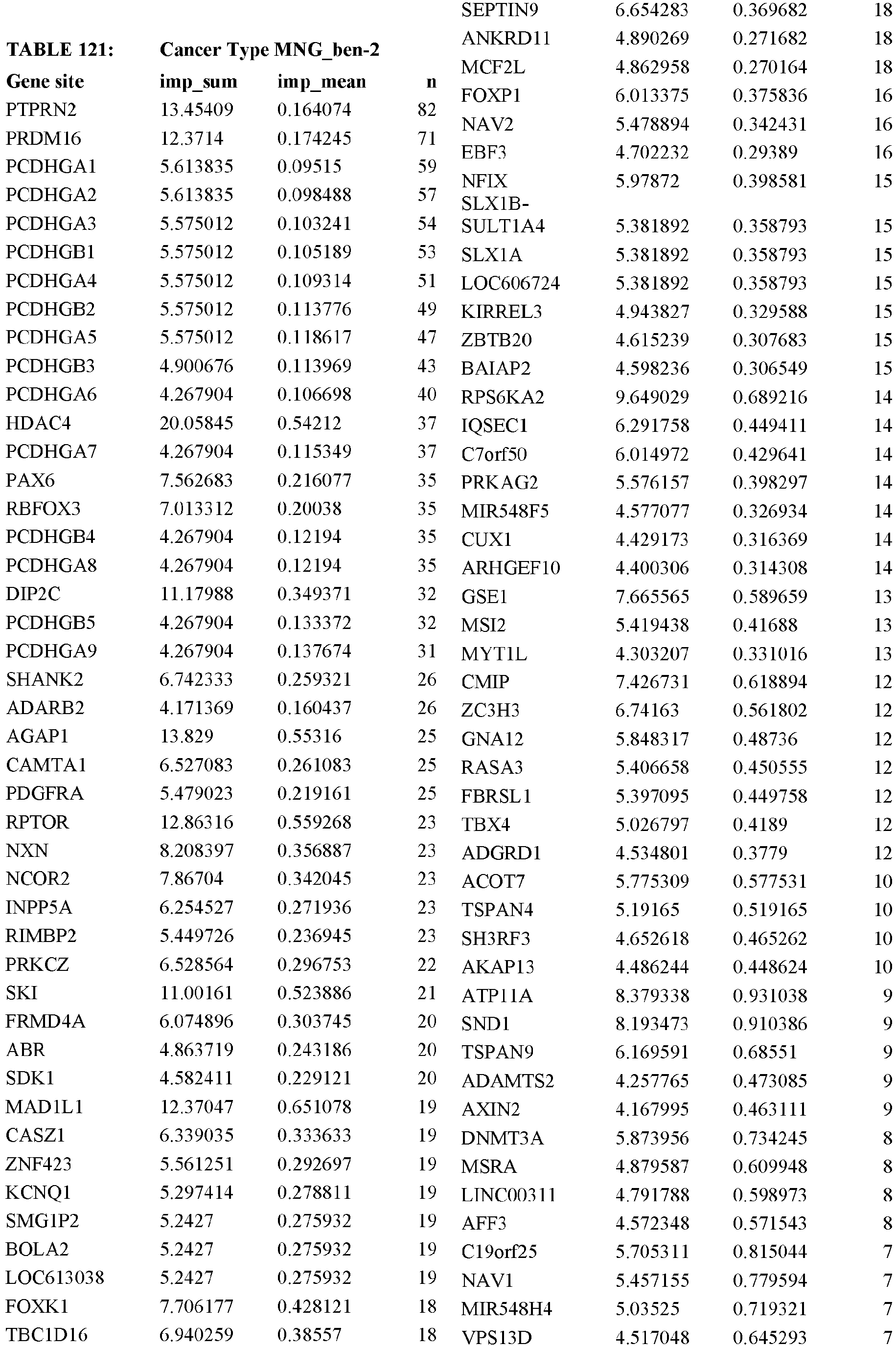

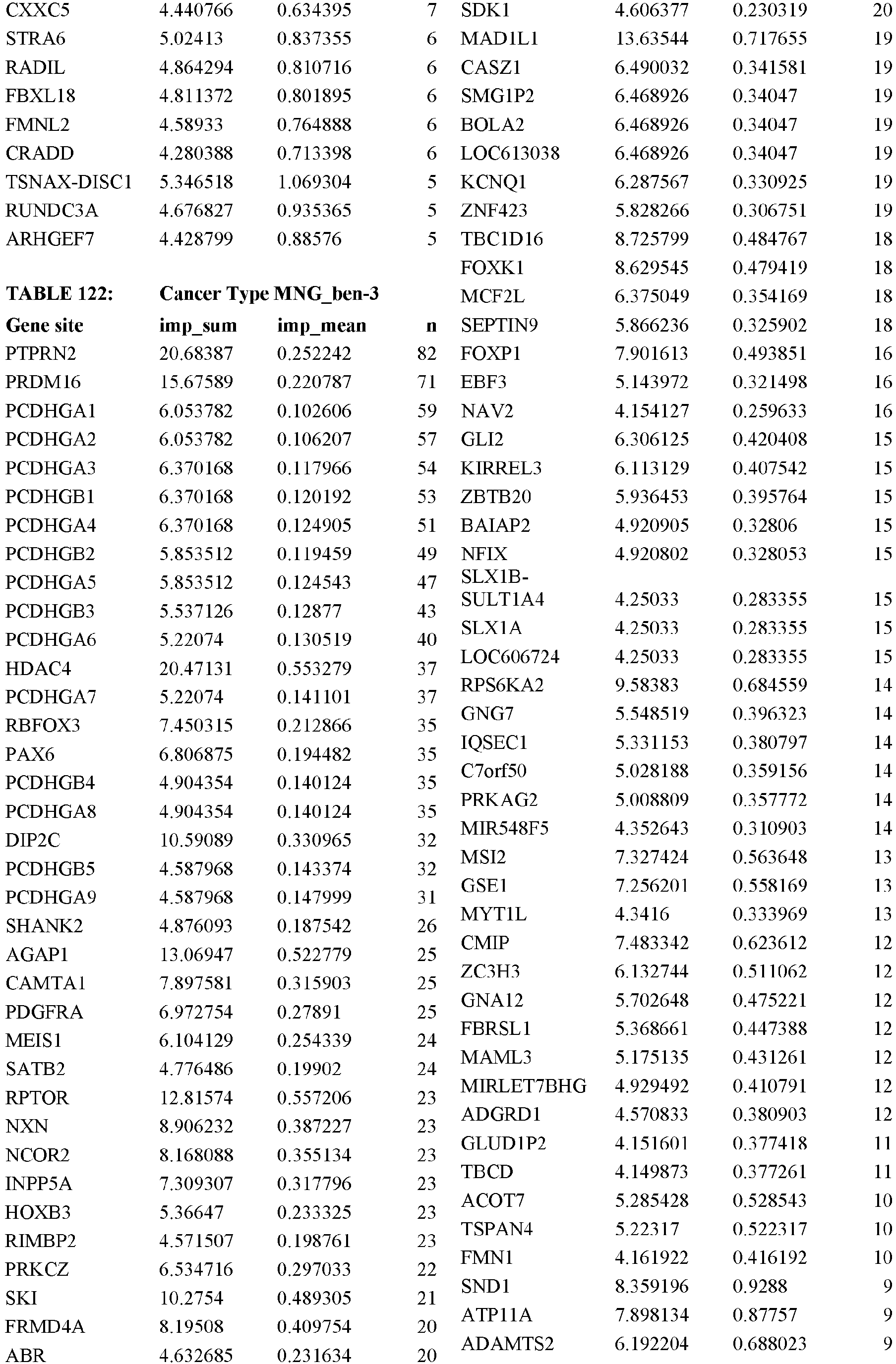

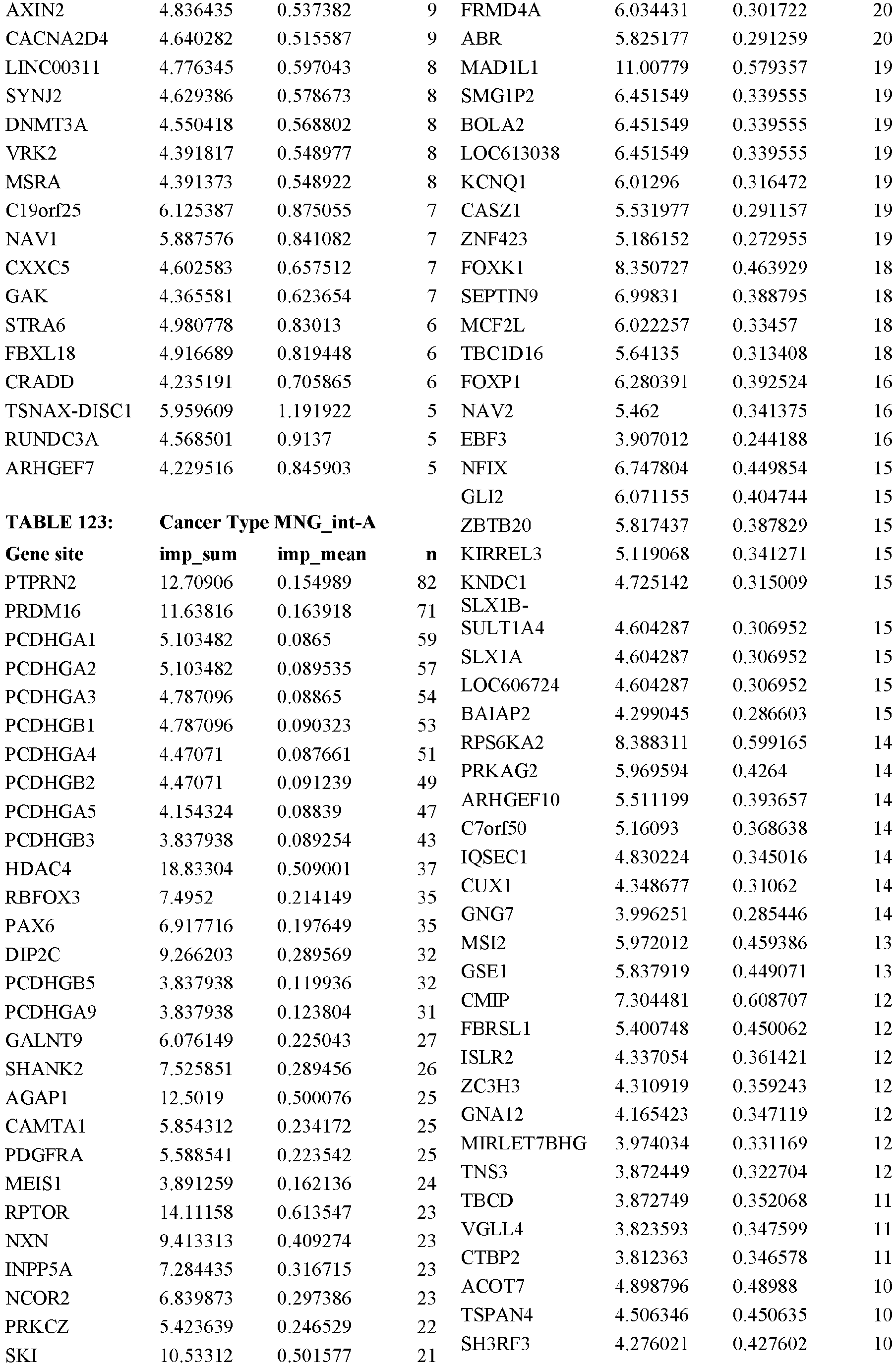

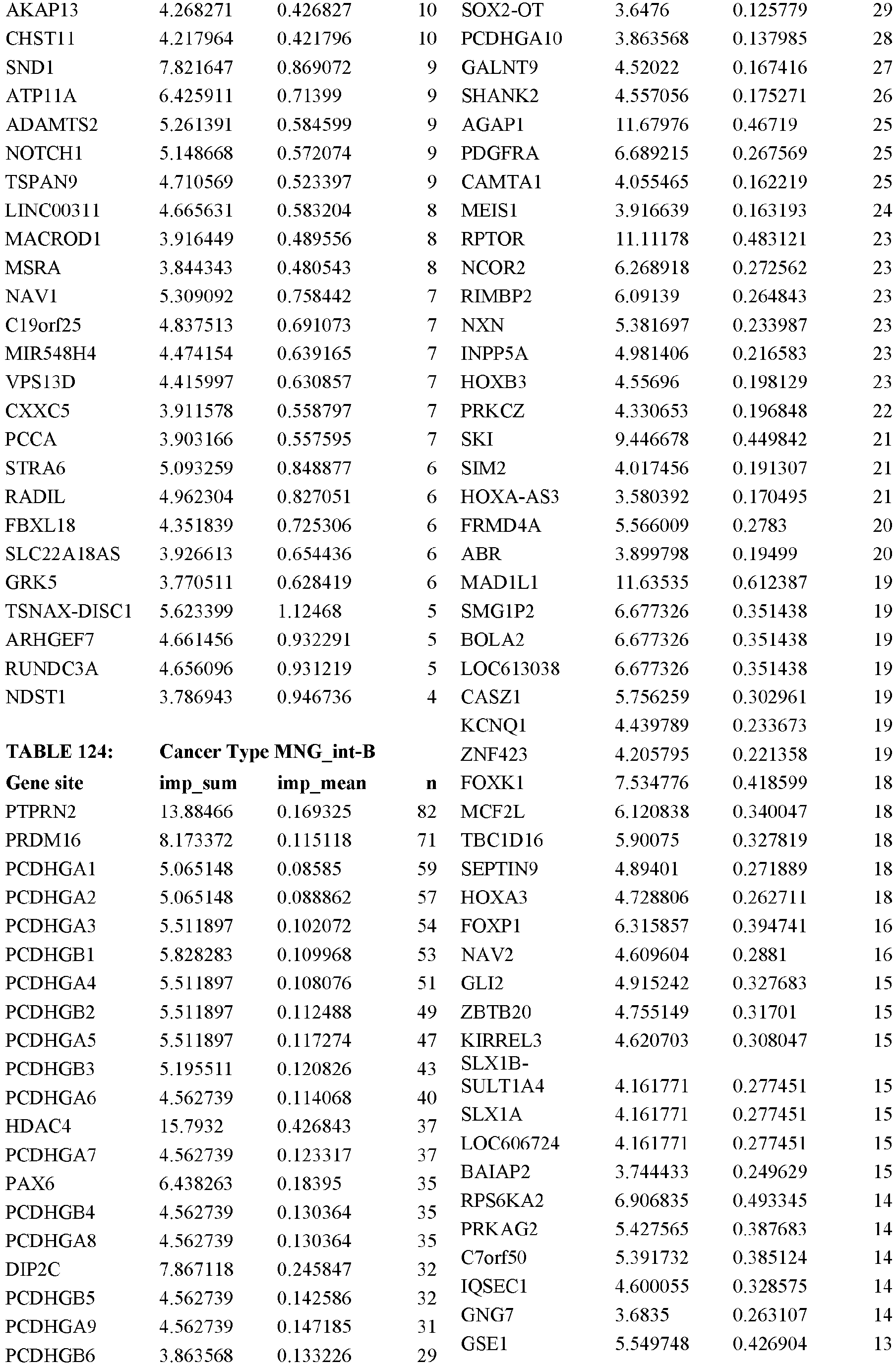

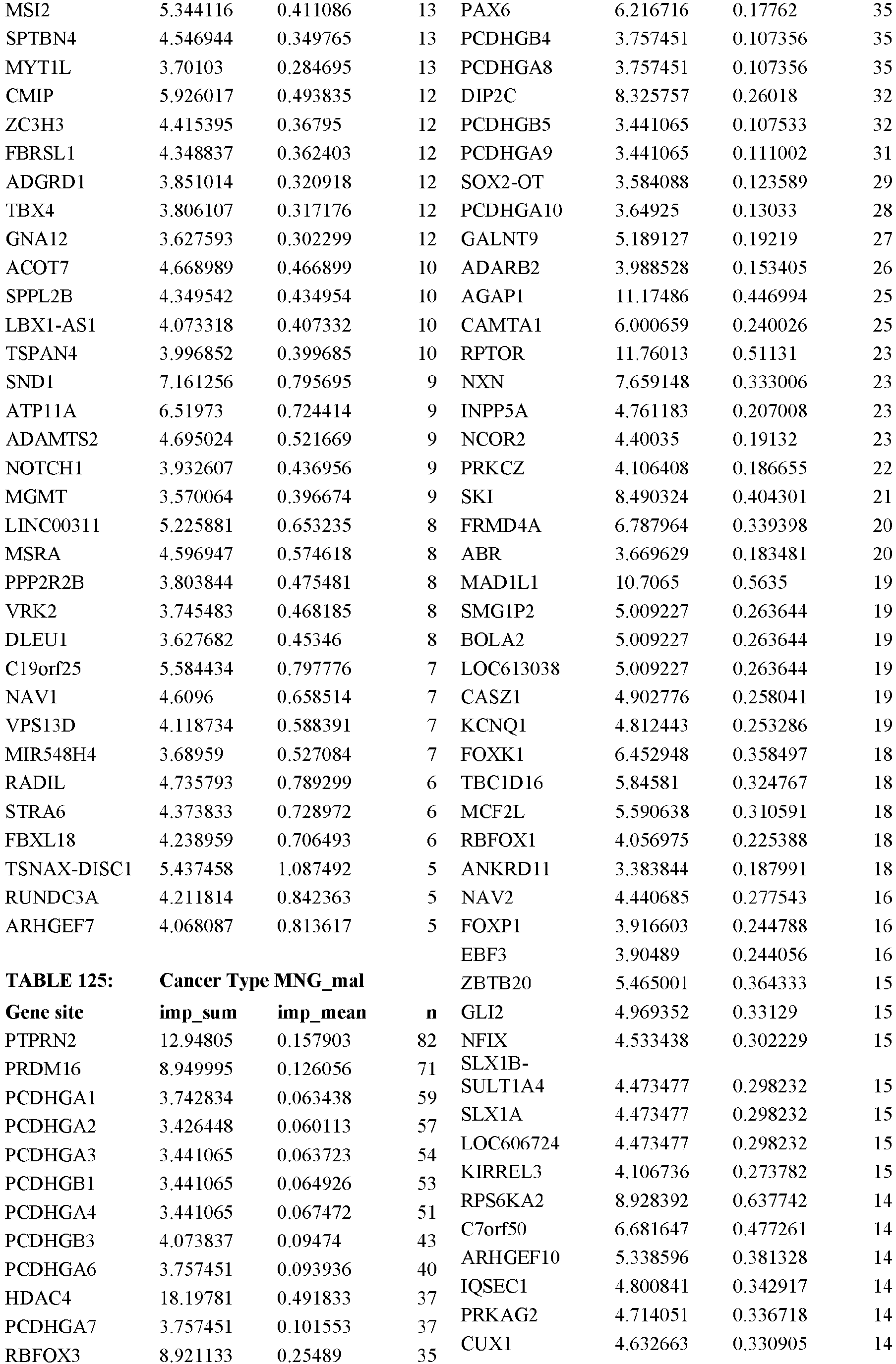

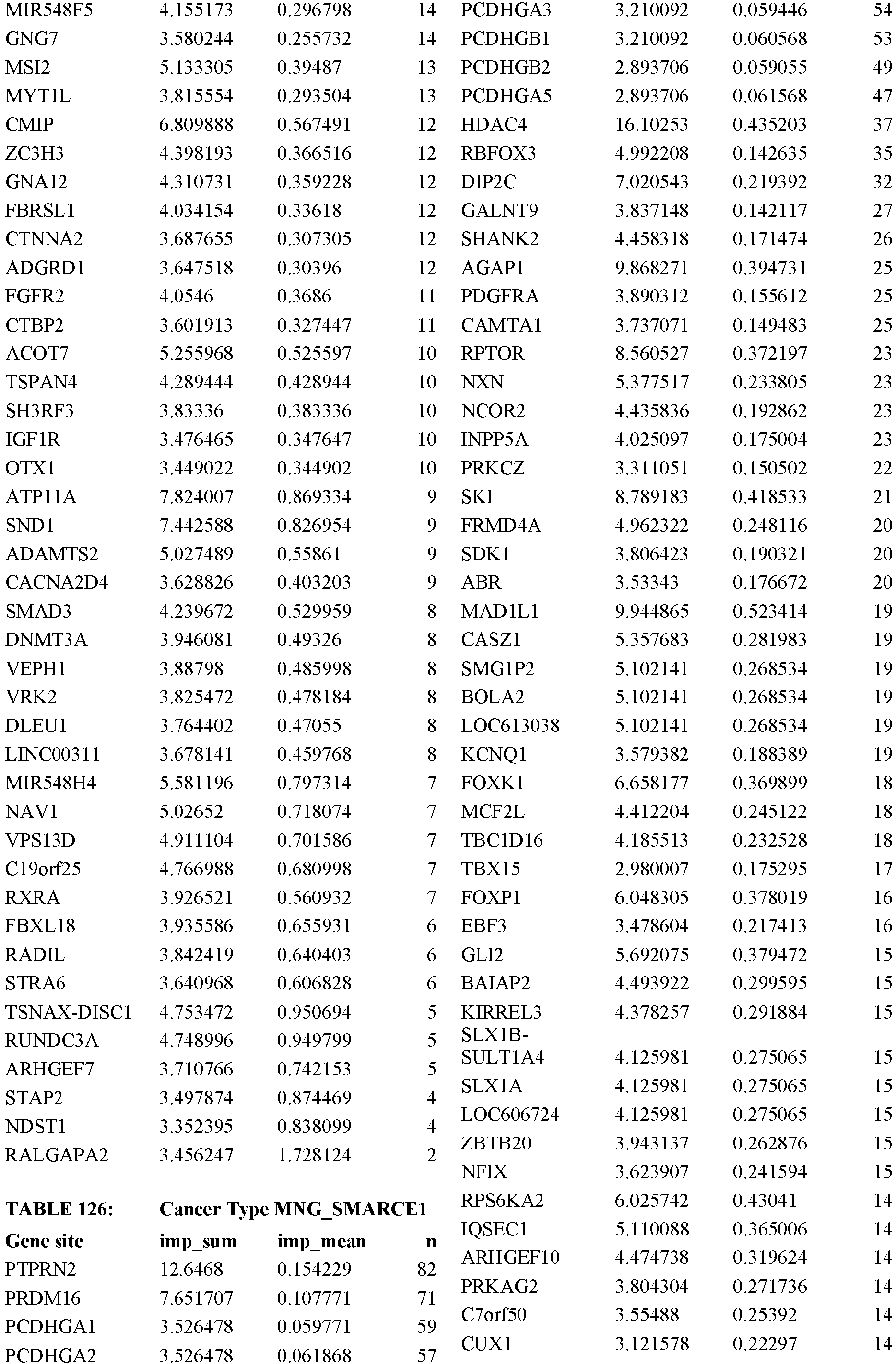

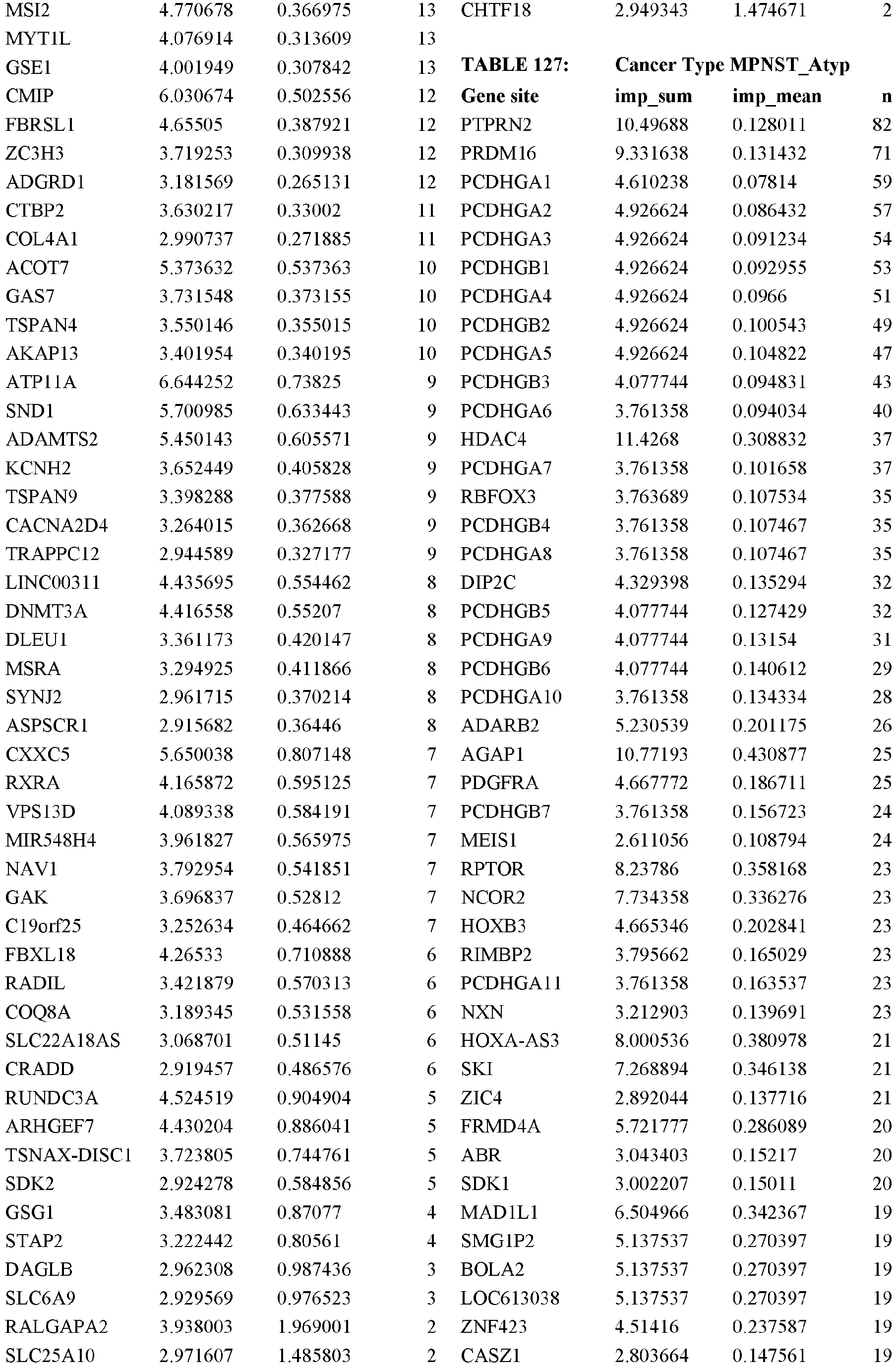

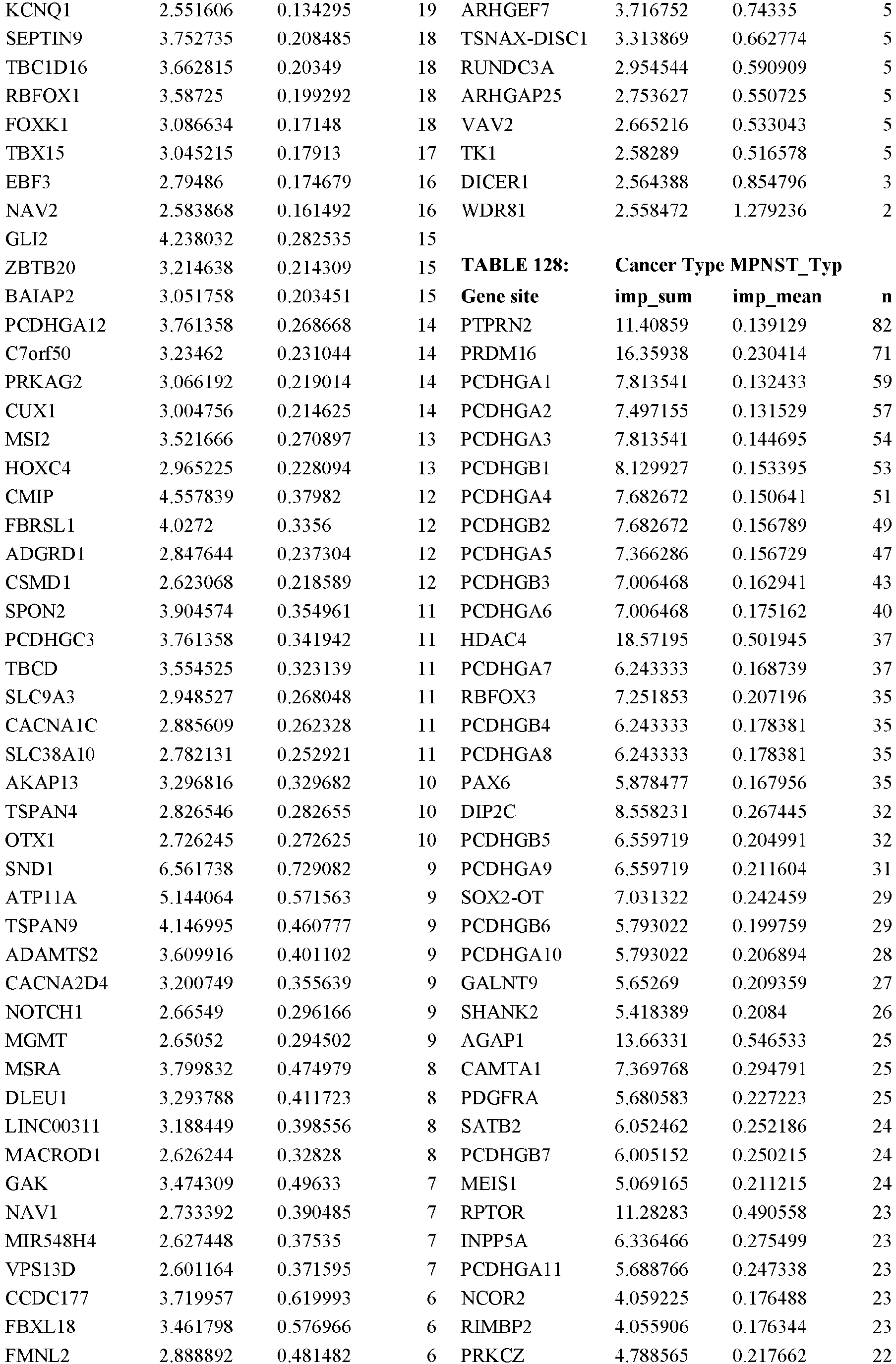

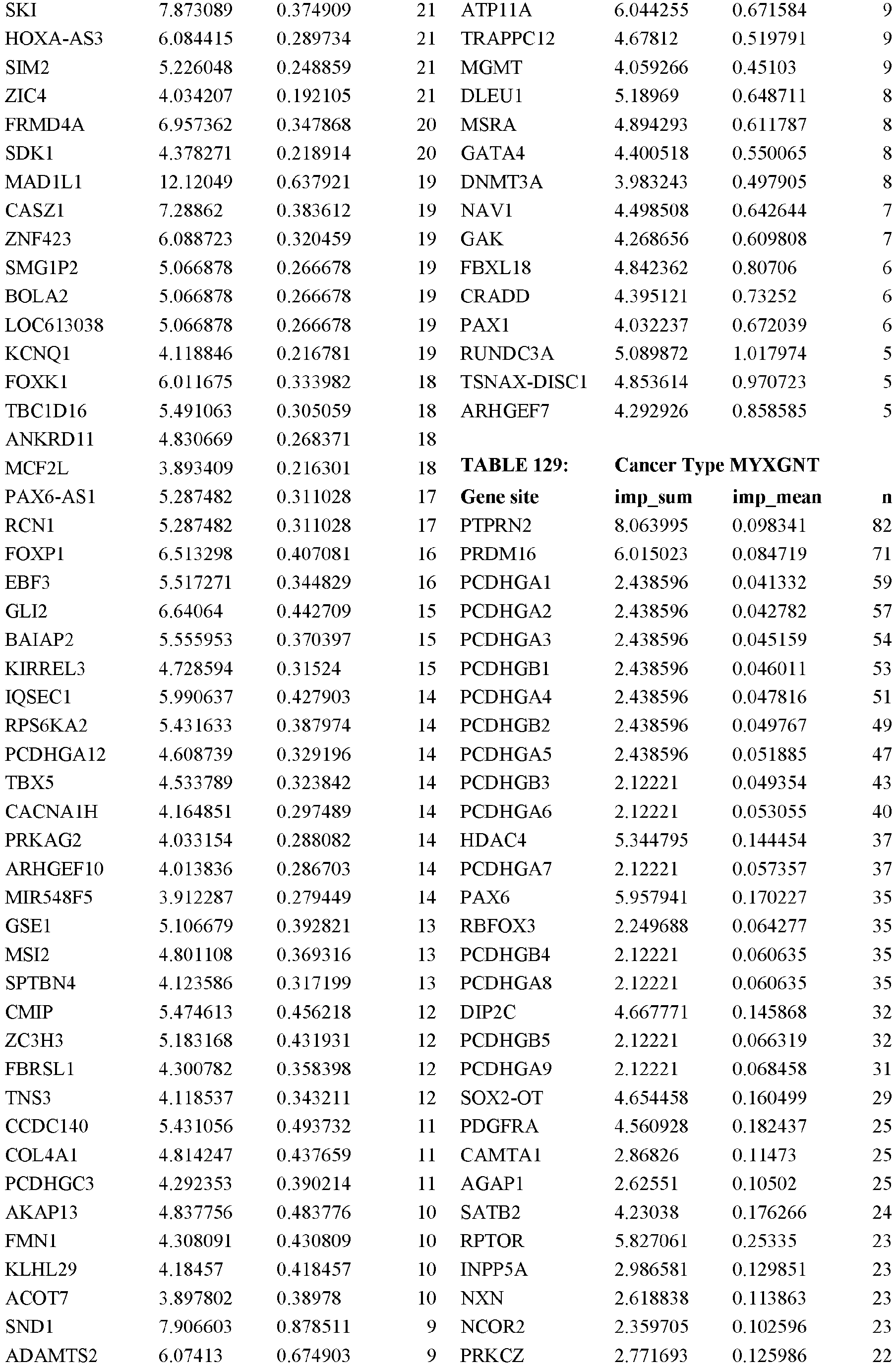

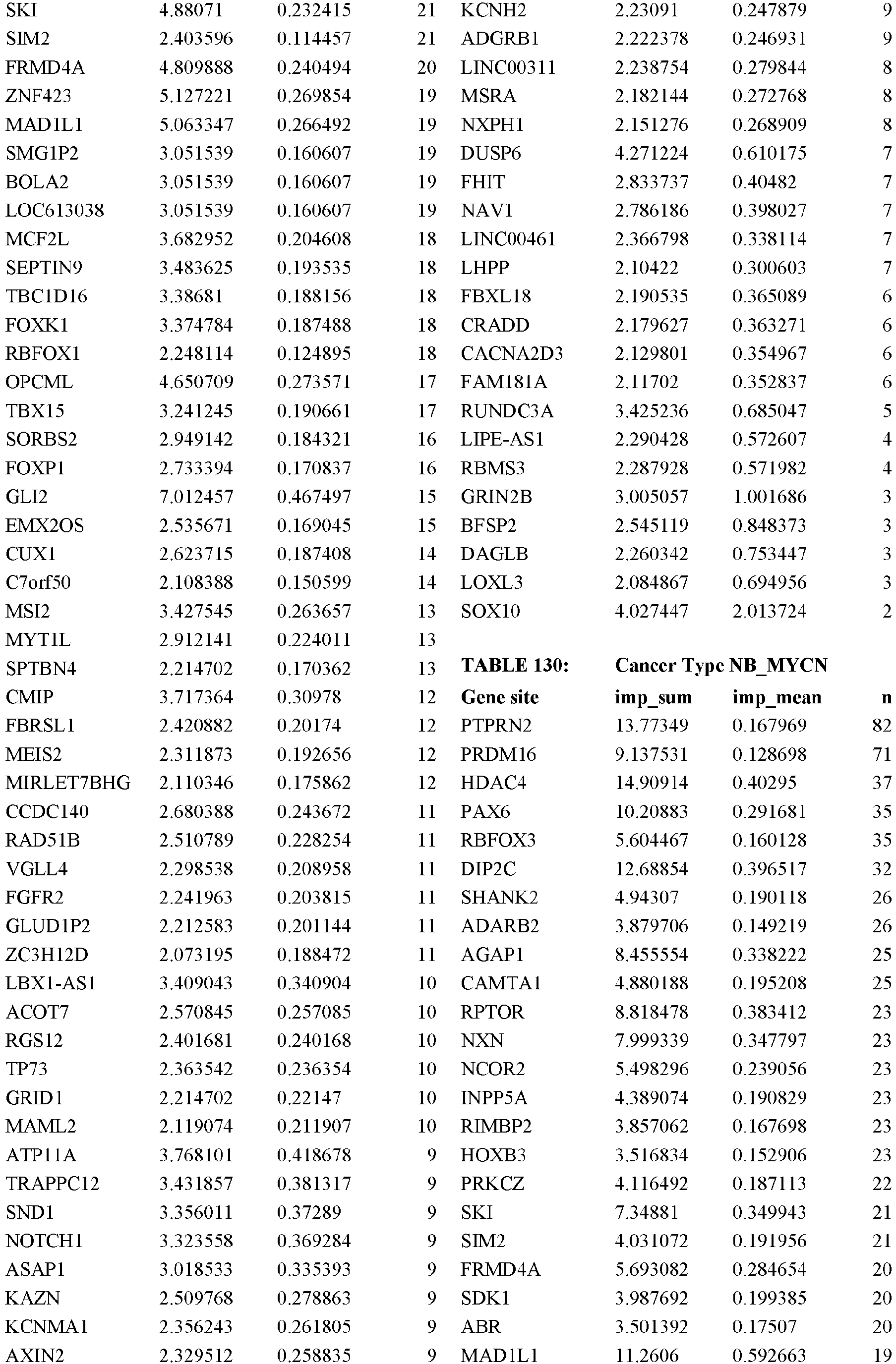

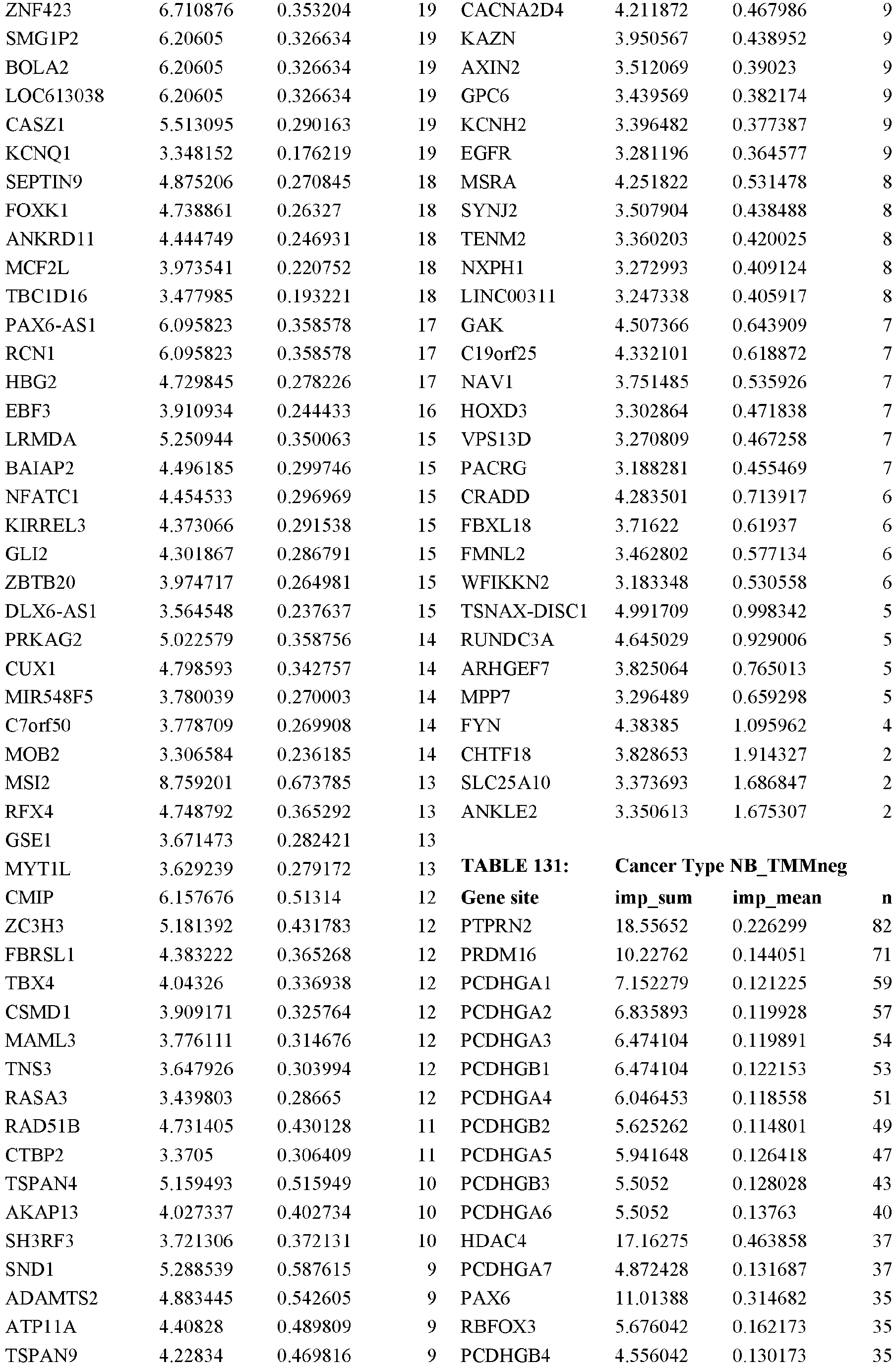

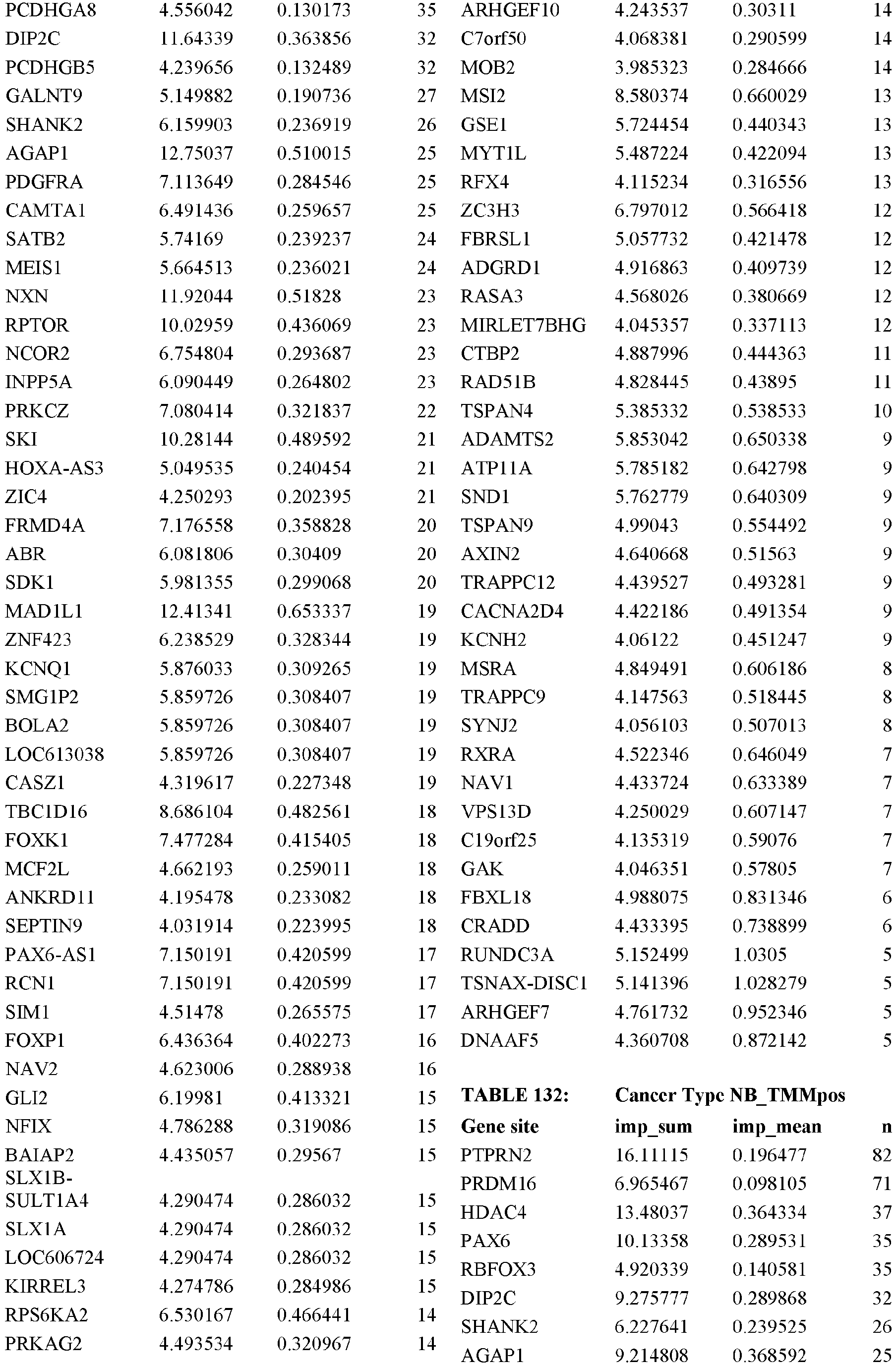

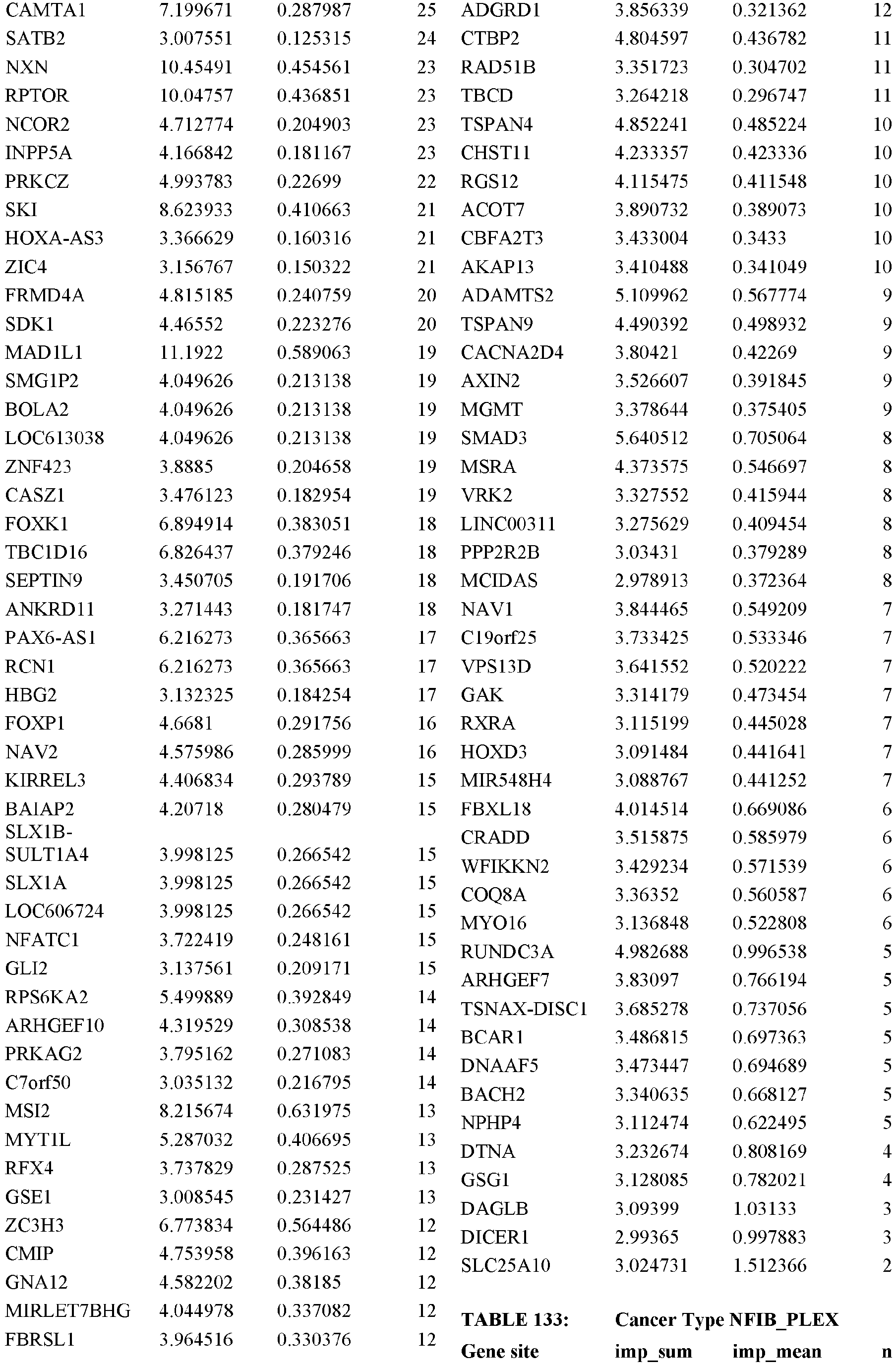

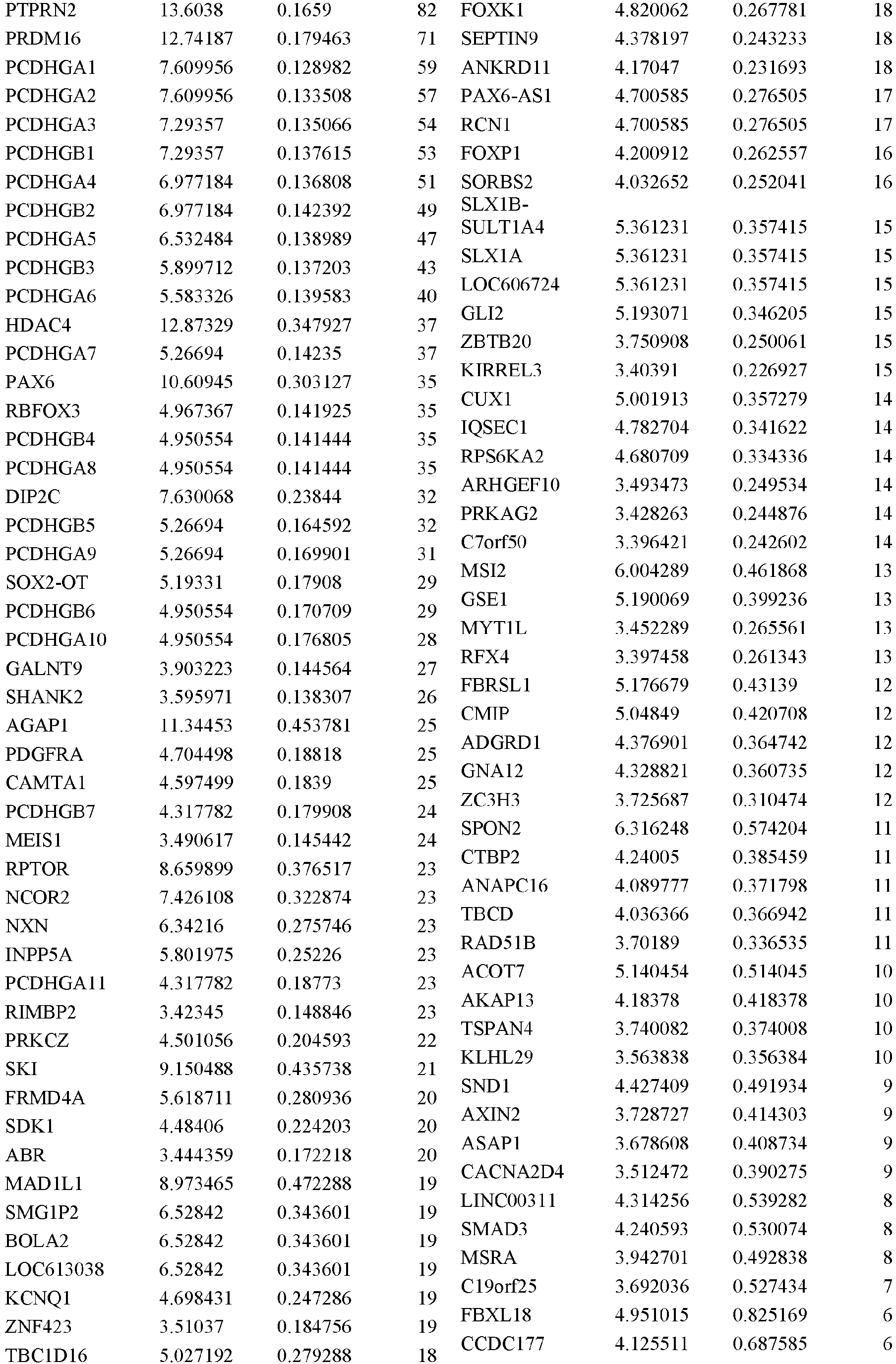

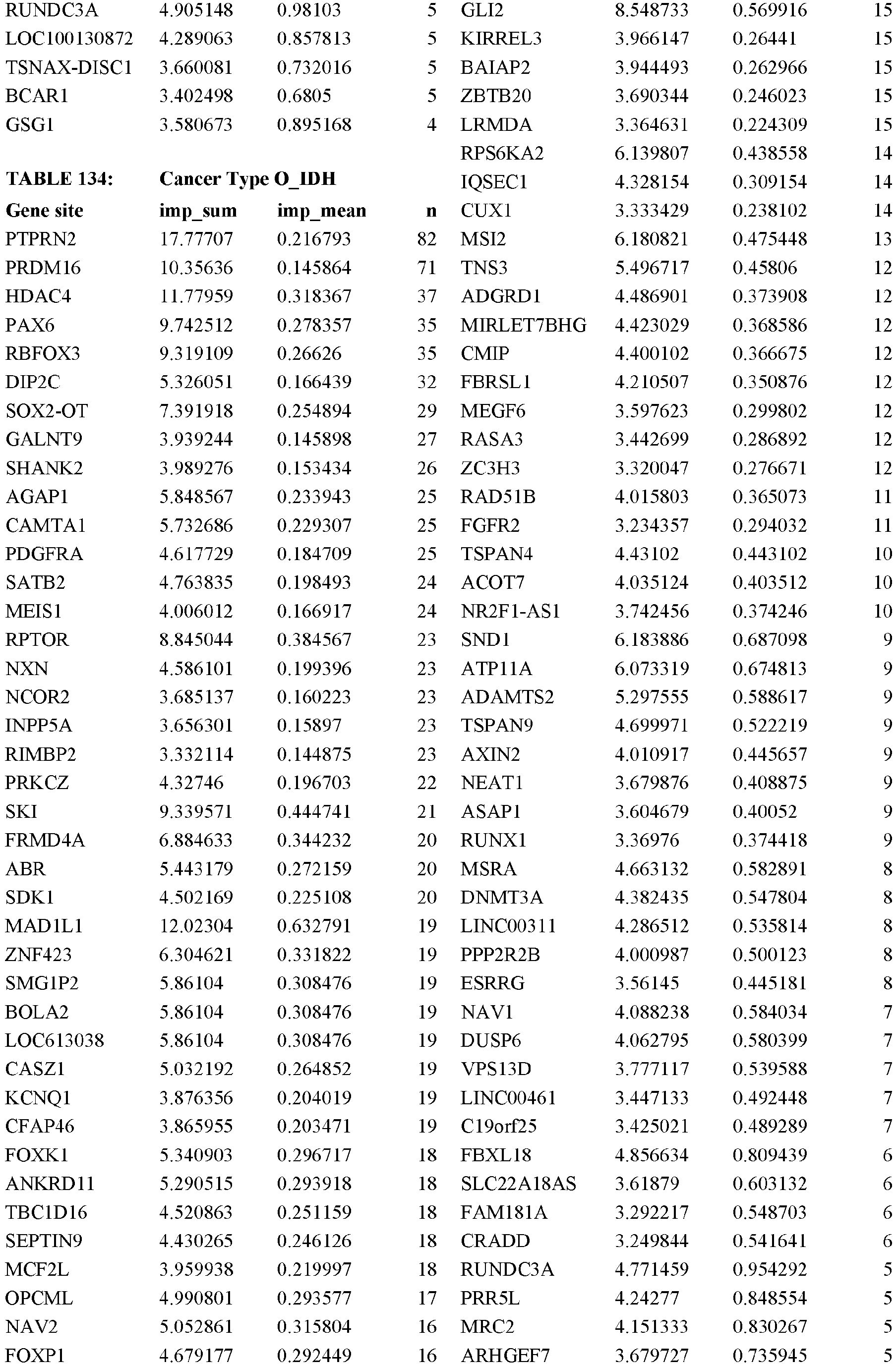

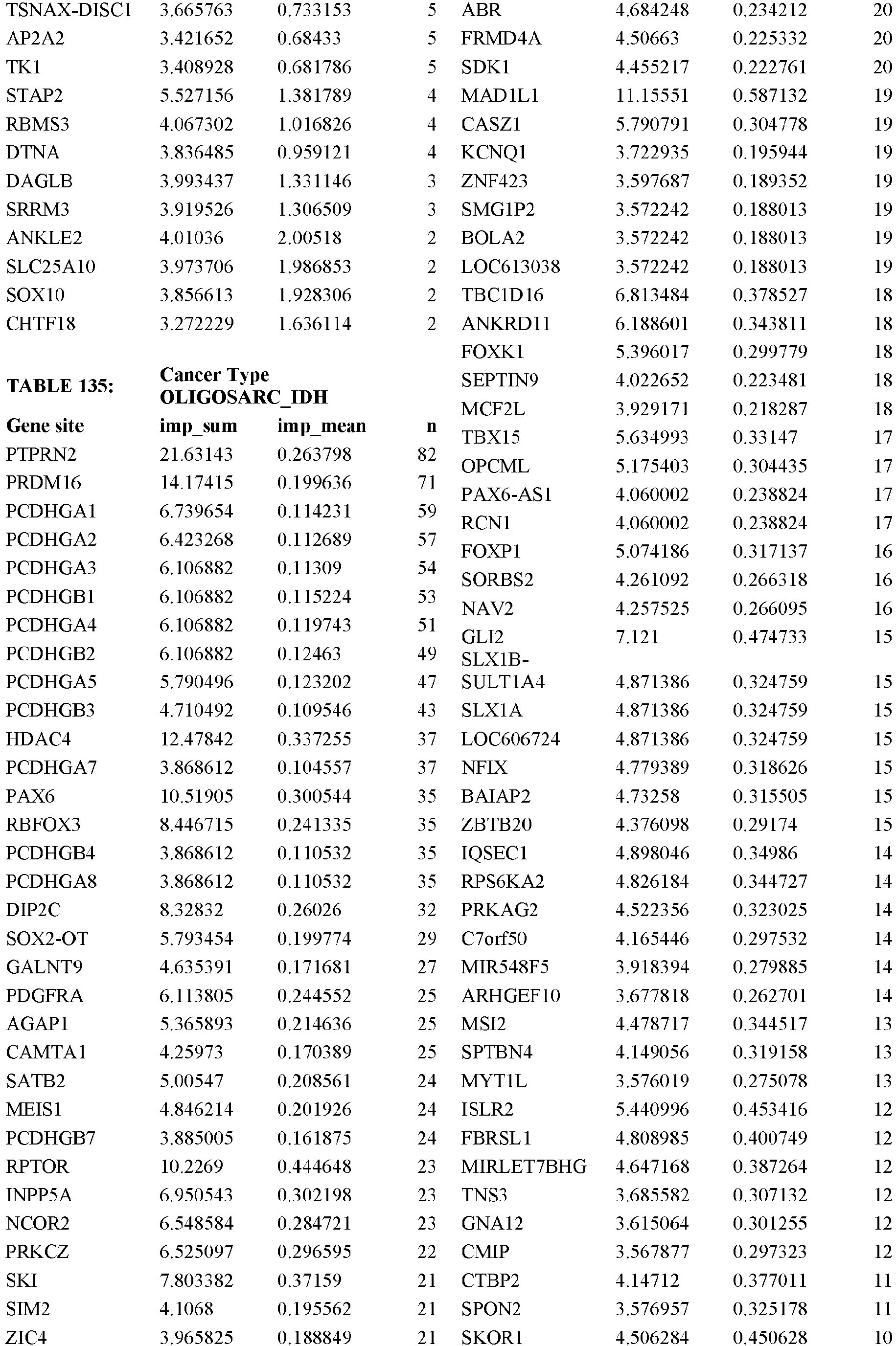

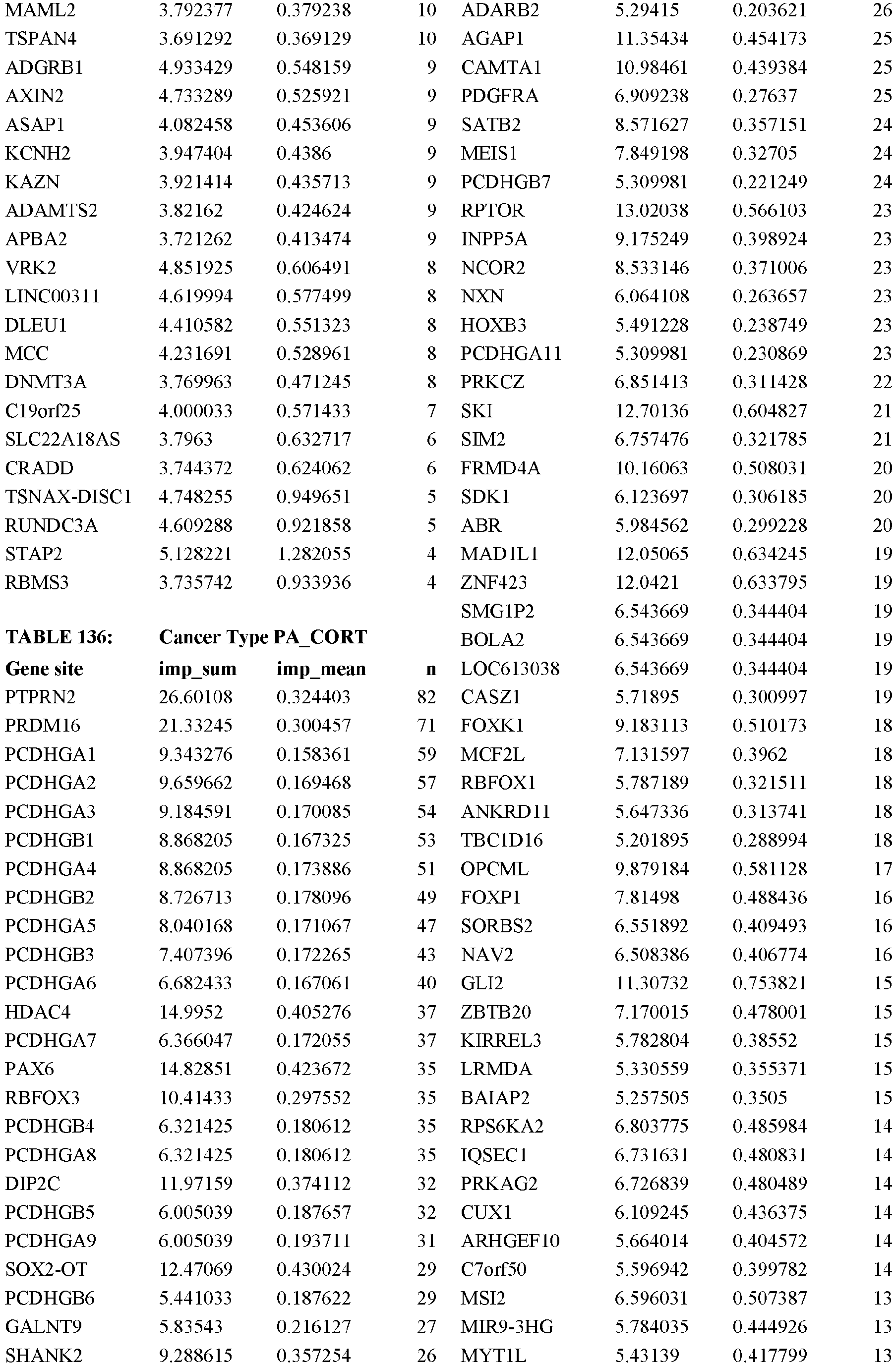

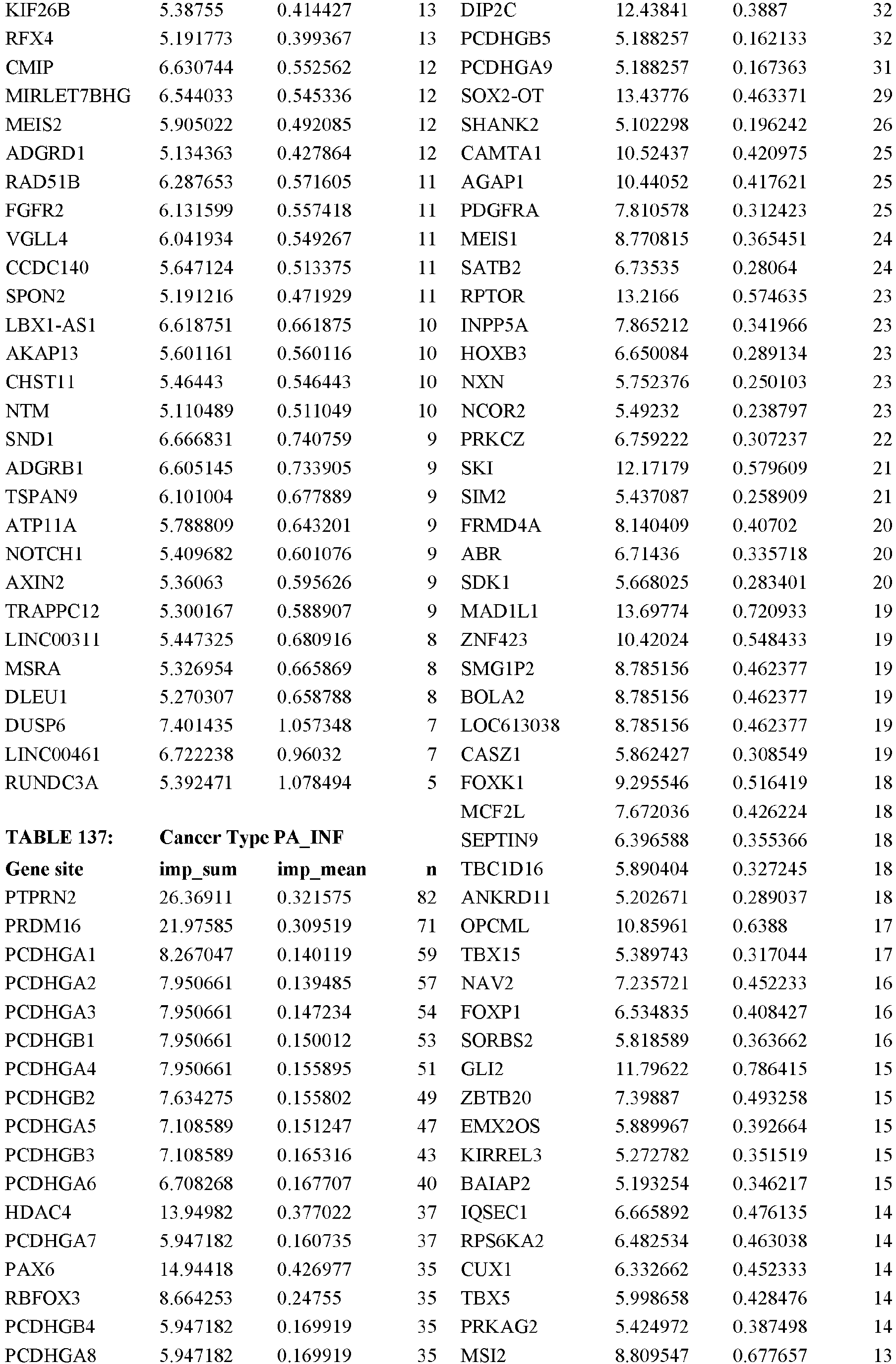

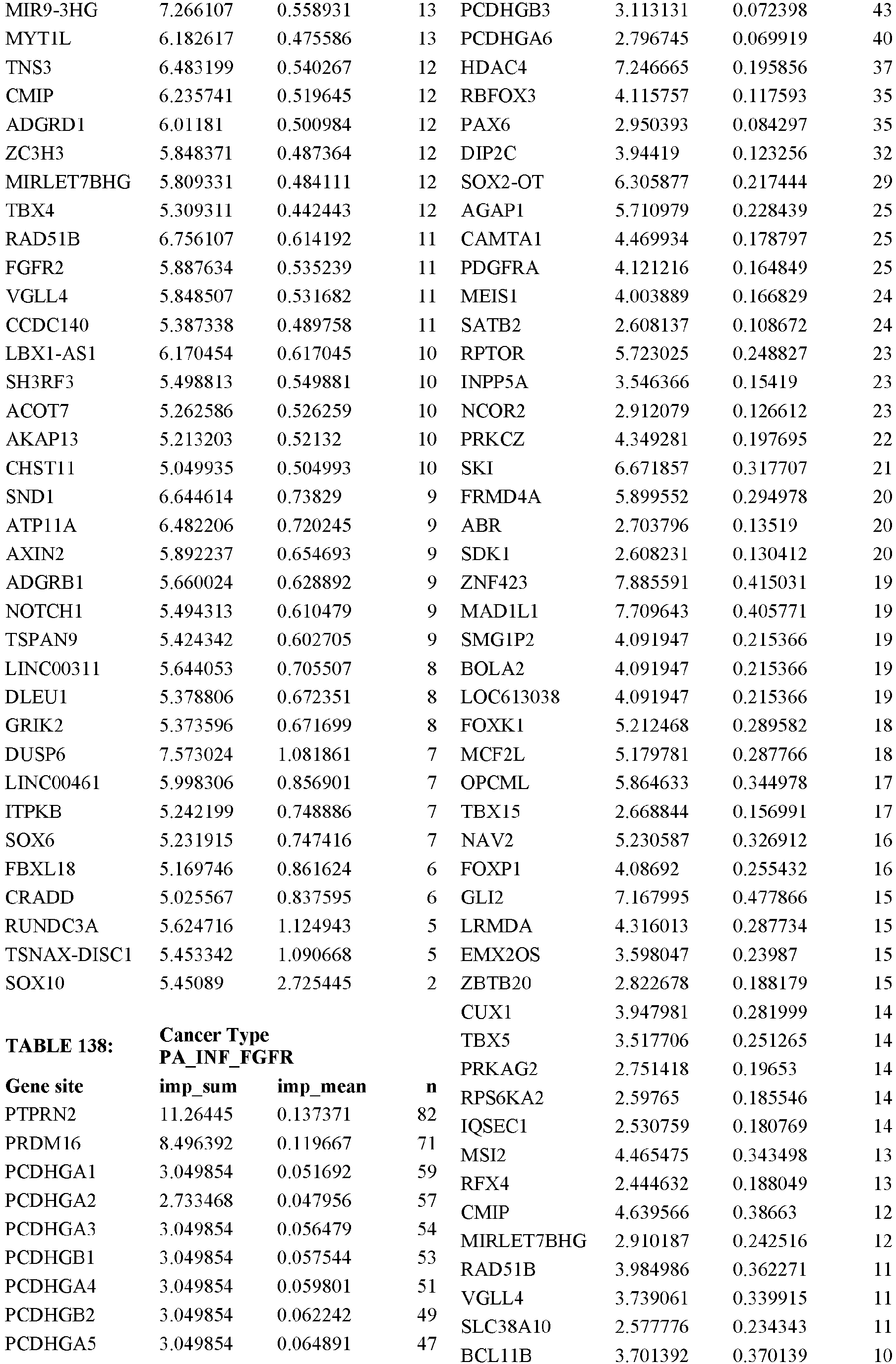

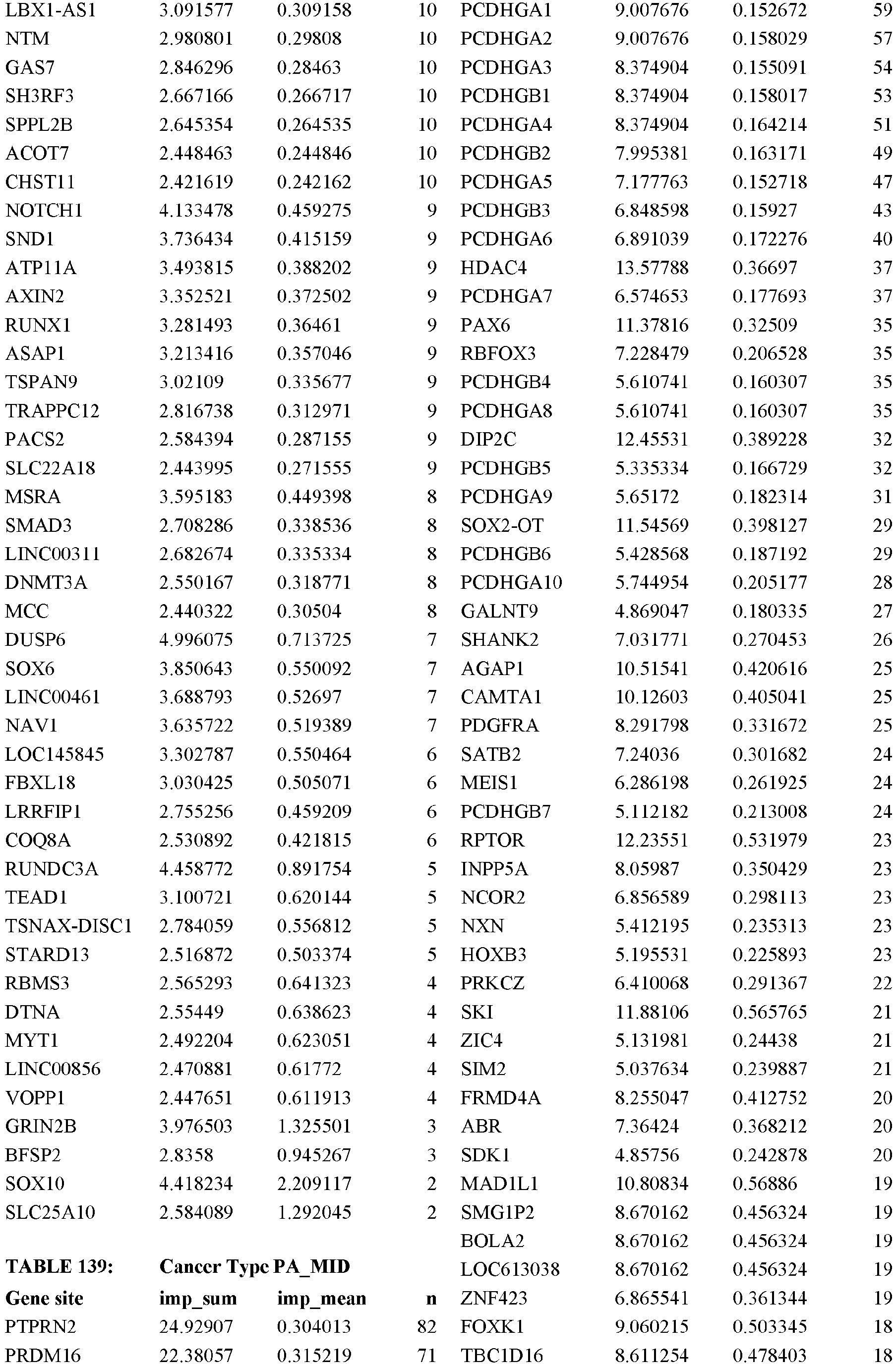

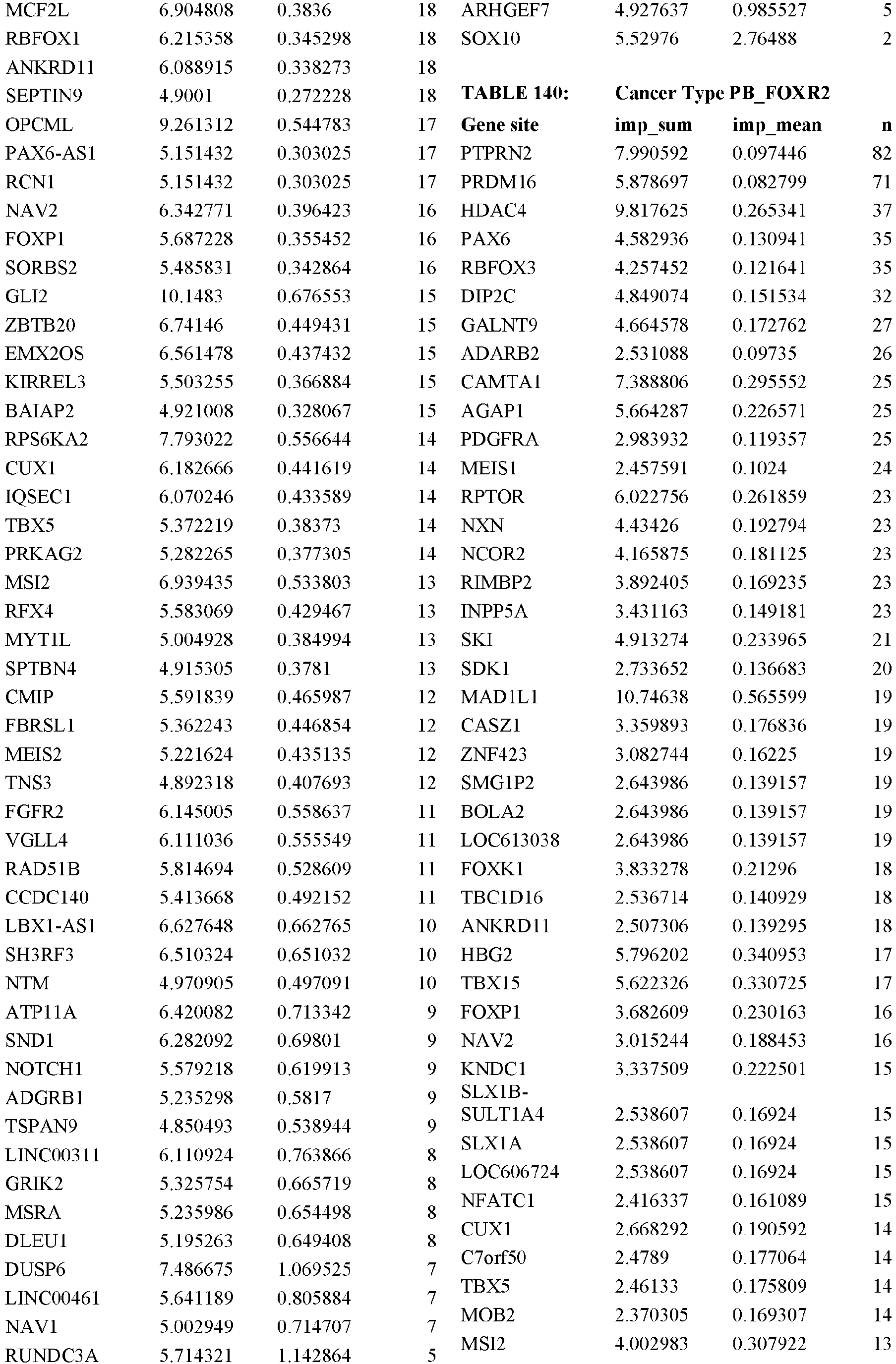

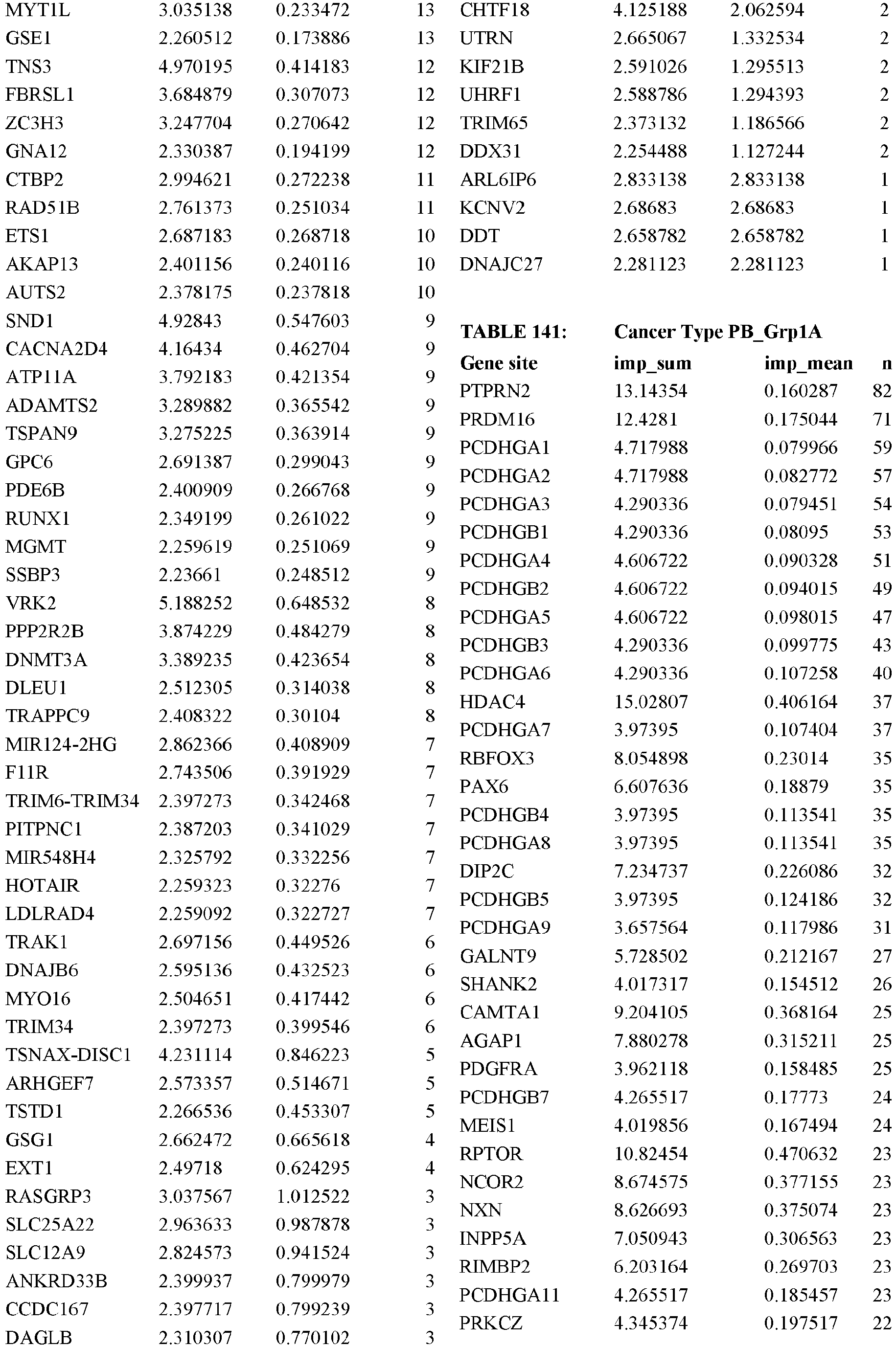

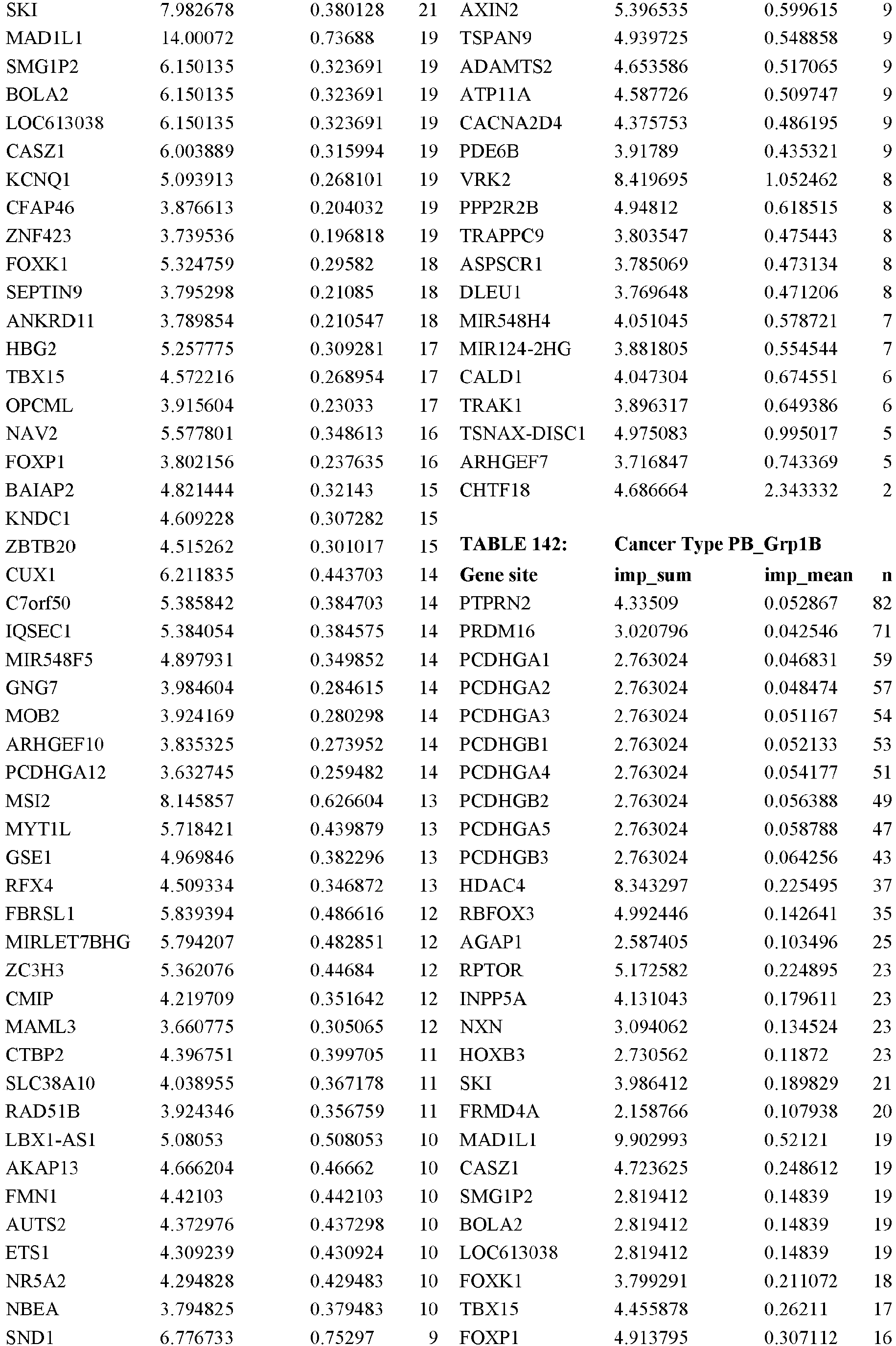

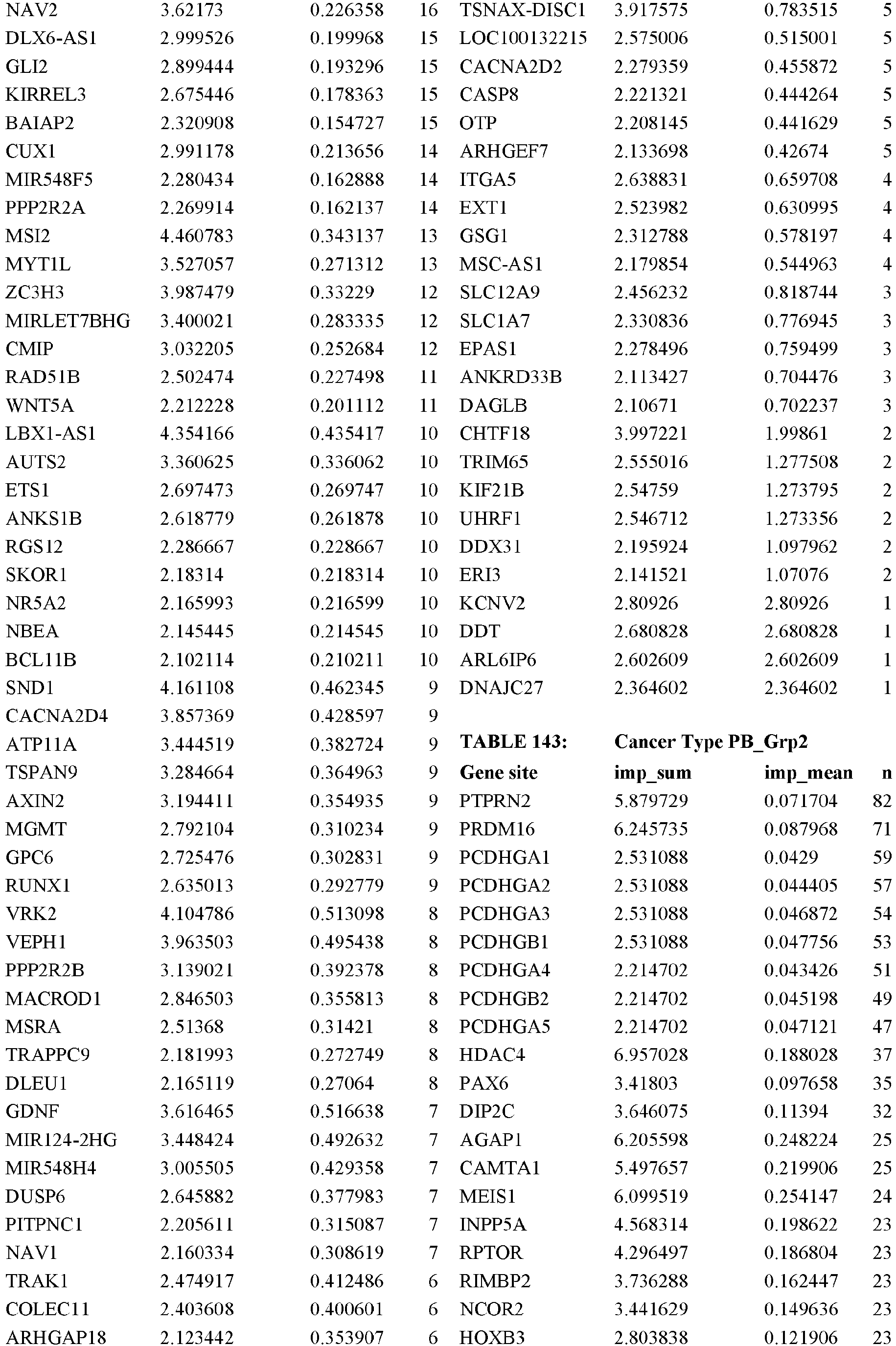

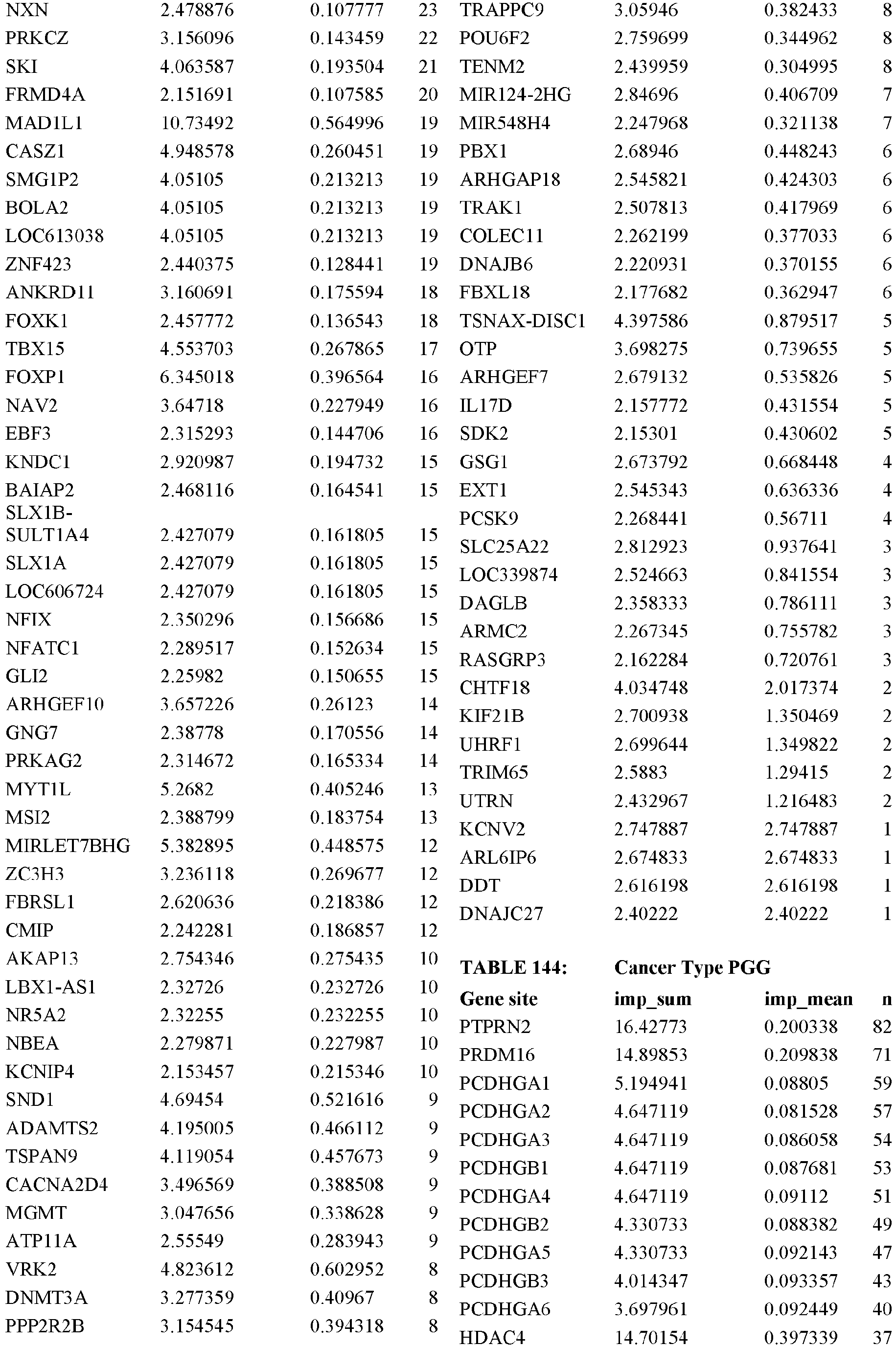

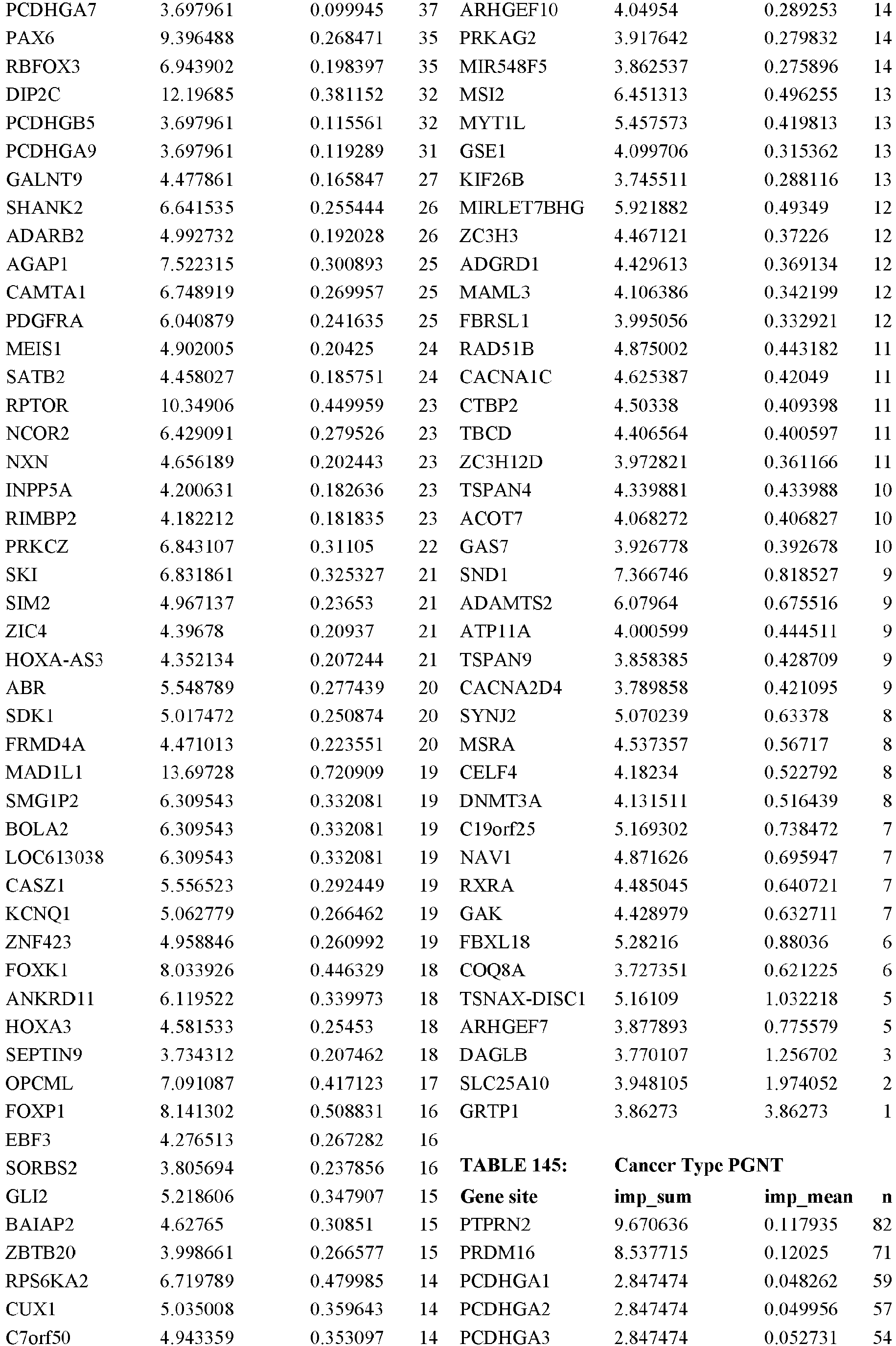

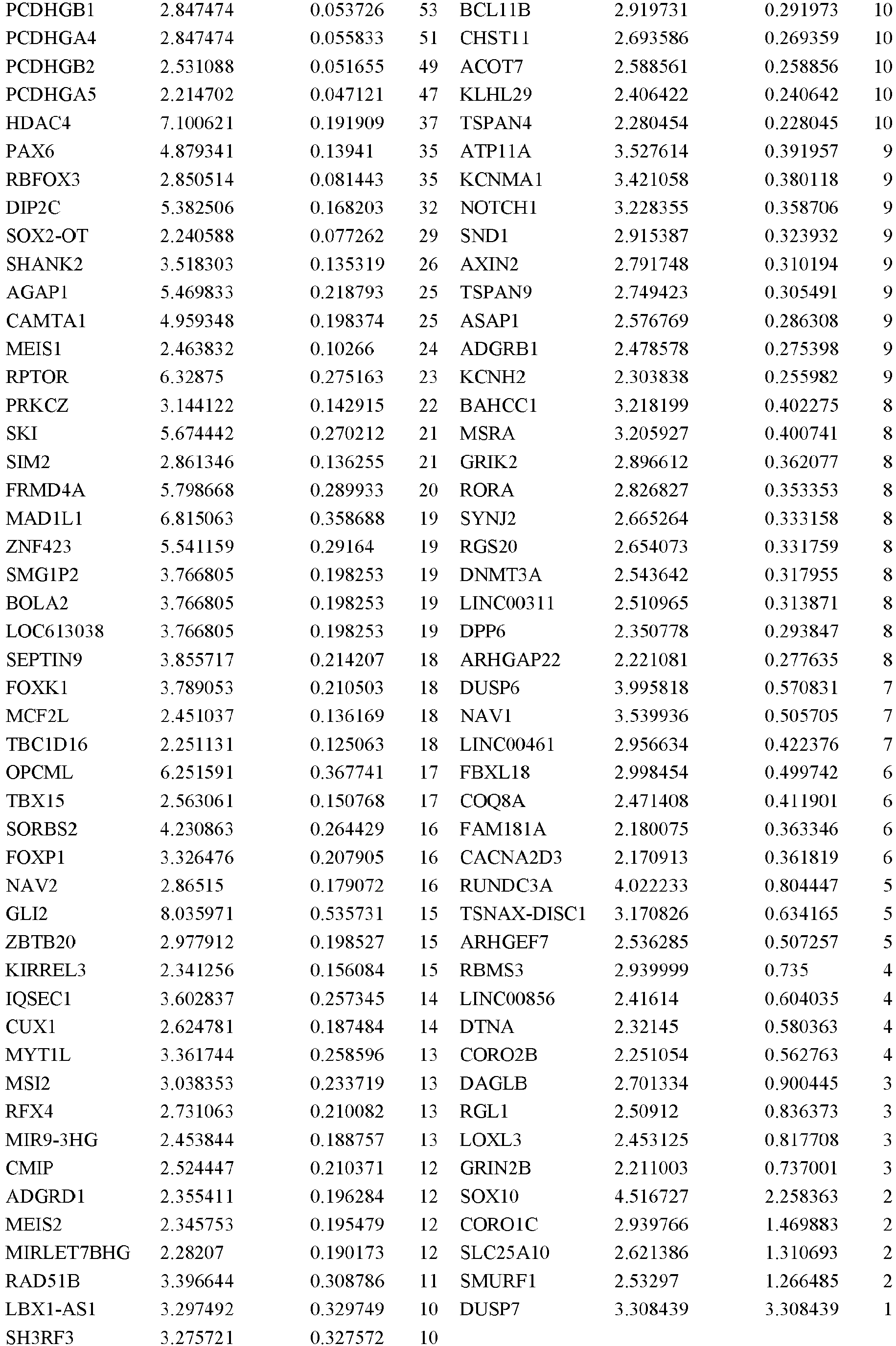

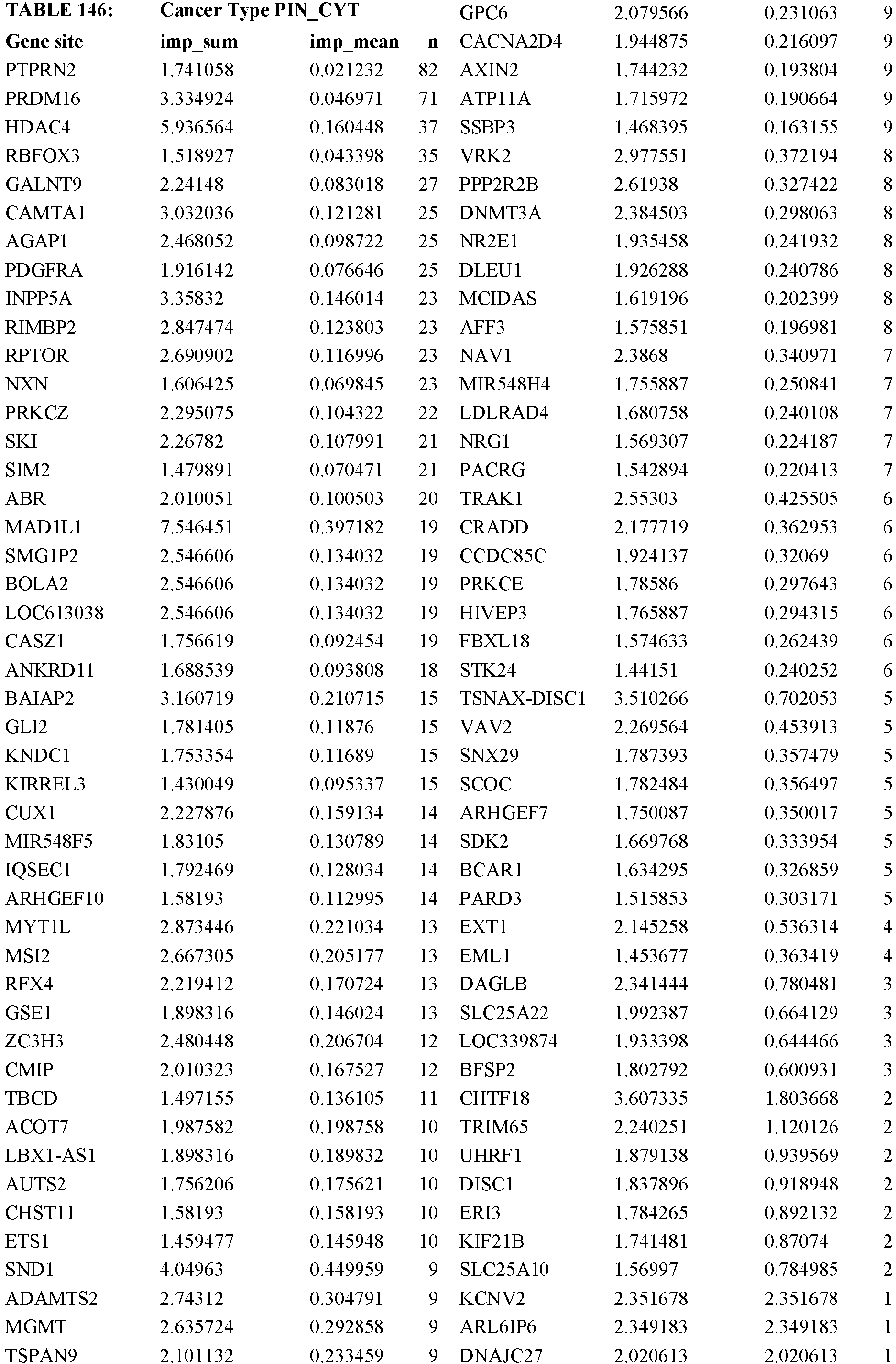

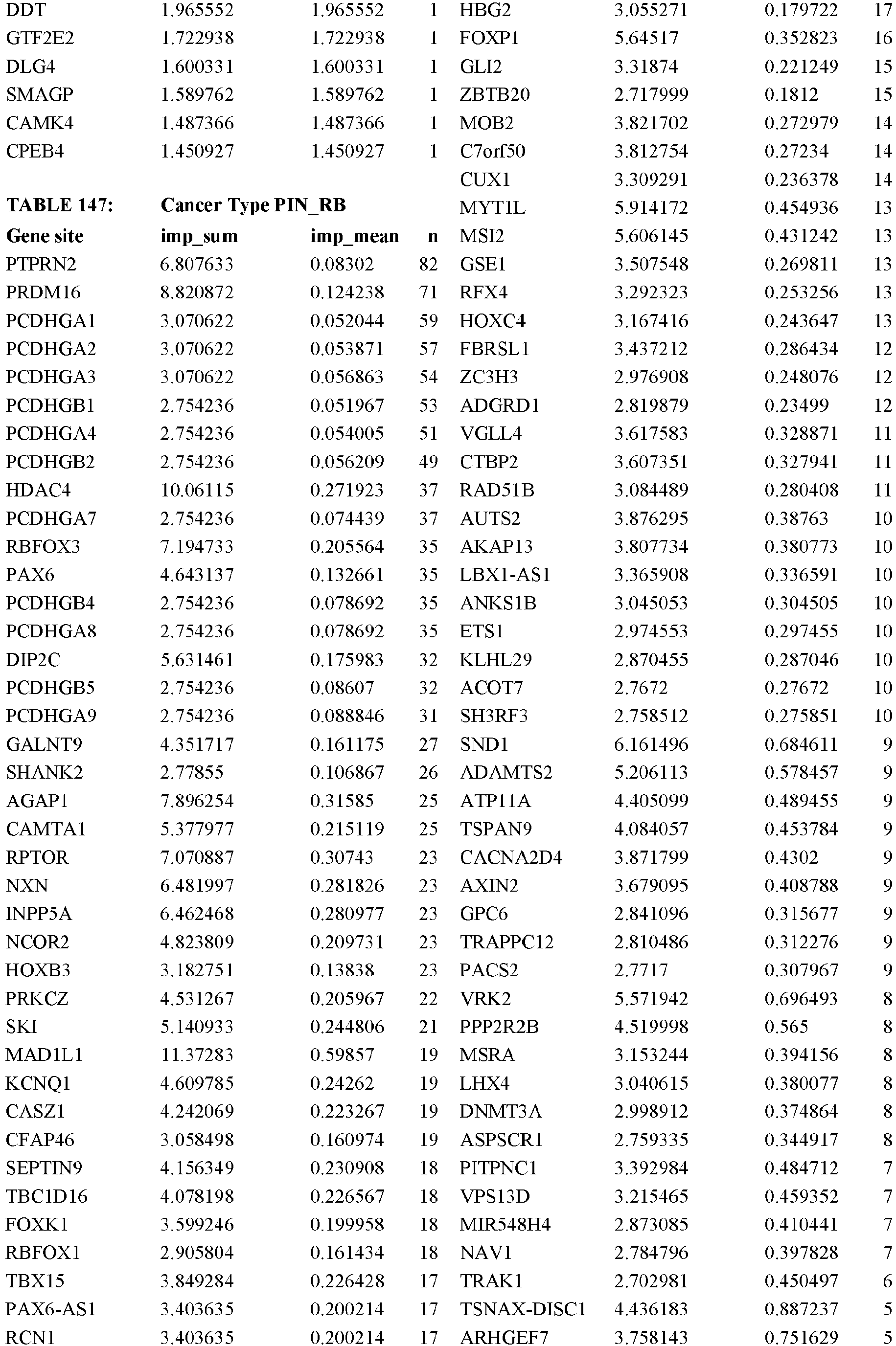

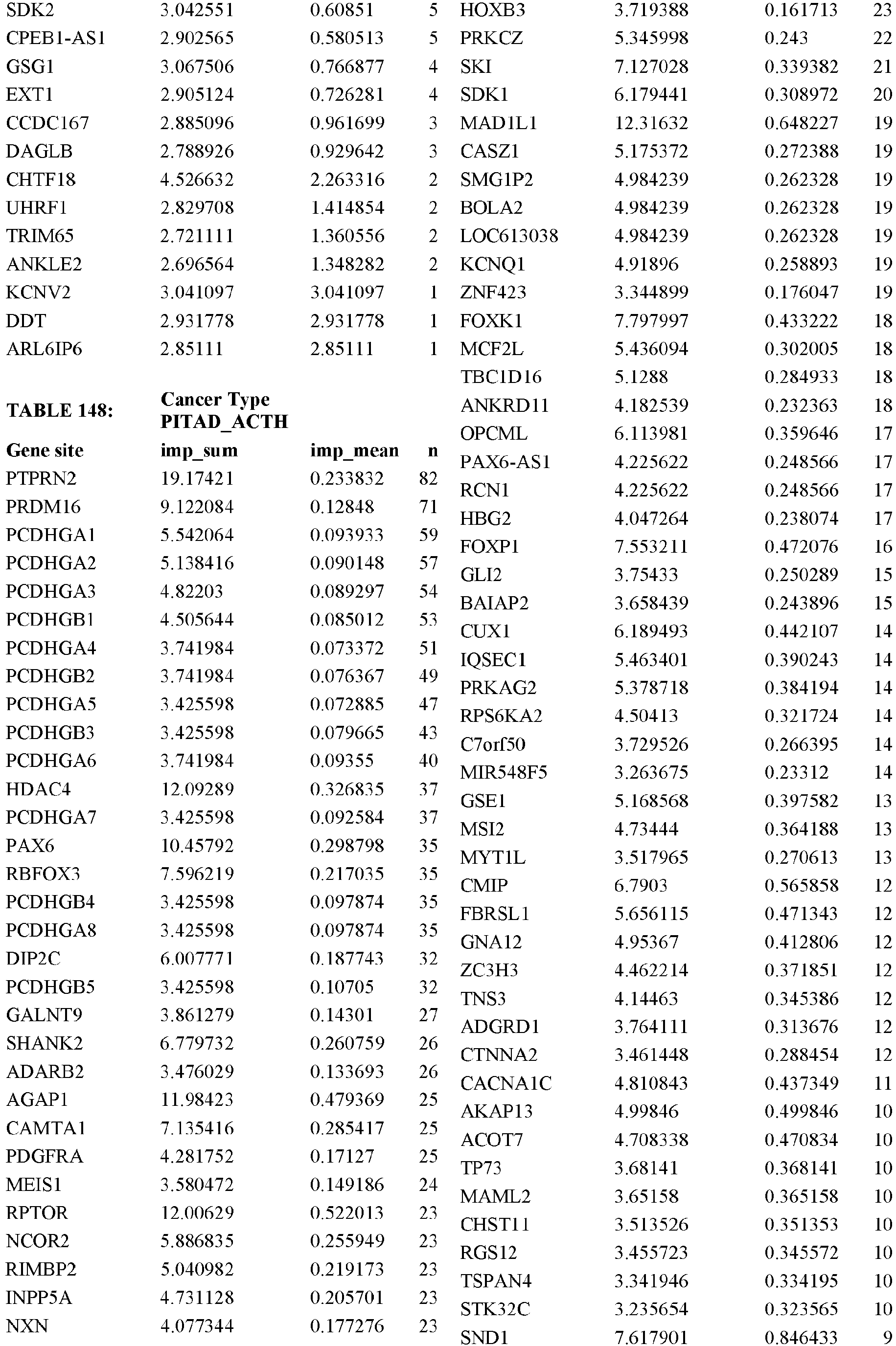

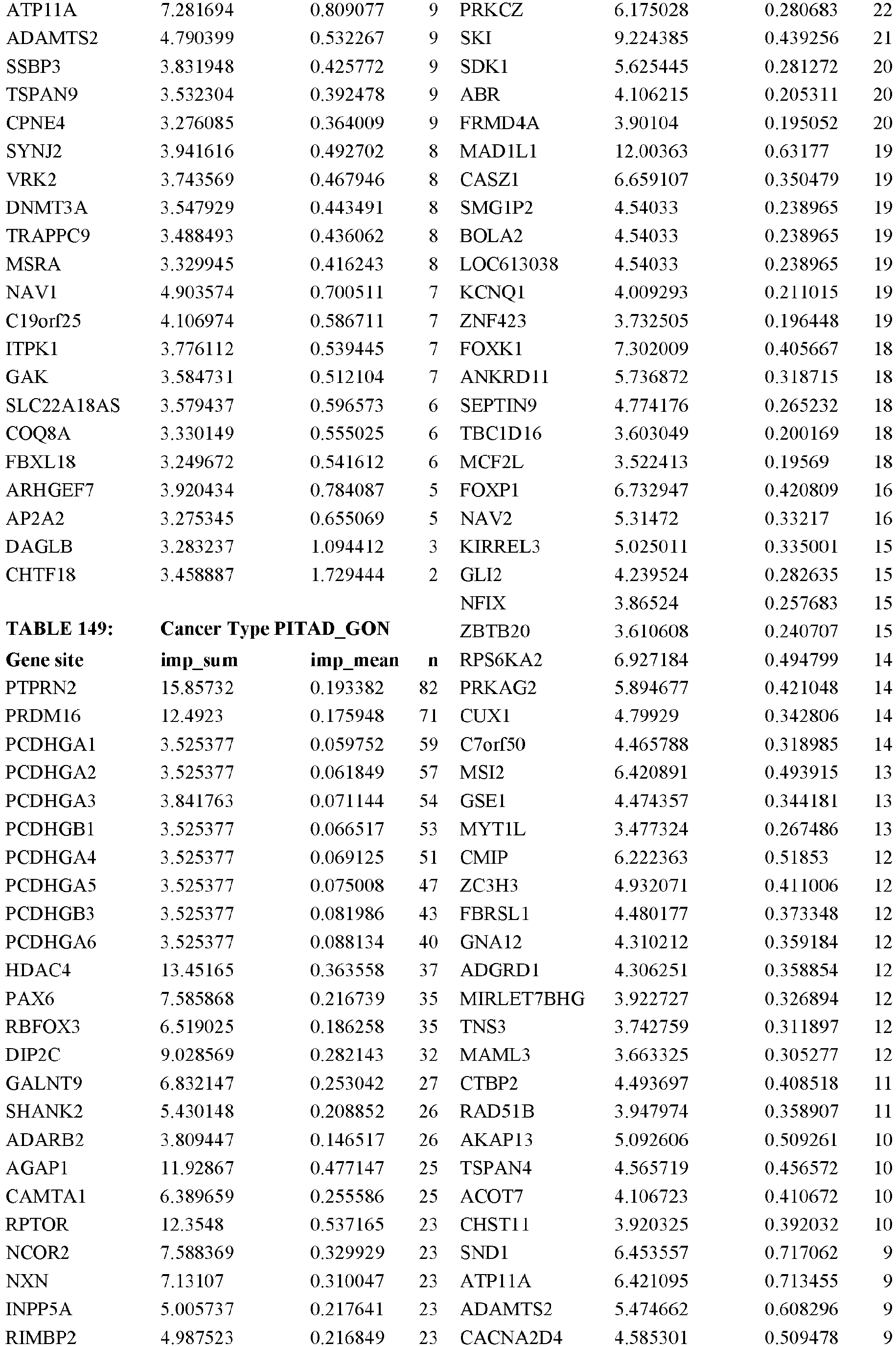

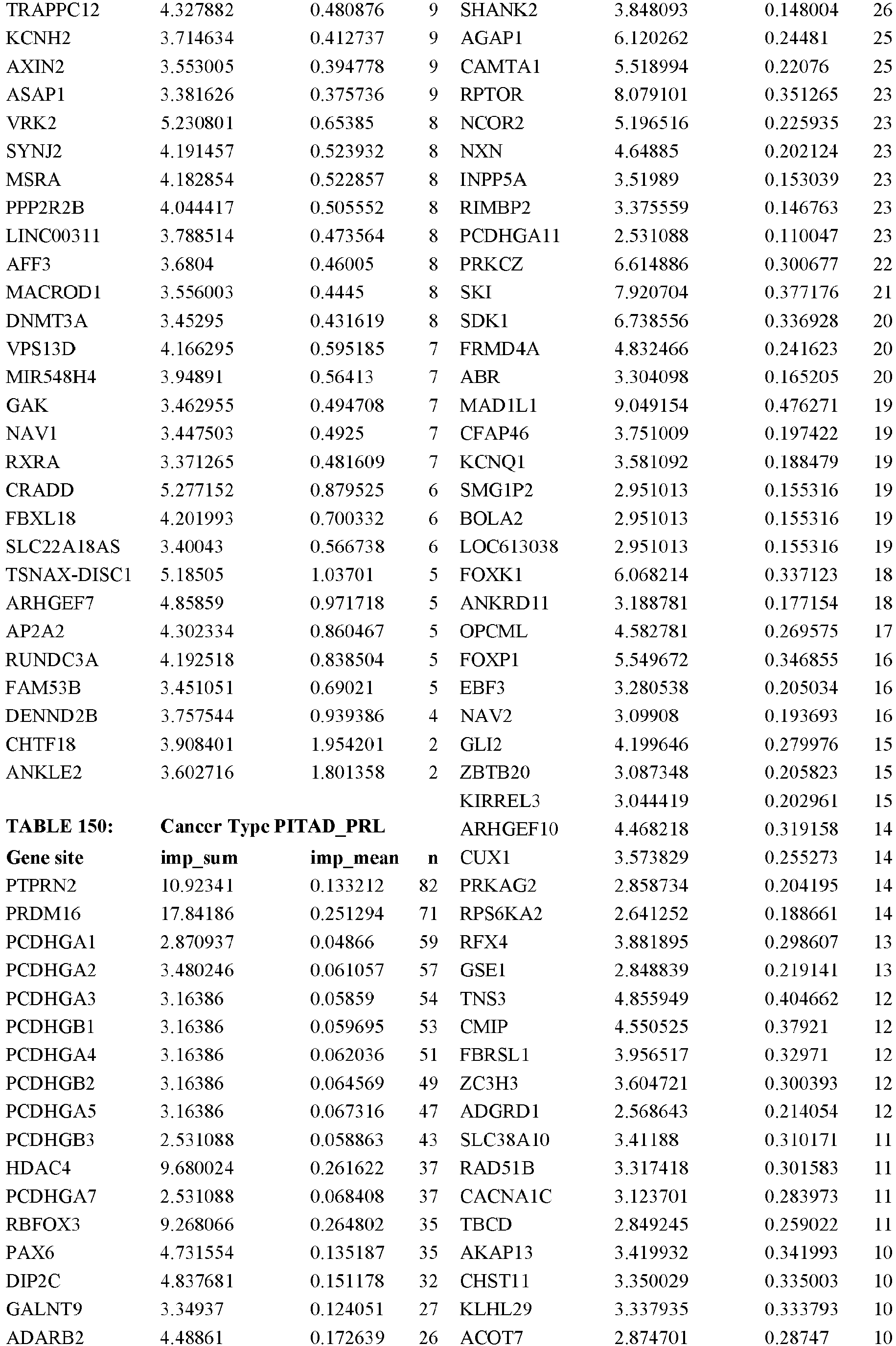

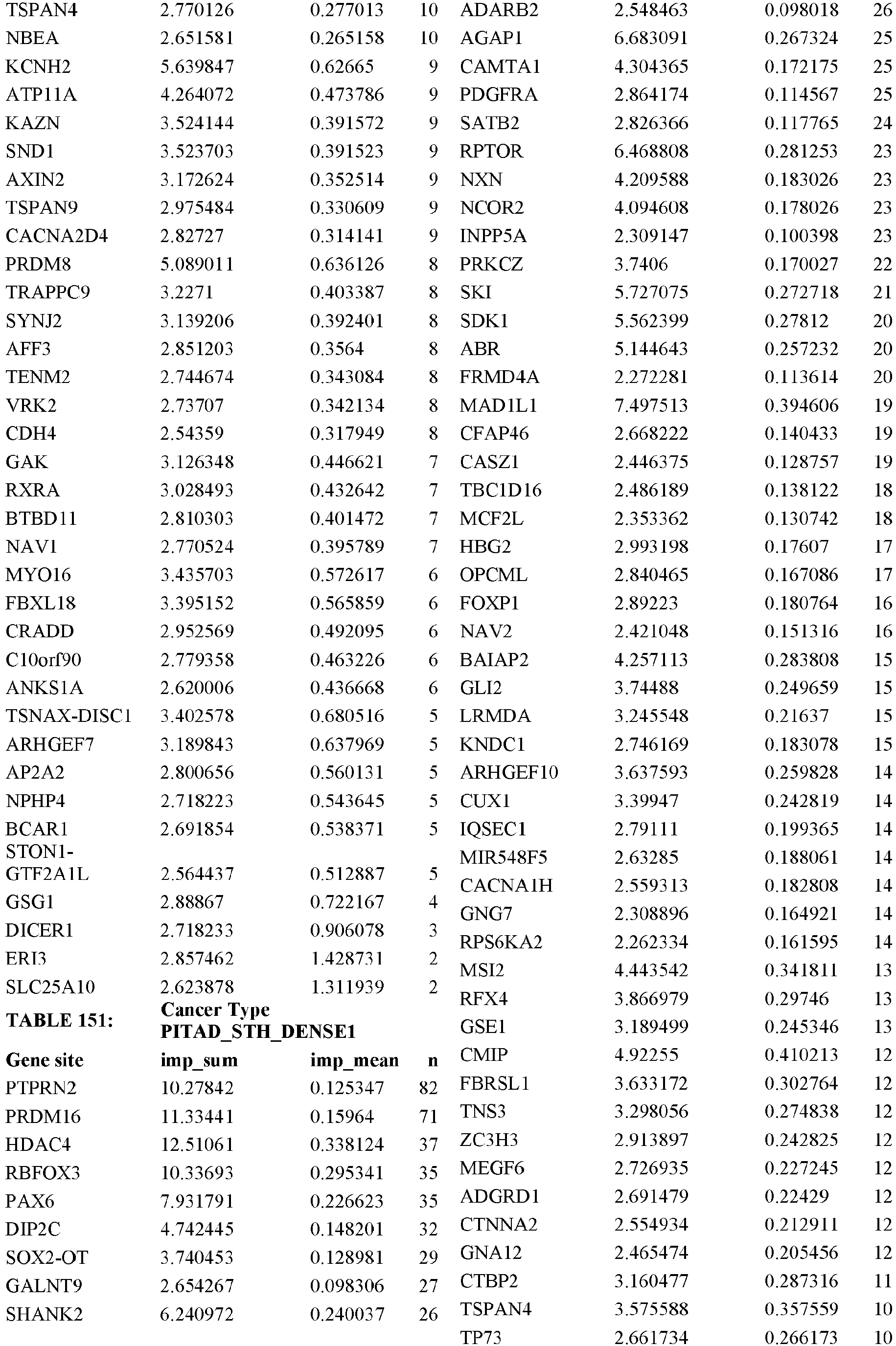

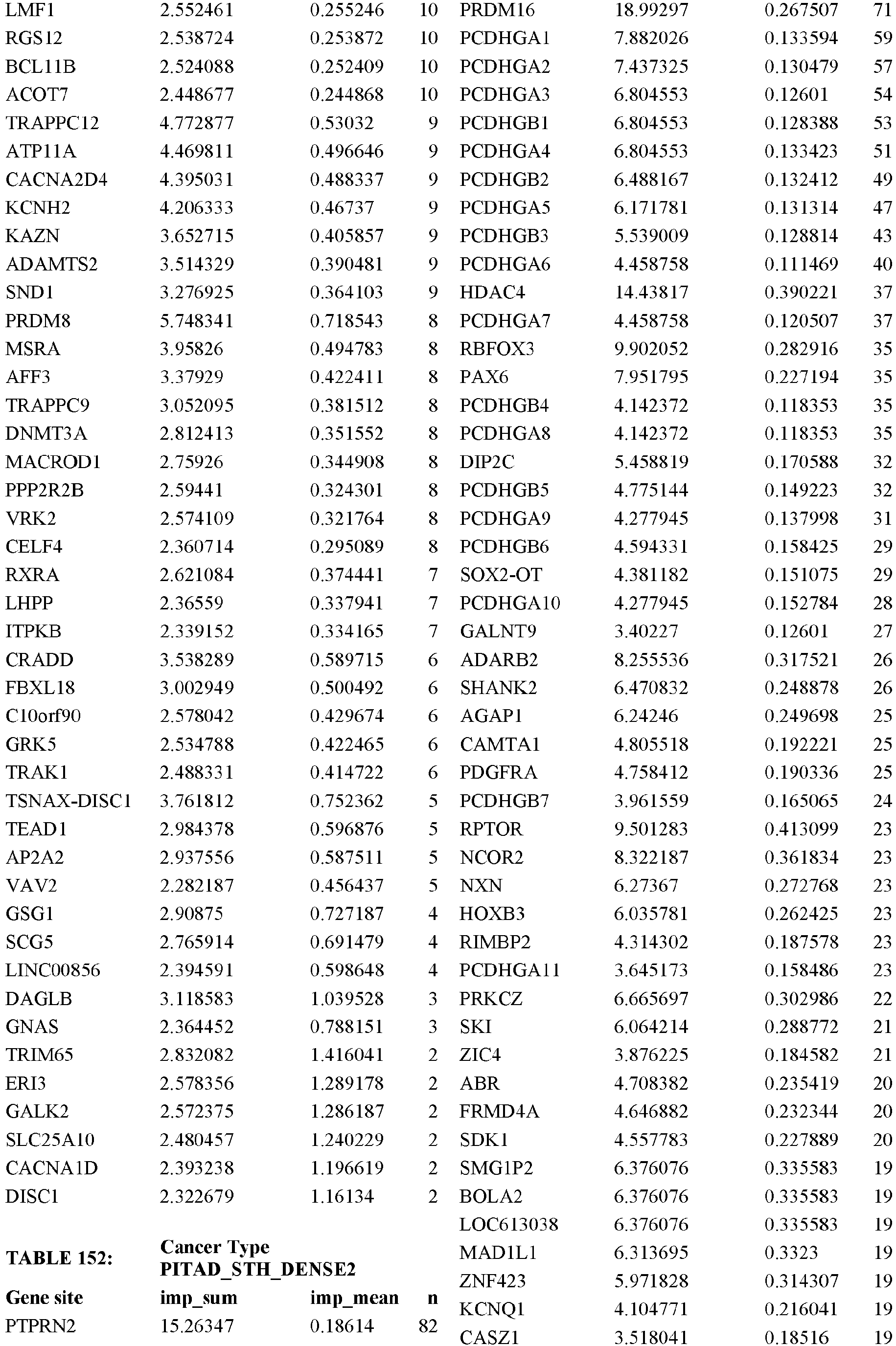

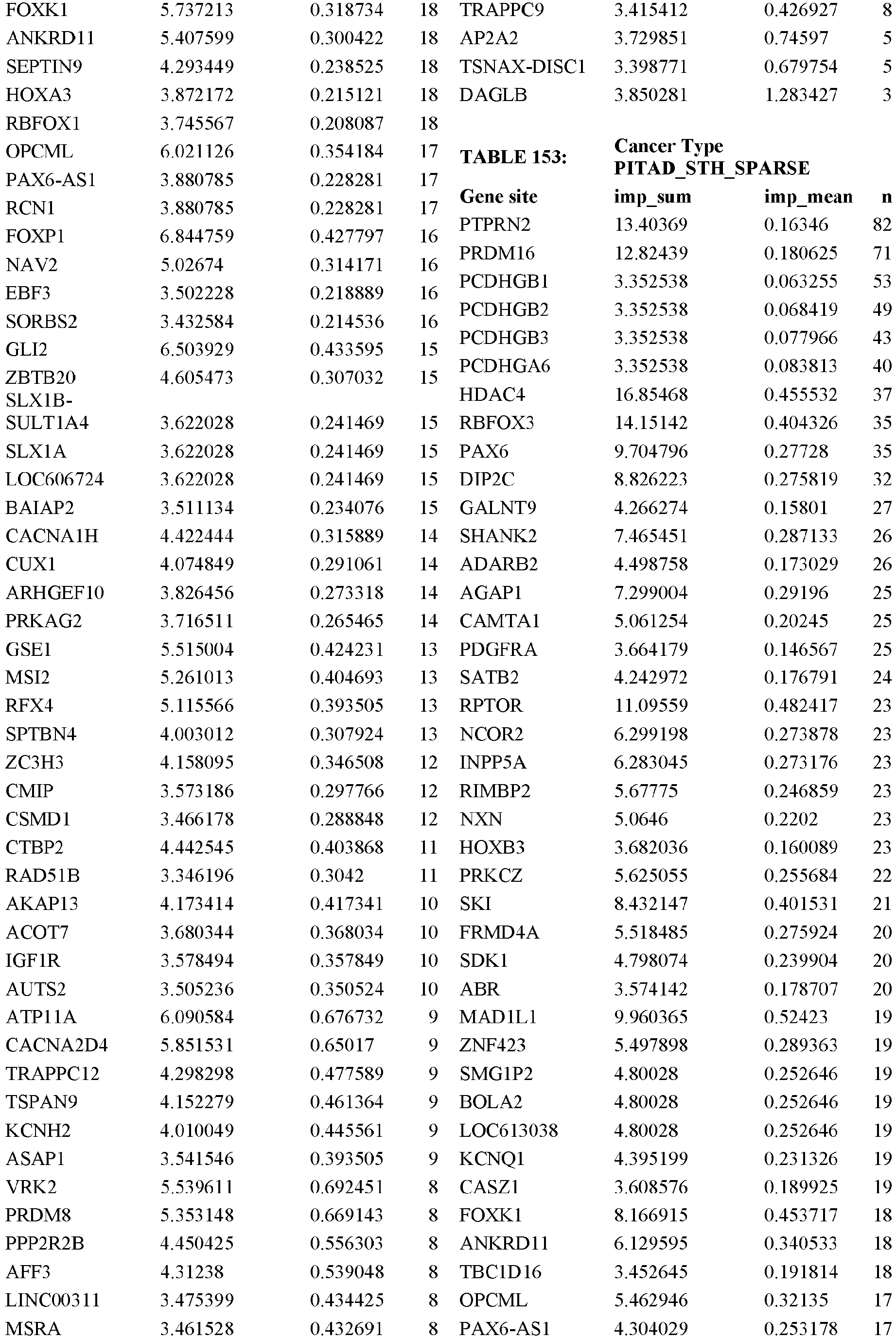

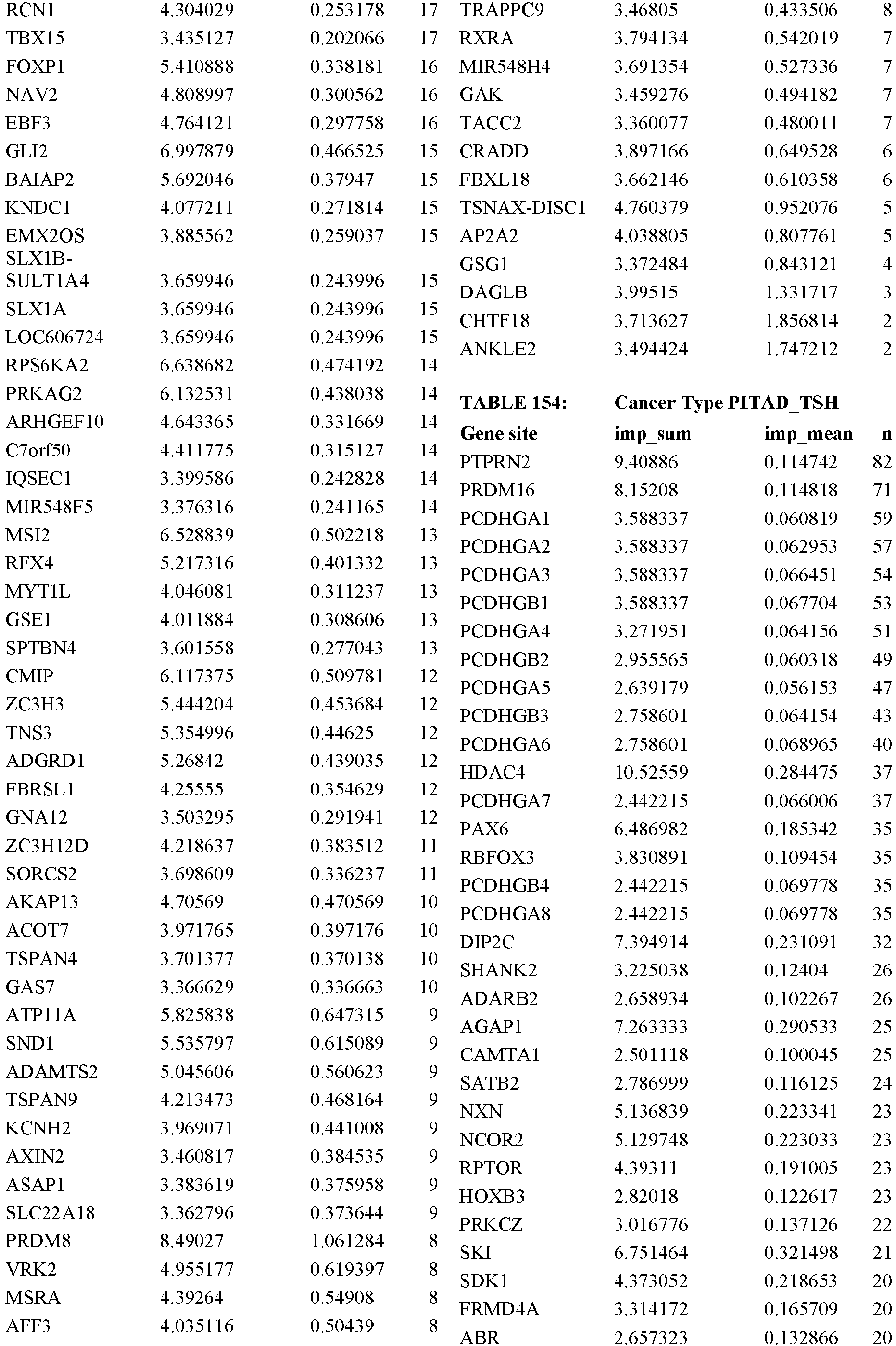

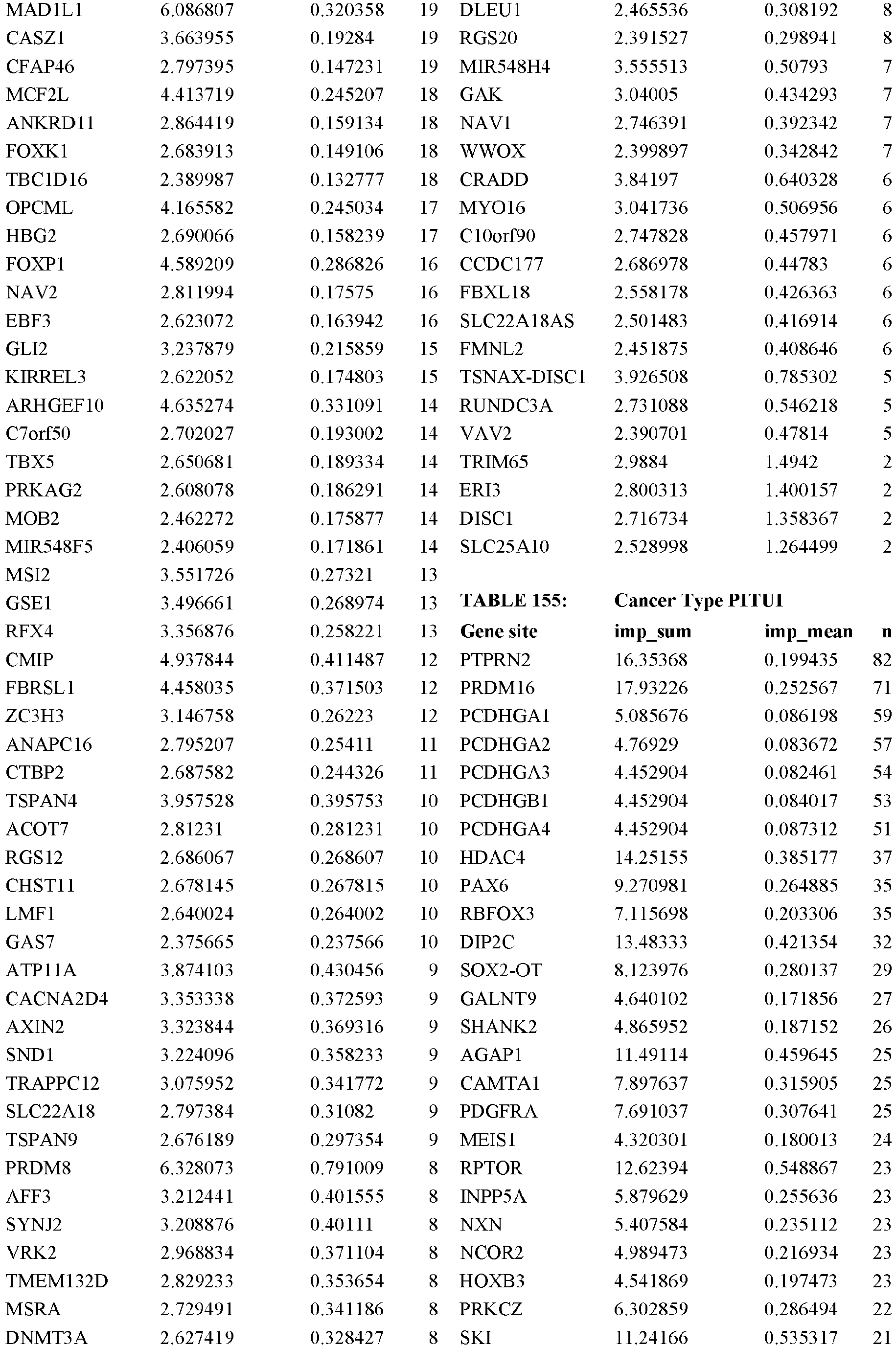

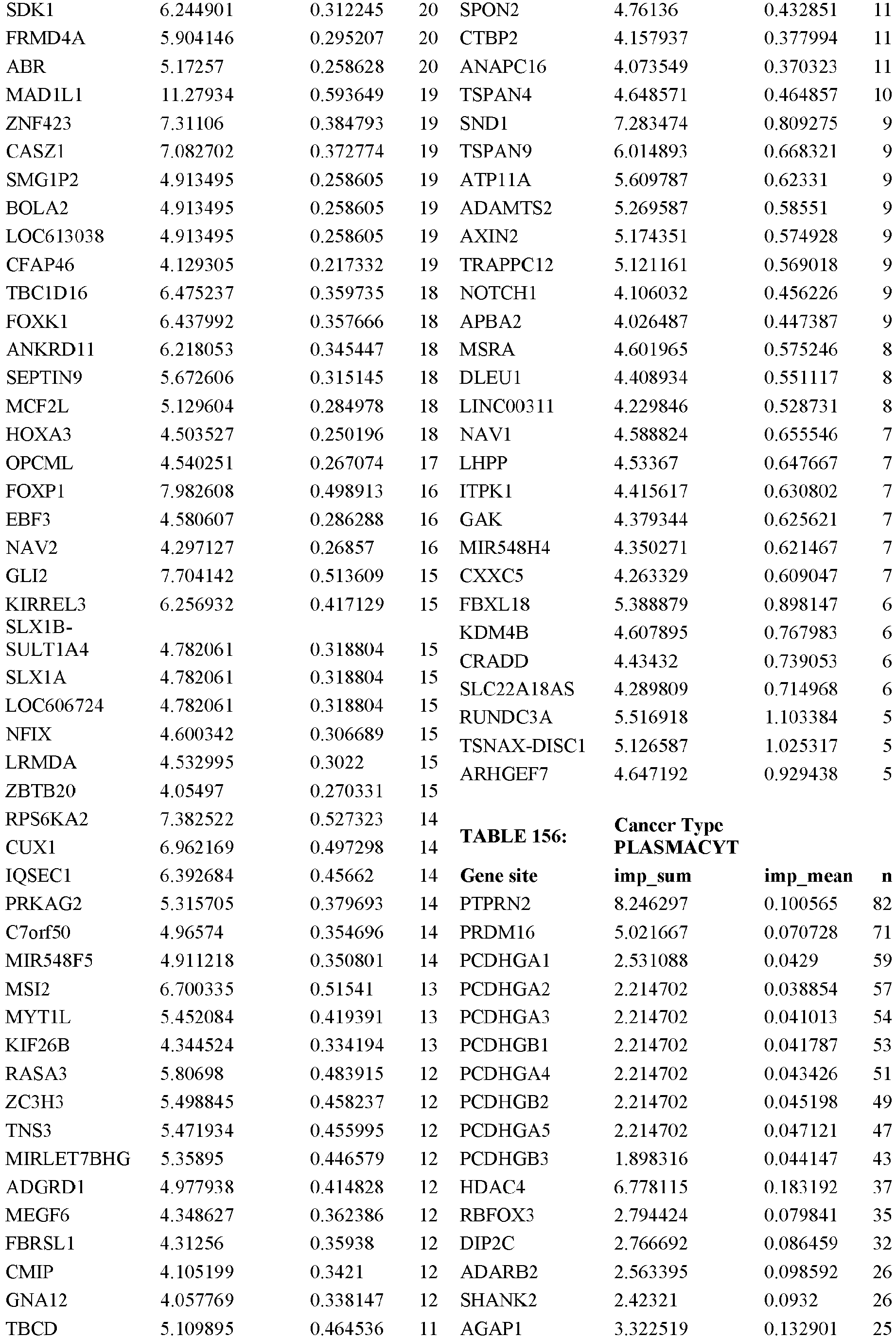

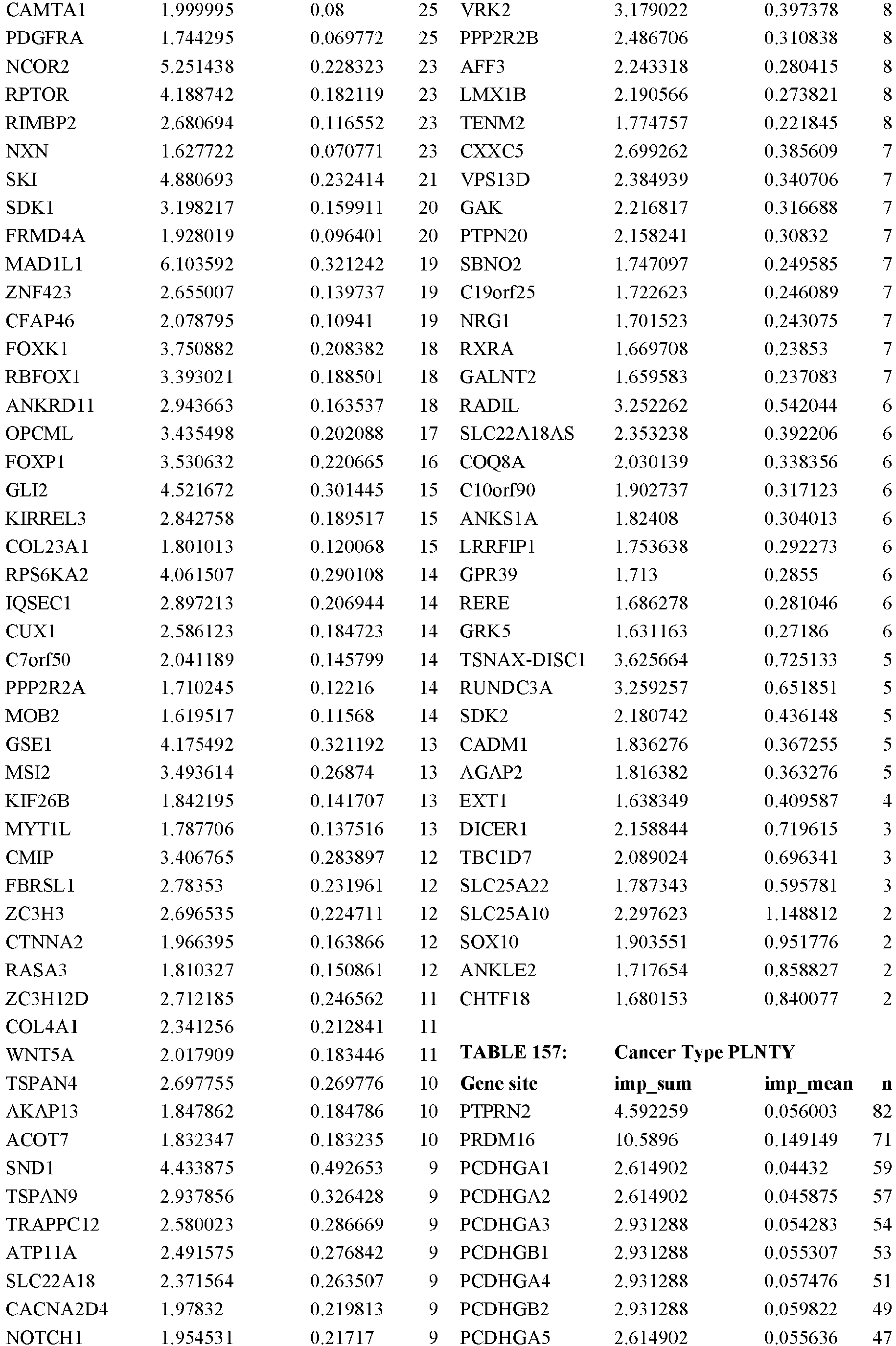

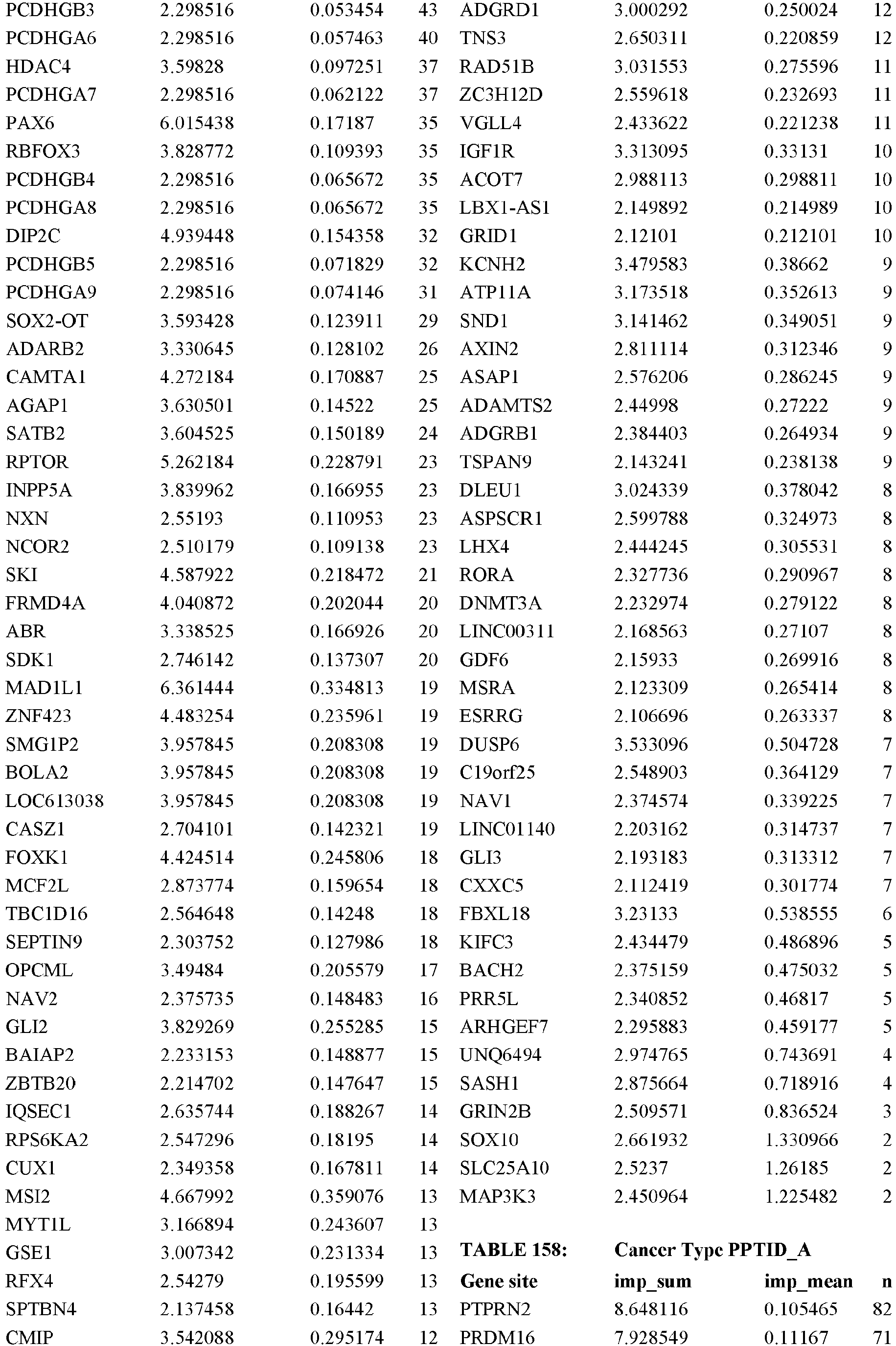

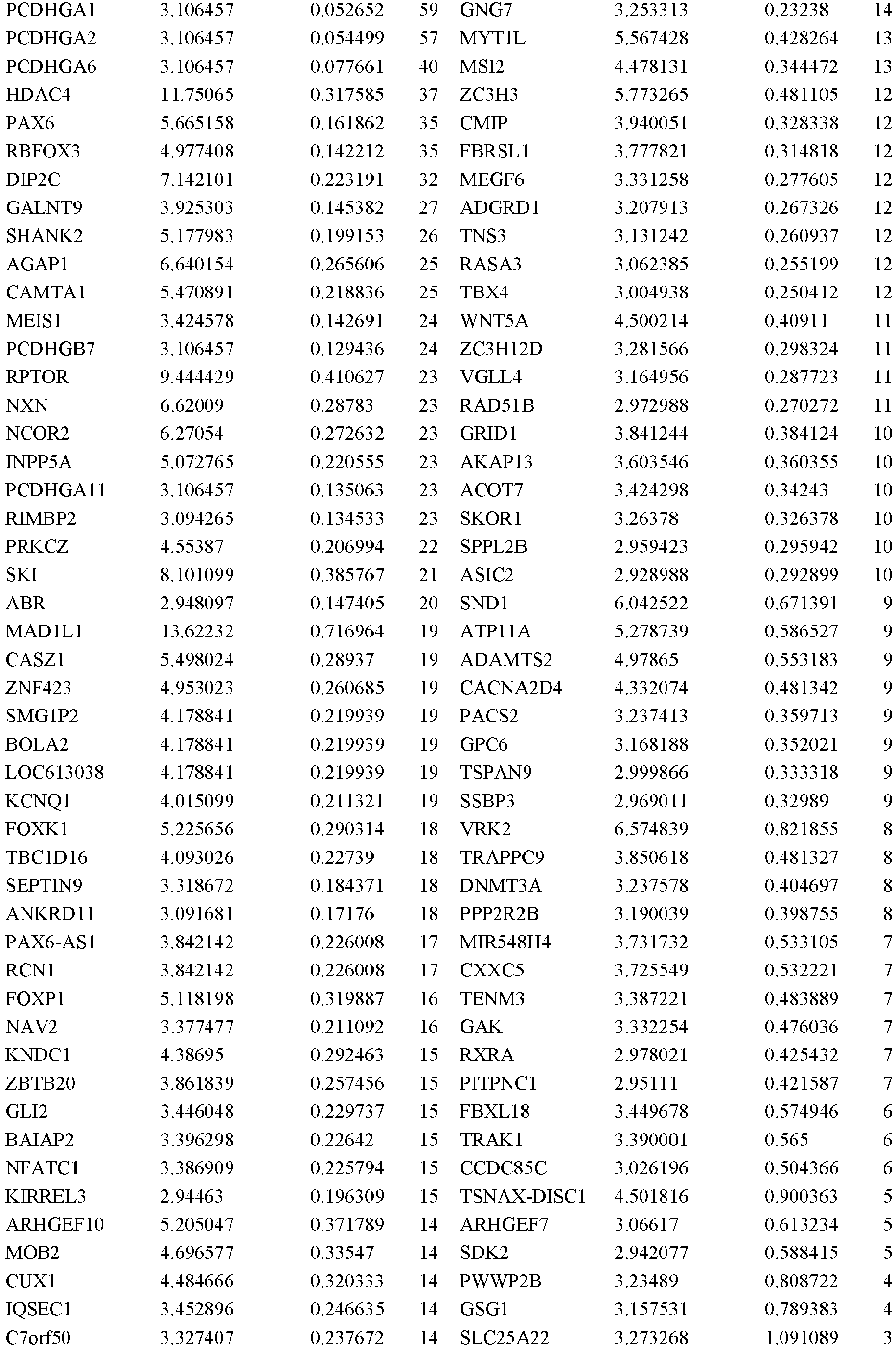

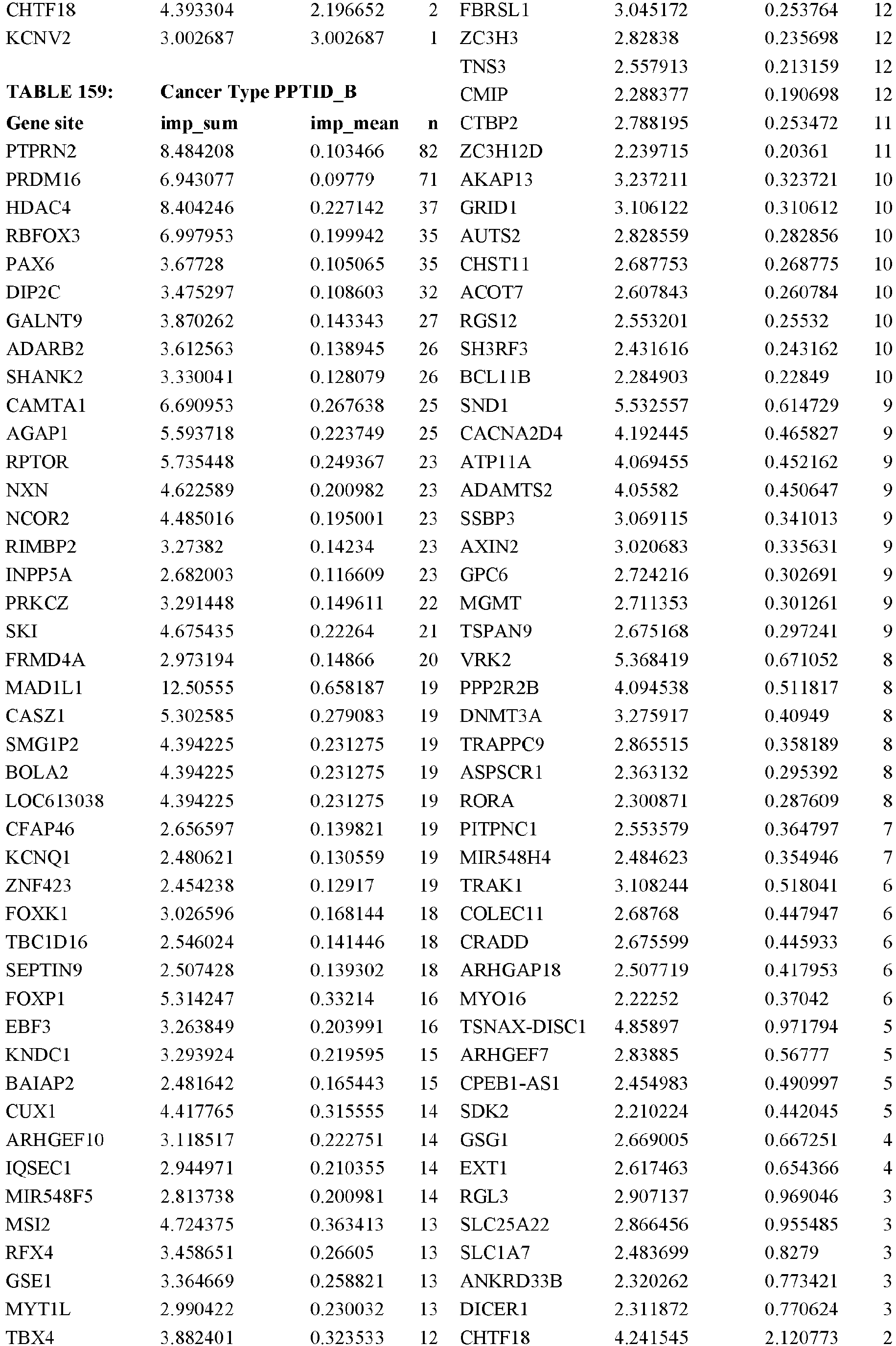

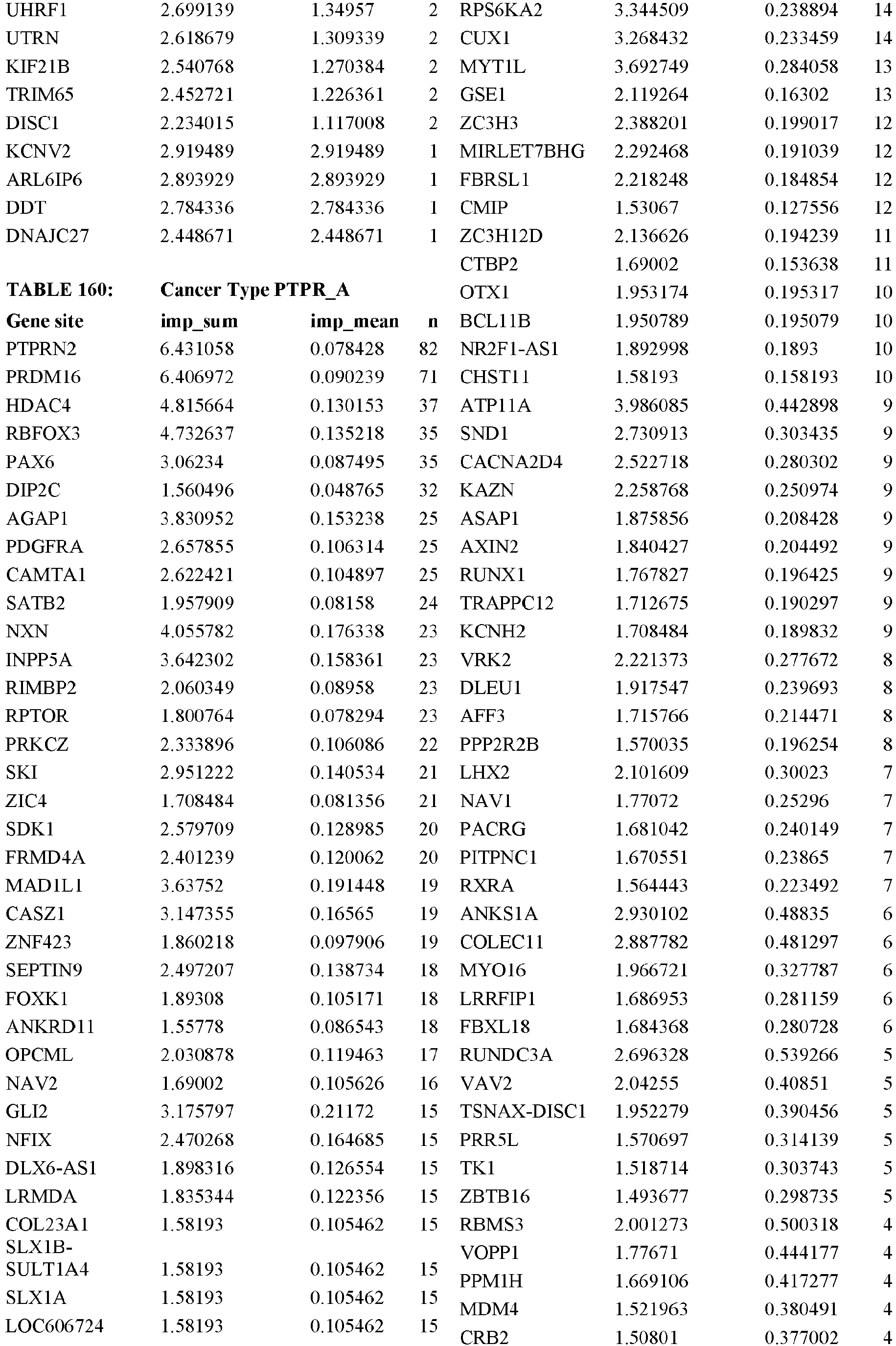

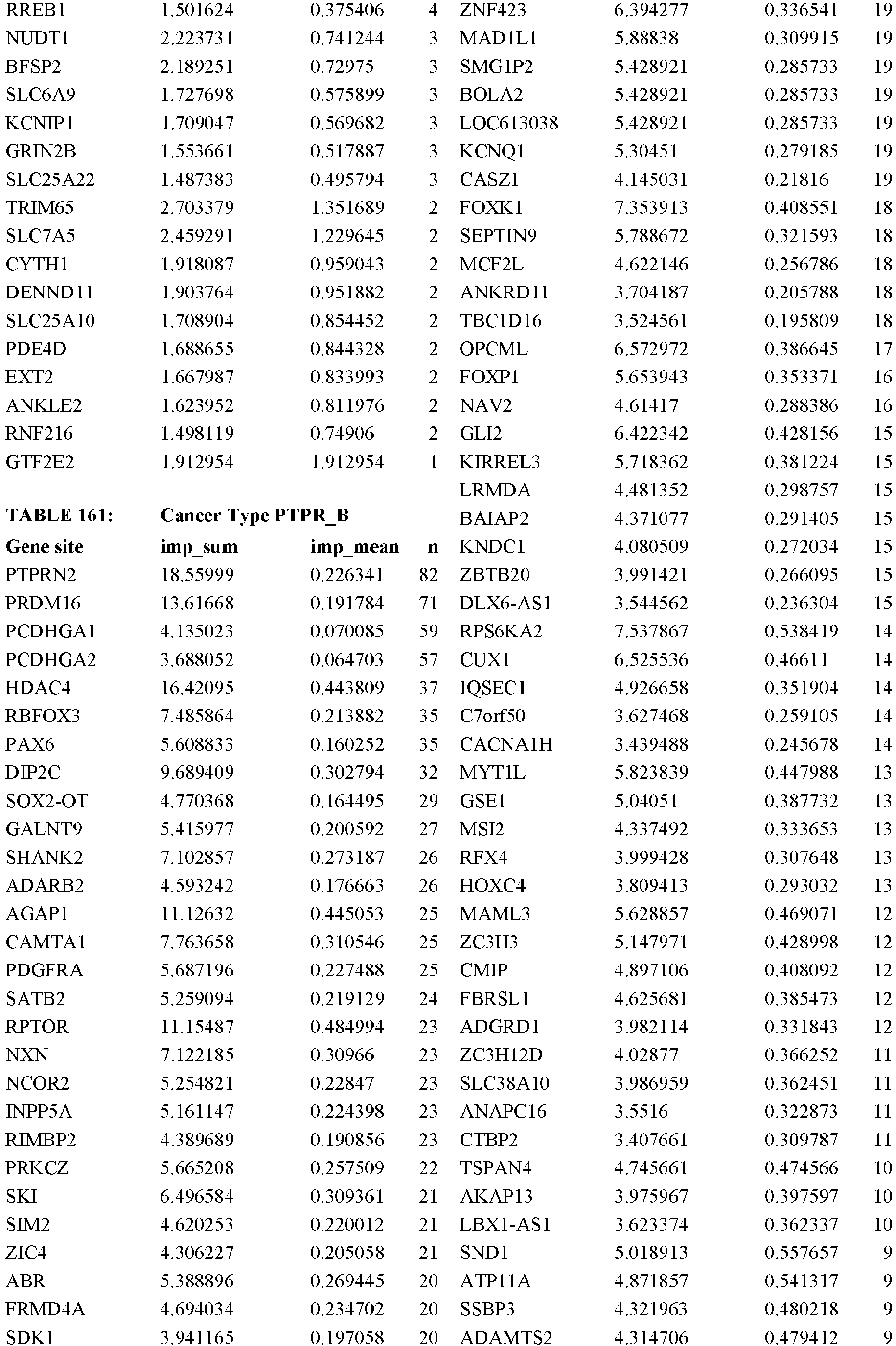

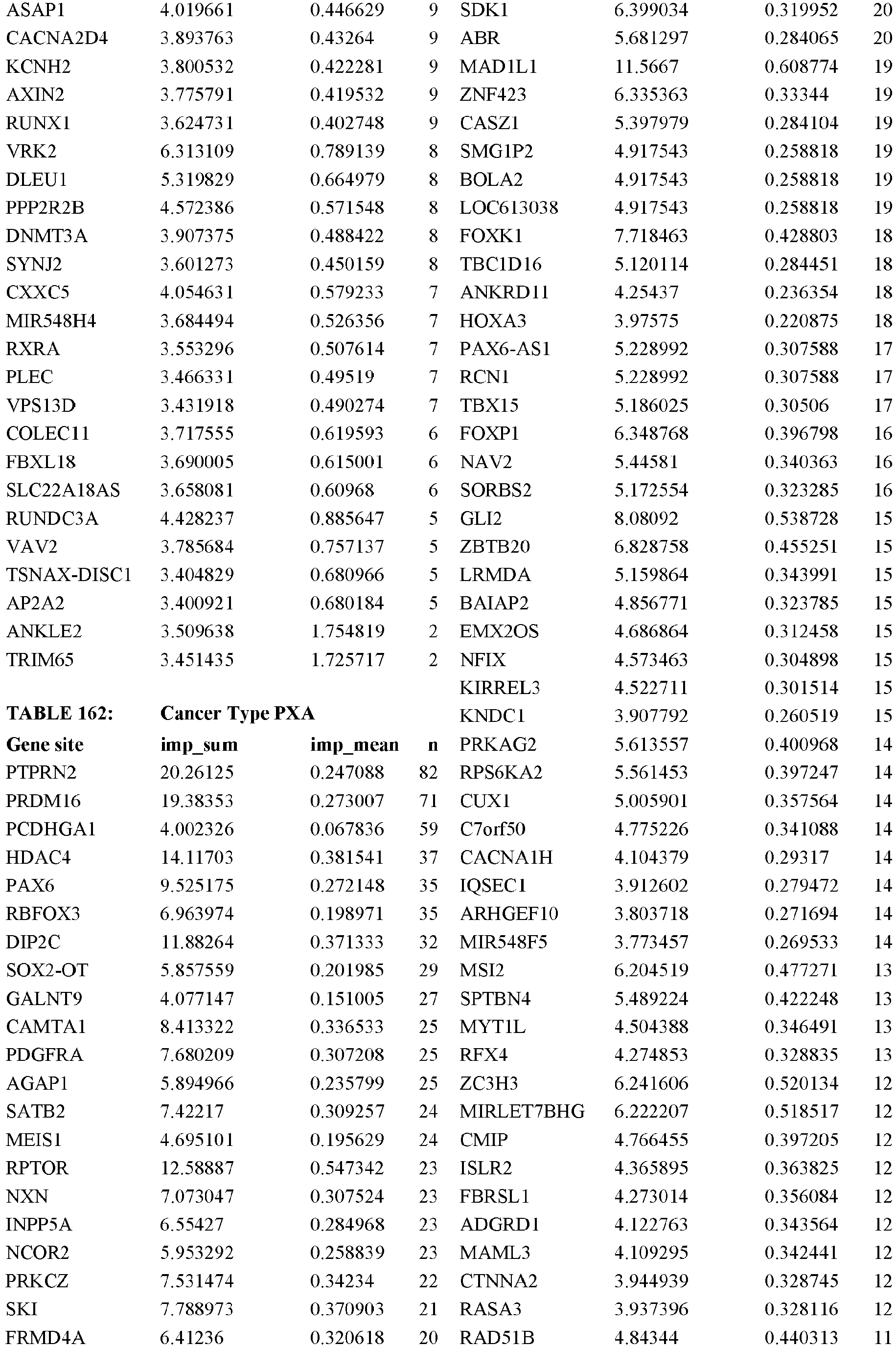

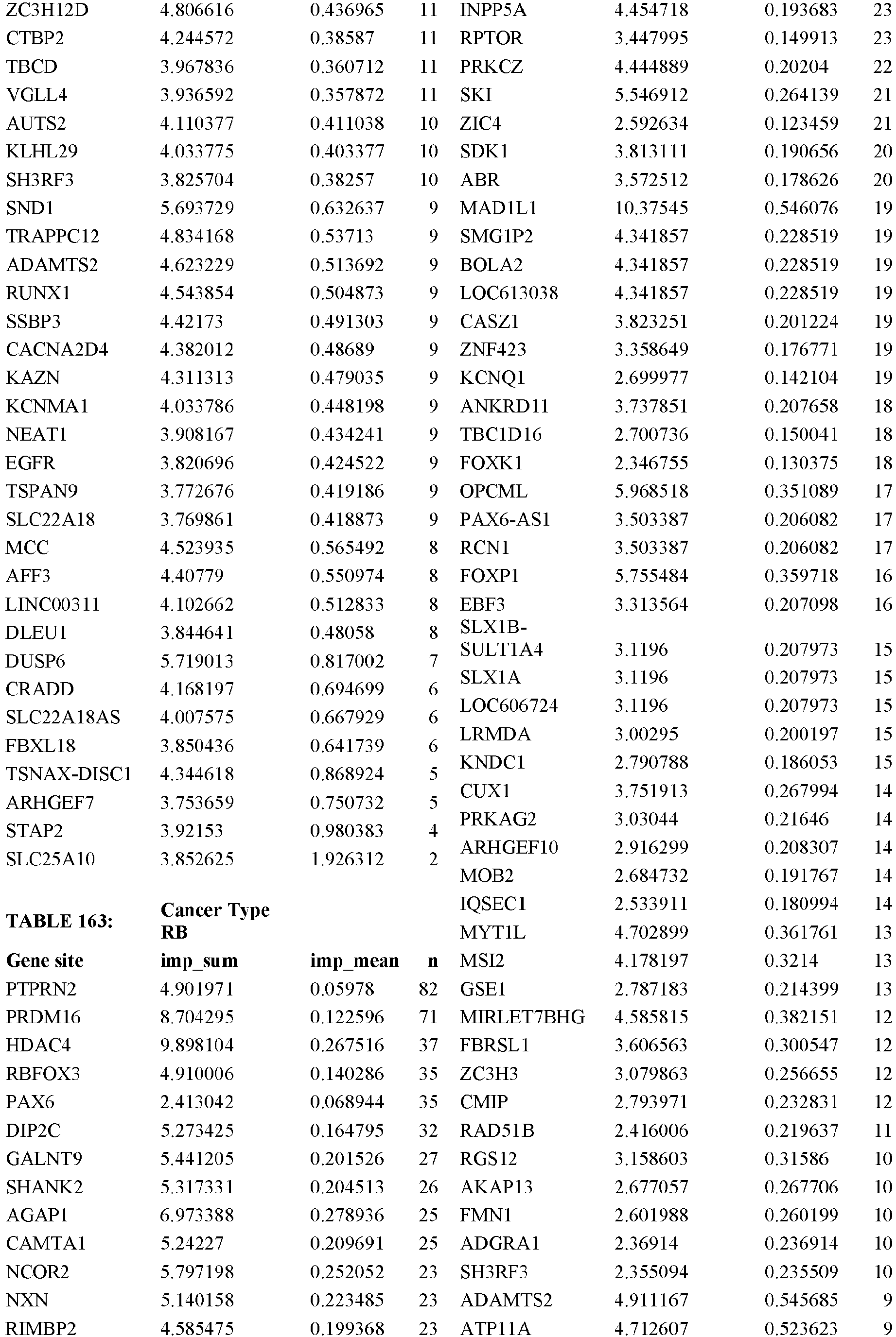

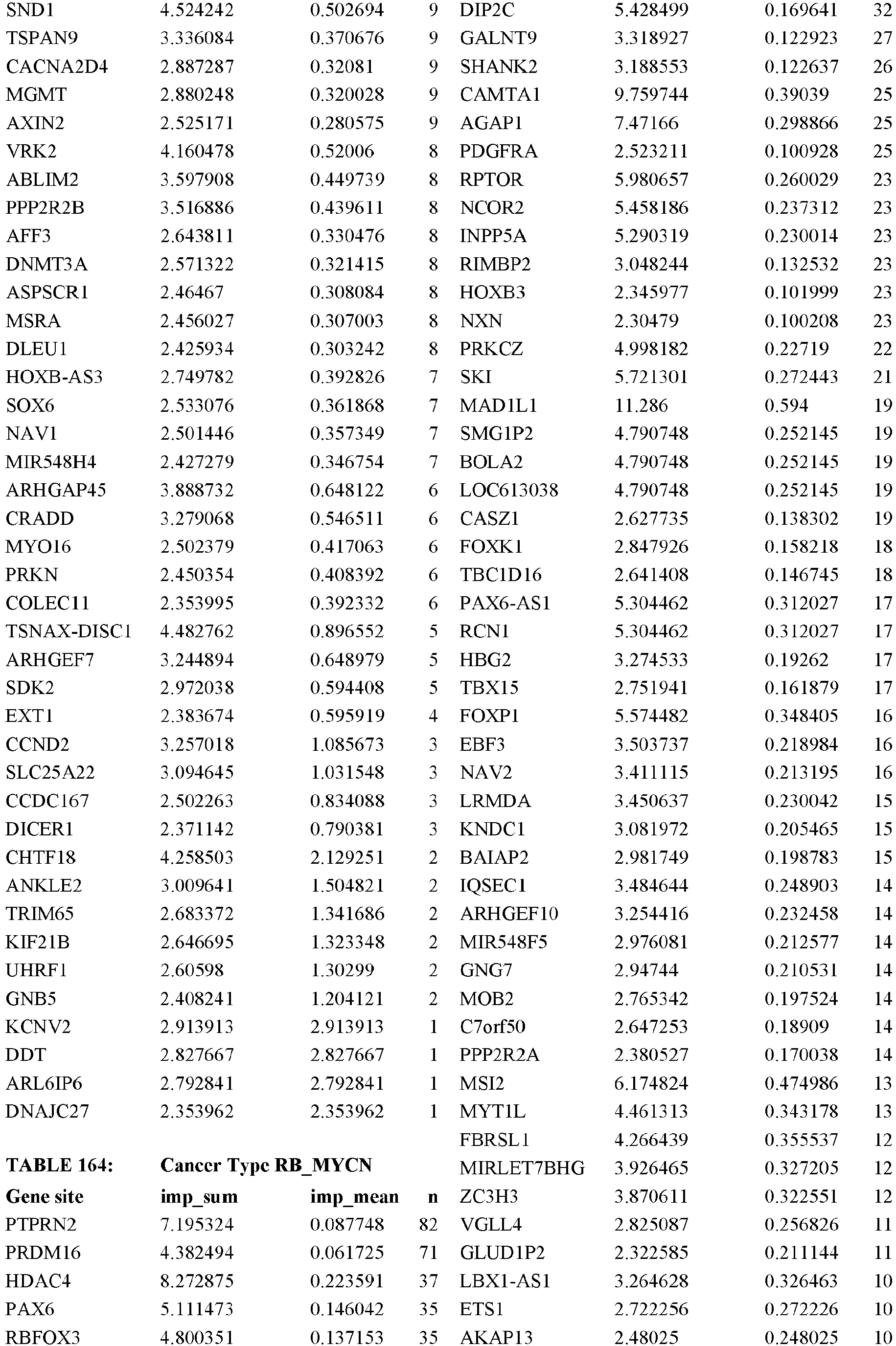

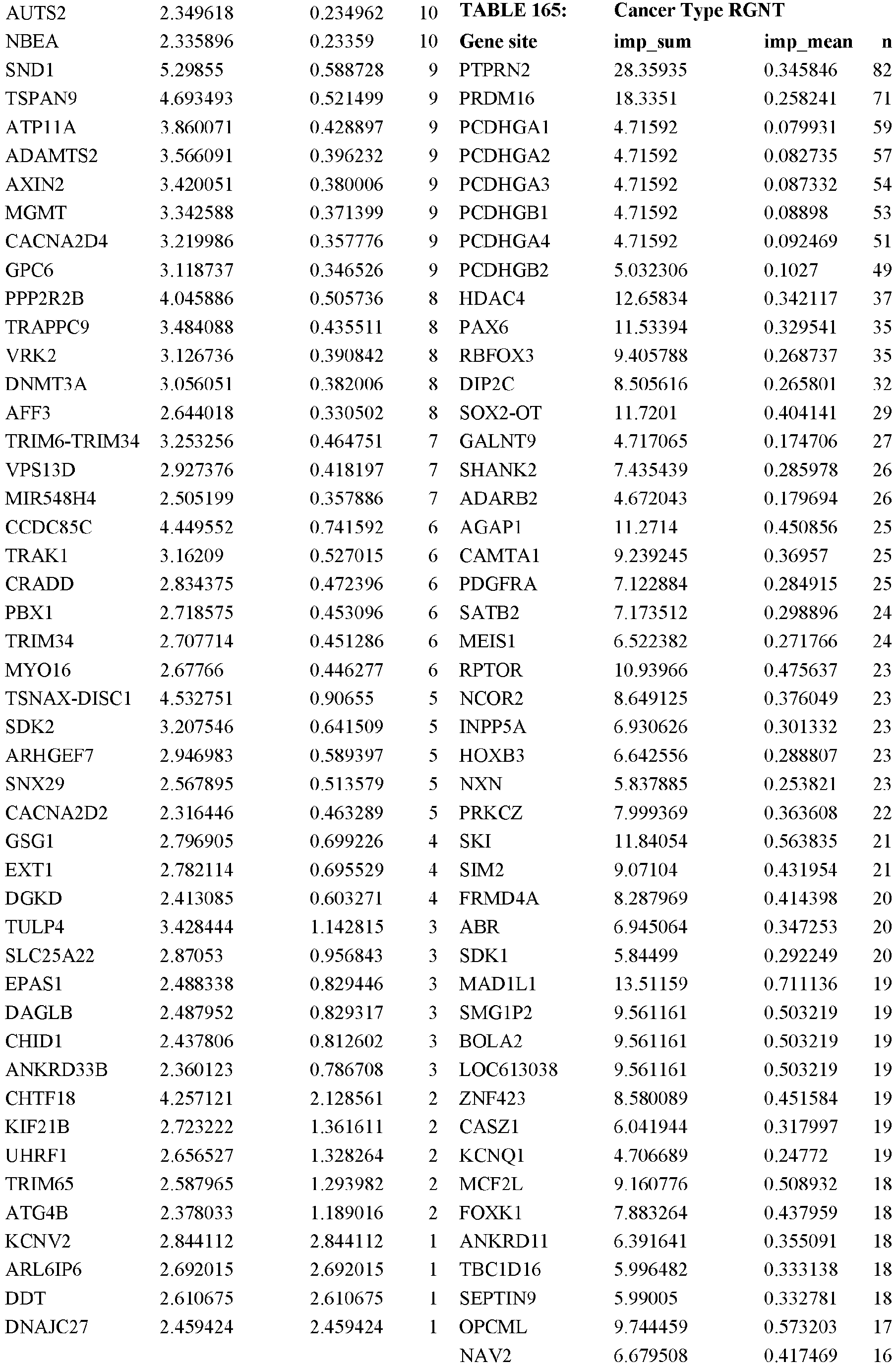

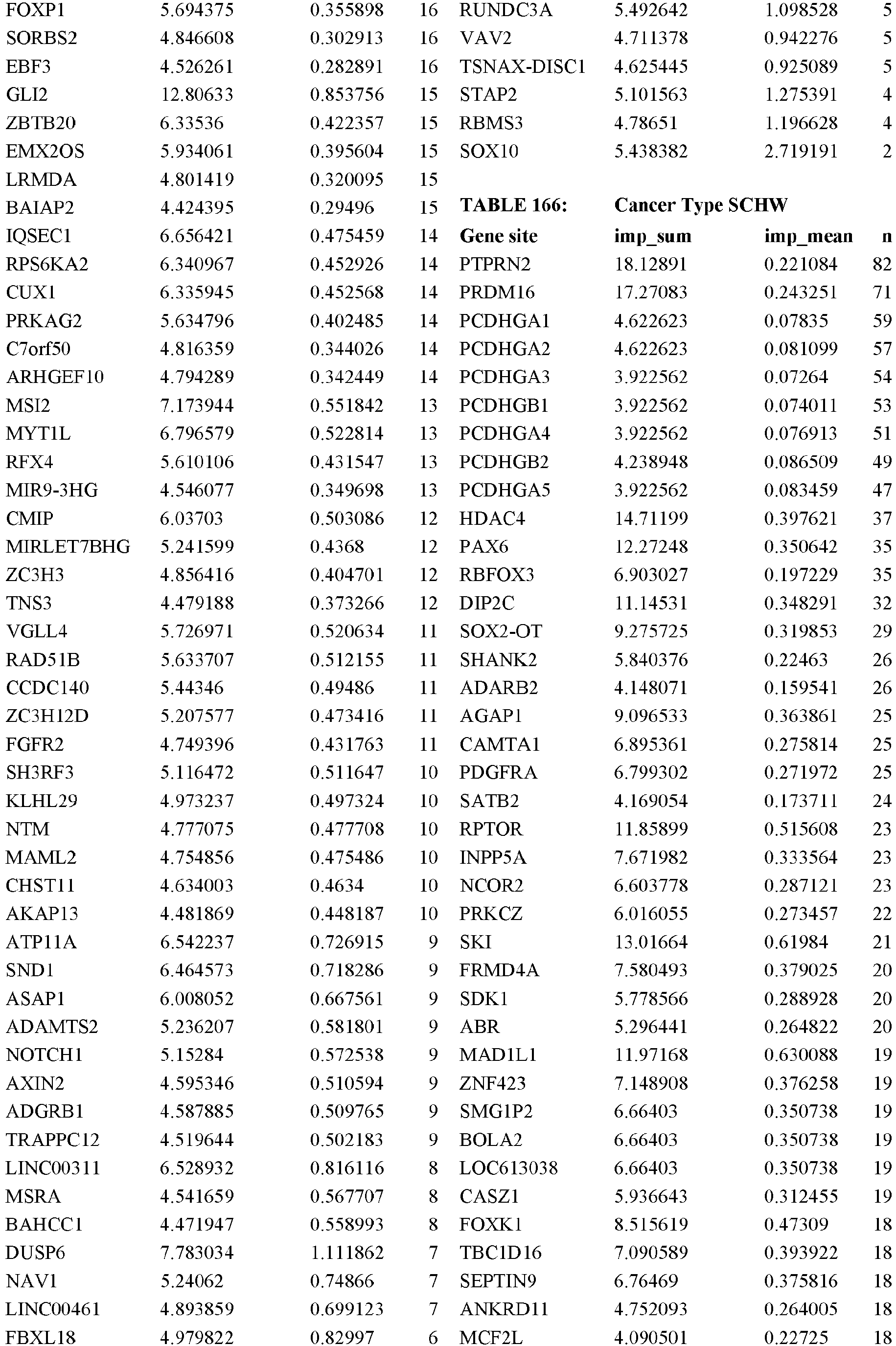

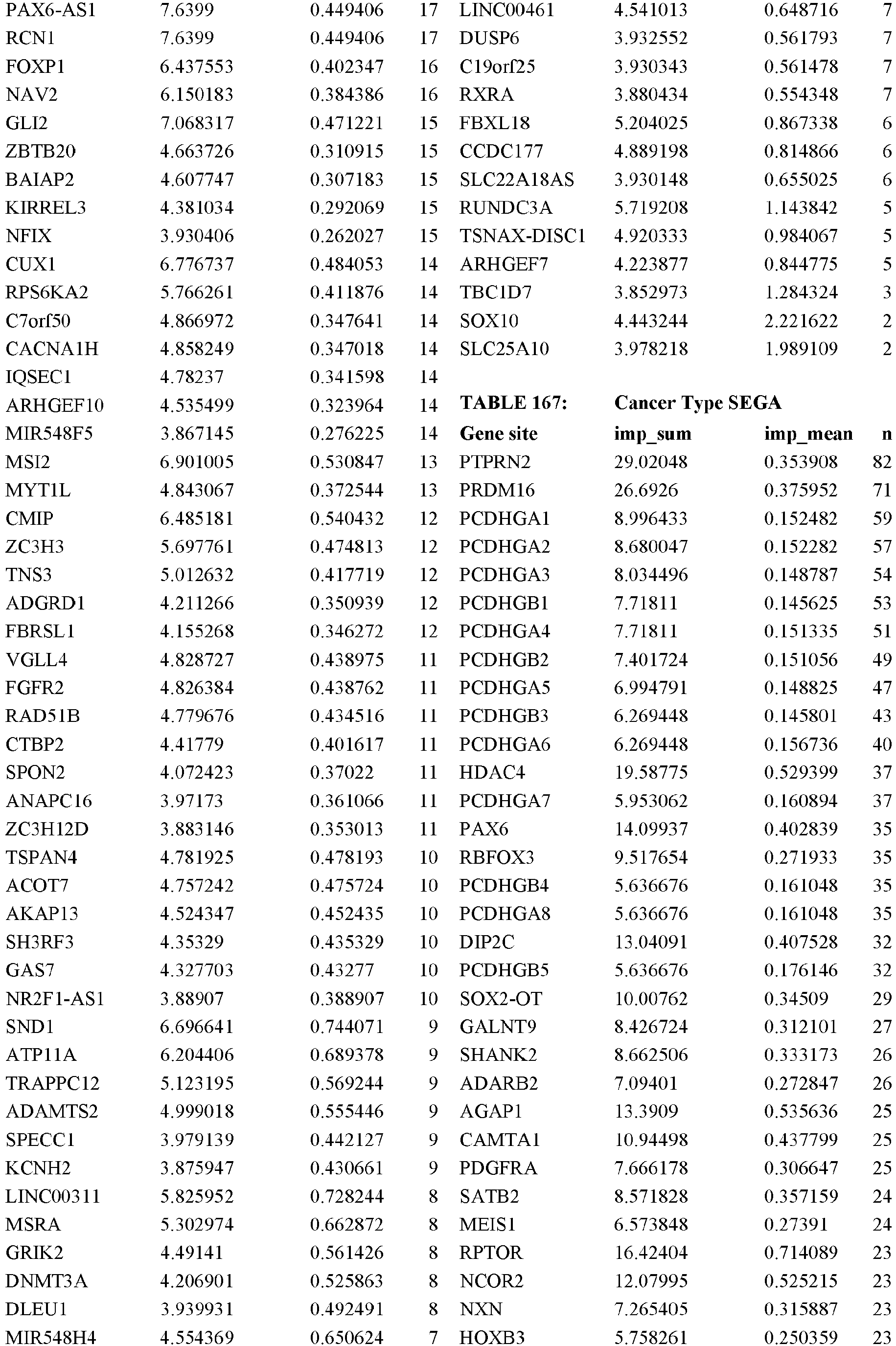

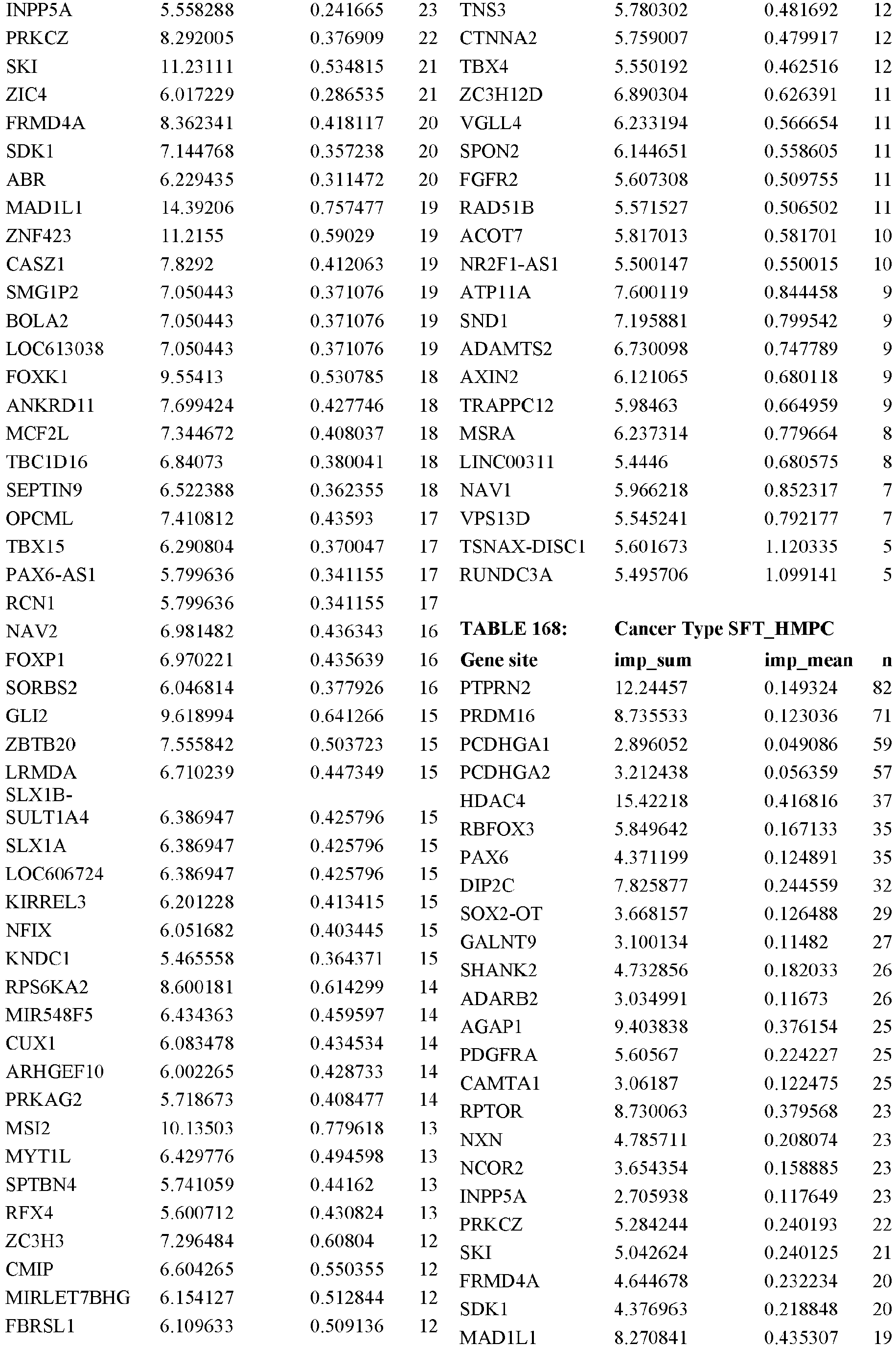

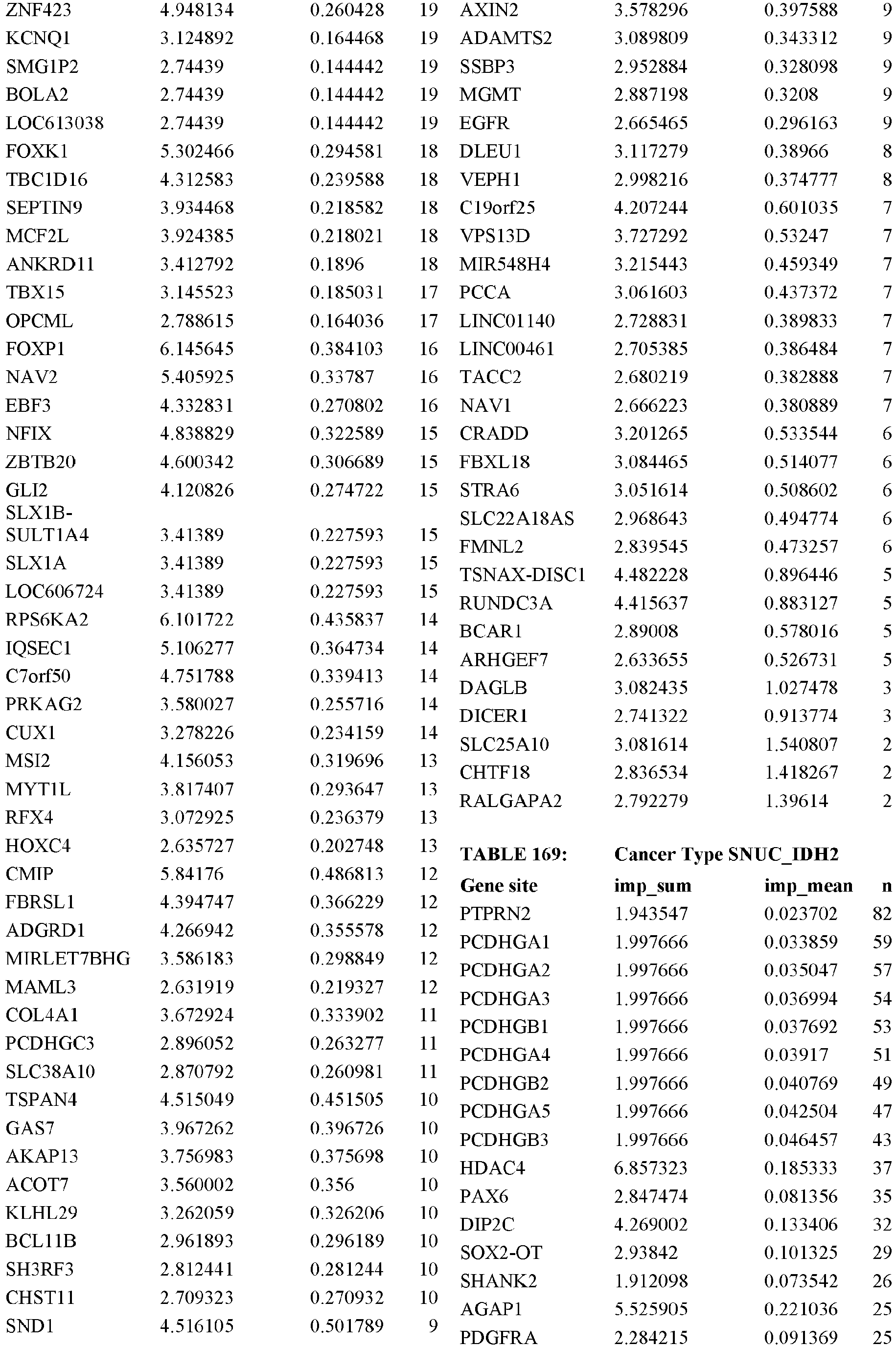

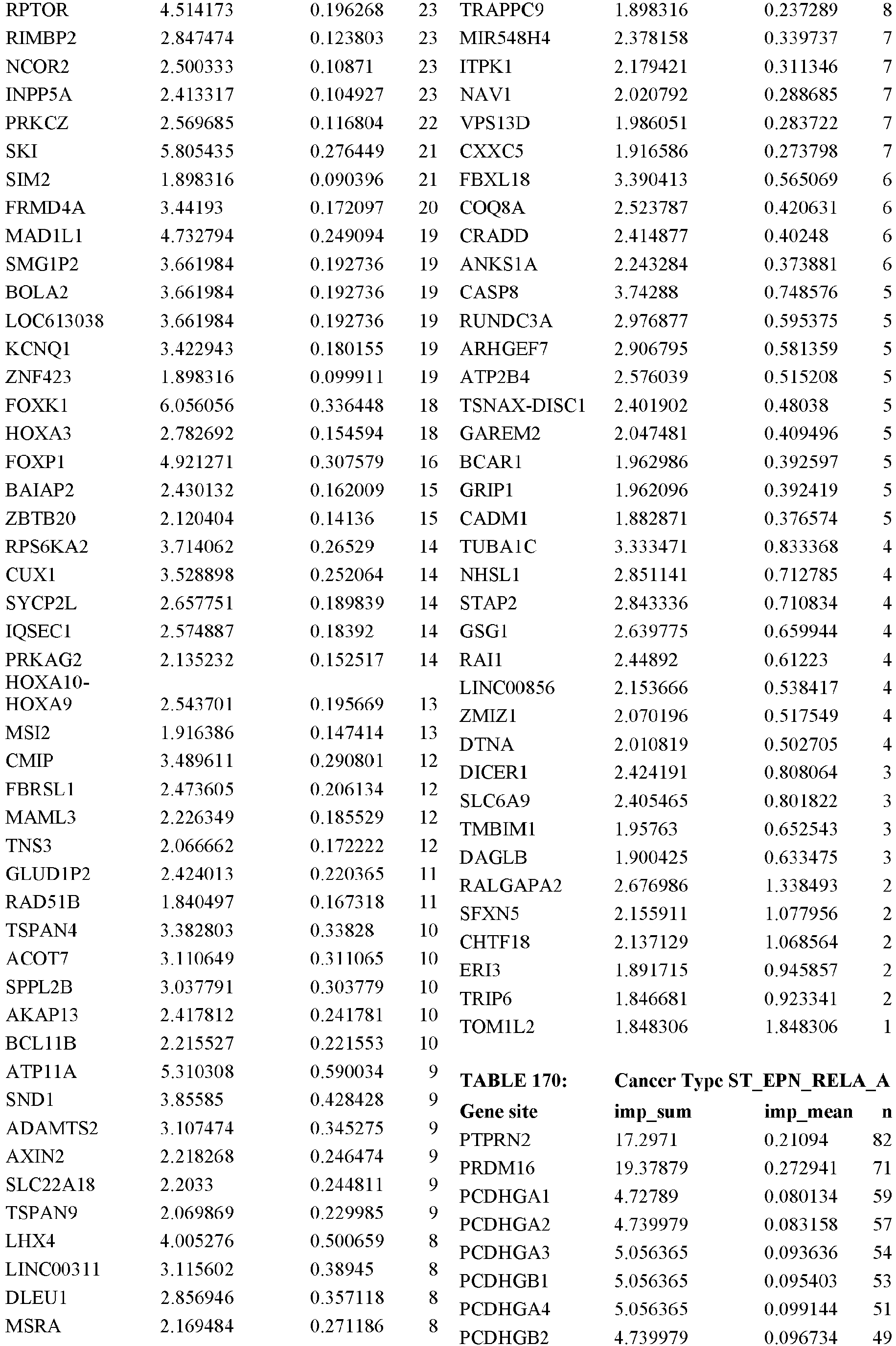

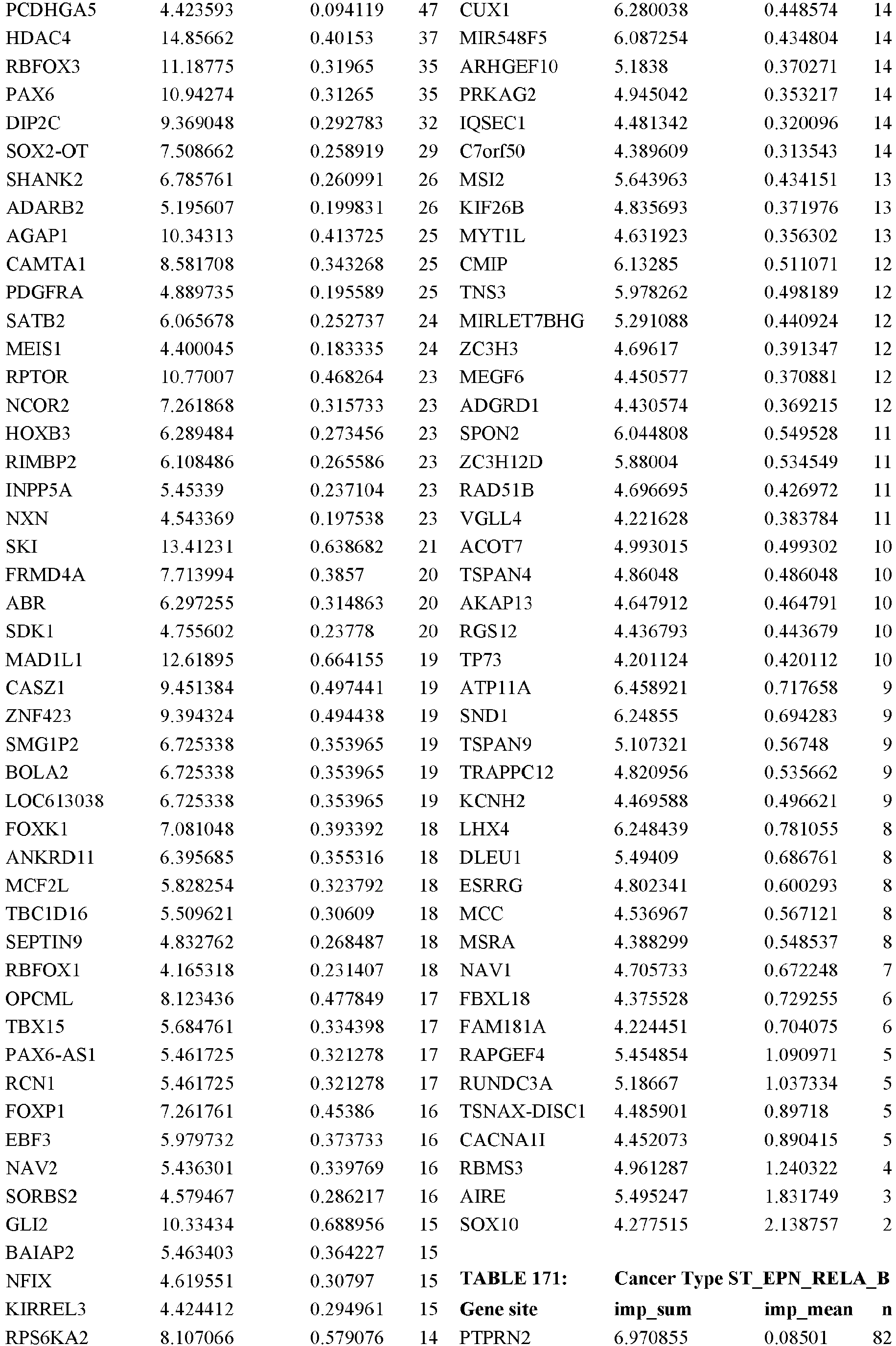

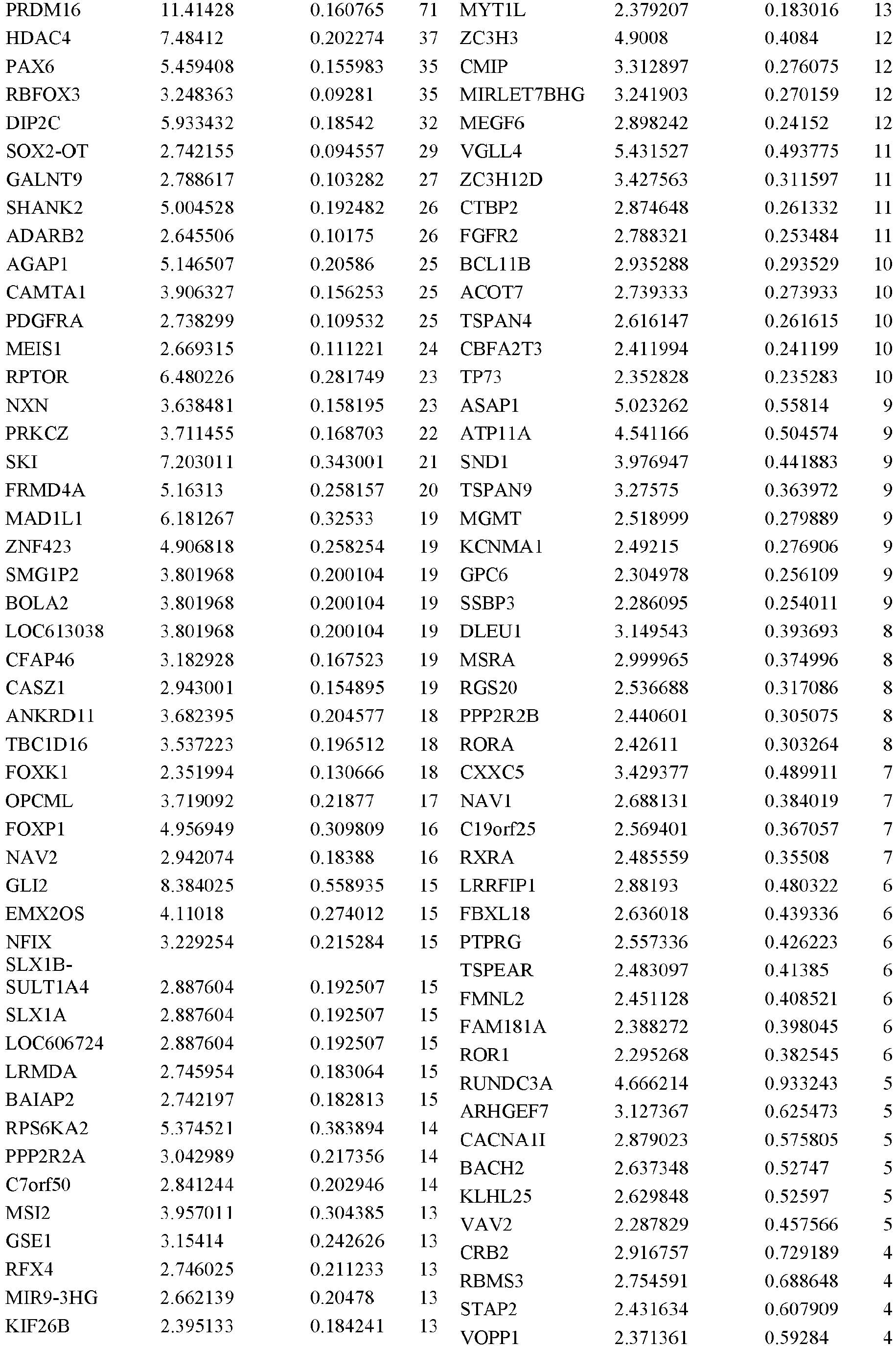

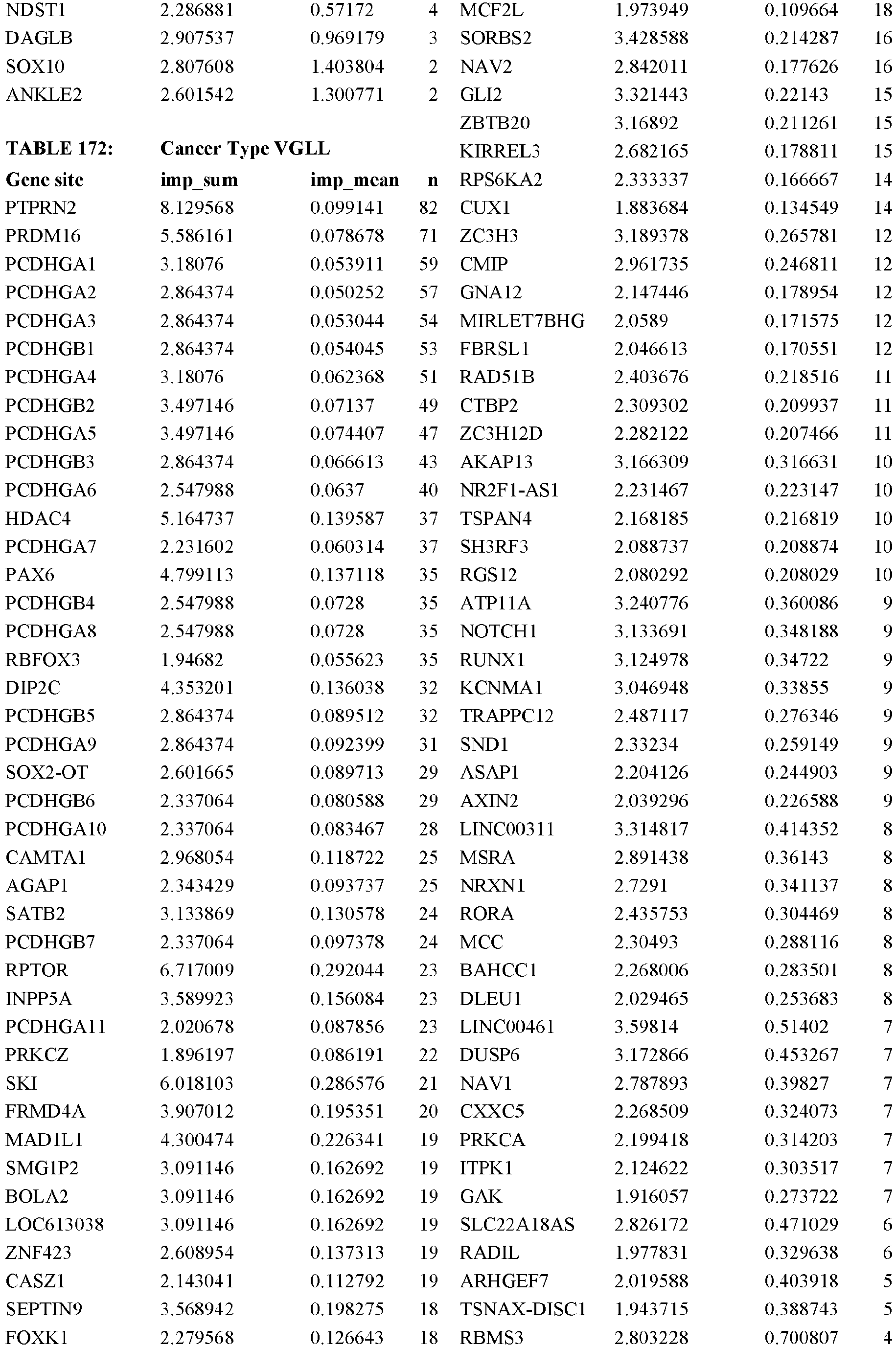

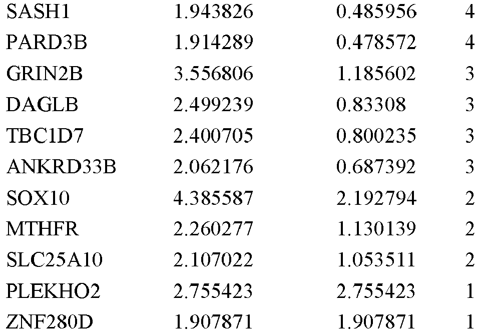

## Claims

1. A computer-implemented method for the diagnostic classification of cancer, the method comprising:
classifying a cancer using a classification algorithm based on biological states of a set of gene sites of a cancer sample, wherein the classification algorithm is trained using data of biological states derived from classified cancer types, wherein the set of gene sites comprises at least 3 gene sites of the cancer sample genome selected from the gene sites in Table 1 (SEQ ID No. 1 to SEQ ID No. 688), and wherein the biological states of the gene sites comprise the biological states of the gene sites as listed in Table 1 (SEQ ID No. 1 to SEQ ID No. 688) and preferably up to 12 kb upstream and/or downstream of each of said gene sites.

2. The computer-implemented method of claim 1, further comprising:
determining a biological state pertaining to each of the at least 3 gene sites of the cancer sample genome; and
determining a biological state pattern of the set of gene sites based on the determined biological states of the at least 3 gene sites.

3. An *in-vitro* method for classification of cancer, comprising
providing a cancer sample;
determining biological states pertaining to at least 3 gene sites of the cancer sample genome, wherein the gene sites are selected from the gene sites in Table 1 (SEQ ID No. 1 to SEQ ID No. 688), and wherein the biological states of the gene sites comprise the biological states of the gene sites as listed in Table 1 (SEQ ID No. 1 to SEQ ID No. 688) and preferably up to 12 kb upstream and/or downstream of each of said gene sites;
determining a biological state pattern based on the determined biological state of each of the at least 3 gene sites; and
classifying a cancer type based on the determined biological state pattern and pre-determined biological state patterns pertaining to different cancer types.

4. The method of claim 3, wherein:
determining a biological state pattern comprises combining the biological states of the gene sites into the biological state pattern; and/or
classifying a cancer type comprises comparing the biological state pattern of the set of gene sites with pre-determined biological state patterns derived from the biological state data.

5. The method of claim 1 to 4, wherein the biological state is selected from a group consisting of epigenetic state, mutation state, copy number and RNA expression, in particular wherein the epigenetic state is a methylation state.

6. The method of any one of claims 1 to 5, wherein the set of gene sites comprises at least 10, preferably at least 20 or at least 30 or at least 40 or at least 50 or at least 60 or at least 70 or at least 80 or at least 90 or at least 100 gene sites or all gene sites in Table 1 (SEQ ID No. 1 to SEQ ID No. 688).

7. The method of any one of claims 1 to 6, wherein the gene sites are the gene sites with the highest values of variable importance in Tables 3 to 172, respectively.

8. The method of any one of claims 1 to 7, wherein the biological states of the gene sites comprise the biological states of the gene sites as listed in Table 1 (SEQ ID No. 1 to SEQ ID No. 688) and up to 10 kb, preferably up to 8 kb or up to 6 kb or up to 4 kb or up to 2 kb, upstream and/or downstream of the genes.

9. The method of any one of claims 1 to 7, wherein the biological states of the gene sites comprise exclusively the biological states of the gene sites as listed in Table 1 (SEQ ID No. 1 to SEQ ID No. 688) without any bases upstream and/or downstream of the gene sites.

10. The method of any one of claims 2 to 9, wherein the biological state is a methylation state and the biological state pattern is a methylation state pattern.

11. The method of any one of claims 1 to 10, wherein the cancer is a cancer of the central nervous system or a sarcoma.

12. The method of any one of claims 1 to 11, wherein the cancer is a cancer listed in Table 2.

13. The method of any one of claims 1 to 12, further comprising:
determining a further biological state different from the biological states and pertaining to at least one of the gene sites pertaining to the cancer sample genome, wherein the further biological state is selected from the group consisting of epigenetic state, mutation state, RNA expression and copy number; and
correlating the further biological state of the at least one gene site pertaining to the cancer sample genome with the classified cancer type.

14. A computer-readable storage medium having computer-executable instructions stored, that, when executed, cause a computer to perform a method according to claim 1 or 2.

15. A system for diagnosing cancer, comprising:
one or more processors; and
a memory coupled to the one or more processors and comprising instructions executable by the one or more processors to implement the method according to claim 1 or 2.
